# EUROPEAN PATENT APPLICATION

(11) **EP 0 037 644 A1**
(43) Date of publication of application: **14.10.1981**
(21) Application number: 81301044.4
(22) Date of filing: 12.03.1981
(51) Int. Cl.: A23L 1/226, C11D 3/50, A23G 3/00, A24B 15/34, A61K 7/46, C07C 47/34, C07C 61/13, C07C 67/34, C07C 69/74, C07C 103/19

(54) **Carbonyl norbornanes, organoleptic uses thereof and processes for preparing same**

(30) Priority: 25.03.1980 US 133870; 22.05.1980 US 152187; 17.07.1980 US 152188; 13.11.1980 US 206466; 13.11.1980 US 206632
(71) Applicant: INTERNATIONAL FLAVORS & FRAGRANCES INC., New York New York 10019 (US)
(72) Inventor: Klemarczyk, Philip T., Middlesex County New Jersey 08857 (US); Sanders, James Milton, Monmouth County New Jersey 07721 (US); Vinals, Joaquin F., Monmouth County New Jersey 07701 (US); Schmitt, Frederick Louis, Monmouth County New Jersey 07735 (US); Granda, Edward J., Monmouth County New Jersey 07726 (US); Luccarelli Jr., Domenick, Neptune,Monmouth County New Yersey 07753 (US); Vock, Manfred Hugo, Monmouth County New Jersey 07760 (US)
(74) Representative: Brown, John David

(57) **Abstract**

Described are a series of compounds defined according to structure: 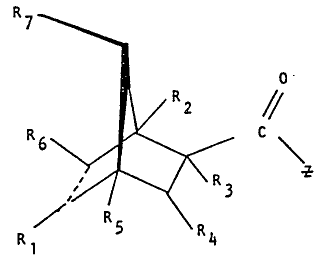 wherein the dashed line represents a carbon-carbon single bond or a carbon-carbon double bond; wherein Z is in the alternative one of the moieties: 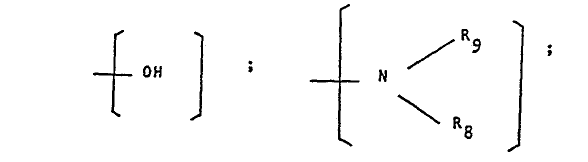 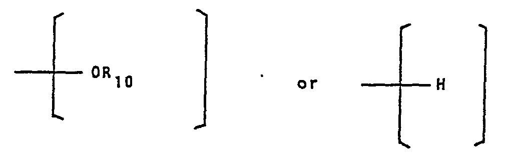 wherein R₁, R₂, R₃, R₄, R₅. R₆ and R₇ are each inthe alternative hydrogen or methyl; wherein R₈ and R₉ are in the alternative the same or different and are hydrogen orC₁-C₄ alkyl; with the proviso that one of R₁, R₂, R₅, R₆ or R₇ is methyl and the other of R₁, R₂, R₅, R₆ and R₇ is hydrogen; and with the additional proviso that R₃ and R₄ are not both methyl and uses thereof for augmenting or enhancing the organoleptic properties of consumable materials including foodstuffs, chewing gums, toothpastes, medicinal products, smoking tobaccos, chewing tobaccos, perfumes, perfumed articles, colognes, smoking tobaccos or smoking tobacco articles, as well as catalytic and thermal processes for preparing such compounds by means of Diels-alder reactions between arcylic compounds and monomethyl substituted cyclopentadienes or mixtures thereof.

## Description

It has been discovered that (1) solid and liquid foodstuff, chewing gum, medicinal product and toothpaste compositions therefor having fruity, anise-like, burnt fruit, raspberry, seedy, sweet, grapefruit-like, berry-like, red berry-like, blueberry-like, spicy, black pepper-like, herbaceous, clove-like, vermouth-like, strawberry-like, wild strawberry-like, patchouli-like, eucalyptus, green, camphoraceous and balsamic aroma and flavor characteristics and bitter taste characteristics; (2) novel smoking tobacco, smoking tobacco article and smoking tobacco flavoring compositions having spicy, cooling, clove-like, cinnamon bark-like, sweet, fruity, berry-like, juicyfruit, woody, piney, blueberry, banana, green, herbaceous, strawberry-like and dill aroma and taste nuances prior to and on smoking in both the mainstream and the sidestream; and (3) novel perfume compositions, colognes and perfumed articles (e.g., solid or liquid anionic, cationic, nonionic or zwitterionic detergents, fabric softeners and dryer-added fabric softener articles) having intense and long lasting fruity, banana, creamy, camphoraceous, strawberry-like, raspberry, reseda body-like, herbaceous, sweet, spicy, woody, eucalyptol-like, rum/butterscotch- like, balsamic, green, minty, spearmint, spicy, caraway, basil, powdery, castoreum-like, anise, borneol-like, cut grass-like and medicinal aromas with strong apple-like, herbaceous, camphor, minty and calamnus-like undertones may be provided by utilization of one or more of the substituted carbonyl norbornane derivatives having the aeneric structure: 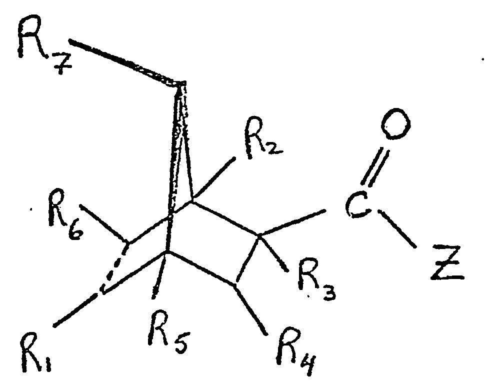 wherein the dashed line represents a carbon-carbon single bond or a carbon-carbon double bond; wherein Z is in the alternative one of the moieties: 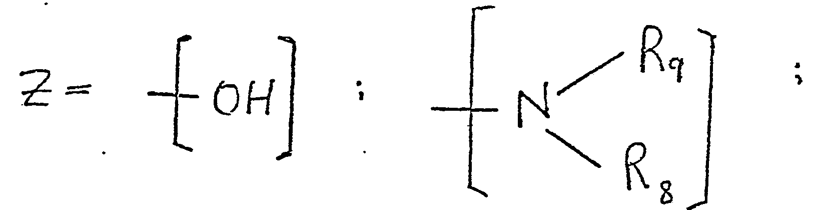 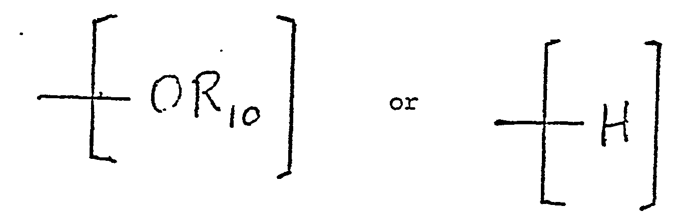 wherein R₁, R₂, R₃, R₄, R₅, R₆ and R₇ are each in the alternative hydrogen or methyl; wherein R₈ and R₉ are in the alternative the same or different and are hydrogen or C₁-C₄ alkyl; with the proviso that one of R₁, R₂, R₅, R₆ or R₇ is methyl and the other of R₁, R₂, R₅, R₆ and R₇ is hydrogen; and with the additional proviso that R₃ and R₄ are not both methyl, in foodstuffs, chewing gums, medicinal products, perfume compositions, perfumed articles, colognes and smoking tobaccos as well as smoking tobacco substitutes.

Unless otherwise specified, representations herein are intended to indicate "cis" isomers, "trans" isomers, mixtures of "cis" and "trans" isomers and "endo" isomers and "exo" isomers with respect to the norbornane ring moiety and dextro and levorotatory isomers as well as racemic mixtures of optical isomers of the carbonyl norbornane derivatives of our invention.

Thus, for example, the generic structure: 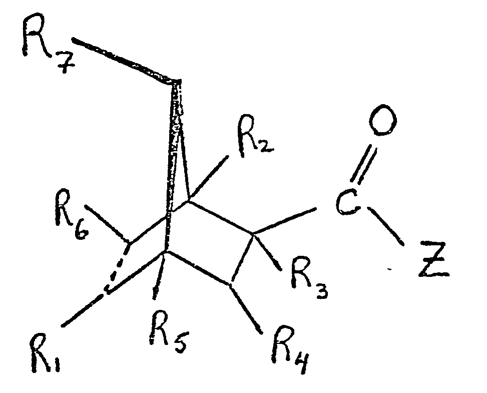 is intended to mean both "endo" and "exo" isomers having the structures: 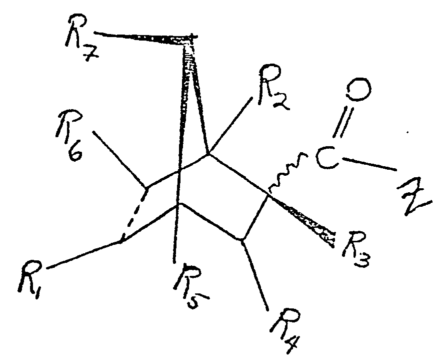 and

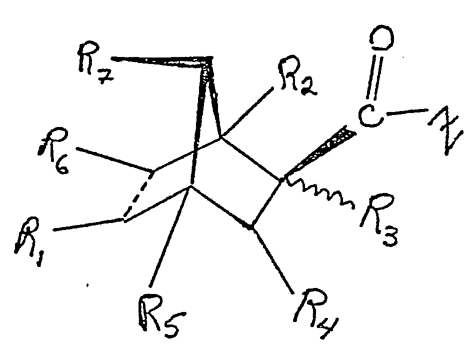

The novel substituted carbonyl norbornane derivatives of our invention useful as indicated supra may be produced by reacting a 1-methylcyclopentadiene or a 2-methylcyclopentadiene or a methylcyclopentadiene having one of the structures defined according to the generic structure: 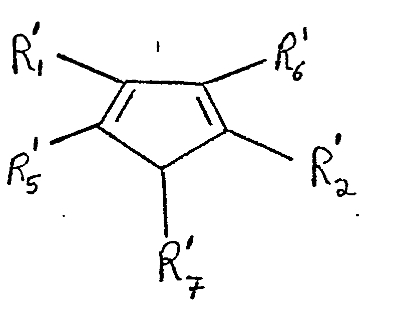 with acroline, an alkyl acrylate or an acrylamide derivative defined according to the generic structure: 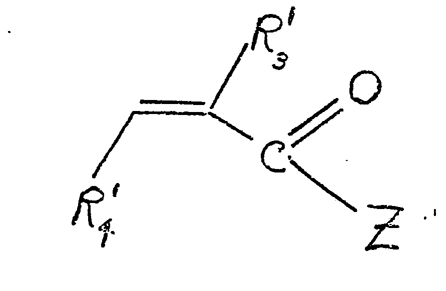 in the presence of a specific Lewis acid catalyst at relatively low temperatures (e.g., 0-50°C) or in the absence of a catalyst at relatively high temperatures (e.g., 170-250°C) at high pressures.

When using the catalytic process of our invention, an isomer mix will be produced which contains isomers in proportions and (in some cases) having structures different from the proportions and (in some cases) structures produced using the high temperature reaction of our invention (which reaction is carried out in the absence of catalyst).

The reactant having the structure: 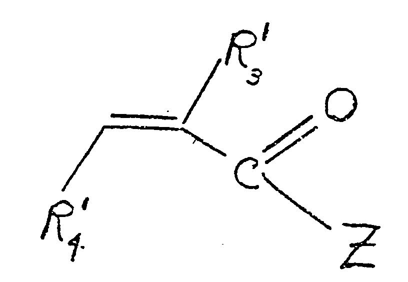 represents a "cis" isomer or a "trans" isomer or a mixture of "cis" and "trans" isomers. Thus, the "trans" isomer may be represented by the structure: 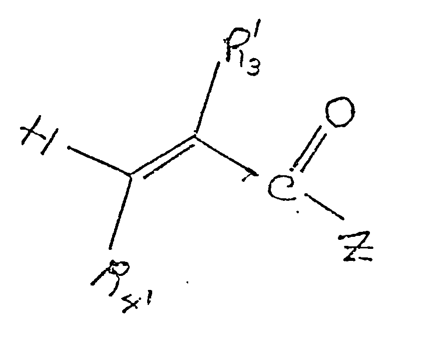 and the "cis" isomer may be represented by the structure: 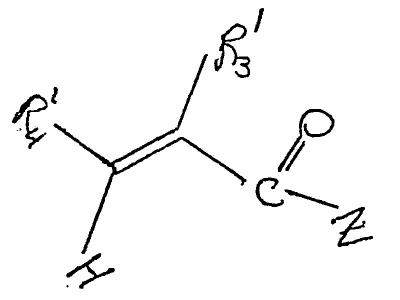 wherein R₁', R₂', R₃', R₄', R₅', R₆' and R₇' represent hydrogen or methyl and Z represents the moieties having the structures: 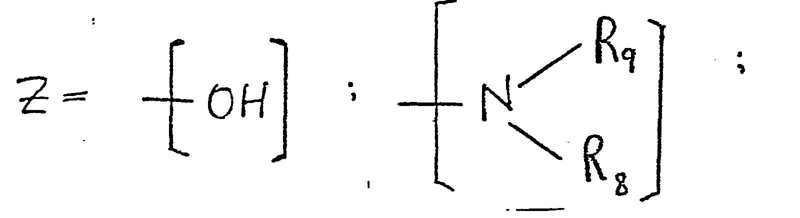 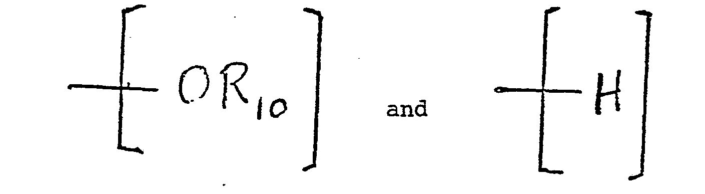 wherein R₈ and R9 represent C₁-C₃ lower alkyl and R ₁₀ represents C₁-C₄ lower alkyl.

When performing the process for preparing compounds useful in the practice of our invention where the use of a catalyst is required, the catalyst for the reaction may be an alkyl aluminum dihalide or a dialkyl aluminum halide for example RAlC1₂ of R₂AlCl wherein R represents Bethyl, ethyl or n-propyl or i-propyl. The preferred catalyst is ethyl aluminum dichloride. Other Lewis acids such as aluminum trichloride, stannic chloride, zinc chloride, ferric chloride and titanium tetrachloride have been attempted to be used but such attempts have proved to be unsuccessful with minimal or no yields of product being produced. The temperature range of the reaction may vary from about 0°C up to about 50°C with ambient temperatures from 20 up to 30⁰C being preferred. The reaction pressure will not affect the yield but conveniently and economically, a reaction pressure of atmospheric is preferred. Thus, the catalyst for the reaction may be defined according to the formula: R'ₘ AlClₙ wherein R' is C₁-C₃ alkyl and the sum, m + n = 3; with m being 1 or 2 and n being 1 or 2.

The reaction mass resulting from the catalytic process is a mixture containing compounds substantially in its entirety compounds containing unsaturation in the norbornane moieties defined according to the generic structures: 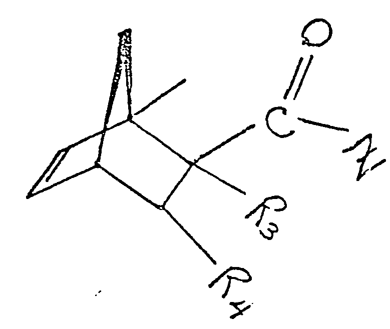 and

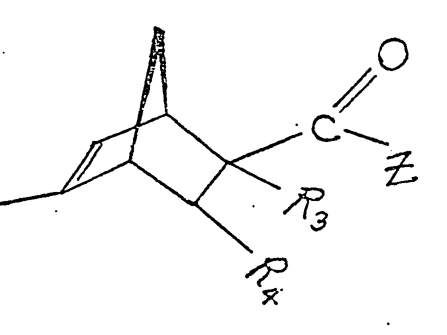

When performing the reaction for preparing compounds useful in the practice of our invention in the absence of catalysts (that is where no catalyst is required), the reaction temperature is in the range of from about 170°C up to about 250°C with a temperature of 200°C being preferred. When the reaction is carried out at such high temperatures, the reaction is carried out under pressures greater than atmospheric in a closed pressurized system. The pressure of reaction may vary from about 10 psig up to about 10 psig depending on the vapor pressure of the reaction mass and depending upon (a) the temperature at which it is desired to carry out the reaction and (b) the time period over which the reaction is carried out. Thus, reaction pressures of between 10 psig and 100 psig will not in and of themselves affect the yield of product although too high a temperature of reaction will affect the yield of product in view of the higher degree of decomposition and too low a reaction temperature will affect the yield of product in that the reaction will take an inordinately long period of time in order to achieve a given desired yield. Thus, it appears that the optimum reaction temperature in the absence of catalyst is 200°C. Reaction temperatues below 170°C will not be high enough to effect the initiating decomposition of the dimethyl dicyclopentadiene starting material which it is most practical to use as a precursor of the dimethyl dicyclopentadiene.

Depending upon the identity of the moiety represented as "(-Z)'', the reaction mass resulting from the "thermal non-catalytic" process may either be a mixture of compounds containing unsaturation in the norbornane moiety such as those defined according to the generic structures: 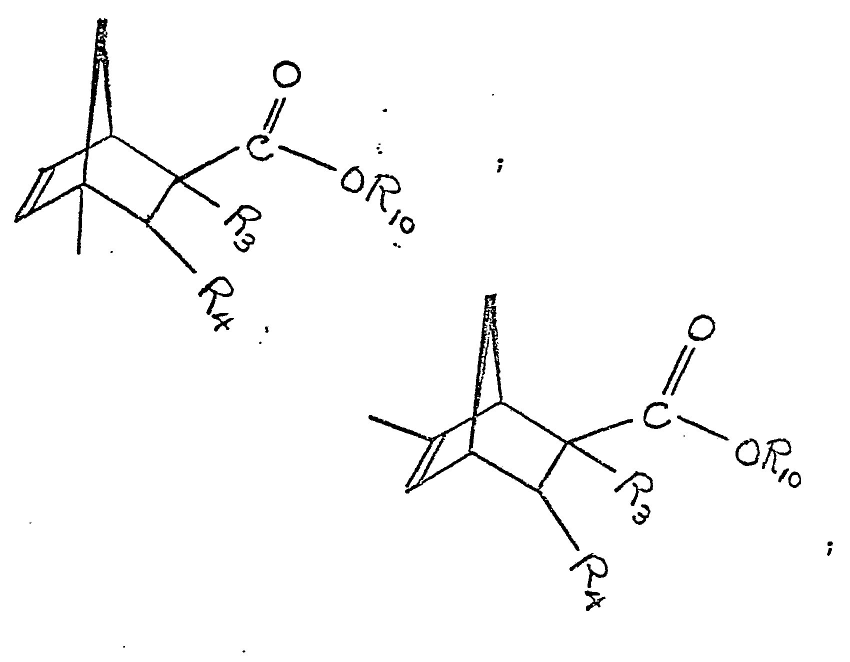 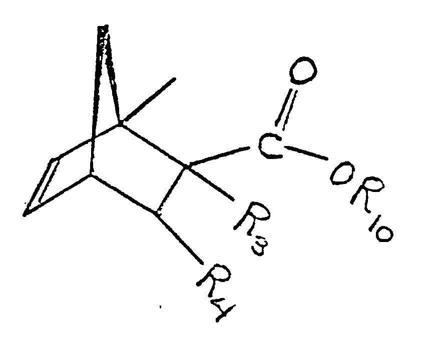 and 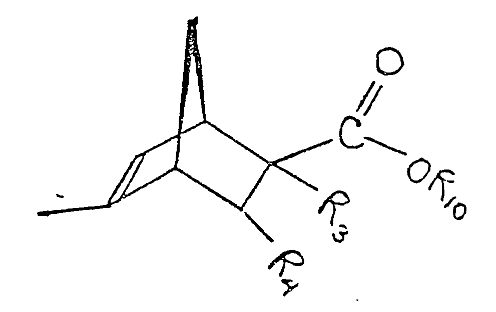 wherein R₃, R₄ and R₁₀ are as defined above (in the case of Z being -OR10 for example) or a substantially pure isomer such as the case where Z is the structure:

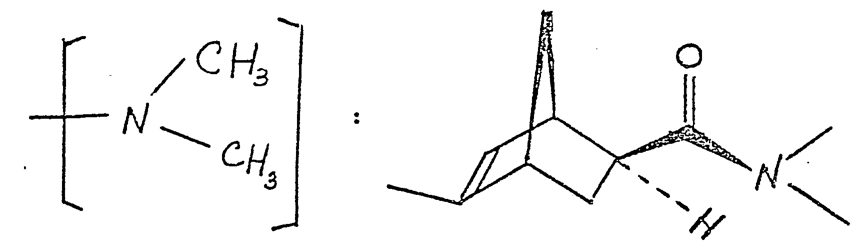

The above unsaturated compounds may be used "as is" for their individual organoleptic properties or mixtures of compounds may be used "as is" for their organoleptic properties or the compounds individually or in admixture may be further hydrogenated using hydrogen gas at super atmospheric pressures. When it is desired to use the compounds "as is" for their organoleptic properties, it is preferable in most cases to refine the reaction mass as by fractional distillation or high pressure liquid chromatography thereby creating one or more products usable as set forth above.

When it is desired to carry out a hydrogenation, the hydrogenation is carried out preferably on mixtures of products containing compounds having unsaturation prepared by the above named Diels-Alder reactions. Thus, when carrying out the hydrogenation on a mixture of products containing compounds resulting from the catalytic process and having the structures: 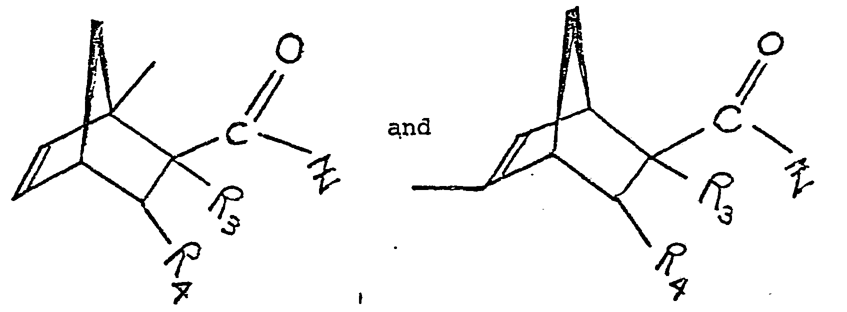 wherein R₃, R₄ and Z are defined as above, compounds having the structures: 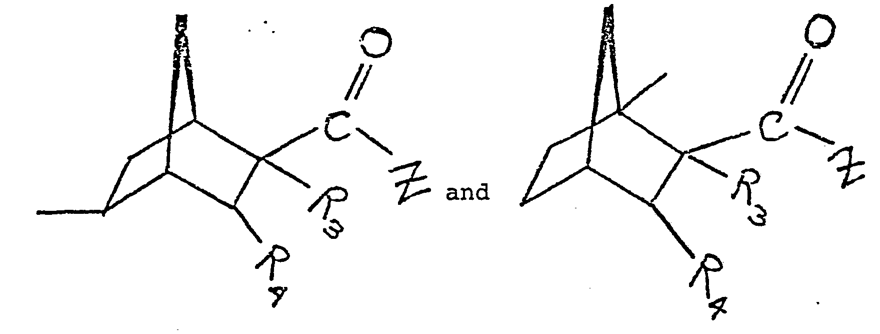 will be produced.

When carrying out the hydrogenation on, for example, a mixture of products containing the compounds resulting from the non-catalytic, thermal process and having the structures: 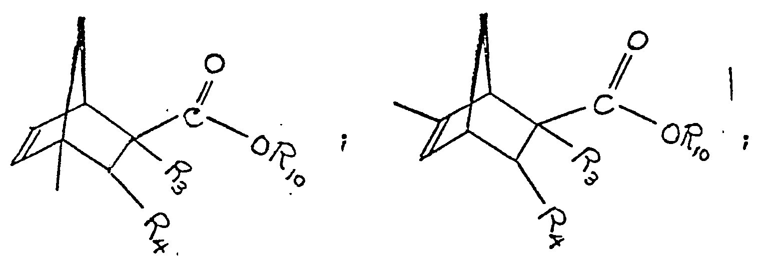 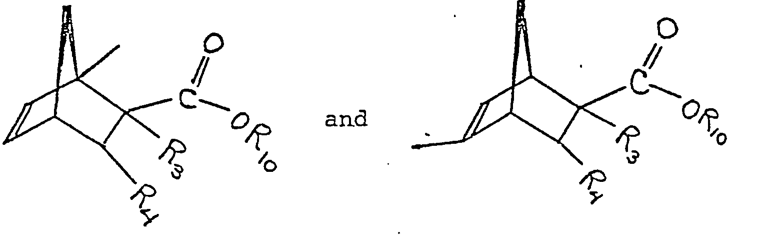 the compounds haying the structures; 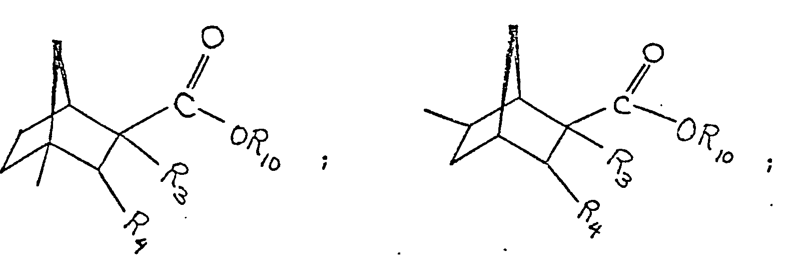 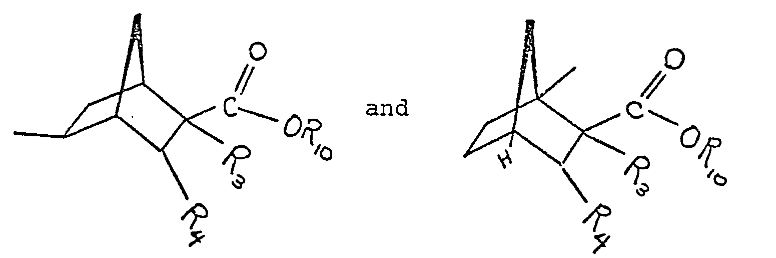 will be produced.

In either case, it is preferable to carry out the hydrogenation at pressures of from about 20 psig up to about 2,000 psig with a pressure range of from about 40 up to about 80 psig being preferred. It is also preferable for the reaction to be carried out in the presence of a catalyst such as Raney Nickel, palladium on carbon, palladium on calcium carbonate, palladium on barium sulfate and platinum. When using the palladium salt catalyst, it is preferred to use from about 3% up to about 12% palladium on salt, for example 5% palladium on calcium carbonate.

Thus, the reaction sequence which is embodied within our invention when using the catalytic procedure may be illustrated as follows: 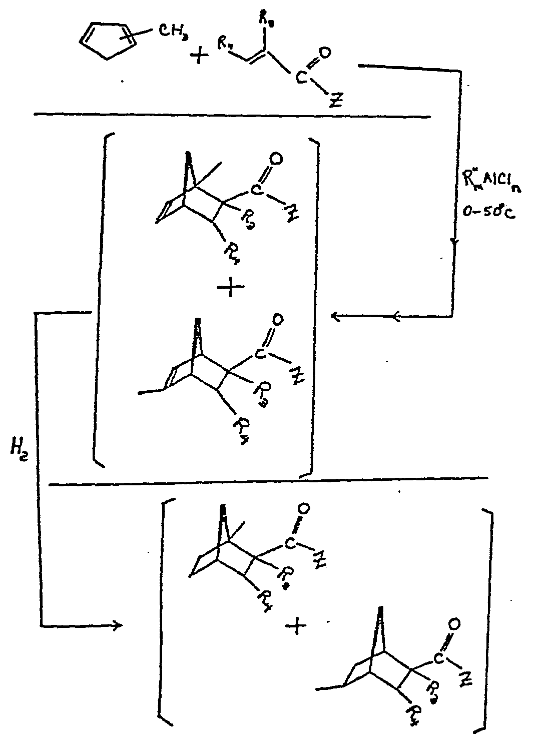 wherein one of R₃ or R₄ is hydrogen and the other of R₃ or R₄ is methyl; wherein R₁₀ represents C₁-C₄ lower alkyl; wherein Z represents one of the structures: 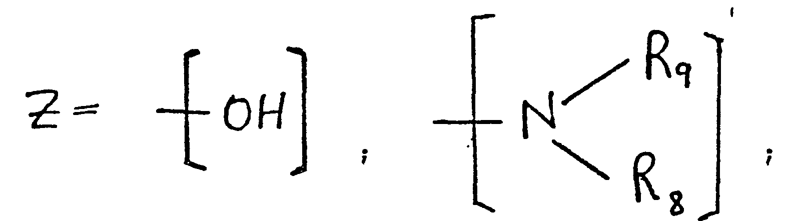 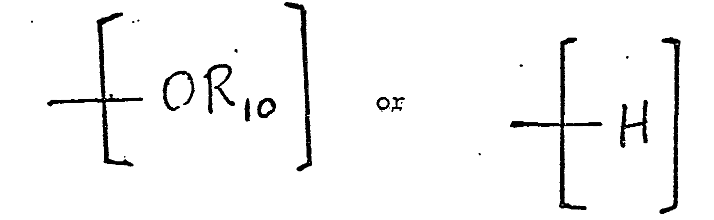 wherein R₈ or R₉ represents C₁-C₃ lower alkyl; and wherein m + n = 3 with m = 1 or 2 and n = 1 or 2.

Furthermore, the reaction sequence when embodied within our invention when using the high temperature non-catalytic Diels-Alder reaction may be illustrated as follows: 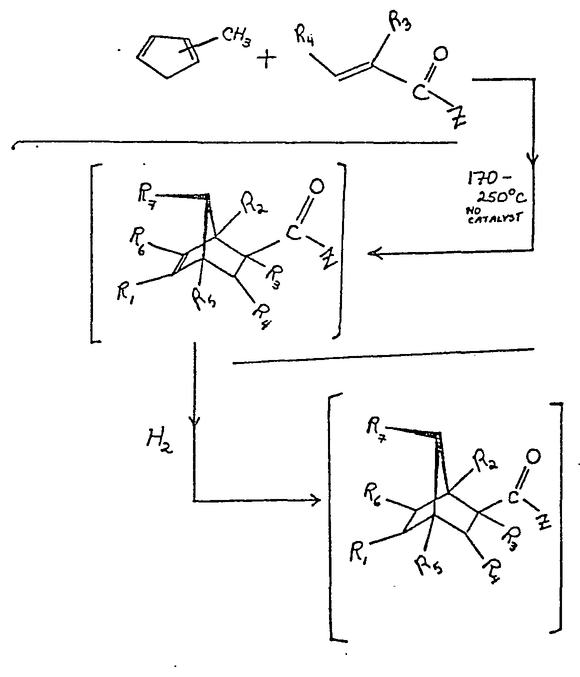 wherein R₁, R₂, R₃, R₄, R₅, R₆, R₇ and Z are as defined above.

### (a) The formation of norbornyl esters (wherein Z = (OR₁₀)

The novel substituted norbornyl ester derivatives of our invention useful as indicated supra may be produced by reacting a 1-methylcyclopentadiene or 2-methylcyclopentadiene having one of the structures: 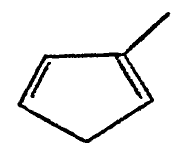 or 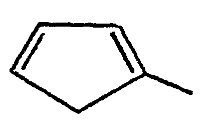 or a mixture of these methylcyclopentadienes defined according to the generic structure: 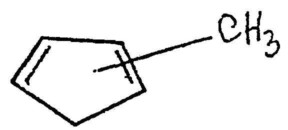 with an alkyl acrylate derivative defined according to the generic structure: 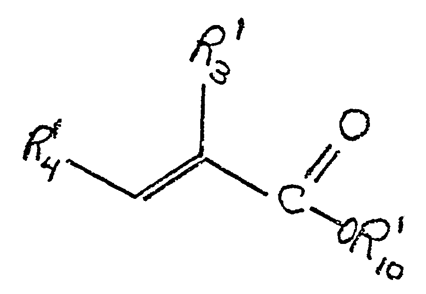 wherein one of R₃' or R₄' is methyl and the other of R₃' or R₄' is hydrogen and R₁₀' represents C₁-C₄ alkyl in the presence of a specific Lewis acid catalyst at relatively low temperatures (e.g., 0-50°C) or in the absence of a catalyst at relatively high temperatures (e.g., 170-250°C). When using the catalytic process of our invention, an isomer mix will be produced which contains isomers in proportions and (in some cases) having structures different from the proportions and (in some cases) structures produced using the high temperature reaction of our invention (which reaction is carried out in the absence of catalyst).

The reactant having the structure: 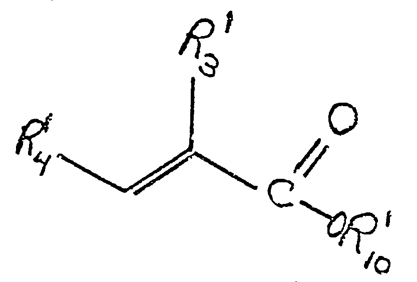 represents a "cis" isomer or a "trans" isomer or a mixture of "cis" and "trans" isomers. Thus, the "trans" isomer may be represented by the structure: 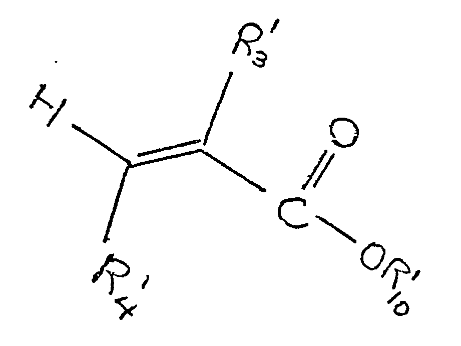 and the "cis" isomer may be represented by the structure:

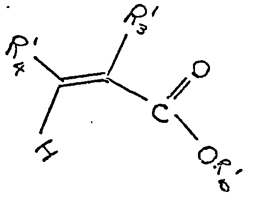

When performing the process for preparing compounds useful in the practice of our invention where the use of a catalyst is required, the catalyst for the reaction may be an alkyl aluminum dihalide or a dialkyl aluminum halide for example RAlC12 or R₂AlCl wherein R represents methyl, ethyl or n-propyl or i-propyl. The preferred catalyst is ethyl aluminum dichloride. Other Lewis acids such as aluminum trichloride, stannic chloride, zinc chloride, ferric chloride and titanium tetrachloride have been attempted to be used but such attempts have proved to be unsuccessful with minimal or no yields of product being produced. The temperature range of the reaction may vary from about 0°C up to about 50°C with ambient temperatures from 20 up to 30°C being preferred. The reaction pressure will not affect the yield but conveniently and economically a reaction pressure of atmospheric is preferred. Thus, the catalyst for the reaction may be defined according to the formula: Rₘ' AlClₙ wherein R' is C₁-C₃ alkyl and the sum, m + n = 3; with m being 1 or 2 and n being 1 or 2.

The reaction mass resulting from the catalytic process is a mixture of compounds containing unsaturation in the norbornane moieties defined according to the generic structures:

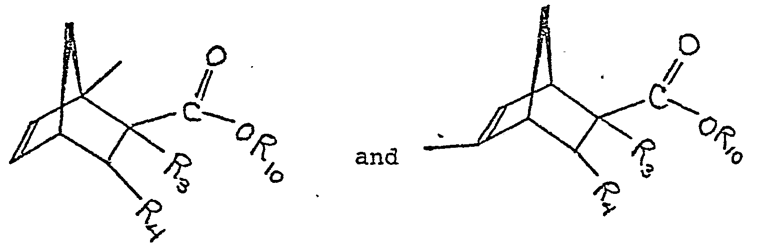

When performing the reaction for preparing compounds useful in the practice of our invention in the absence of catalysts (that is where no catalyst is required), the reaction temperature is in the range of from about 170°C up to about 250°C with a temperature of 200°C being preferred. When the reaction is carried out at such high temperatures, the reaction is carried out under pressures greater than atmospheric in a closed pressurized system. The pressure of reaction may vary from about 10 psig up to about 100 psig depending on the vapor pressure of the reaction mass and depending upon the temperature at which it is desired to carry out the reaction. Thus, a reaction pressure of between 10 psig and 100 psig will not in and of themselves affect the yield of product although too high a temperature of reaction will affect the yield of product in view of the higher degree of decomposition and too low a reaction temperature will affect the yield of product in that the reaction will take an inordinately long period of time in order to achieve a given yield. Thus, it appears that the optimum reaction temperature in the absence of catalyst is 200°C. Reaction temperatures below 170°C will not be high enough to effect the initiating decomposition of the dimethyl dicyclopentadiene starting material which is the most practical to use as a precursor of the dimethyl dicyclopentadiene.

The reaction mass resulting from the "thermal non cataly- . tic" process is also a mixture of compounds containing unsaturation in the norbornane moiety defined according to the generic structures: 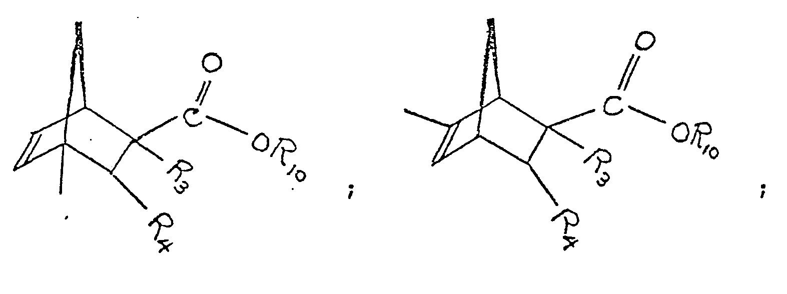 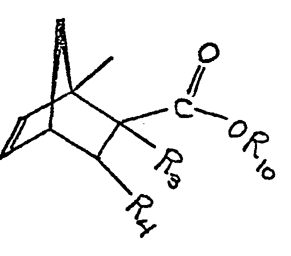 and 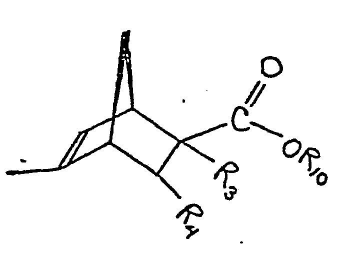 wherein R₃, R₄ and R₅ are as defined above.

The above unsaturated compounds may be used "as is" for their individual organoleptic properties or mixtures of compounds may be used "as is" for their organoleptic properties or the compounds individually or in admixture may be further hydrogenated using hydrogen gas at super atmospheric pressures. When it is desired to use the compounds "as is" for their organoleptic_properties, it is preferable to refine the reaction mass as by fractional distillation or high pressure liquid chromatography thereby creating one or more products usable as set forth above.

When it is desired to carry out a hydrogenation, the hydrogenation is carried out preferably on mixtures of products containing compounds having unsaturation prepared by the above named Diels-Alder reactions. Thus, when carrying out the hydrogenation on a mixture of products containing compounds resulting from the catalytic process and having the structures: 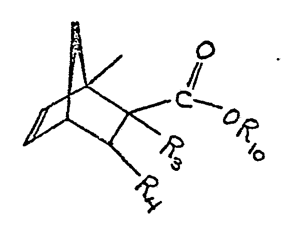 and 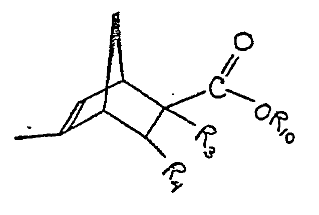 wherein R₃, R₄ and R₅ are defined as above, compounds having the structures; 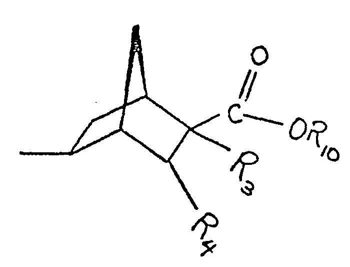 and 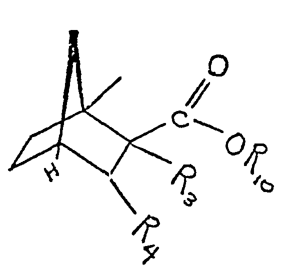 will be produced.

When carrying out the hydrogenation on a mixture of products containing the compounds resulting from the non-catalytic, thermal process and having the structures: 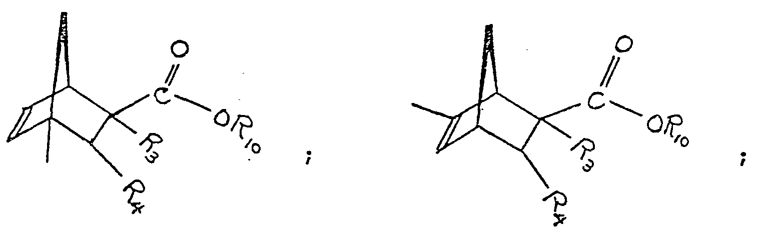 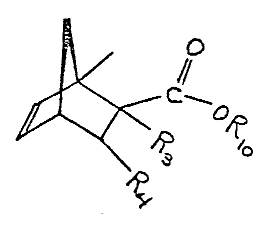 and 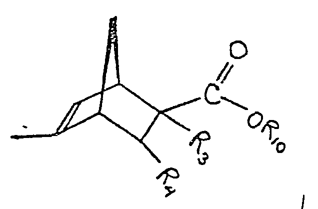 the compounds having the structures: 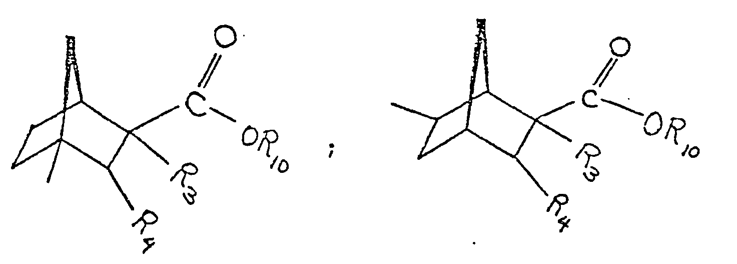 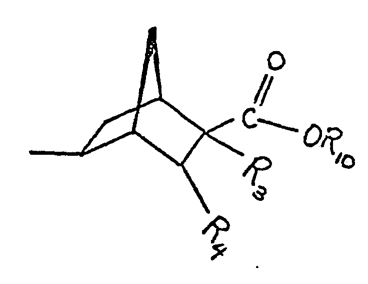 and 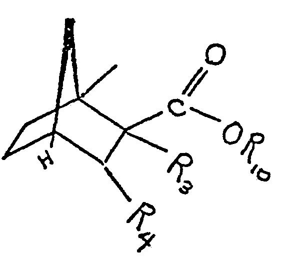 will be produced.

In either case, it is preferable to carry out the hydrogenation at pressures of from about 20 psig up to about 2,000 psig with a pressure range of from about 40 up to about 80 psig being preferred. It is also preferable for the reaction to be carried out in the presence of a catalyst such as Raney Nickel, palladium on carbon, palladium on calcium carbonate, palladium on barium sulfate and platinum. When using the palladium salt catalyst, it is preferred to use from about 3% up to about 12% palladium salt, for example 5% palladium on calcium carbonate.

Thus, the reaction sequence which is embodied within our invention when using the catalytic procedure may be illustrated as follows: 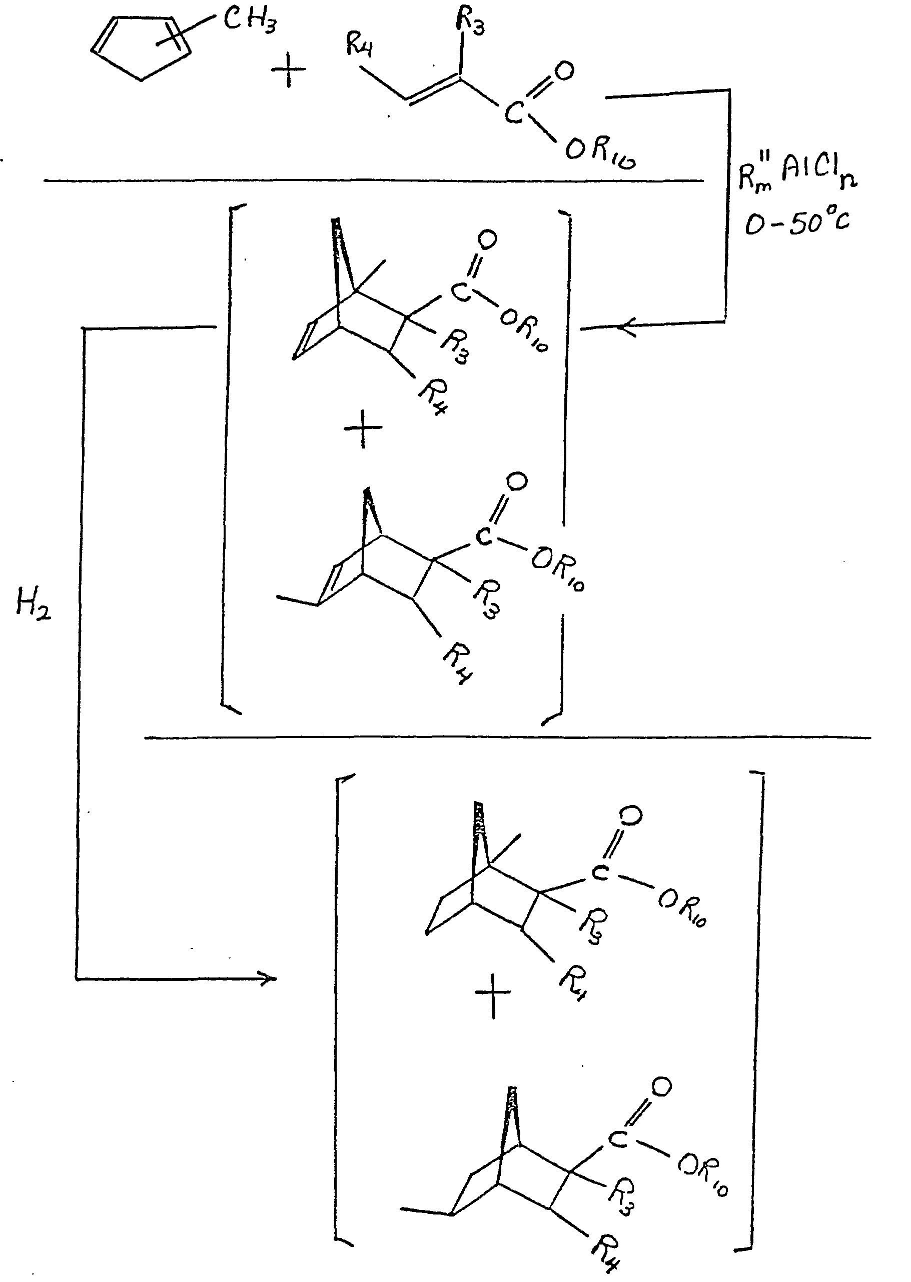 wherein one of R₃ or R₄ is hydrogen and the other of R₃ or _{R4} is methyl; wherein _{R10} represents C₁-C₄ lower alkyl; wherein R" represents C₁-C₃ alkyl; and wherein m + n = 3 with m being equal to 1 or 2 and n being equal to 1 or 2.

Furthermore, the reaction sequence which is embodied within our invention when using the high temperature non-catalytic thermal Diels-Alder reaction may be illustrated as follows: 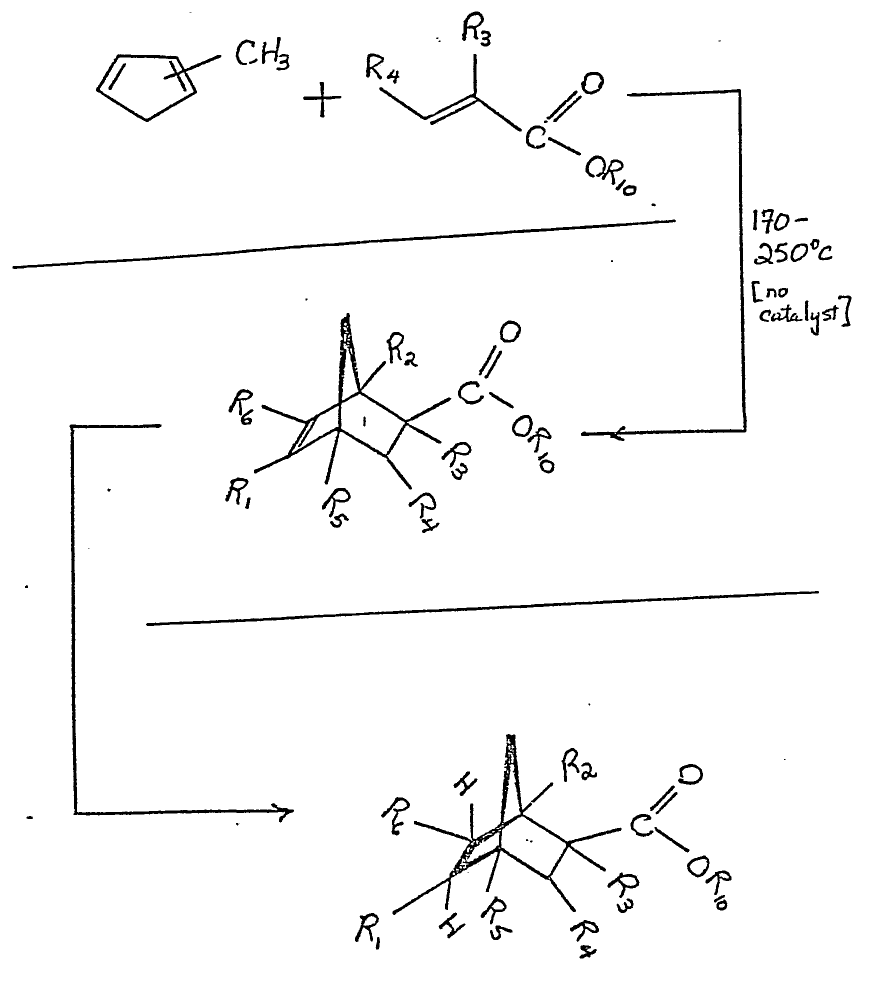 wherein R₁, R₂, R₃, R₄, R₅, R₆ and R₁₀ are as defined above.

The following tables set forth the products covered by our invention and the alkyl acrylate precursor reactant starting materials to be reacted with the methylcyclopentadienes to produce said products and in addition sets forth the organoleptic properties of the products of our invention:

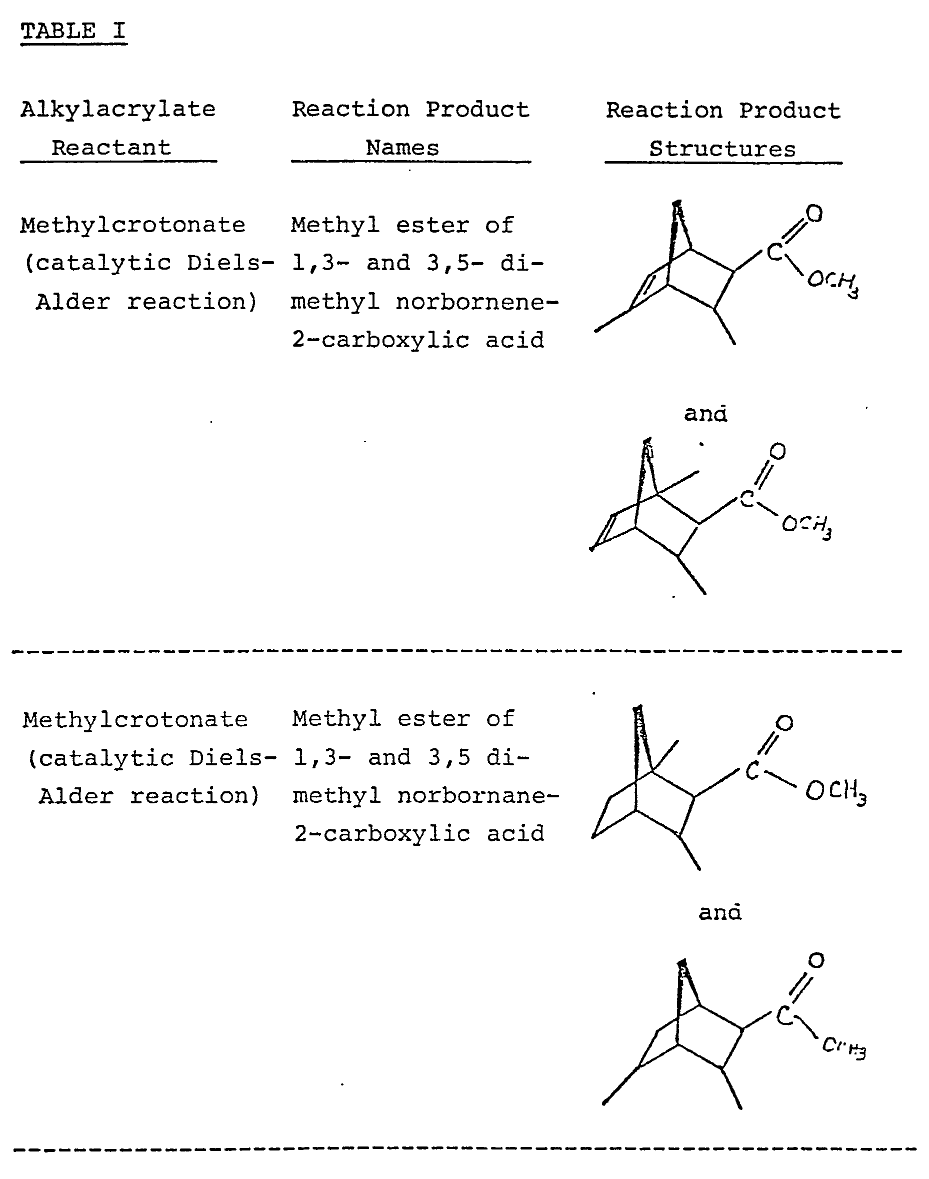

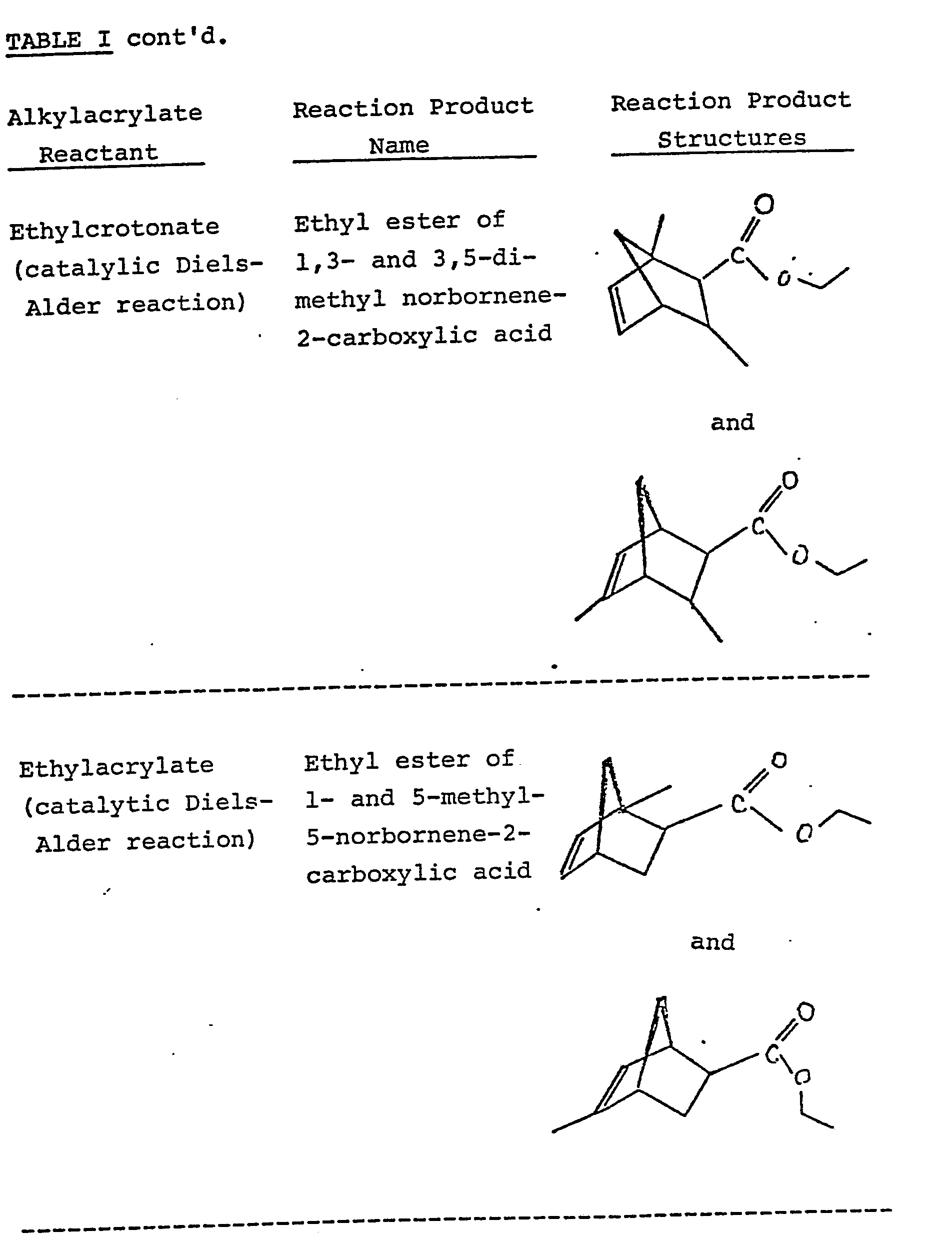

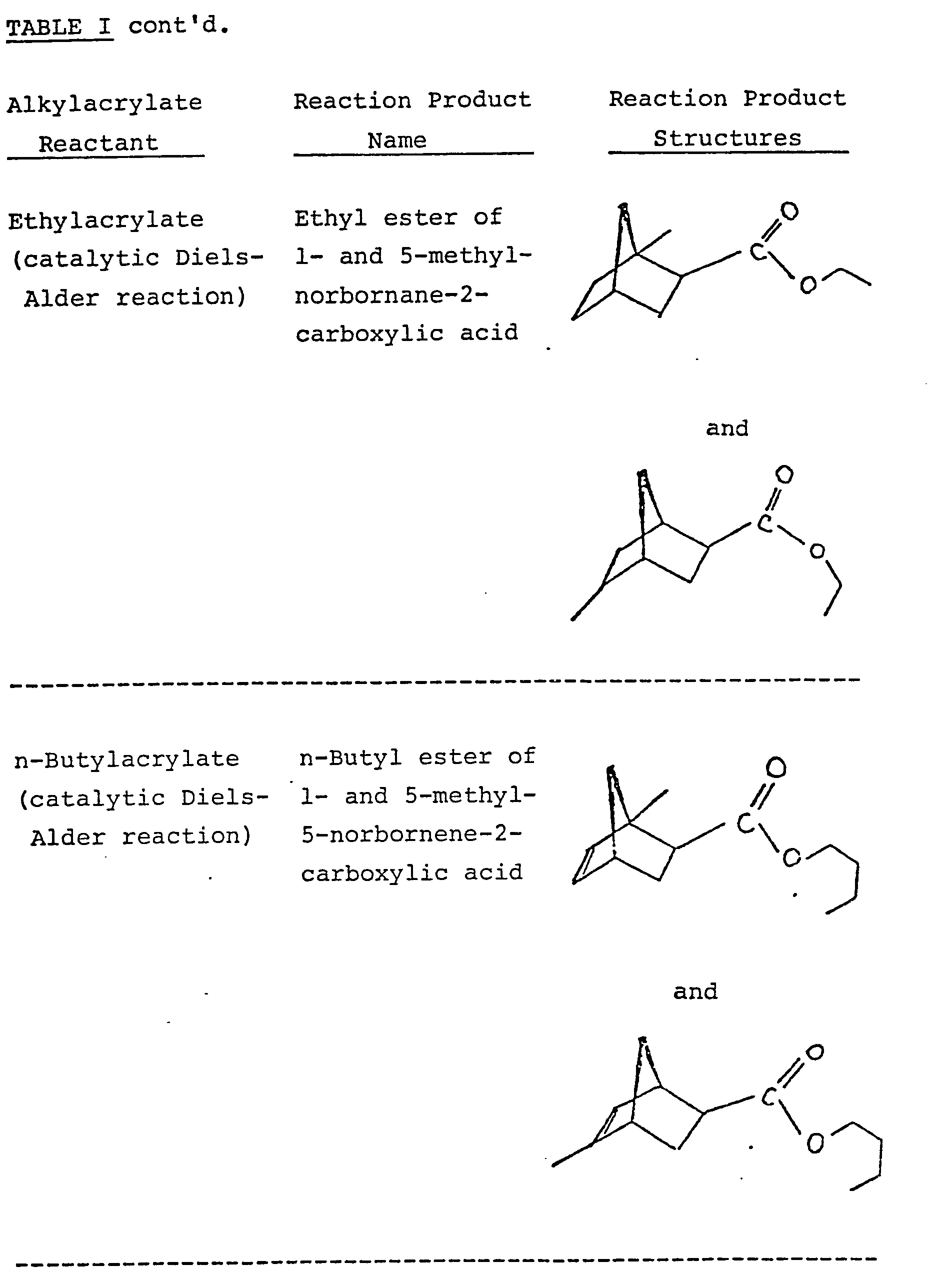

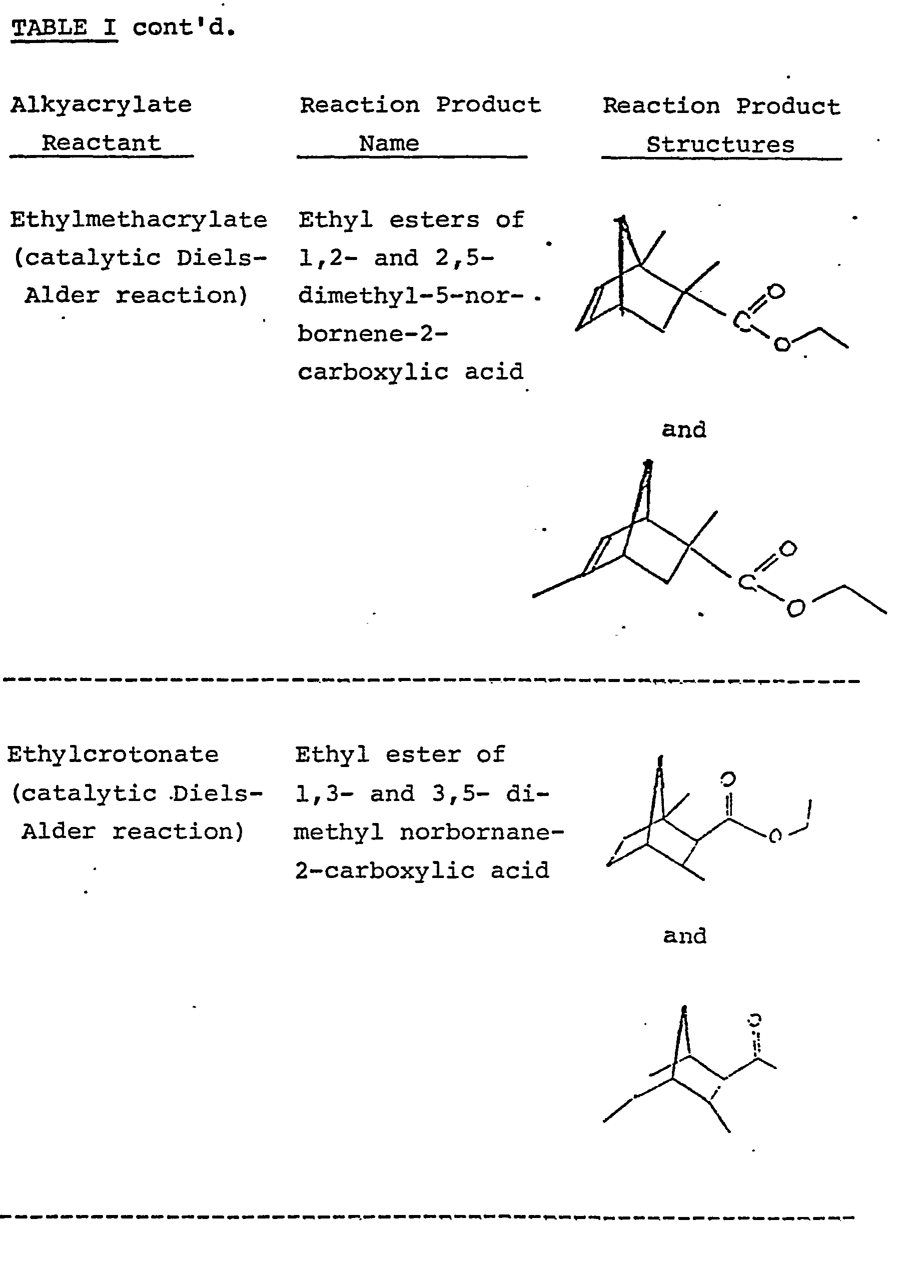

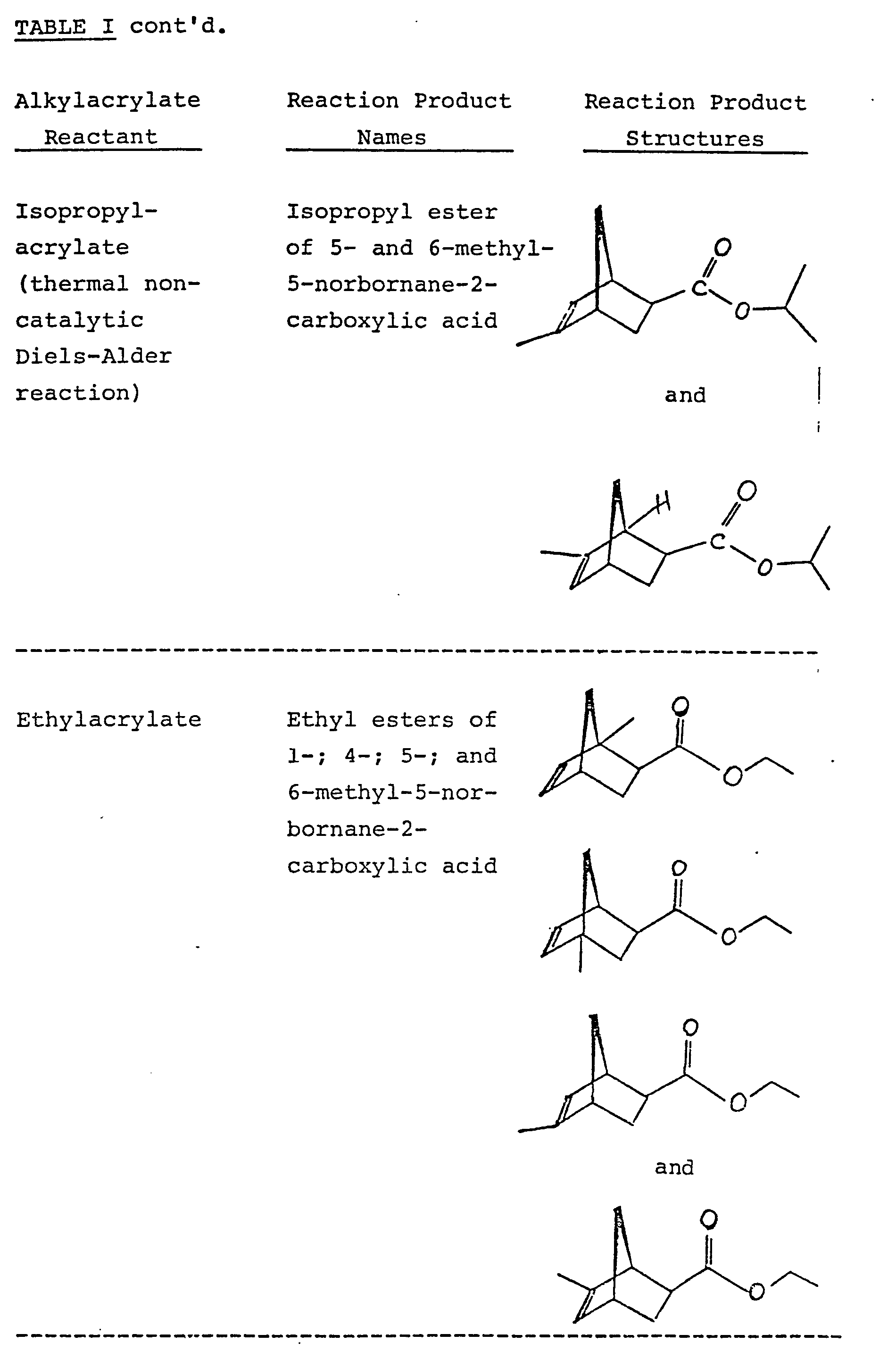

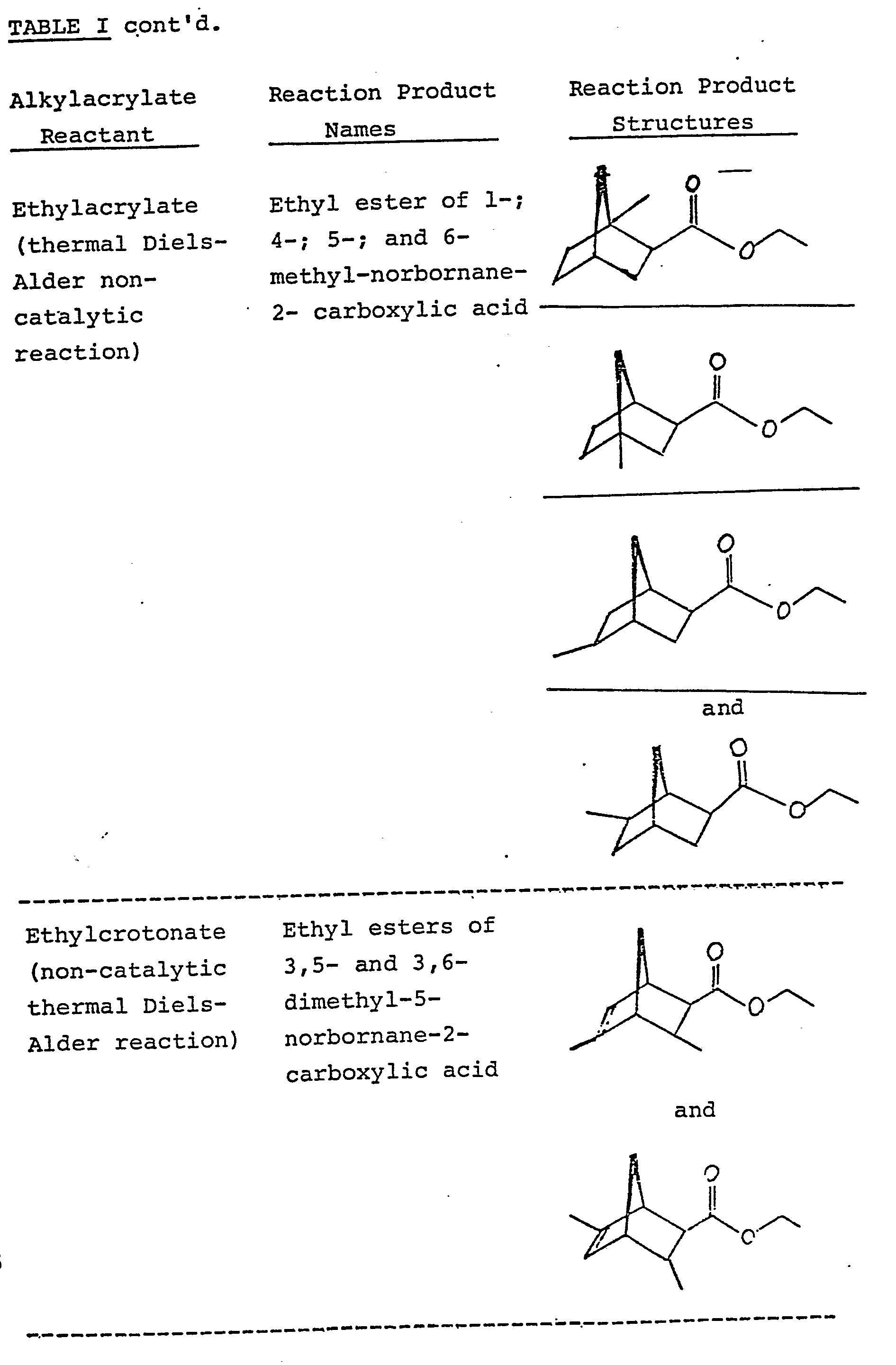

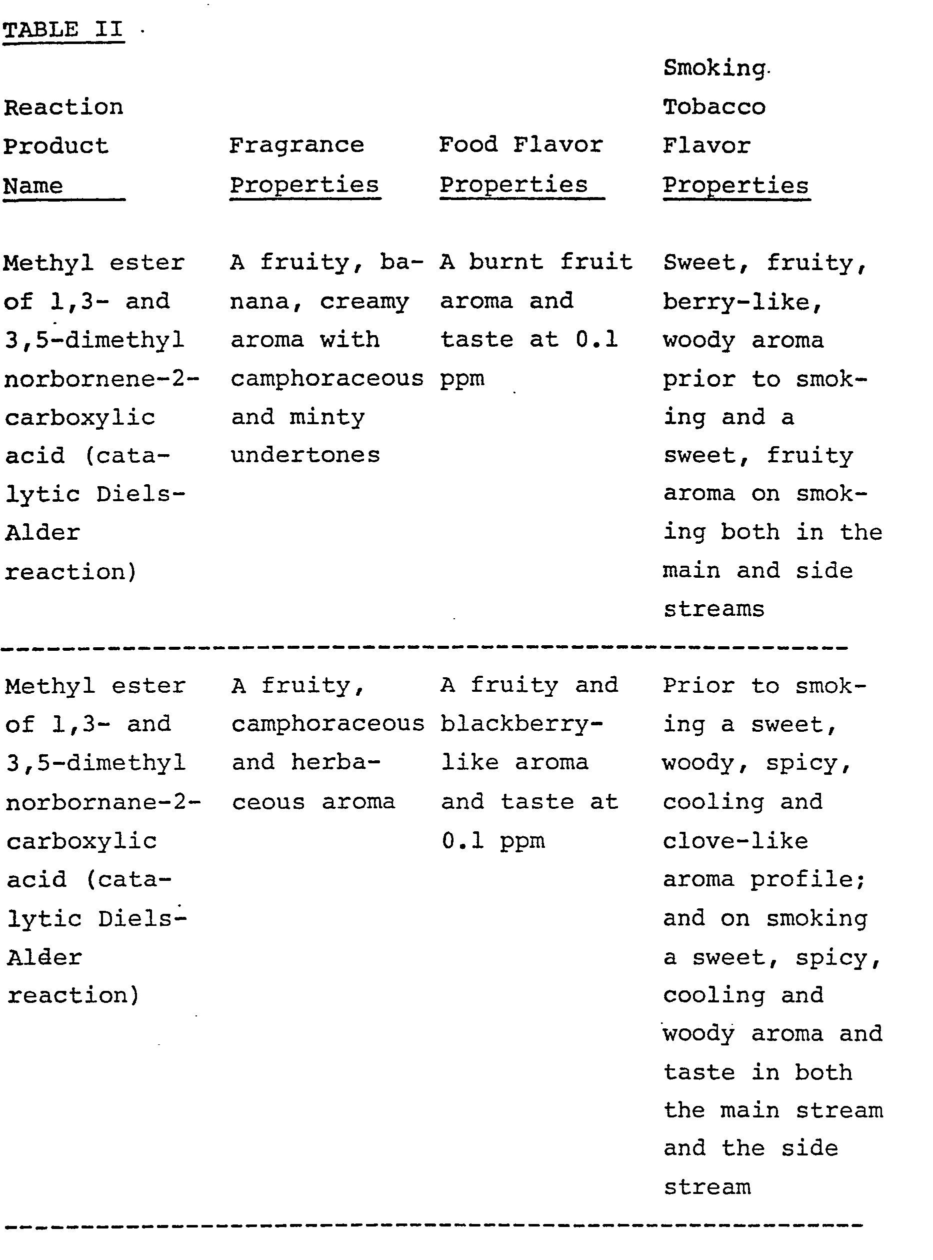

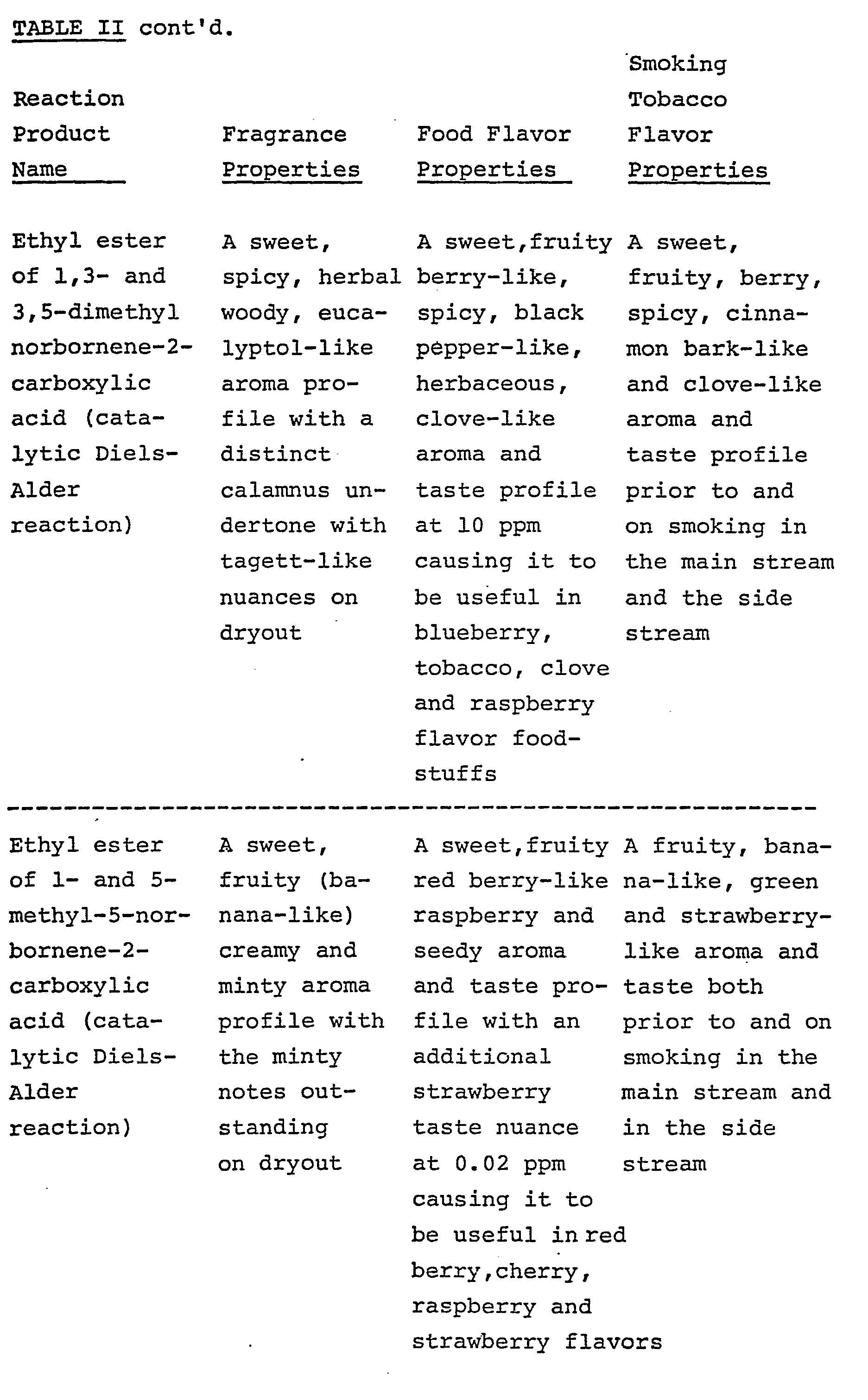

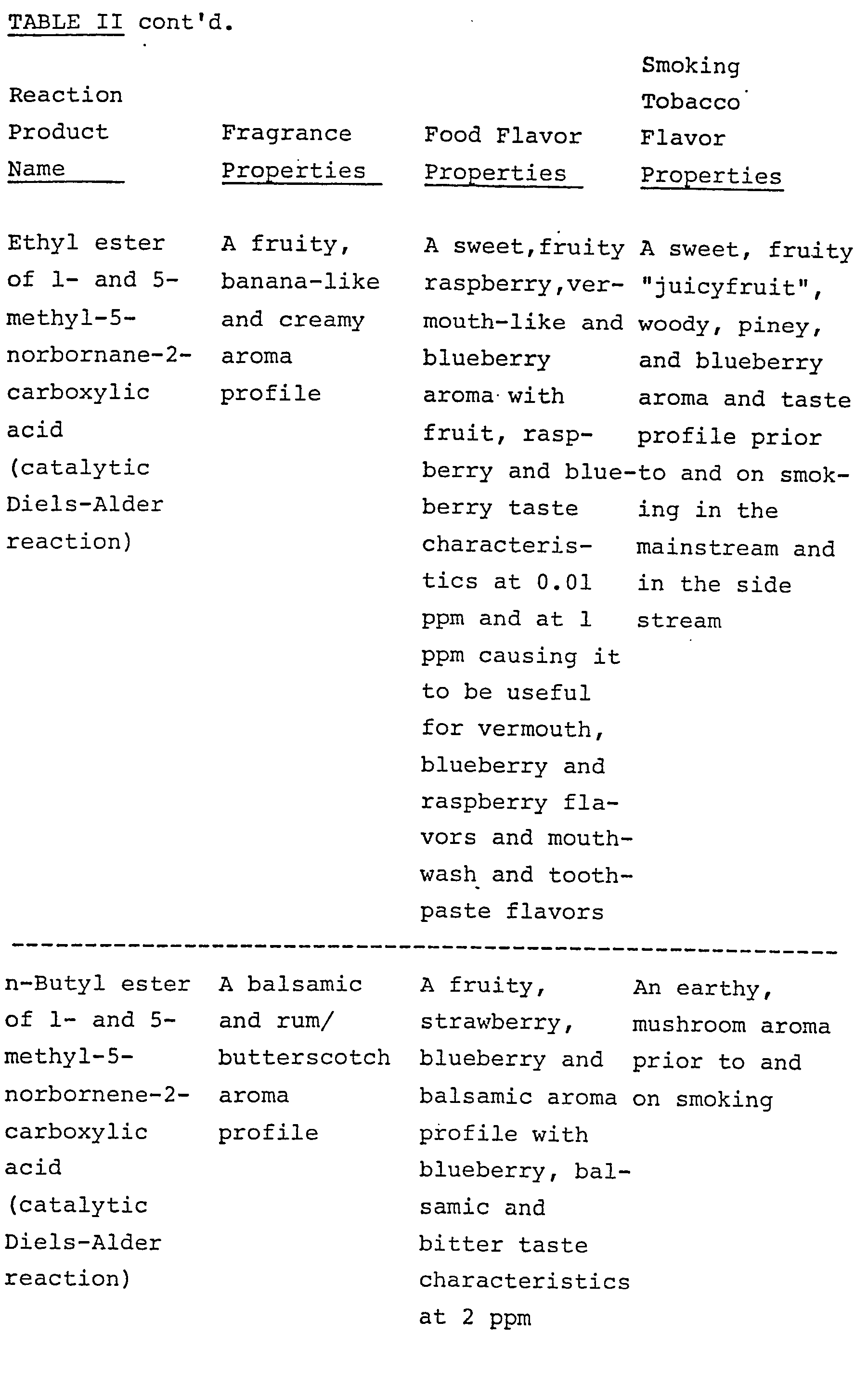

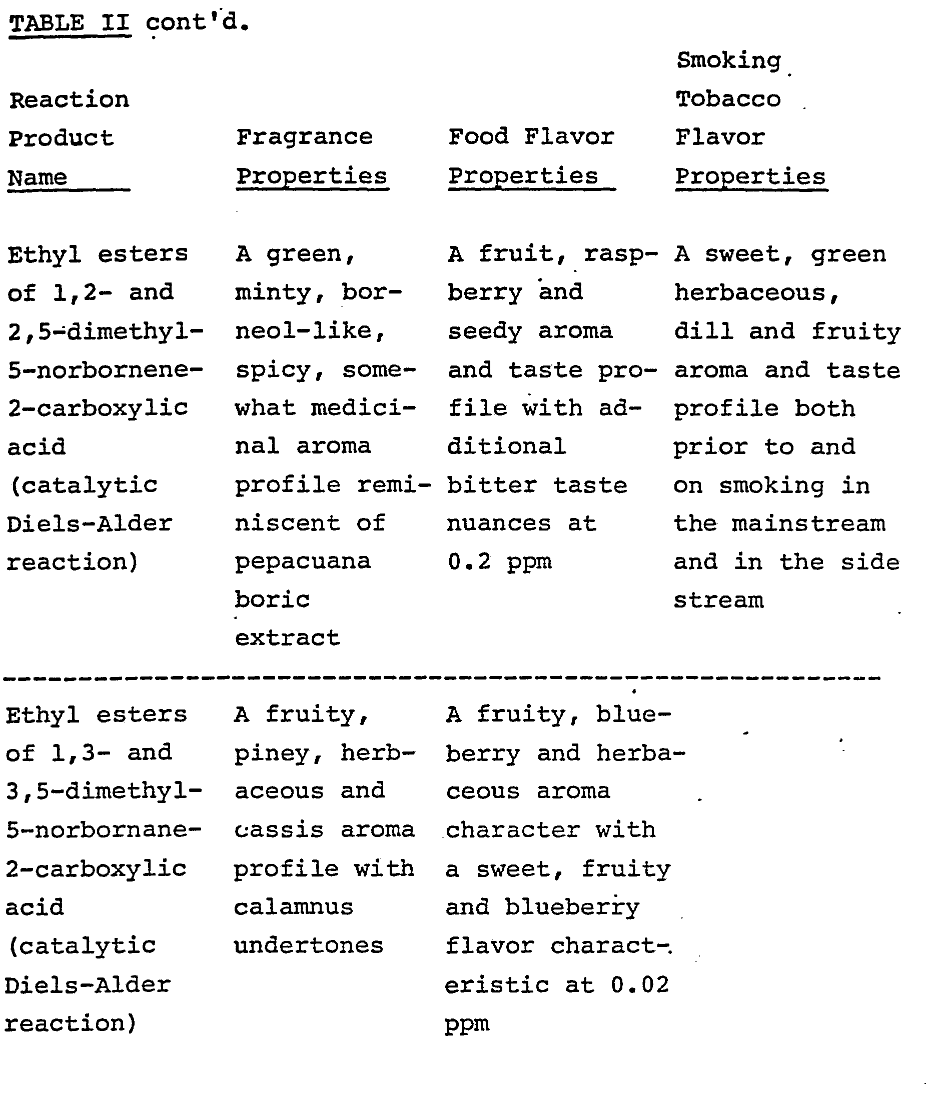

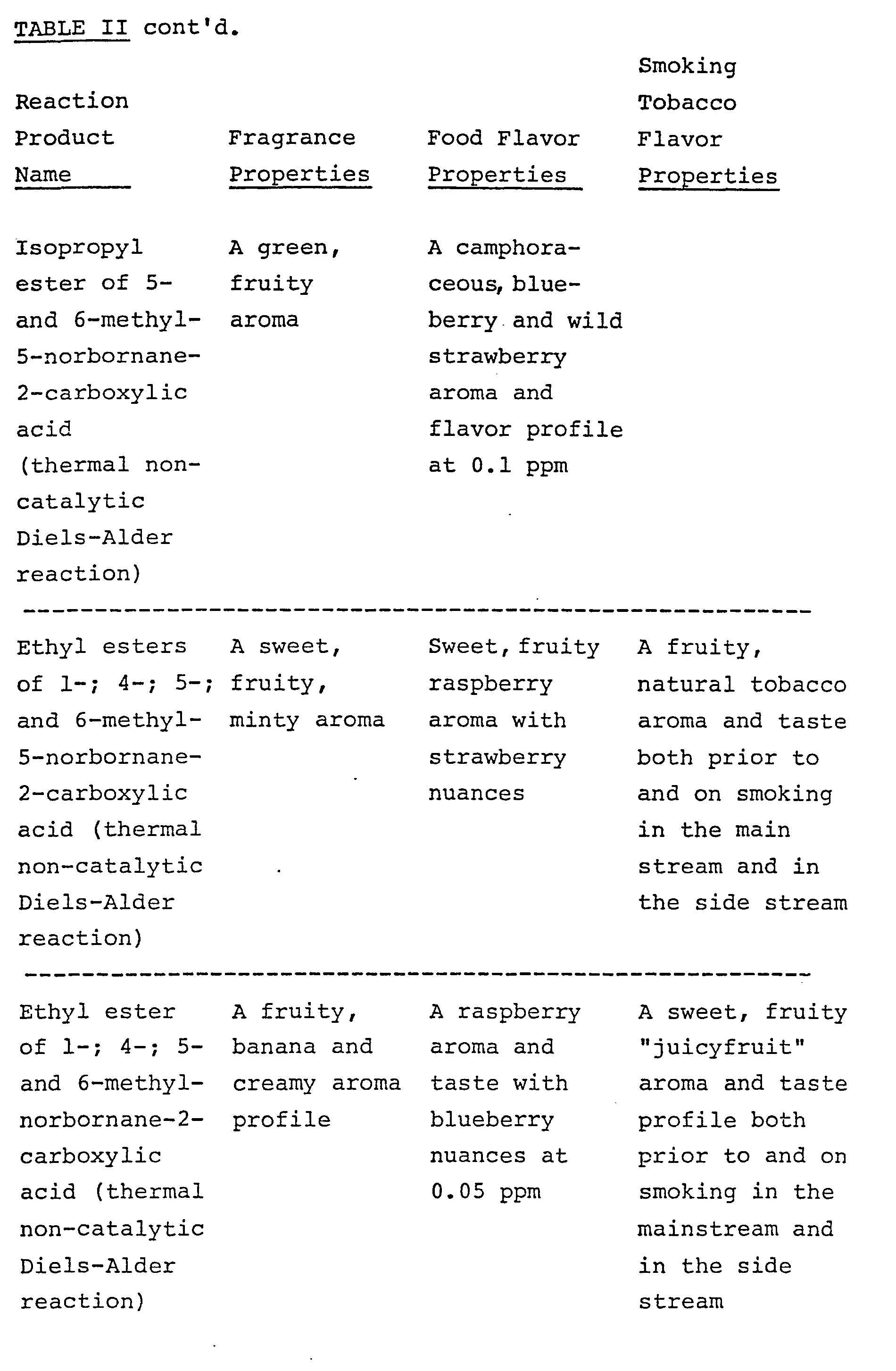

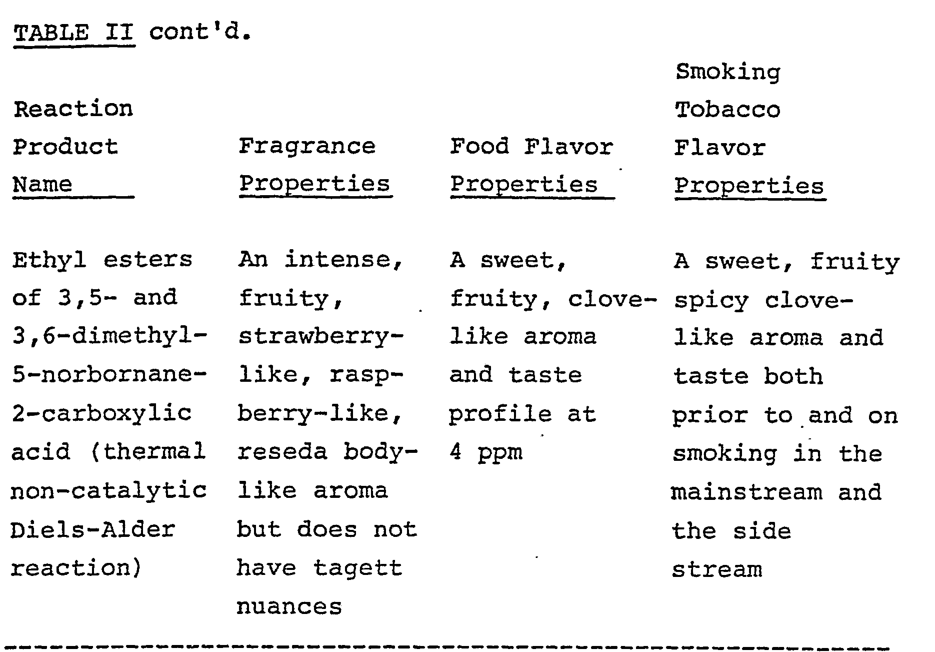

### (b) The aldehydes; wherein Z = (̵H)

The substituted norbornane carboxaldehydes of our invention may be prepared by first reacting an unsaturated aldehyde having the generic structure: 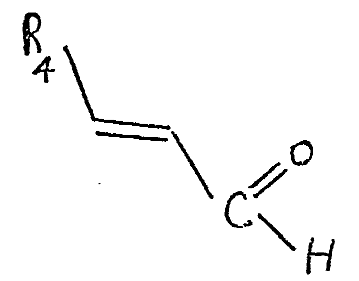 wherein R₂ is hydrogen or methyl and wherein when R₂ is methyl the foregoing structure represents cis or trans or mixtures of cis and trans isomers with 1-methyl-1,3-cyclopentadiene having the structure: 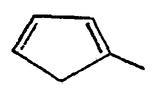 or a mixture of 1- and 2-methyl-l,3-cyclopentadiene signified by the structure: 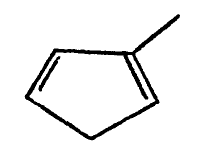 which are all freshly prepared from cracking methylcyclopentadiene dimer by passing the methylcyclopentadiene dimer through a distillation column at 175°C which distillation column is packed preferably with Berle Saddles or Raschig Rings. The reaction of the methylcyclopentadiene(s) with the unsaturated aldehyde takes place at a temperature of between 0⁰ and 50°C in the presence of an alkyl aluminum halide catalyst having the structure: 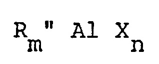 wherein R" is C₁-C₃ alkyl, preferably ethyl; X is chloro or bromo; m + n = 3 with m being 1 when n is 2 and m being 2 when n is 1, the reaction preferably takes place in the presence of a solvent such as toluene, and in the presence of a promoter such as triethanolamine. The products of the reaction are compounds having the generic structures: 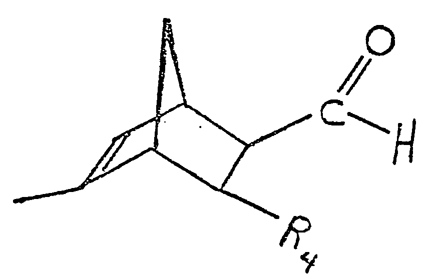 and 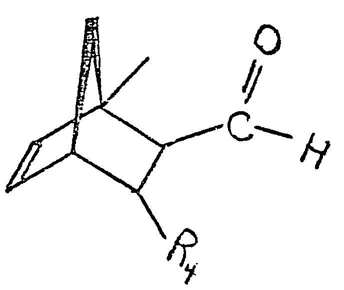 wherein R₂ is methyl or ethyl. The resulting products can be used in admixture for their organoleptic properties; or they can be separated as by fractional distillation to yield pure materials which can be used for their organoleptic properties; or the mixture can be used for further reaction with hydrogen to form the mixture of compounds having the structures: 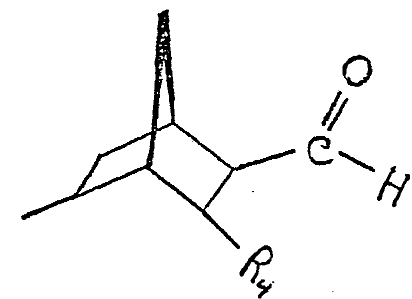 and 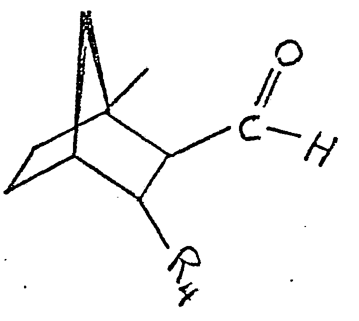 or the separated compounds may be individually hydrogenated to form the corresponding hydrogenated materials having the structures: 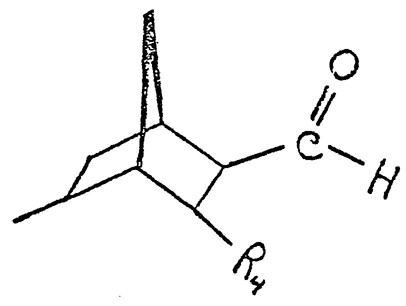 and

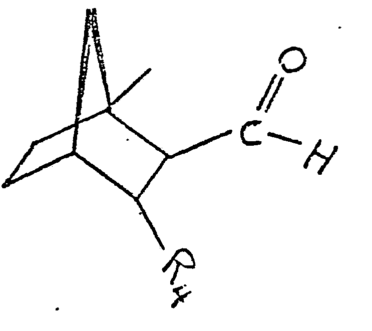

When the compounds having the structures: 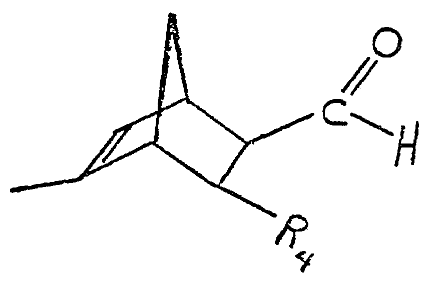 and 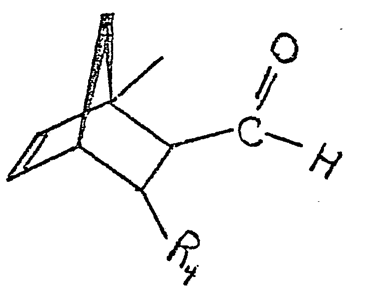 are hydrogenated, it is preferable to carry out the hydrogenation at a pressure between 25 psig and 100 psig, with a pressure of about 50 psig being most preferable. It is also preferred to carry out the hydrogenation at a temperature of between about 15°C and about 50°C, with ambient temperatures, e.g., 25-30⁰C being most preferable. Furthermore, the time of reaction is a function of the maximum amount of hydrogen absorption taking place thus, when a hydrogen pressure of 50 psig is used and a temperature of 25⁰C is used in the reaction, the time of maximum absorption is 5.5 hours.

The catalyst used in the hydrogenation may either be Raney Nickel or a supported palladium catalyst such as palladium on carbon or palladium on calcium carbonate. When the supported catalyst is used in the hydrogenation reaction, it is preferred to use from about 3% up to about 10% by weight palladium on support, preferably about 5% palladium on, for example, carbon or calcium carbonate.

At the end of the reaction, the reaction product is fractionally distilled to yield fractions containing at least one compound generically defined according to the structure: 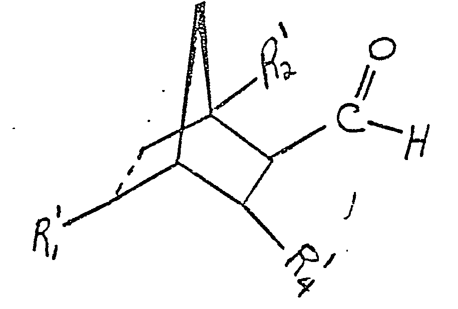 wherein one of Rₗ' or R₃' is methyl and the other is hydrogen, and wherein R₂' is methyl or hydrogen. These compounds can be further separated into their respective stereoisomers by reacting the corresponding aldehyde with a dextro or laevo amine and then, either fractionally distilling or fractionally crystallizing the resultant stereoisomers followed by subsequent hydrolysis.

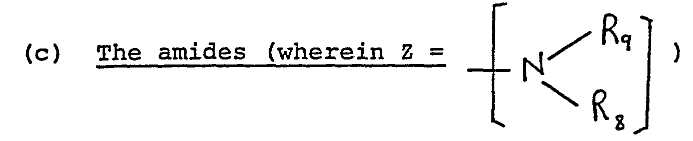

The novel substituted norbornane derivatives of our invention useful as indicated supra, may be produced by reacting a 1-methylcyclopentadiene or 2-methylcyclopentadiene having one of the structures: 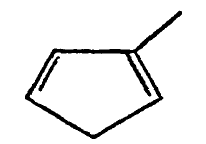 or 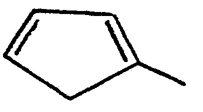 or a mixture of these methylcyclopentadienes defined according to the generic structure: 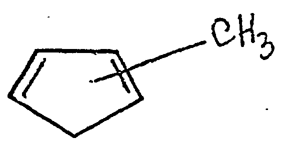 with an alkyl acrylamide derivative defined according to the generic structure:

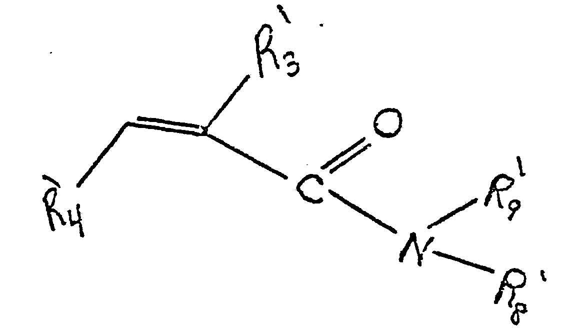

Generically, the methylcyclopentadiene derivative struct- . ures may be indicated by the generic structure:

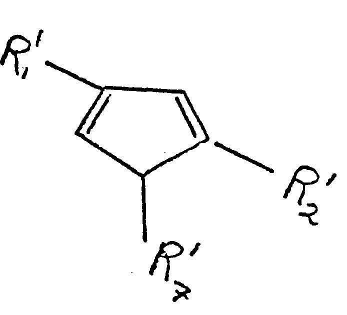

The substituted acrylamide structure represents a "cis" isomer or a "trans" isomer, or a mixture of "cis" and "trans" isomers. Thus, the "trans" isomer may be represented by the structure: 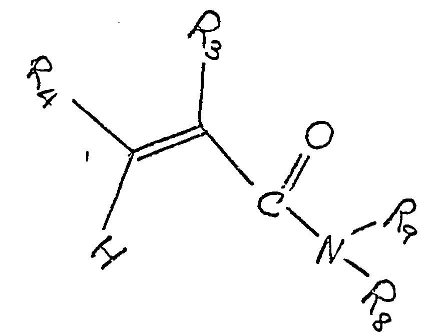 and the "cis" isomer may be represented by the structure:

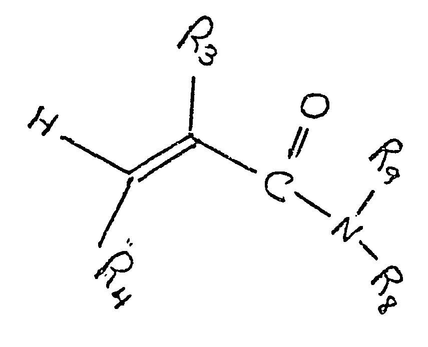

The Diels-Alder.reaction may be carried out using a specific Lewis acid catalyst at low temperatures (e.g., 0-50°C) or in the absence of a catalyst, at elevated temperatures (e.g., 170-250°C). Different isomers and proportions of isomers are produced depending on whether a catalytic reaction is carried out or a non-catalytic thermal _{D}iels-Alder reaction is carried out as will be seen in the examples and subsequent paragraphs, infra.

Thus, when it is desired to carry out a "catalytic" Diels-Alder reaction, the catalyst for this reaction may be an alkyl aluminum dihalide or a dialkyl aluminum halide for example, RAlC1₂ or R₂AlCl wherein R represents methyl, ethyl or n-propyl or i-propyl. The preferred catalyst is ethyl aluminum dichloride. Other Lewis acids such as aluminum trichloride, stannic chloride, zinc chloride, ferric chloride and titanium tetrachloride have been attempted to be used but such attempts have proved to be unsuccessful with minimal or no yields in the product being produced. The temperature range of the reaction may vary from about 0°C up to about 50°C with ambient temperatures from 20 up.to 30°C being preferred. The reaction pressure will not affect the yield, but conveniently and economically, a reaction pressure of atmospheric is preferred. Thus, the catalyst for the reaction may be defined according to the formula: 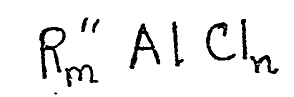 wherein R' is C₁-C₃ alkyl and the sum, n + n = 3, with m being 1 or 2 and n being 1 or 2. The resulting reaction mass is a mixture of compounds containing unsaturation in the norbornane moiety defined according to the generic structure:

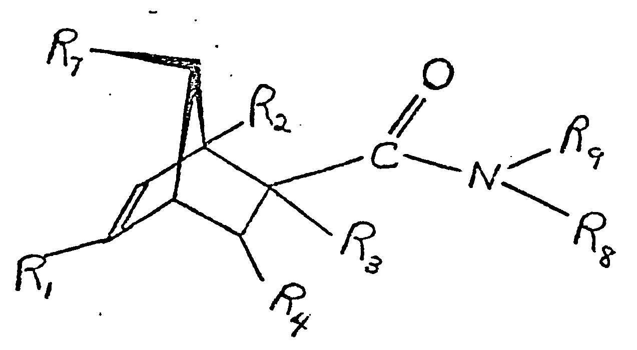

These compounds may be used "as is" for their organoleptic properties or the mixture of compounds may be further hydrogenated using hydrogen gas at supra-atmospheric pressures. When it is desired to use the compounds "as is", for their organoleptic properties, it is preferable to refine the reaction mass as by fractional distillation thereby creating a product usable as set forth above.

When the hydrogenation is carried out on a mixture of compounds containing compounds having the structures defined according to the genus: 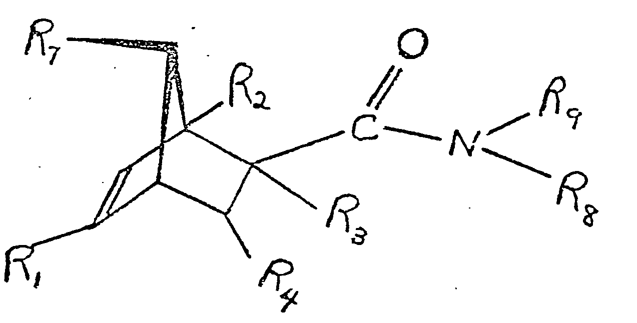 compounds containing the structures: 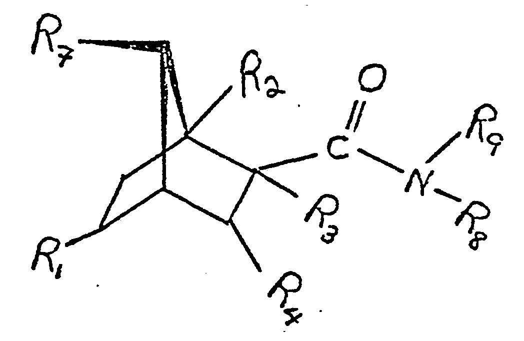 will be produced. It is preferable to carry out the hydrogenation at pressures of from about 20 psig up to about 2000 psig with a pressure range of from about 40 up to about 80 psig being preferred. It is also preferable for the reaction to be carried out in the presence of a catalyst such as Raney nickel, palladium on carbon, palladium on calcium carbonate, palladium on barium sulfate, and platinum. When using the palladium salt catalyst, it is preferred to use from about 3% up to about 12% palladium on salt, for example 5% palladium on calcium carbonate.

The reaction sequence including the catalytic Diels-Alder reaction which is embodied within our invention is generically set forth as follows: 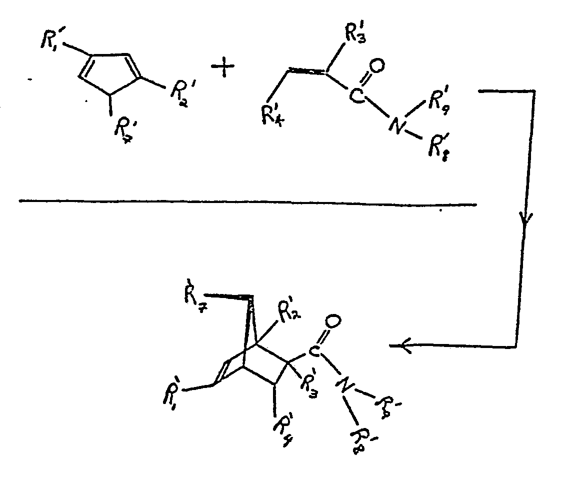 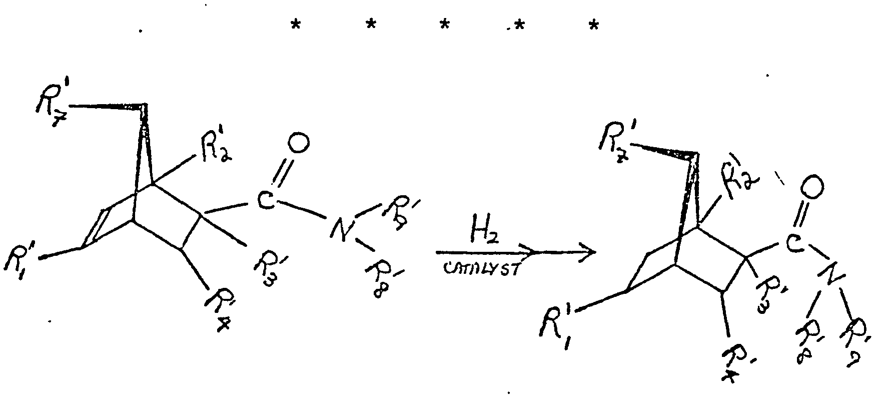 wherein R₂ is methyl or hydrogen; R₆ is methyl or hydrogen; R₁, R₃ or R₇ represent methyl or hydrogen, with the provisc that one of R₁, R₃ and R₇ represent methyl and the other two of R₁, R₃ and R₇ represent hydrogen; wherein R₄ and R5 are the same or different and each represents hydrogen or C₁-C₃ lower alkyl. When it is desired to carry out the thermal non-catalytic _{D}iels-Alder reaction, the reaction takes place at temperatures of between 170°C and 250°C at elevated pressures in order to prevent the constituents of the reaction mass from evaporating. Too low a temperature of reaction will either give rise to inordinately long times of reaction, yields which are not commercially feasible since they are too low, or no reaction at all. However, temperatures above 250°C are considered too high in that although the reaction is speeded up, the high temperature gives rise to an uncontrollable reaction and decomposition of reaction product which further gives rise to separation problems when attempting to obtain the substantially pure reaction product usable for its organoleptic properties. It has also been found that when carrying out the thermal reaction in certain instances, this reaction is surprisingly, unexpectedly, unobviously and advantageously more specific than the low temperature catalytic Diels-Alder reaction. Thus, when reacting the methylcyclopentadiene dimer mixture with dimethylacrylamide according to the reaction sequence: 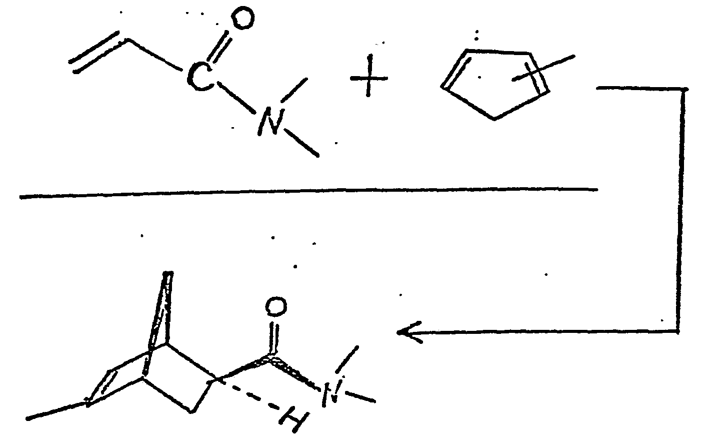 the reaction product having the structure: 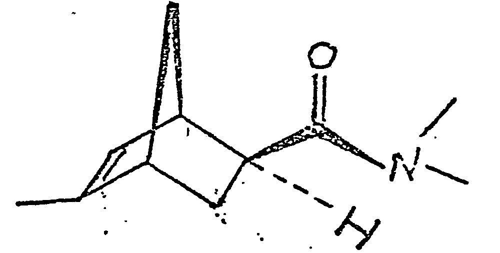 is produced without producing any other isomers thereof. This reaction product is the "endo" isomer of a specific isomeric product that would be expected to be one of many reaction products but that is, in fact, the only isomer produced.

Notwithstanding the foregoing statement, however, both the catalytic Diels-Alder reaction and the non-catalytic thermal Diels-Alder reaction, as a general rule, give rise to mixtures of isomers albeit different mixtures of isomers in different proportions.

When the norbornane derivatives of our invention are used as food flavor adjuvants, the nature of the co-ingredients included with each of the said norbornane derivatives in formulating the product composition will also serve to alter, modify, augment or enhance the organoleptic characteristics of the ultimate foodstuff treated therewith.

As used herein in regard to flavors, the terms "alter", "modify" and "augment" in their various forms mean "supplying or imparting flavor character or note to otherwise bland, relatively tasteless substances or augmenting the existing flavor characteristic where a natural flavor is deficient in some regard or supplementing the existing flavor impression to modify its quality, character or taste".

The term "enhance" is used herein to mean the intensification of a flavor or aroma characteristic or note without the modification of the quality thereof. Thus, "enhancement" of a flavor or aroma means that the enhancement agent does not add any additional flavor note.

As used herein, the term "foodstuff" includes both solid and liquid ingestible materials which usually do, but need not, have nutritional value. Thus, foodstuffs include soups, convenience foods, beverages, dairy products, candies,vegetables, cereals, soft drinks, snacks and the like.

As used herein, the term "medicinal product" includes both solids and liquids which are ingestible non-toxic materials which have medicinal value such as cough syrups, cough drops, aspirin and chewable medicinal tablets.

The term "chewing gum" is intended to mean a composition which comprises a substantially water-insoluble, chewable plastic gum base such as chicle, or substitutes therefor, including jelutong, guttakay, rubber or certain comestible natural or synthetic resins or waxes. Incorporated with the gum base in admixture therewith may be plasticizers of softening agents, e.g., glycerine; and a flavor composition which incorporates one or more of the norbornane derivatives of our invention, and in addition, sweetening agents which may be sugars, including sucrose or dextrose and/or artificial sweeteners such as cyclamates or saccharin. Other optional ingredients may also be present.

Substances suitable for use herein as co-ingredients or flavoring adjuvants are well known in the art for such use, being extensively described in the relevant literature. It is a requirement that any such material be "in- gestibly" acceptable and thus non-toxic and otherwise non- deleterious particularly from an organoleptic standpoint whereby the ultimate flavor and/or aroma of the consumable material used is not caused to have unacceptable aroma and taste nuances. Such materials may in general be characterized as flavoring adjuvants or vehicles comprising broadly stabilizers, thickeners, surface active agents, conditioners, other flavorants and flavor intensifiers.

Stabilizer compounds include preservatives, e.g., sodium chloride; antioxidants, e.g., calcium and sodium ascorbate, ascorbic acid, butylated hydroxyanisole (mixture of 2- and 3-tertiary-butyl-4-hydroxyanisole), butylated hydroxytoluene (2,6-di-tertiary-butyl-4-methylphenol), propyl gallate and the like and sequestrants, e.g., citric acid.

Thickener compounds include carriers, binders, protective colloids, suspending agents, emulsifiers and the like, e.g., agar agar, carrageenan; cellulose and cellulose derivatives such as carboxymethyl cellulose and methyl cellulose; natural and synthetic gums such as gum arabic, gum tragacanth; gelatin, proteinaceous materials; lipids; carbohydrates; starches, pectins and emulsifiers, e.g., mono- and diglycerides of fatty acids, skim milk powder,. hexoses, pentoses, disaccharides, e.g., sucrose corn syrup and the like.

Surface active agents includes emulsifying agents, e.g., fatty acids such as capric acid, caprylic acid, palmitic acid, myristic acid and the like, mono- and diglycerides of fatty acids, lecithin, defoaming and flavor-dispersing agents such as sorbitan monostearate, potassium stearate, hydrogenated tallow alcohol and the like.

Conditioners include compounds such as bleaching and maturing agents, e.g., benzoyl peroxide, calcium peroxide, hydrogen peroxide and the like; starch modifiers such as peracetic acid, sodium chlorite, sodium hypochlorite, propylene oxide, succinic anhydride and the like, buffers and neutralizing agents, e.g., sodium acetate, ammonium bicarbonate, ammonium phosphate, citric acid, lactic acid, vinegar and the like; colorants, e.g., carminic acid, cochineal, tumeric and curcuma and the like; firming agents such as aluminum sodium sulfate, calcium chloride and calcium gluconate; texturizers, anti-caking agents, e.g., aluminum calcium sulfate and tri-basic calcium phosphate; enzymes, yeast foods, e.g., calcium lactate and calcium sulfate; nutrient supplements, e.g., iron salts such as ferric phosphate, ferrous gluconate and the like, riboflavin, vitamins, zinc sources such as zinc chloride, zinc sulfate and the like.

Other flavorants and flavor intensifiers include aldehydes, esters, natural oils, alcohols, sulfides, ketones, lactones, carboxylic acids and hydrocarbons such as heliotropin, terpinenol-4, benzaldehyde, anisaldehyde, phenyl acetaldehyde, benzyl formate, benzyl acetate, cis-3-hexenyl benzoate, methyl hexanoate, hexanal, eucalyptol, eugenol, acetaldehyde, ethyl acetate, ethyl butyrate, turpentine gum oil, limonene, gum camphor, isobornyl acetate, borneol, cinnamic aldehyde, cuminic aldehyde, furfural, methyl cinnamate, cassia oil, vanillin, maltol, parahydroxybenzyl acetate, dimethyl sulfide, alphaionone, acetic acid, isobutyl acetate, acetone, butyric acid, formic acid, valeric acid, amyl acetate, amyl butyrate, anethol, benzyl salicylate, diacetyl, dimethyl anthranilate, ethyl methylphenylglycidate, ethyl succinate, ethyl valerate, geraniol, cis-3-hexen-l-ol, 2-hexenyl acetate, 2-hexenyl butyrate, hexyl butyrate, 4-(p-hydroxyphenyl)-2-butanone, beta-ionone, isobutyl cinnamate, jasmine, lemon essential oil, methyl butyrate, methyl caproate, methyl disulfide, methyl p-naphthyl ketone, orris butter, rose absolute, terpenyl acetate, gamma-undecalactone, vanilla and alcohol.

The specific flavoring adjuvant selected for use may be either solid or liquid depending upon the desired physical form of the ultimate product, i.e., foodstuff, whether simulated or natural,-and should, in any event, (i) be organoleptically compatible with the norbornane derivatives of our invention by not covering or spoiling the organoleptic properties (aroma and/or taste) thereof; (ii) be non-reactive with the norbornane derivatives of our invention and (iii) be capable of providing an environment in which the norbornane derivatives can be dispersed or admixed to provide a homogeneous medium. In addition, selection of one or more flavoring adjuvants, as well as the quantities thereof will depend upon the precise organoleptic character desired in the finished product. Thus, in the case of flavoring compositions, ingredient selection will vary in accordance with the foodstuff, chewing gum, medicinal product or toothpaste to which the flavor and/or aroma are to be imparted, modified, altered or enhanced. In contradistinction, in the preparation of solid products, e.g., simulated foodstuffs, ingredients capable of providing normally solid compositions should be selected such as various cellulose derivatives.

As will be appreciated by those skilled in the art, the amount of norbornane derivatives employed in a particular instance can vary over a relatively wide range, depending upon the desired organoleptic effects to be achieved. Thus, correspondingly, greater amounts would be necessary in those instances wherein the ultimate food composition to be flavored is relatively bland to the taste, whereas relatively minor quantities may suffice for purposes of enhancing the composition merely deficient in natural flavor or aroma. The primary requirement is that the amount selected to be effective, e.i., sufficient to alter, modify, or enhance the organoleptic characteristics of the parent composition, whether foodstuff per se, chewing gum, per se, medicinal product per se, toothpaste per se, or flavoring composition.

The use of insufficient quantities of norbornane derivatives will, of course, substantially vitiate any possibility of obtaining the desired results while excess quantities prove needlessly costly and, in extreme cases, may disrupt the flavor-aroma balance, thus proving self- defeating. Accordingly, the terminology "effective amount" and "sufficient amount" is to be accorded a significance in the context of the present invention consistent with the obtention of desired flavoring effects.

Thus, and with respect to ultimate food compositions, chewing gum compositions, medicinal product compositions and toothpaste compositions, it is found that quantities of norbornane derivatives ranging from a small but effective amount, e.g., 0.01 parts per million up to about 100 parts per million based on total composition are suitable. Concentrations in excess of the maximum quantity stated are not normally recommended, since they fail to prove commensurate enhancement of organoleptic properties. In those instances, wherein the norbornane derivatives are added to the foodstuff as an integral component of a flavoring composition, it is, of course, essential that the total quantity of flavoring composition employed be sufficient to yield an effective norbornane derivative concentration in the foodstuff product.

Food flavoring compositions prepared in accordance with the present invention preferably contain the norbornane derivatives in concentrations ranging from about 0.01% up to about 15% by weight based on the total weight of the said flavoring composition.

The composition described herein can be prepared according to conventional techniques well known as typified by cake batters and fruit drinks and can be formulated by merely admixing the involved ingredients within the proportions stated in a suitable blender to obtain the desired consistency, homogeneity of dispersion, etc. Alternatively, flavoring compositions in the form of particulate solids can be conveniently prepared by mixing the norbornane derivatives with, for example, gum arabic, gum tragacanth, carrageenan and the like, and thereafter spray-drying the resultant mixture whereby to obtain the particular solid product. Prepared flavor mixes in powder form e.g., a fruit-flavored powder mix are obtained by mixing the dried solid components, e.g., starch, sugar and the like and norbornane derivatives in a dry blender until the requisite degree of uniformity is achieved.

It is presently preferred to combine with the norbornane derivatives of our invention, the following adjuvants:
Heliotropin;
Terpinenol-4;
Benzaldehyde;
Anisaldehyde;
Phenylacetaldehyde;
Benzyl formate;
Benzyl acetate;
-Cis-3-hexenyl benzoate;
Methyl hexanoate;'
Hexanal;
Eucalyptol;
Eugenol;
Acetaldehyde;
Ethyl acetate;
Ethyl butyrate;
Turpentine gum oil;
Limonene;
Gum camphor;
Isobornyl acetate;
Borneol;
Cinnamic aldehyde;
Cuminic aldehyde;
Furfural;
Methyl cinnamate;
Cassia oil;
Vanillin;
Malto;
Paranydroxybenzylacetone;
Dimethyl sulflde;
Alpha-ionone;
Acetic acid;
Isobutyl acetate;
Acetone;
Butyric acid;
Formic acid;
Valeric acid;
Amyl acetate;
Amyl butyrate;
Anethol;
Benzyl salicylate;
Diacetyl;
Dimethyl anthranilate;
Ethyl methylphenylglycidate;
Ethyl succinate;
Ethyl valerate;
Geraniol;
Cis-3-hexen-l-ol;
2-Hexenyl acetate;
2-Hexenyl butyrate;
Hexyl butyrate;
4-(p-Hydroxyphenyl)-2-butanone;
Beta-ionone;
Isobutyl cinnamate;
Jasmine;
Lemon essential oil;
Methyl butyrate;
Methyl capronate;
Methyl disulfide;
Methyl p-naphthyl ketone;
Orris butter;
Rose absolute;
Terpenyl acetate;
Gamma-undecalactone;
Vanilla; ana
Alcohol.

An additional aspect of our invention provides an organoleptically improved smoking tobacco product and additives therefor, as well as methods of making the same which overcome problems theretofore encountered in which desired spicy, cooling, clove-like, cinnamon bark-like, sweet, fruity, berry-like, juicyfruit; woody, piney, blueberry, banana, green, herbaceous, strawberry-like, and dill flavor characteristics of natural tobacco (prior to smoking and on smoking in the mainstream and in the sidestream) as well as cooling effects, are created or enhanced or modified or augmented and may be readily controlled and maintained at the desired uniform level regardless of variations in the tobacco components of the blend.

This invention further provides improved tobacco additives and methods whereby various desirable natural aromatic tobacco flavoring characteristics with spicy, cooling, clove-like, cinnamon bark-like, sweet, fruity, berry-like, juicyfruit, woody, piney, blueberry, banana, green, herbaceous, strawberry-like and dill notes may be imparted to smoking'tobacco products and may be readily varied and controlled to produce the desired uniform flavoring characteristics.

In carrying out this aspect of our invention, we add to smoking tobacco materials or a suitable substitute therefor (e.g., dried lettuce leaves) an aroma and flavor additive containing as an active ingredient one or more norbornane derivatives of our invention.

In addition to the norbornane derivatives of our invention other flavoring and aroma additives may be added to the smoking tobacco material or substitute therefor either separately or in mixture with the norbornane derivatives as follows:
I. Synthetic Materials
   Beta-ethyl-cinnamaldehyde;
   Eugenol;
   Dipentene;
   Damascenone;
   Maltol;
   Ethyl maltol;
   Delta undecalactone;
   Delta decalactone;
   Benzaldehyde;
   Amyl acetate;
   Ethyl butyrate;
   Ethyl valerate;
   Ethyl acetate;
   2-Hexenol-1;
   2-Methyl-5-isopropyl-l,3-nonadiene-8-one;
   2,6-Dimethyl-2,6-undecadiene-10-one;
   2-Methyl-5-isopropylacetophenone;
   2-Hydroxy-2,5,5,8a-tetramethyl-1-(2-hydroxyethyl)-decahydronaphthalene;
   Dodecahydro-3a,6,6,9a-tetramethylnaphtho-(2,l-b-)-furan
   4-Hydroxyhexanoic acid, gamma lactone; ana
   Polyisoprenoid hydrocarbons defined in Example V of U.S. Patent No. 3,589,372, issued on 6-29-71.
II. Natural oils
   Celery seed oil;
   Coffee extract;
   Bergamot oil;
   Cocoa extract;
   Nutmeg oil; and
   Origanum oil

An.aroma and flavoring concentrate containing one or more norbornane derivatives of our invention and, if desired, one or more of the above indicated additional flavoring additives may be added to the smoking tobacco material, to the filter or to the leaf or paper wrapper. The smoking tobacco material may be shredded, cured, cased and blended tobacco material or reconstituted tobacco material or tobacco substitutes (e.g.; lettuce leaves) or mixtures thereof. The proportions of flavoring additives may be varied in accordance with taste but insofar as enhancement or the imparting of spicy and/or cooling and/or clove-like and/or cinnamon bark-like and/or sweet and/or fruity and/ or berry-like and/or juicyfruit and/or woody and/or piney and/or blueberry and/or banana and/or green and/or herbaceous and/or strawberry-like and/or dill notes, we have found that satisfactory results are obtained if the proportion by weight of the sum total of norbornane derivative(s) to smoking tobacco material is between 50 ppm and 1,500 ppm (0.015% - 0.15%). We have further found that satisfactory results are obtained if the proportions by weight of the sum total of norbornane derivative used to flavoring material is between 1,500 and 15,000 ppm (0.15%-1.5%).

Any convenient method for incorporating the norbornane derivative(s) into the tobacco product may be employed. Thus, the norbornane derivative(s) taken alone or along with other flavoring additives may be dissolved in a suitable solvent such as ethanol, diethyl ether and/or vola- tive organic solvents and the resulting solution may either be spread on the cured, cased and blended tobacco material or the tobacco material may be dipped into such solution. Under certain circumstances, a solution of the norbornane derivative(s) taken alone or taken further together with other flavoring additives as set forth above, may be applied by means of a suitable applicator such as a brush or roller on the paper or leaf wrapper for the smoking product, or it may be applied to the filter by either spraying, or dipping, or coating.

Furthermore, it will be apparent that only a portion of the tobacco or substitute therefor need be treated and the thus treated tobacco may be blended with other tobaccos before the ultimate tobacco product is formed. In such cases, the tobacco treated by have the norbornane derivative(s) in excess of the amounts or concentrations above indicated so that when blended with other tobaccos, the final product will have the percentage within the indicated range.

In accordance with one specific example of our invention an aged, cured and shredded domestic burley tobacco is sprayed with a 20% ethanol solution of the mixture of 1,3-and 3,5-dimethyl-2-norbornane carboxylic acid methyl esters having the structures: 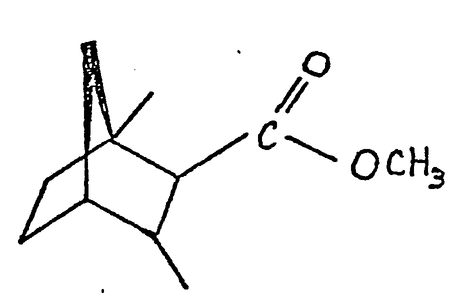 and 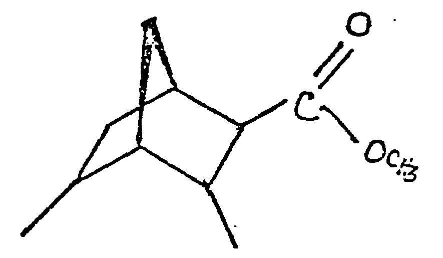 in an amount of mixture to provide tobacco composition containing 800 ppm by weight of the mixture of the two esters on a dry basis. Thereafter the alcohol is removed by evaporation.and the tobacco is manufactured into cigarettes by the usual techniques. The cigarette when treated as indicated has a desired and pleasing aroma which is detectable in the mainstream and the sidestream when the cigarette is smoked. This aroma is described as being sweeter, more aromatic, more tobacco-like having sweet, spicy, cooling, woody and clove-like notes.

In accordance with a second specific example of our invention an aged, cured and shredded domestic burley tobacco is sprayed with a 20% ethanol solution of the compound having the structure: 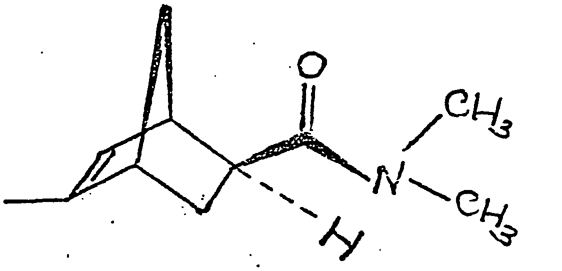 in an amount of mixture to provide tobacco composition containing 800 ppm by weight of the mixture of the amide on a dry basis. Thereafter the alcohol is removed by evaporation and the tobacco is manufactured into cigarettes by the usual techniques. The cigarette when treated as indicated has a desired and pleasing aroma which is detectable in the mainstream and the sidestream when the cigarette is smoked. This aroma is described as being sweeter, fruity, more aromatic, more tobacco-like, having sweet, fruity notes.

While our invention is particularly useful in the manufacture of smoking tobacco, such as cigarette tobacco, cigar tobacco and pipe tobacco, other tobacco products, formed from sheeted tobacco dust or fines may also be used. Likewise, the norbornane derivative(s) of our invention can be incorporated with materials such as filter tip materials (e.g., cellulose acetate filters wherein spicy, cooling, clove-like, cinnamon bark-like, sweet, fruity, berry-like, juicyfruit, woody, piney, blueberry, banana, green, herbaceous, strawberry-like and/or dill effects are desired), seam paste, packaging materials and the like which are used along with tobacco to form a product adapted for smoking. Furthermore, the norbornane derivative(s) can be added to certain tobacco substitutes of natural or synthetic origin (e.g., dried lettuce leaves) and, accordingly, by the term "tobacco" as used throughout this specification is meant any composition intended for human consumption by smoking or otherwise, whether composed of tobacco plant parts or substitute materials or both.

The norbornane derivative(s) and one or more auxiliary perfume ingredients, including, for example, hydrocarbons, alcohols, ketones, aldehydes, nitriles, esters, lactones or cyclic esters, synthetic essential oils and natural essential oils, may be admixed so that the combined odors of the individual components produce a pleasant and desired fragrance, particularly and preferably in fruity, banana, creamy, camphoraceous, herbaceous, strawberry-like, raspberry-like, reseda body-like, sweet, spicy, woody, eucalyptol-like, tagett-like, rum/butterscotch, balsamic, green, and/or minty fragrances. Such perfume compositions usually contain (a) the main note or the "bouquet" or foundation stone of the composition; (b) modifiers which round off and accompany the main note; (c) fixatives which include odorous substances which lend a particular note to the perfume throughout all stages of evaporation and substances which retard evaporation; and (d) topnotes which are usually low boiling fresh-smelling materials.

In perfume compositions, it is the individual components which contribute to their particular olfactory characteristics, however the overall sensory effect of the perfume composition will be at least the sum total of the effects of each of the ingredients. Thus, one or more of the norbornane derivative(s) of our invention can be used to alter, modify or enhance the aroma characteristics of a perfume composition, for example, by utilizing or moderating the olfactory reaction contributed by another ingredient in the composition.

-The amount of norbornane derivative(s) of our invention which will be effective in perfume compositions as well as perfumed articles and colognes depends on many factors, including the other ingredients, their amounts and the effects which are desired. It has been found that perfume compositions containing as little as 0.01% of norbornane derivative(s) or even less (e.g., 0.005%) can be used to impart a fruity, banana, creamy, camphoraceous, strawberry-like, raspberry-like, reseda body-like, herbaceous, sweet, spicy, woody, eucalyptol-like, rum/butterscotch, balsamic, green, spearmint, caraway, basil, powdery, castoreum-like, anise, minty, cut grass-like, borneol-like, tagette-like and/or medicinal odor with camphor, apple-like, herbaceous, minty and calamnus-like nuances to soaps, detergents (including anionic, nonionic, cationic and zwitterionic solid or liquid detergents), cosmetics, fabric softeners, dryer-added fabric softener articles, fabric whiteners and optical brighteners or other products. The amount employed can range up to 70% of the fragrance components and will depend on considerations of cost, nature of the end product, the effect desired on the finished product and the particular fragrance sought.

The norbornane derivative(s) of our invention are useful (taken alone or together with other ingredients in perfume compositions) in detergents and soaps, space odorants- and deodorants, perfumes, colognes, toilet water, bath preparations, such as lacquers, brilliantines, pomades and shampoos; cosmetic preparations, such as creams, deodorants, hand lotions and sun screens; powders such as talcs, dusting powders, face powders and the like. As little as 0.005% of the norbornane derivative(s) (in the ultimate perfumed article) will suffice to impart an intense, fruity, banana, creamy, camphoraceous, strawberry-like, raspberry-like, reseda body-like, herbaceous, sweet, spicy, woody, eucalyptol-like, rum/butterscotch, balsamic, green, spearmint, caraway, basil, powdery, castoreum-like, anise, minty, cut grass-like, borneol-like, tagette-like and/or medicinal note to various types of perfumed articles. Generally no more than 3% of the norbornane derivative(s) based on the ultimate end product (perfumed article) is required.

In addition, the perfume composition or fragrance composition of our invention can contain a vehicle, or carrier for the norbornane derivative(s). The vehicle can be a liquid such as a non-toxic alcohol, a non-toxic glycol or the like. The carrier can also be an absorbent solid, such as a gum (e.g., gum arabic), or components for encapsulating the composition (such as gelatin).

It will thus be apparent that the norbornane derivative(s) of our invention can be utilized to alter, modify or enhance sensory properties, particularly organoleptic properties such as flavor(s) and/or fragrance(s) of a wide variety of consumable materials.

The following examples serve to illustrate processes for specifically producing the norbornane derivative(s) useful in our invention.

The following examples also serve to illustrate specific embodiments of our invention.

It will be understood that these examples are illustrative and the invention is to be considered to be restricted thereto only as indicated in the appended claims.

All parts and percentages given herein are by weight unless otherwise specified.

### Example A

Into a 2 liter distillation flask equipped with eighteen inch column and packed with Berle Saddles, a small Rushover Head, a nitrogen purge and a large receiver cooled using an isopropyl alcohol flask dry-ice bath is placed 10 grams of Primol ®and 1 kilogram of methylcyclopentadiene dimer.

The reaction mass is heated to 150°C and the temperature is controlled with a "Thermowatch" ® (Registered trademark of Instruments for Research and Industry of Cheltenham, Pa.), using a head thermometer. A mixture of 1-methyl-1,3-cyclopentadiene and 2-methyl-l,3-cyclopentadiene is collected in the receiver which is maintained at a temperature of -78°C. The product has a vapor temperature (head temperature) of 60°C and a pot temperature of 173°C. Distillation is carried out until the head temperature reaches 82°C and the pot temperature reaches 187°C, the distillation being carried out at atmospheric pressure.

### Example I

Preparation of l,3-dimethyl-5-norbornene-2-carboxylic acid methyl ester and 3,5-dimethyl-5-norbornene-2-carboxylic acid methyl ester (catalytic Diels-Alder reaction)

### Reaction:

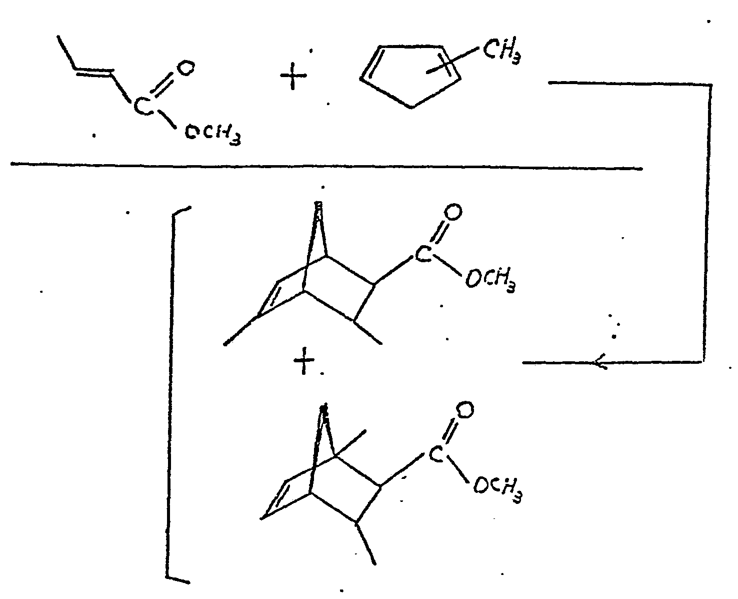

Into a 3 liter flask equipped with stirrer, thermometer, condenser, addition funnel and cooling bath are charged 1000 ml toluene, 500 grams methyl crotonate, 45 grams ethyl aluminum dichloride. The resulting mixture is cooled to 20-25°C. Over a period of 50 minutes while maintaining the reaction mass at 24-25°C, 320 grams of methyl cyclopentadiene (mixture of 1-methyl-1,3-cyclopentadiene and 2-methyl-l,3-cyclopentadiene (50% in toluene solution) is added. After the completion of the addition of the methyl cyclopentadiene mixture, the reaction mass is stirred for a period of 3 hours at 21-25°C. At the end of the three hour period, the reaction mass is heated to 40°C and the temperature is maintained at 40°C for a period of 2.7 hours. At the end of the 2.7 hour period the reaction mass is cooled to 20°C and is poured into one liter of 10% hydrochloride acid solution. The reaction mass now exists in two phases. The aqueous phase is separated from the organic phase. The aqueous phase is extracted with 500 ml toluene. The toluene extract and organic phase are combined. The resulting organic-phase is then washed as follows:
One 1 liter portion water;
One 1 liter portion saturated sodium carbonate;
One 1 liter portion water.

The toluene is then removed under reduced pressure. The resulting organic material is then distilled in a 12 inch Goodloe column yielding the following fractions:

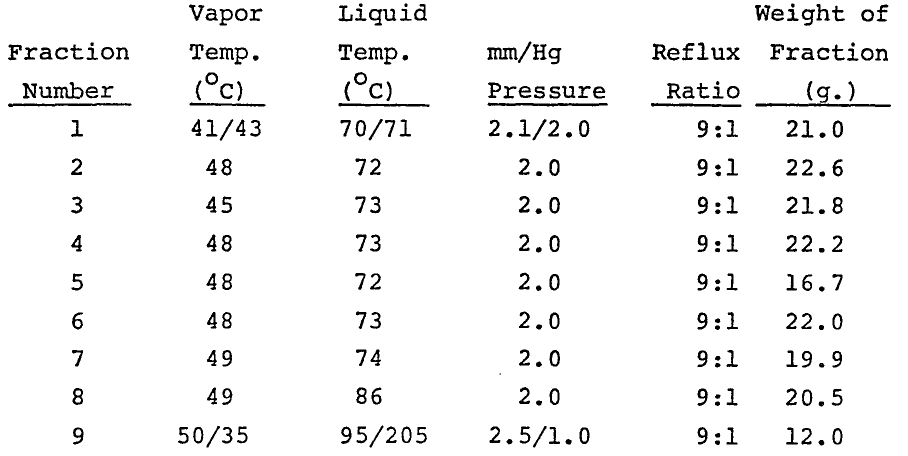

Fractions 4-7 are bulked for purposes of flavor evaluation. The bulked fractions 4-7 as analyzed by NMR, IR and mass spectral analysis contain a mixture of compounds having the structures: 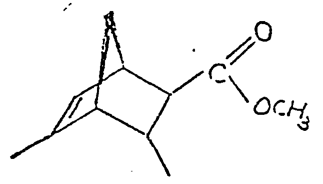 and

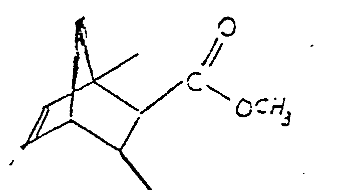

Fractions 4-7 bulked has a burnt fruit aroma and flavor characteristic at 0.1 ppm.

Fractions 4-7 bulked has a fruity, banana, creamy aroma with camphoraceous, minty undertones.

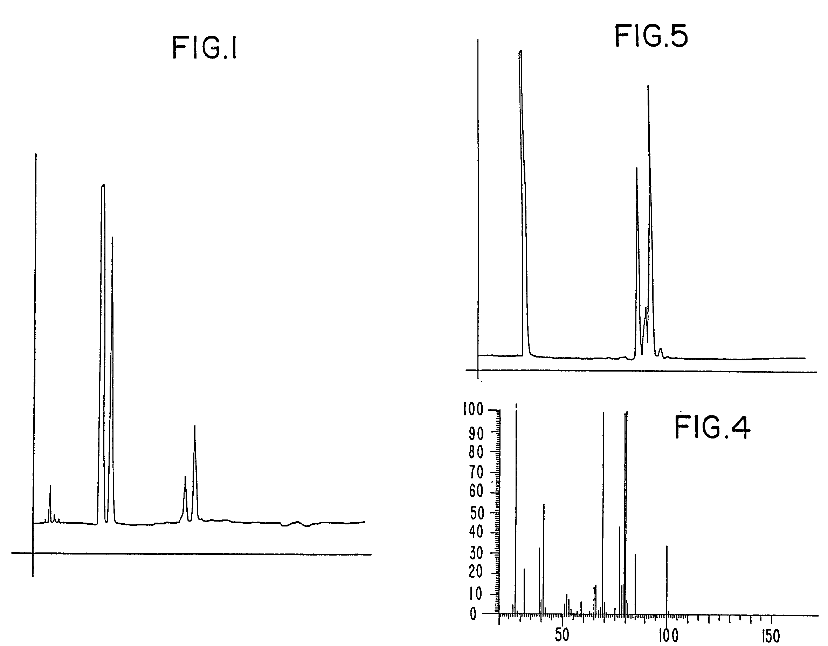

Figure 1 represents the GLC profile for the reaction product of Example I containing the compounds having the structures: 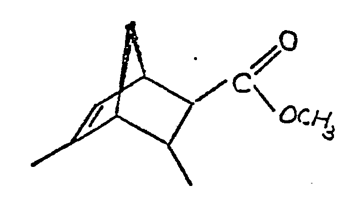 and 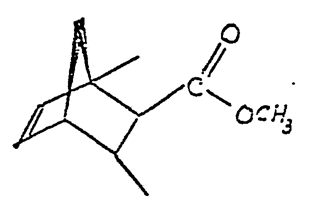

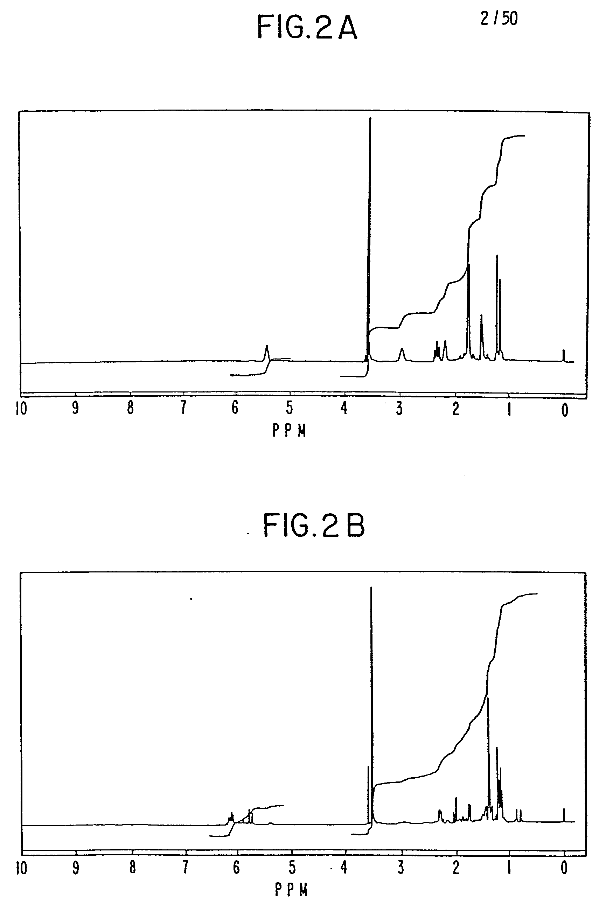

Figure 2A represents the NMR spectrum for fraction 8 of the foregoing distillation containing only the compound having the structure: 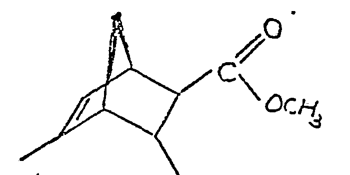
Figure 2B represents the NMR spectrum for fraction 2 of the foregoing distillation containing the compound having the structure: 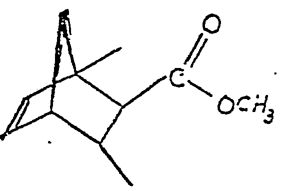

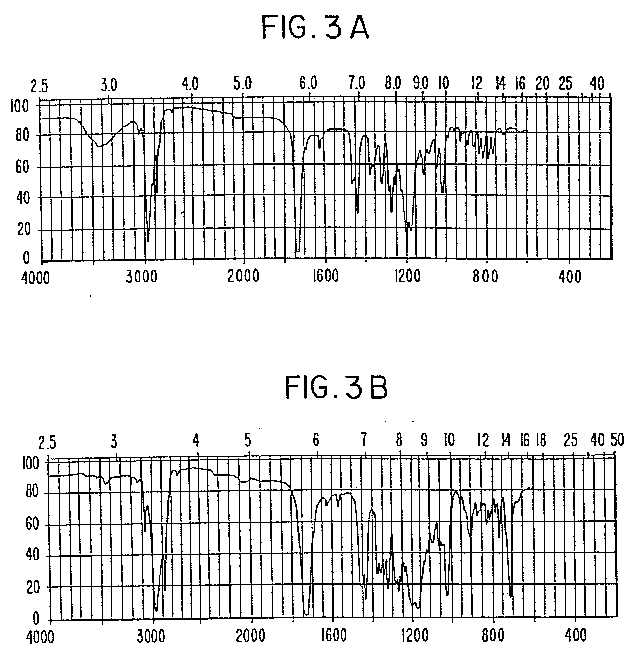

Figure 3A represents the infra-red spectrum for fraction 8 of the foregoing distillation containing only the compound having the structure: 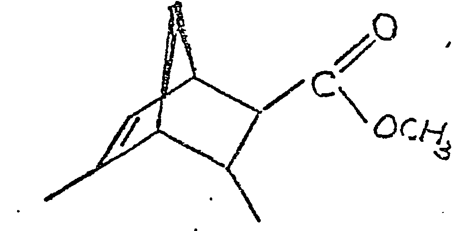
Figure 3B represents the infra-red spectrum for fraction 2 of the foregoing distillation containing only the compound having the structure: 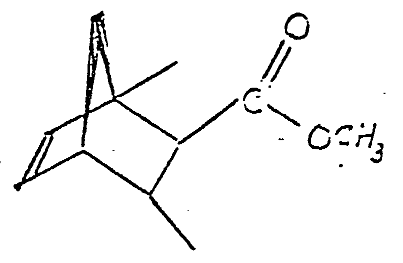

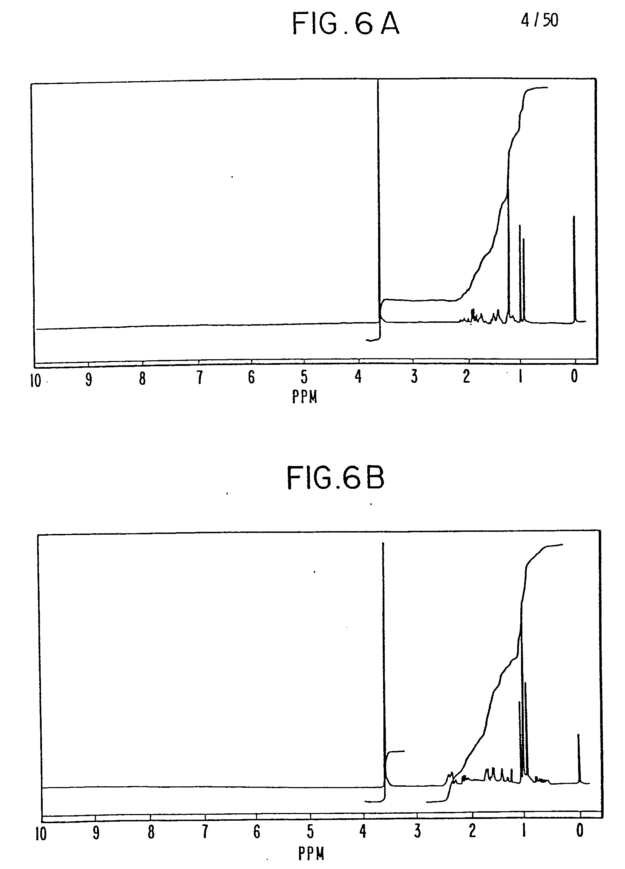

Figure 4 represents the mass spectrum for the reaction product of Example I containing the compounds having the structures: 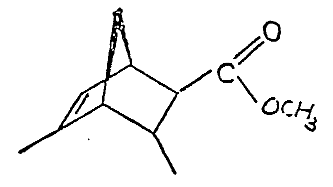 and

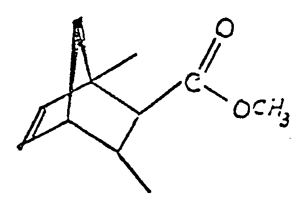

### Example II

### Preparation of 1,3-dimethyl-5-norbornane-2-carboxylic acid methyl ester and 3,5-dimethyl-5-norbornane-2-carboxylic acid methyl ester

Reaction:

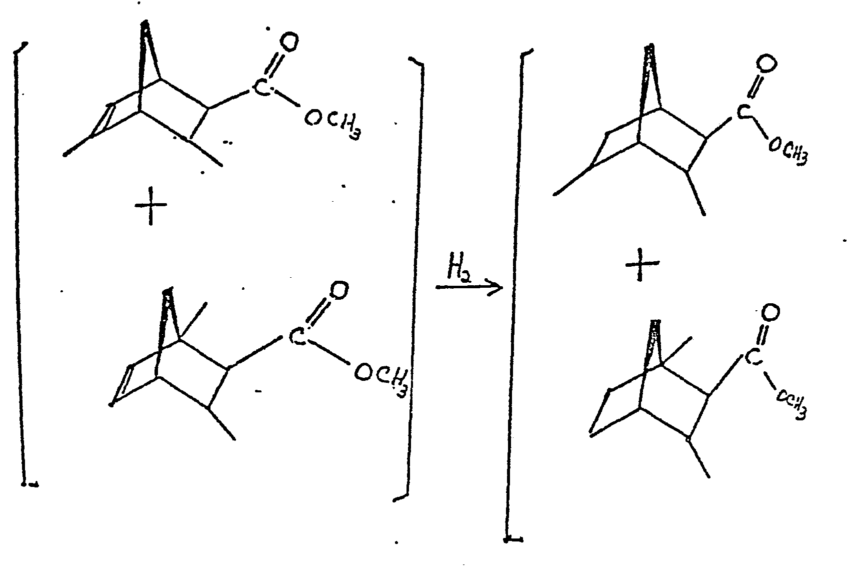

Into a 500 ml pressure bottle held in a Parr shaker is charged 100 ml isopropyl alcohol and 160 grams (0.89 moles) of the methyl cyclopentadiene/methyl crotonate adduct having the structures: 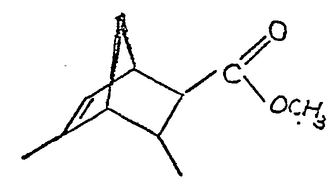 and 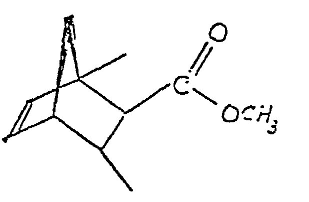 prepared according to Example I and 4 grams of Raney Nickel. The Parr shaker is purged with hydrogen and charged to 50 psig. Shaking is begun and the Parr shaker is recharged with hydrogen when necessary in order to maintain the pressure at 50 psign. A total pressure drop of 74 pounds of hydrogen is observed. A GLC profile (conditions: Carbowax column programmed at 80-220°C at 8°C per minute) indicates some change. NMR analysis indicates that all of the olefin is consumed.

At the end of the reaction, the pressure bottle is opened and the Raney Nickel catalyst is filtered and the solvent is stripped off under reduced pressure. The resulting residue is distilled yielding 122 grams of product. The distillation takes place on a 12" x 2' Goodloe column yielding the following fractions:

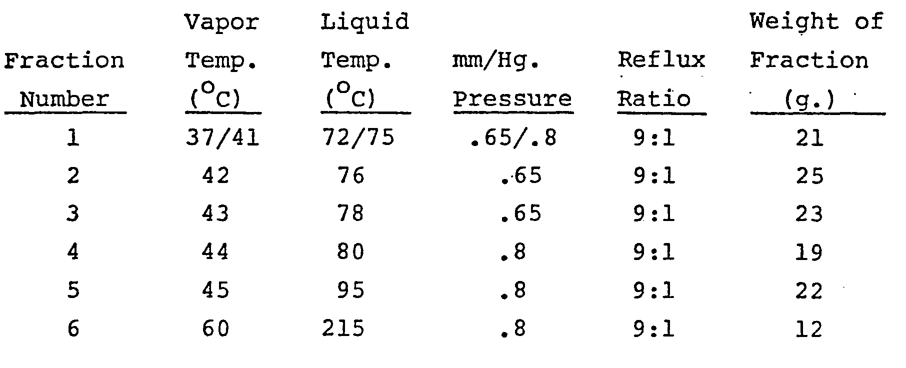

NMR, mass spectral and IR analysis yields the information that the resulting product is a mixture of the compounds having the structures: 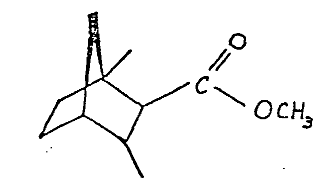 and

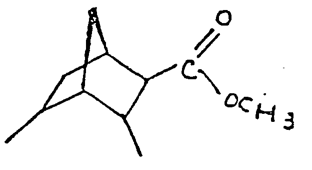

Figure 5 sets forth the GLC profile for the reaction product produced according to this example. Figure 6A sets forth the NMR spectrum for peak 1 of the GLC profile which consists essentially of the compound having the structure:

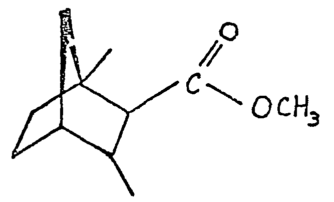

Figure 6B sets forth the NMR spectrum for fraction 6 of the foregoing distillation which consists essentially of the compound having the structure:

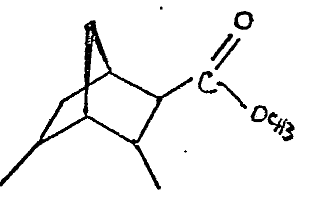

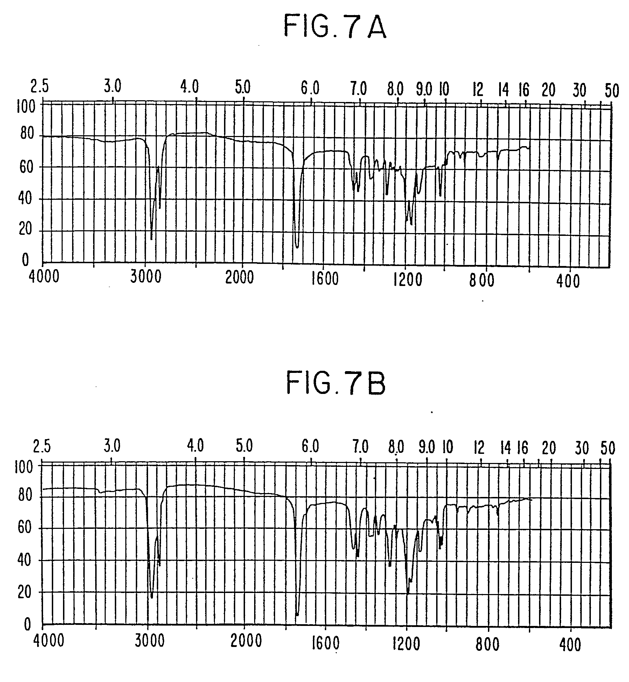

Figure 7A sets forth the infra-red spectrum for peak 1 of the foregoing GLC profile which consists essentially of the compound having the structure:

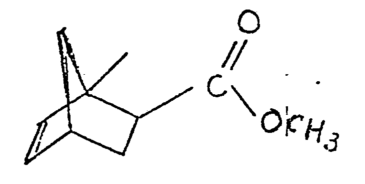

Figure 7B sets forth the infra-red spectrum for fraction 6 of the foregoing distillation which consists essentially of the compound having the structure:

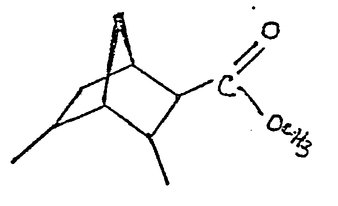

Figure 8 sets forth a mass spectrum for the reaction product of this example which contains the compounds having the structures: 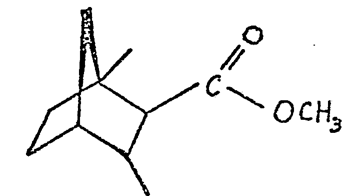 and

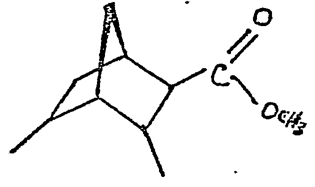

The resulting mixture, from a food flavor standpoint, has a fruity, blueberry aroma and flavor characteristic at 0.1 ppm. The resulting mixture from a fragrance standpoint, has a fruit, camphoraceous and herbaceous aroma profile.

The mixture of this example also has a sweet, woody, spicy, cooling and clove-like aroma profile prior to smoking and a sweet, spicy, cooling and woody aroma and tasteprofile on smoking tobacco articles in both the mainstream and the sidestream.

### Example III

### Preparation of the ethyl ester of 1,3 and 3,5-dimethyl 5-norbornene carboxylic acid (catalytic Diels-Alder reaction)

Reaction: 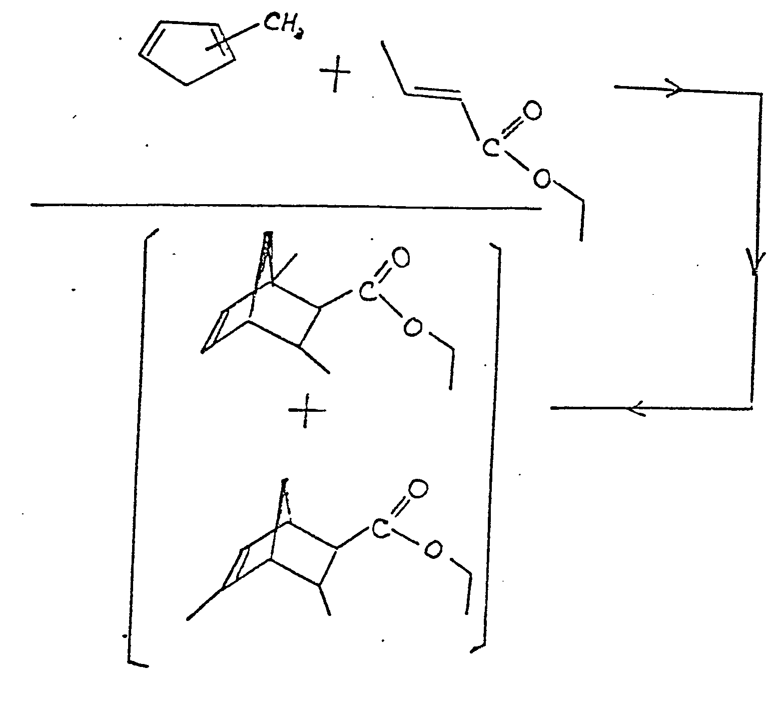 Into a 5 liter reaction vessel equipped with stirrer, thermometer, condenser, addition funnel, nitrogen purge-and cooling bath are added 1000 ml toluene; 600 grams ethyl crotonate; and 50 grams of a 25% solution of ethyl aluminum dichloride in anhydrous toluene. The resulting mixture is cooled to 20-25°C and over a period of 3 hours while maintaining the temperature of the reaction at 20-23°C, 378 grams of methyl cyclopentadiene mixture containing compounds having the structures: 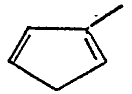 and 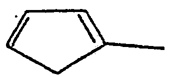 are added.

The reaction mass is then poured into one liter of a 10% aqueous hydrochloric acid solution. The reaction mass now exists in two phases; an aqueous phase and an organic phase. The aqueous phase is removed from the organic phase and the aqueous phase is extracted with one 500 ml volume of toluene. The toluene and organic phases are .combined and washed as follows:
A. One 1 liter portion of water;
B. One 1 liter portion of saturated sodium bicarbonate solution; and
C. One 1 liter portion of water.

The solvent is stripped off via vacuum. The resulting material is then distilled using a 12 inch Goodloe column yielding the following fractions:

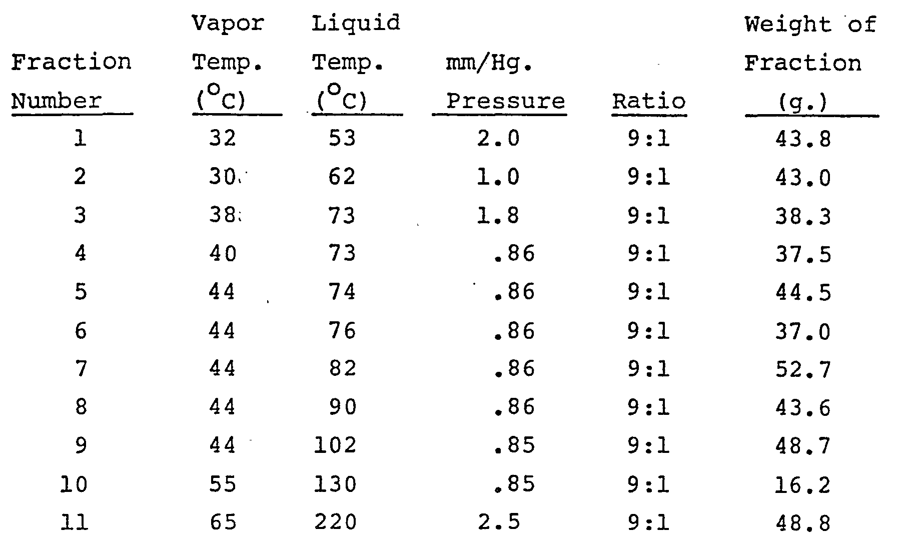

NMR, IR and mass spectral analysis yields the information that the reaction product contains compounds having the structures: 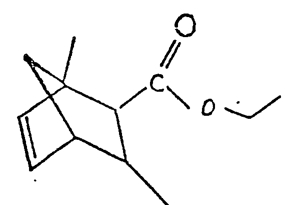 and

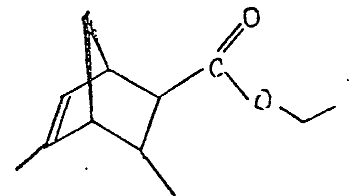

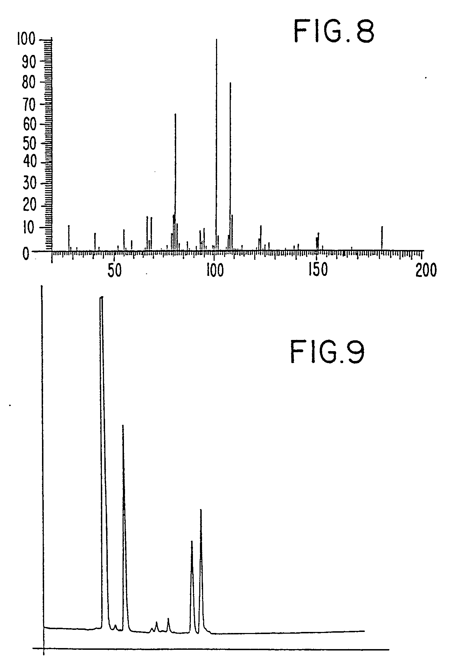

Figure 9 sets forth the GLC profile prior to distillation of the resulting reaction product.

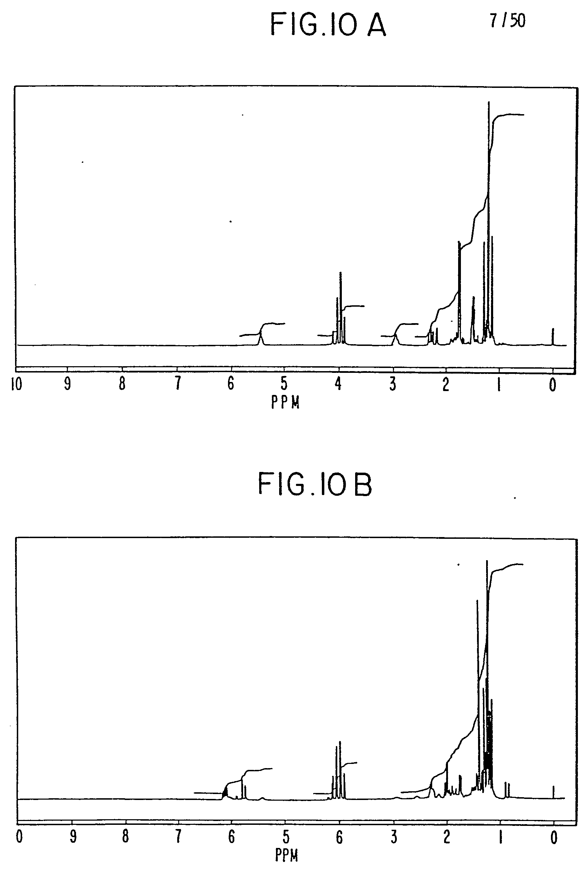

Figure 10A sets forth the NMR spectrum for fraction 10 of the foregoing distillation containing only the product having the structure:

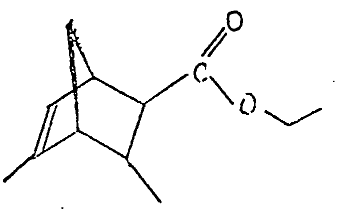

Figure 10B is the NMR spectrum for the compound contained in fraction 6 of the foregoing distillation, having the structure:

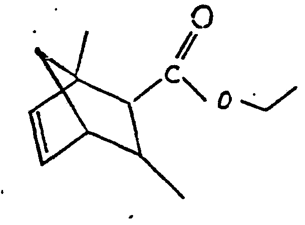

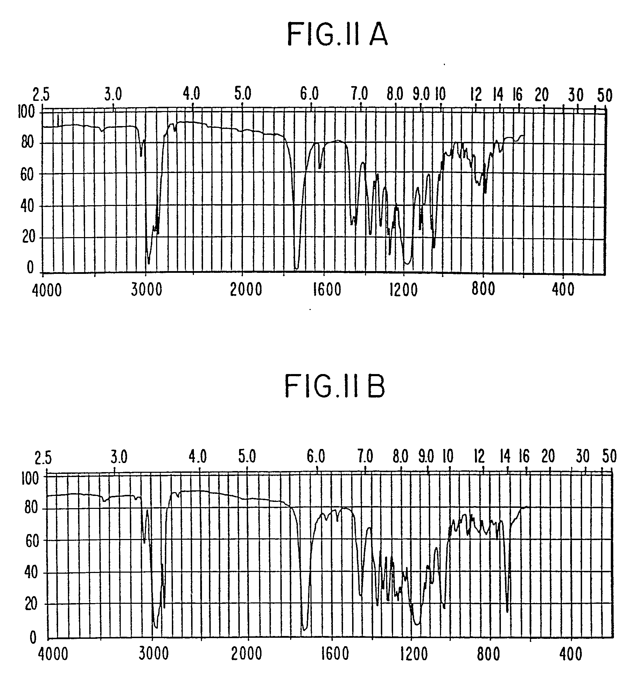

Figure 11A is the infra-red spectrum for the compound of fraction 10 of the foregoing distillation having the structure:

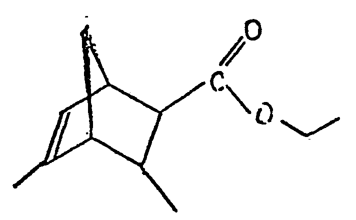

Figure 11B is the infra-red spectrum for the compound contained in fraction 6 of the foregoing distillation, having the structure:

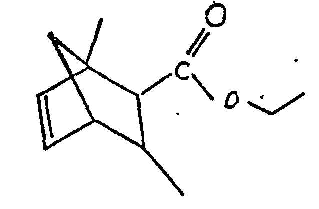

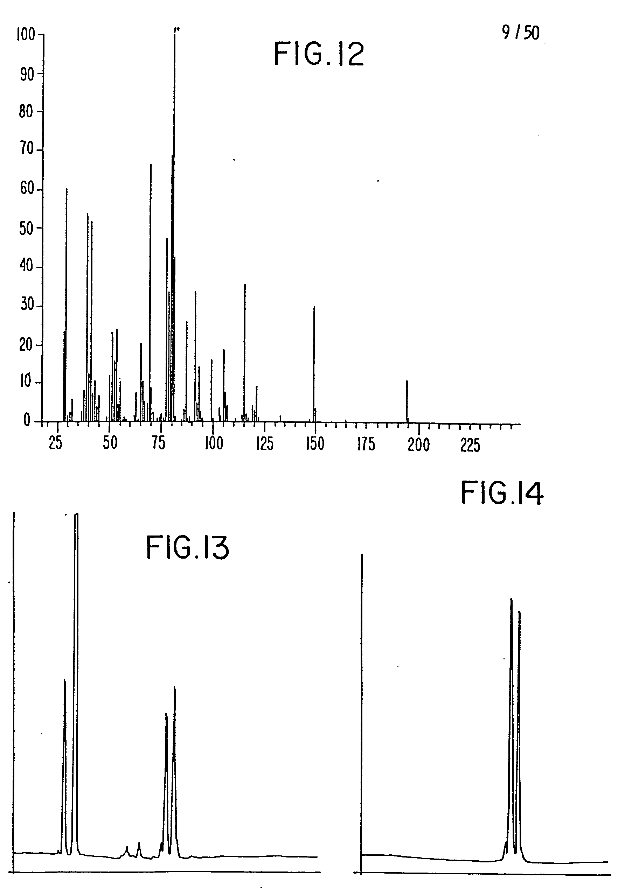

Figure 12 is the mass spectrum for the mixture of compounds produced by the foregoing reaction having the structures: 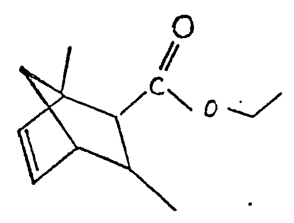 and

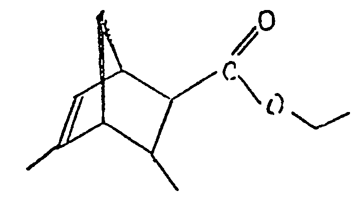

Bulked fractions 5-11 containing the compounds having the structures: 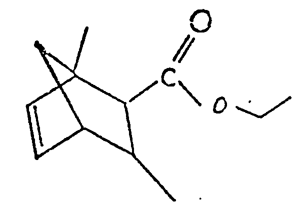 and 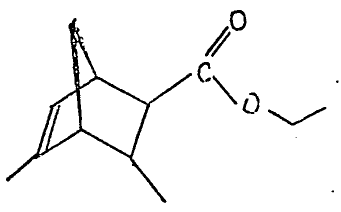 from a fragrance standpoint has a sweet, spicy, herbal, woody, eucalyptol-like aroma with distinct calamnus undertones.

Bulked fractions 5-11 from a food flavor standpoint has a sweet, fruity, berry-like, spicy, black pepper-like, herbaceous and clove-like aroma and taste profile at 10 ppm causing said bulked fractions to be useful in blueberry flavored tobacco (cloves) flavor and raspberry flavor.

Bulked fractions 5-11 from a tobacco flavor standpoint has a sweet, fruity, berry, spicy, cinnamon bark-like and clove-like aroma and taste profile both prior to and on smoking in the mainstream and in the sidestream of smoking tobacco articles such as cigarettes and cigars.

### Example IV

### Preparation of ethyl esters of 1- and 5-methyl-5-norbornene-2-carboxylic acids (catalytic Diels-Alder reaction)

Reaction:

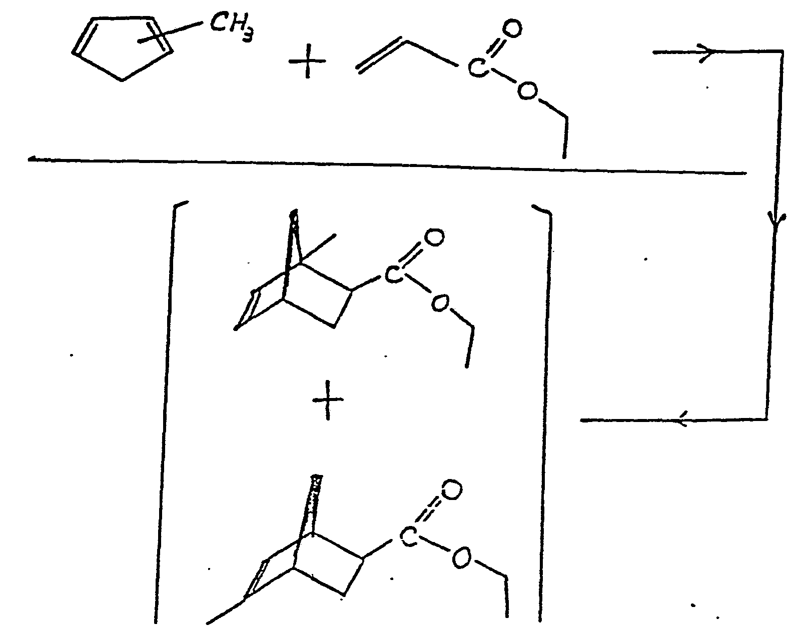

Into a three litex flask equipped with thermometer, stirrer, condenser, addition funnel, cooling bath and nitrogen purge are placed one gram of hydroquinone, 100 ml toluene and 5.0 grams of 25% ethyl aluminum dichloride dissolved in anhydrous toluene. The reaction mass is cooled to about 10°C and, over a period of 30 minutes, 600 grams of ethyl acrylate is added to the resulting mixture. During the addition the temperature of the mixture is maintained at between 10°C and 14°C.

Over a period of three hours while maintaining the reaction temperature at 20-23°C, 389 grams of a mixture of 1-methyl-1,3-cyclopentadiene and 2-methyl-l,3-cyclopentadiene is added to the reaction mixture. At the end of the three hour period GLC analysis indicates the reaction to be complete.

The resulting reaction mass is poured into one liter of.

10% aqueous hydrochloric acid. The reaction mass now exists in two phases; an organic phase and an aqueous phase. The aqueous phase is separated from the organic phase and the organic phase is washed as follows:
A. One 1 liter portion of water;
B. One 1 liter portion of saturated sodium bicarbonate solution;
C. One 1 liter portion of water;
D. One 1 liter portion of water.

The pH of the resulting washing is about six.

The resulting organic layers are combined and the toluene is stripped off under vacuum. Calcium carbonate and Primol ® is added to the reaction mass and the reaction mass is distilled yielding the following fractions on a 12 inch Goodloe column.

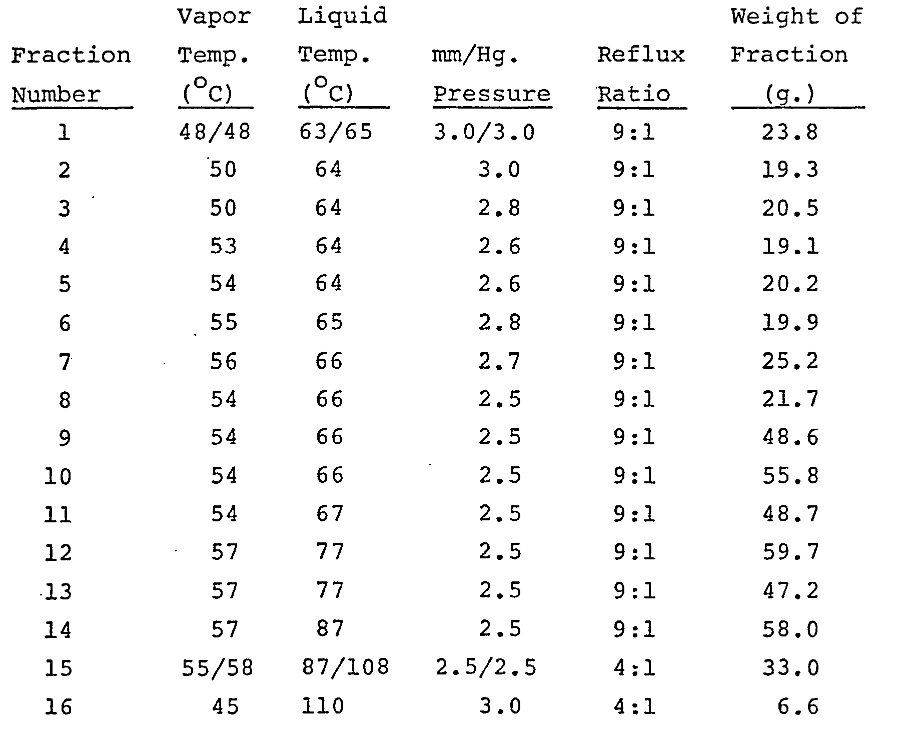

Fractions 7-12 are bulked and NMR, IR and mass spectral analysis yield the information that these fractions consist essentially of a mixture of compounds having the structures: 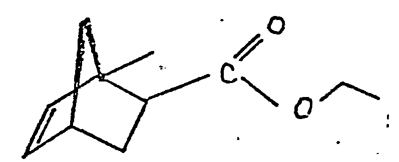 and

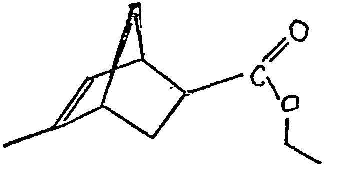

Figure 13 is the GLC profile of the crude reaction product washed, but before distilling. (Conditions: Carbowax column programmed at 80-122°C at 8°C per minute.)

Figure 14 is the GLC profile of fractions 7-12 from the foregoing distillation, bulked.

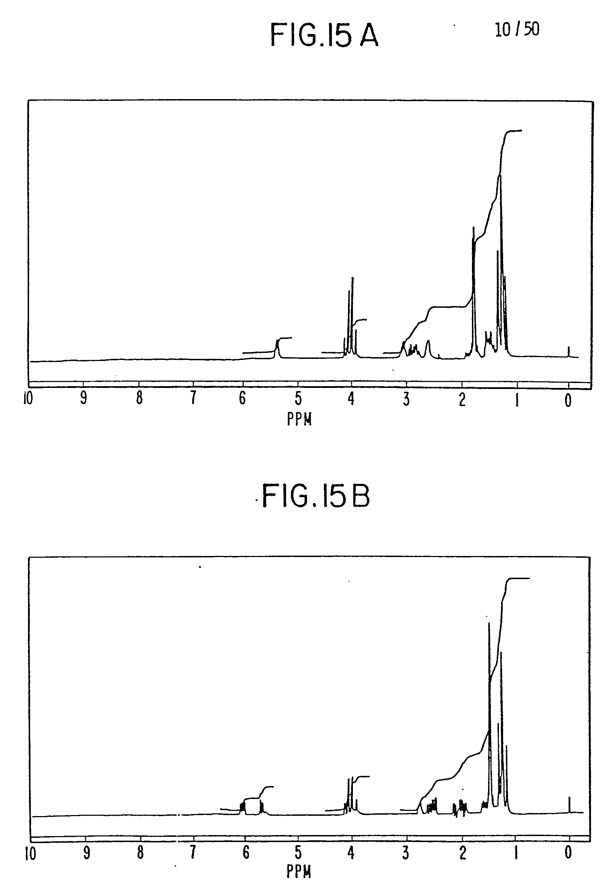

Figure 15A is the NMR spectrum for the compound having the structure:

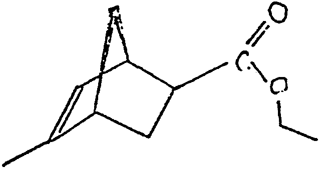

Figure 15B is the NMR spectrum for the compound having the structure:

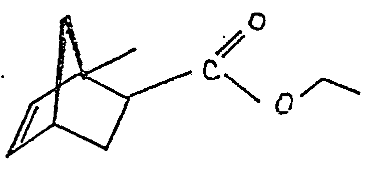

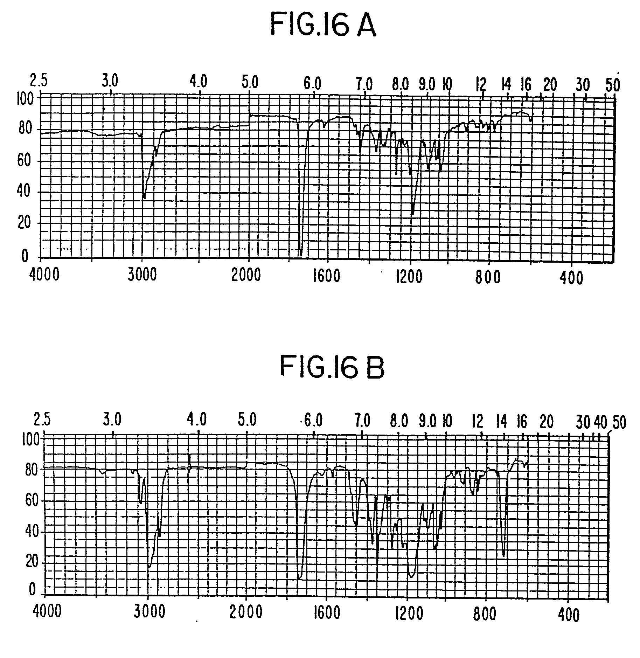

Figure 16A is the infra-red spectrum for the compound having the structure:

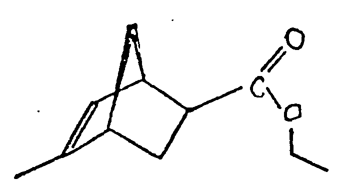

Figure 16B is the infra-red spectrum for the compound having the structure:

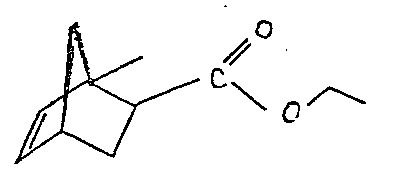

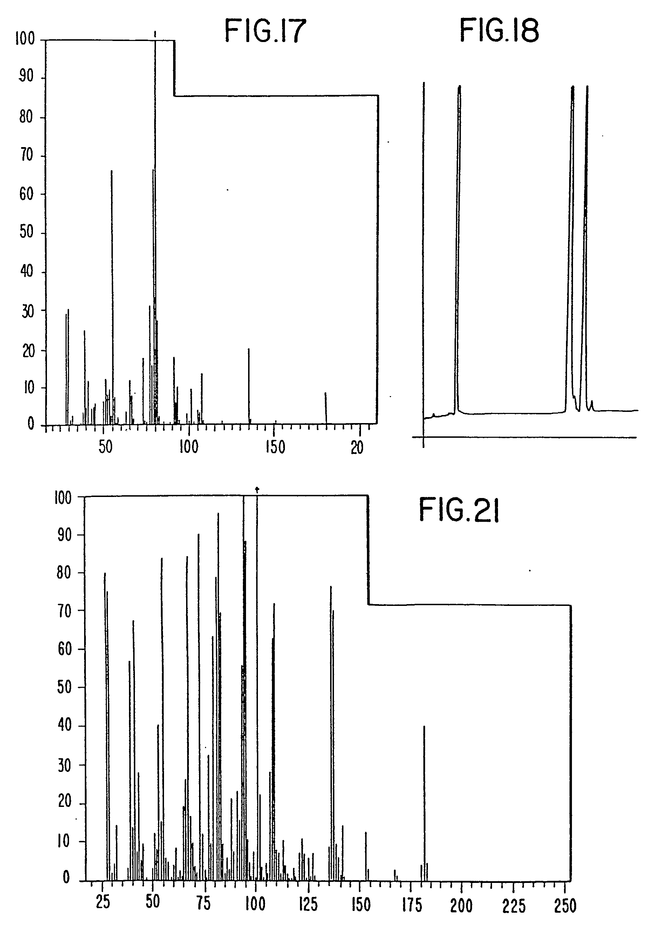

Figure 17 is the mass spectrum for the mixture of compounds having the structures: 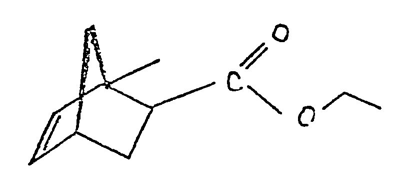 and

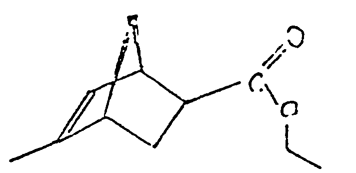

The mixture of compounds having the structures: 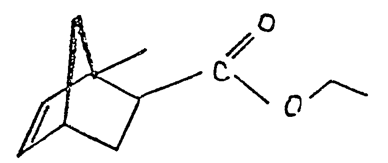 and 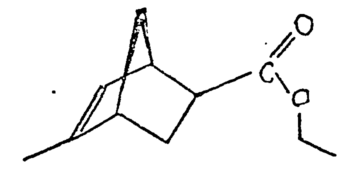 from a fragrance standpoint has a sweet, fruity (banana- like) and creamy aroma profile with minty nuances intense on dryout.

From a food flavor standpoint, the resulting mixture has a sweet, fruity, red berry, raspberry and seedy aroma and taste and in addition a strawberry taste at 0.02 ppm. Thus, this material is useful in red berry, cherry, raspberry and strawberry flavored foodstuffs.

The resulting mixture has a fruity, banana, green and strawberry-like aroma and taste both prior to and on smoking in smoking tobacco articles in both the mainstream and the sidestream.

### Example V

### Preparation of the ethyl esters of 1- and 5-methyl norbornane-2-carboxylic acids

Reaction:

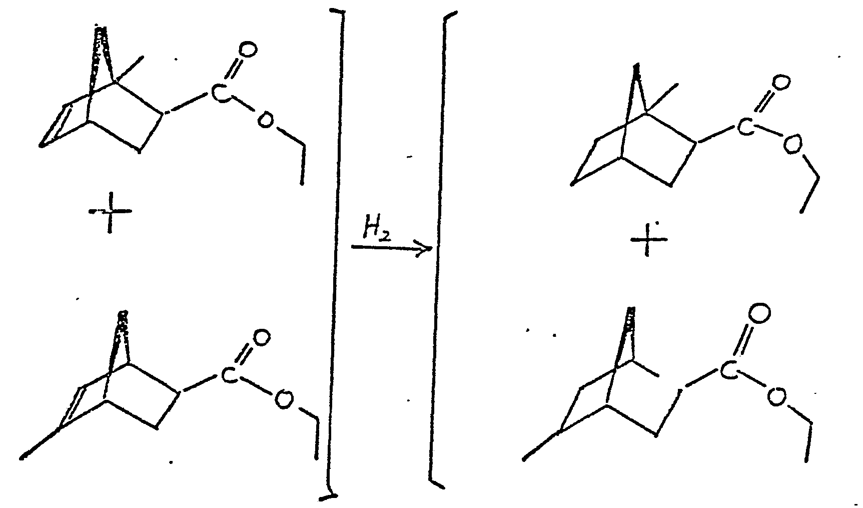

Into a 500 ml pressure bottle attached to a Parr shaker is charged 200 grams (1.11 moles) of the reaction mixture (bulked fractions 7-12) produced according to Example IV consisting essentially of compounds having the structures: 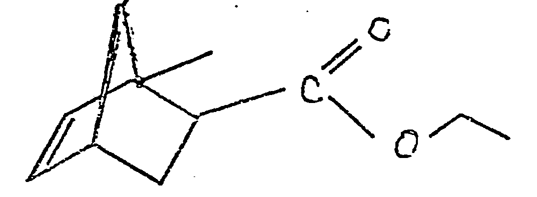 and 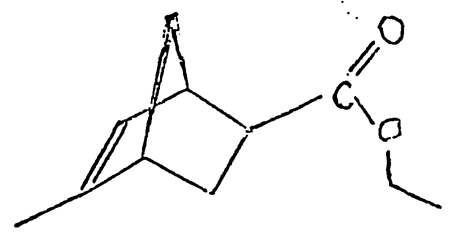 5 grams of Raney Nickel and 125 ml of isopropyl alcohol. The Parr shaker apparatus is purged six times with hydrogen and the 500 ml pressure bottle with contents is pressurized to 50 psig. The bottle is closed and shaking is begun and continued until the hydrogen uptake ceases. This period of time takes 4.25 hours. The 500 ml pressure bottle is recharged when necessary. At the end of the reaction the 500 ml pressure bottle is opened and the reaction mass is filtered and the solvent stripped in vacuum. The resulting residue is distilled on a 1 inch Goodloe column and yields the following fractions:

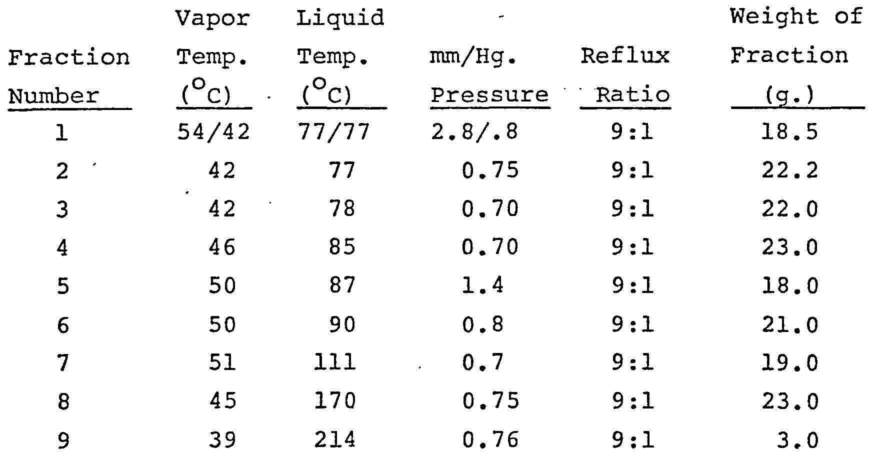

NMR, IR and mass spectral analysis on fractions 1 and 9 yield the information that the resulting mixture contains and consists essentially of the compounds having the structures: 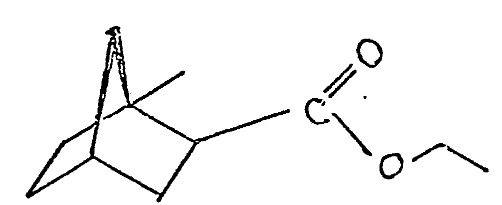 and

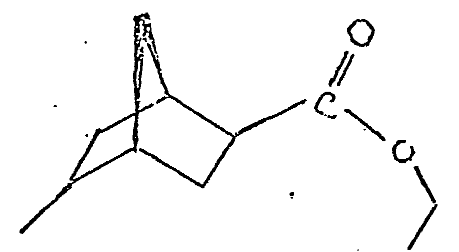

Figure 18 is the GLC profile for the resulting reaction mixture.

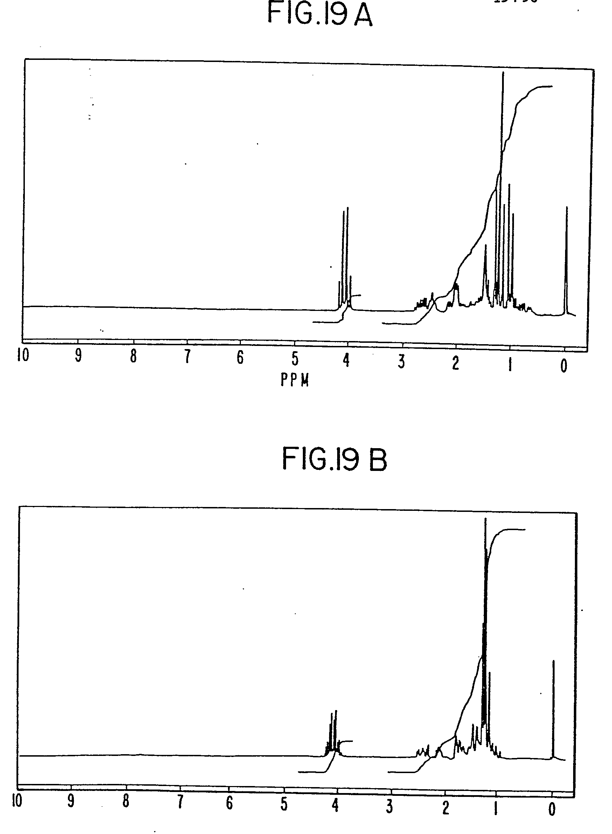

Figure 19A is the NMR spectrum for fraction 9 of the foregoing distillation containing and consisting of the compound having the structure:

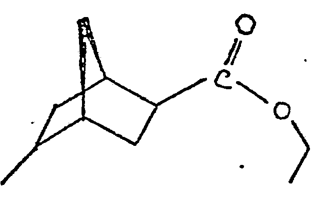

Figure 19B is the NMR spectrum for fraction 1 of the foregoing distillation consisting of the compound having the structure:

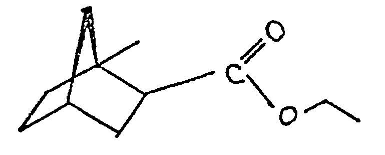

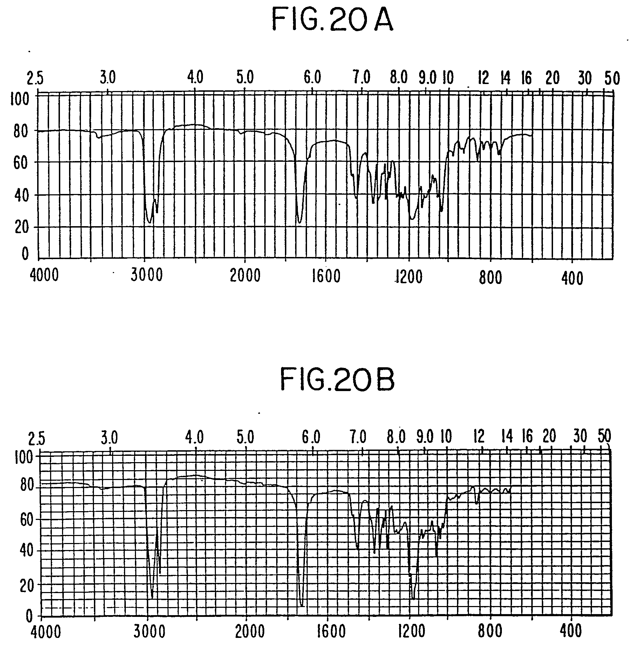

Figure 20A is the infra-red spectrum for fraction 9 of the foregoing distillation consisting of the compound having structure:

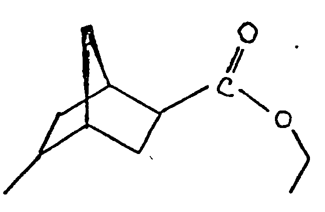

Figure 20B is the infra-red spectrum for fraction 1 of the foregoing distillation consisting of the compound having the structure:

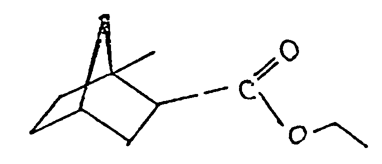

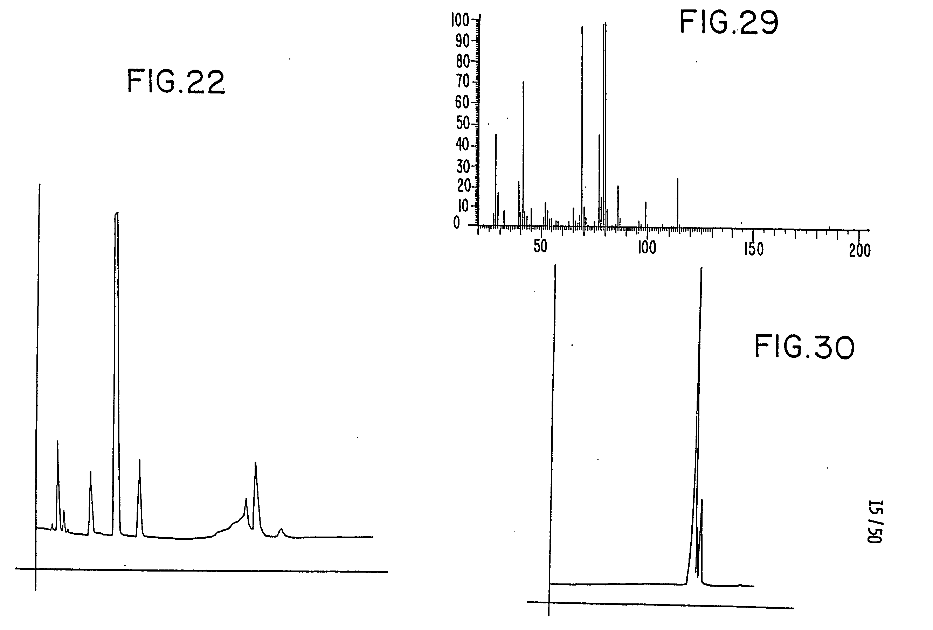

Figure 21 is the mass spectrum for the reaction product produced according to this example containing the mixture of compounds having the structures: 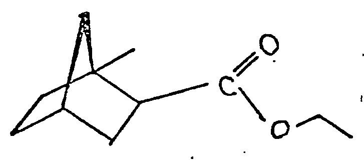 and

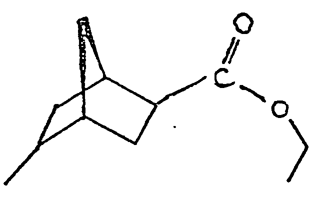

From a fragrance standpoint, bulked fractions 6-8 have a fruity, banana and creamy aroma profile.

From a food flavor standpoint, bulked fractions 6-8 have a sweet, fruity, raspberry, vermouth-like and blueberry aroma profile with a fruity, raspberry and blueberry taste profile at 0.01 ppm and at 1 ppm causing this mixture to be useful for vermouth, blueberry, raspberry mouthwash and toothpaste flavors.

From a tobacco flavor standpoint, bulked fractions 6-8 has, prior to and on smoking in the mainstream and in the sidestream, a sweet, fruity, "juicyfruit", woody, piney and blueberry aroma and taste profile.

### Example VI

### Preparation of 1- and 5-methyl-5-norbornene-2-carboxylic acid n-butyl esters (catalytic Diels-Alder reaction)

Reaction:

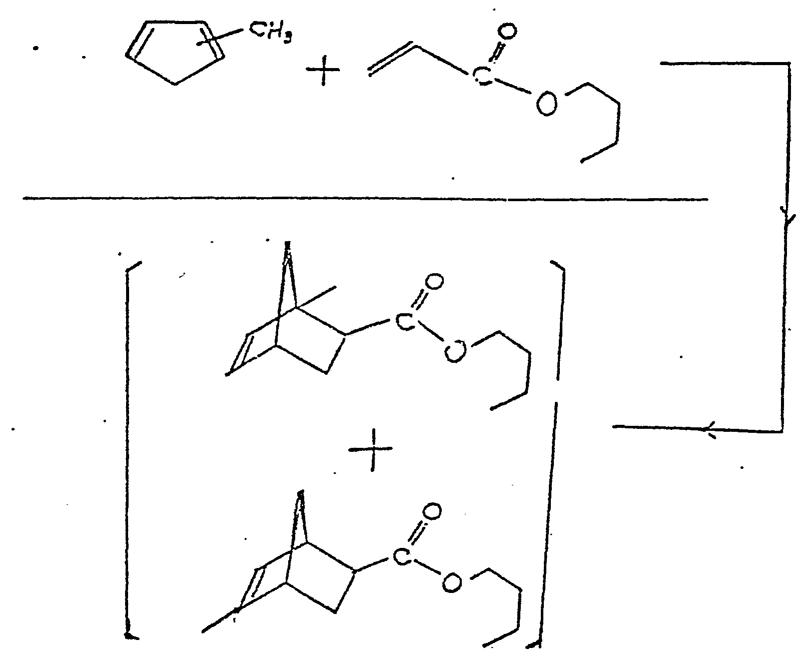

Into a 3 liter reaction flask equipped with stirrer, thermometer, reflux condenser, dropping funnel and cooling bath is placed 700 ml toluene and 60 grams of ethyl aluminum dichloride. While maintaining the temperature of the mixture at 20-25°C, via the dropping funnel is added 300 grams of n-butyl acrylate over a period of 20 minutes.

While keeping temperature at 20-25⁰C over a 40 minute period, 380 grams of a 50% solution of 1-methyl-1,3-cyclopentadiene and 2-methyl-l,3-cyclopentadiene is added to the reaction mass.

After about 2 hours, an additional 30 grams of ethyl aluminum dichloride is added and the reaction mass is stirred for another 1.5 hours while maintaining the temperature thereof at 20-25°C.

Reaction mass is then added to 1 liter of 10% hydrochloric acid. The reaction mass now exists in two phases; and organic phase and an aqueous phase. The organic phase is removed and washed as follows:
A. One 1 liter portion of water;
B. One 1 liter portion of saturated sodium carbonate;
C. One 1 liter portion of water.

The solvent is then removed by vacuum distillation and the resulting product is distilled on a 12" Goodloe column yielding the following fractions:

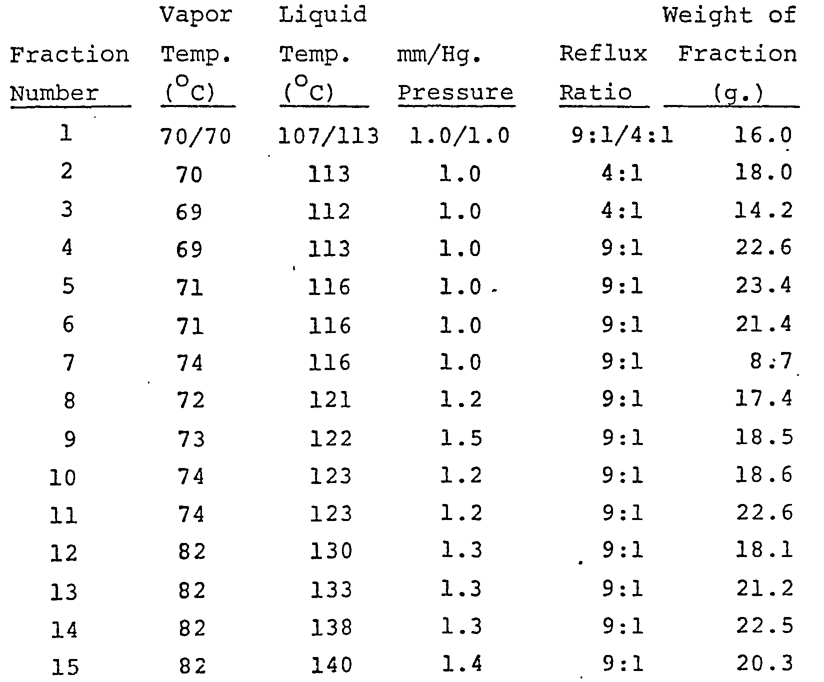

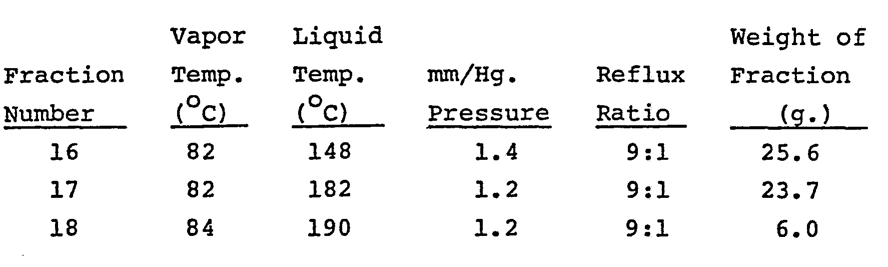

NMR, IR and mass spectral analysis yielding the information that the resulting product is a mixture of compounds having the structures: 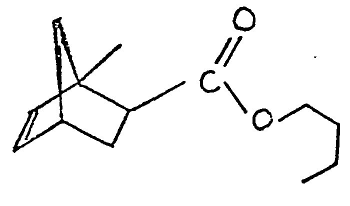 and

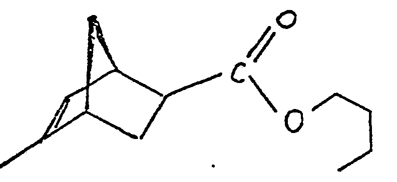

Figure 22 sets forth the GLC profile for the reaction product.

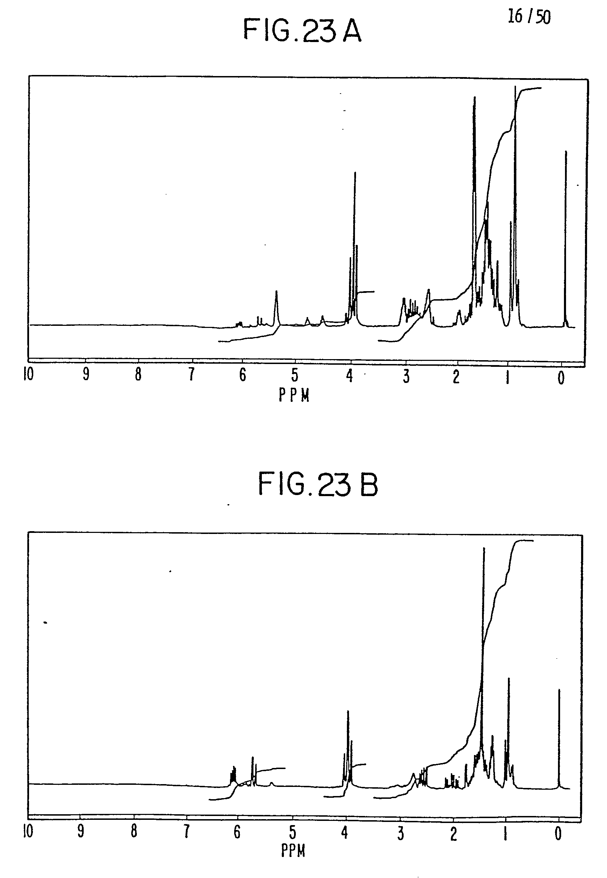

Figure 23A sets forth the NMR spectrum for fraction 14 for the foregoing fractional distillation and this fraction consists of compound having the structure:

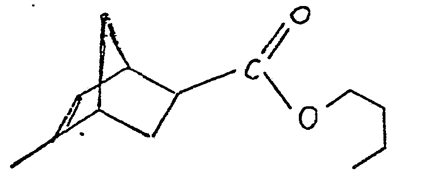

Figure 23B sets forth the NMR spectrum for fraction 4 of the foregoing distillation consisting of the compound having the structure:

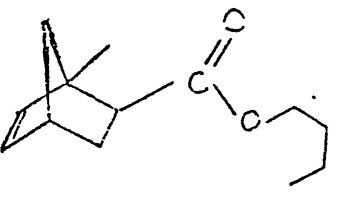

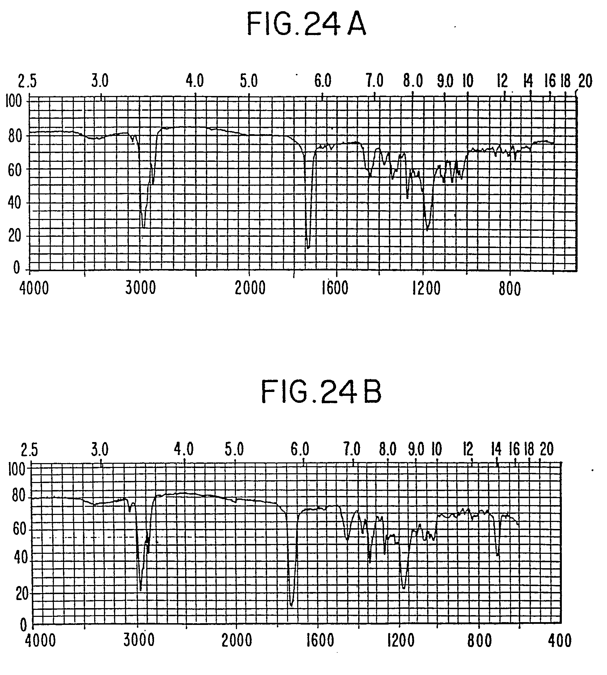

Figure 24A sets forth the infra-red spectrum for fraction 14 of the foregoing distillation consisting of the compound having the structure:

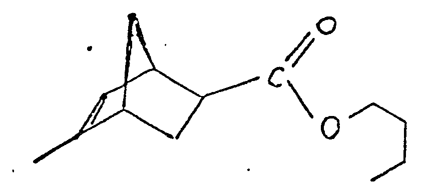

Figure 24B sets forth the infra-red spectrum for fraction 4 of the foregoing distillation consisting of the compound having the structure:

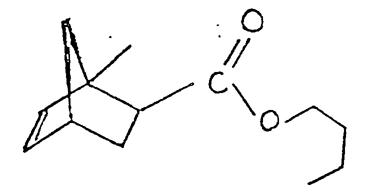

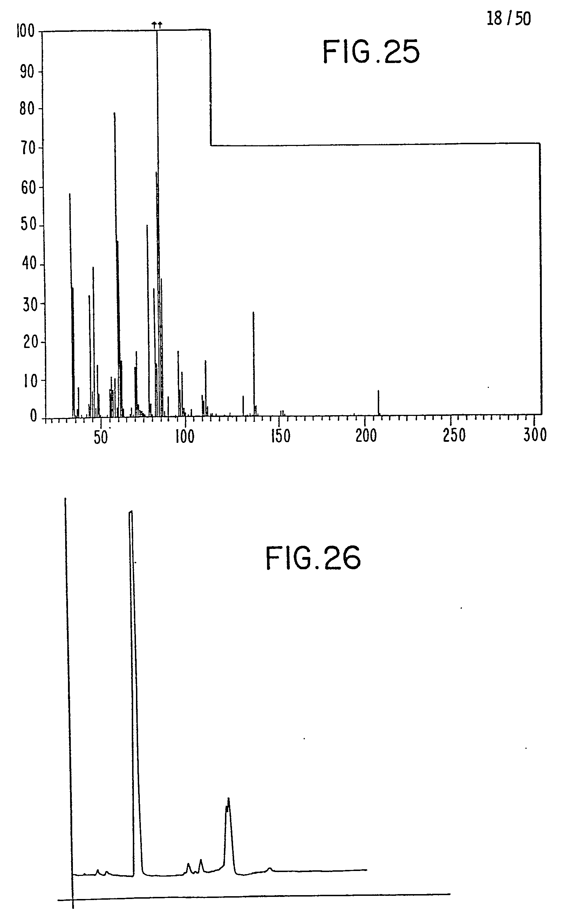

Figure 25 sets forth the mass spectrum for the reaction product containing the compounds having the structures: 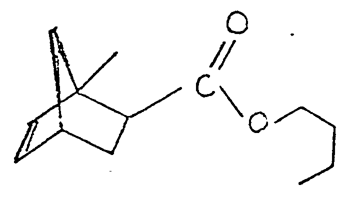 and

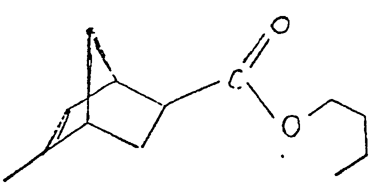

From a fragrance standpoint bulked fractions 2-18 have a balsamic and rum/butterscotch aroma profile.

From a food flavor standpoint, bulked fractions 5-14 have a fruity, strawberry, blueberry, balsamic aroma profile with a blueberry, balsamic and bitter flavor profile at 2 ppm.

From a tobacco flavor standpoint, bulked fractions 5-14 have an earthy and mushroom aroma and taste both prior to and on smoking in the mainstream and in the sidestream.

### Example VII

### Preparation of ethyl esters of 1,2- and 2,5-dimethyl-5-norbornene-2-carboxylic acids (catalytic Diels-Alder reaction)

Reaction:'

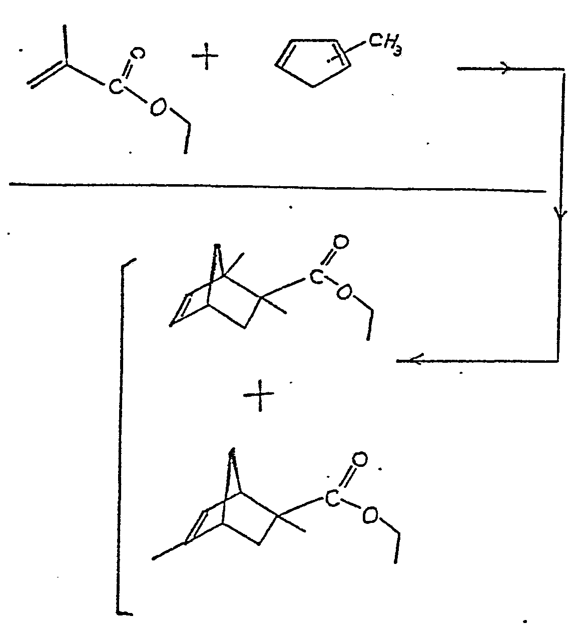

Into a 5 liter flask equipped with condenser, thermometer, addition funnel, nitrogen purge and cooling bath are placed 1000 ml toluene, 50 grams of ethyl aluminum dichloride and 280 grams of ethyl methacrylate. The resulting mixture is cooled to 20-25⁰C and over a period of 3 hours while maintaining the temperature at 23-24°C, 400 grams of a mixture of 1-methyl-1,3-cyclopentadiene and 2-methyl-1,3-cyclopentadiene is added to the reaction mass. The reaction mass is then poured into 1 liter of 10% aqueous hydrochloric acid. The reaction mass now exists in two phases; an aqueous phase and an organic phase. The aqueous phase is extracted with one 500 ml portion of toluene and the toluene extract is combined with the organic layer. The resulting organic phase is washed as follows:
A. One 1 liter portion of water;
B. One 1 liter portion of saturated sodium bicarbonate;
C. One 1 liter portion of water.

The resulting product is then stripped of solvent under vacuum. Primol ® and Ionox ® are then added to the reaction mass. The reaction mass is then distilled on an 18" Goodloe column yielding the following fractions:

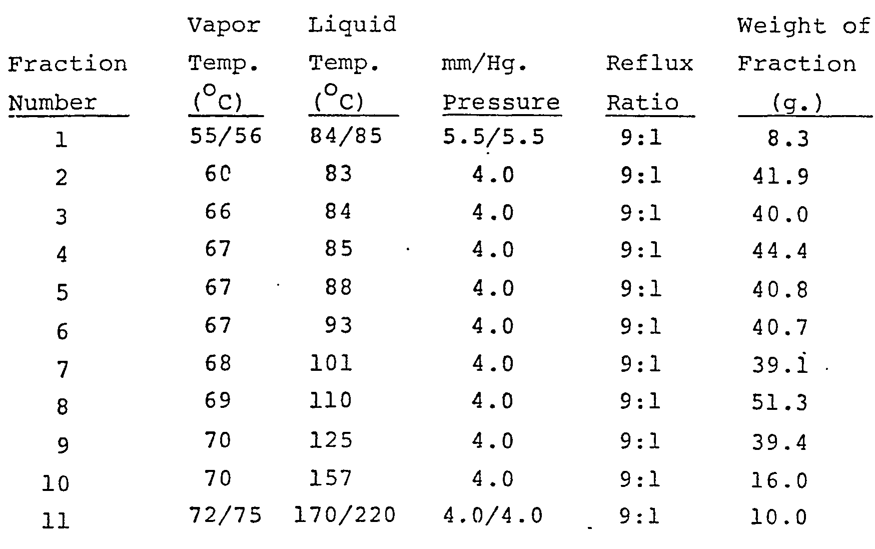

NMR, IR and mass spectral analysis yield the information that the resulting product contains two compounds having the structures: 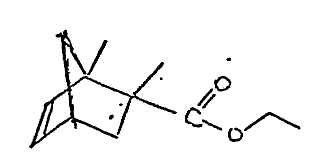 and

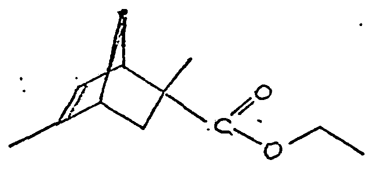

These materials are not separable via ordinary commercial physical separation procedures or commercial unit operations.

Figure 26 sets forth the GLC profile for the resulting mixture containing the compounds having the structures: 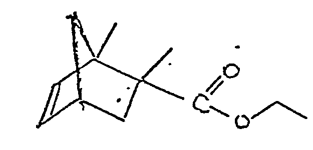 and

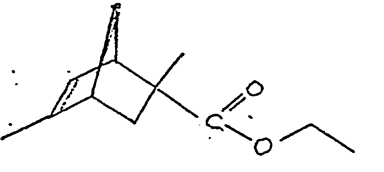

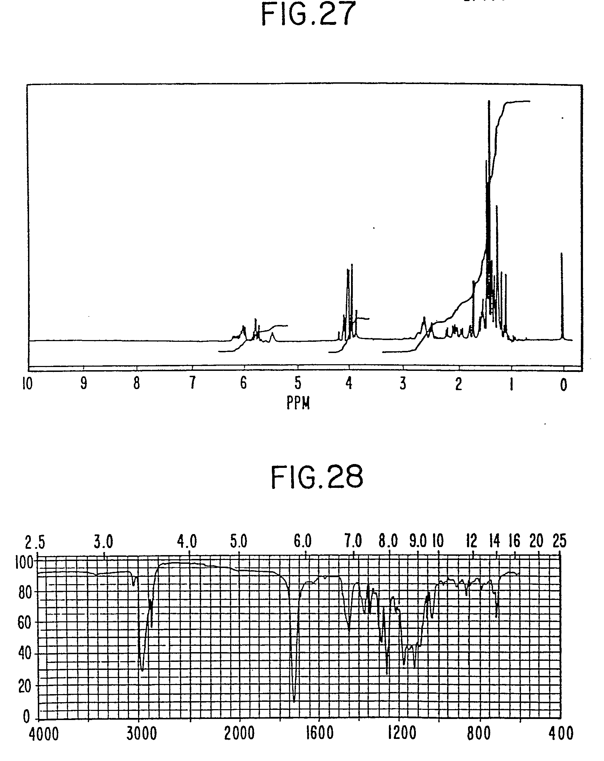

Figure 27 sets forth the NMR spectrum for the mixture of compounds having the structures: 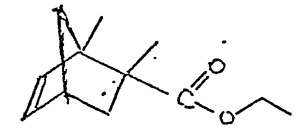 and

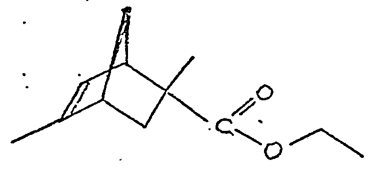

Figure 28 sets forth the infra-red spectrum for the compounds having the structures: 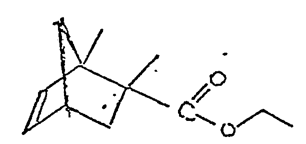 and 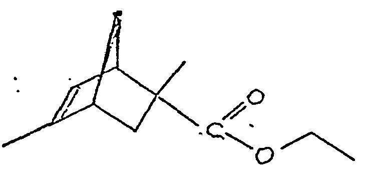 in admixture.

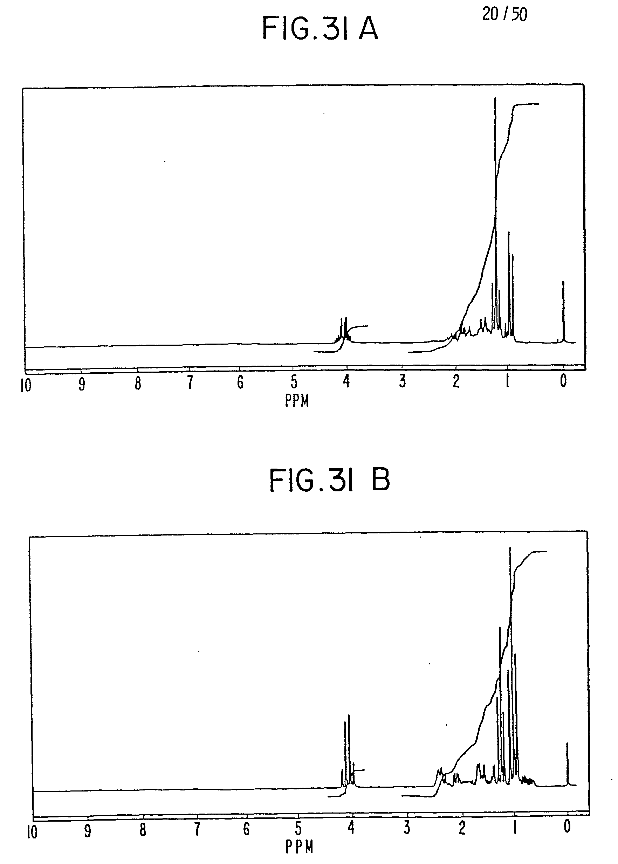

Figure 29 sets forth the mass spectrum for the reaction product as produced above containing and consisting of the structures: 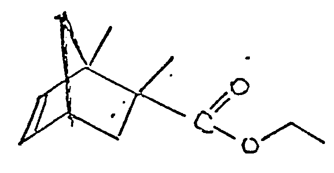 and

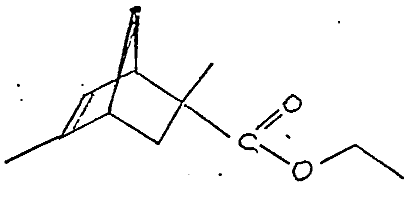

Bulked fractions 4-10 from a fragrance standpoint has a green, minty, spicy aroma with medicinal nuances and a pepacuana bark extract type undertone.

From a food flavor standpoint, the resulting mixture containing the compounds having the structures: 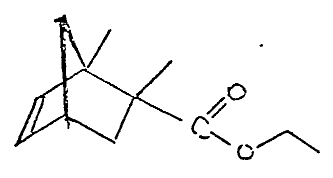 and 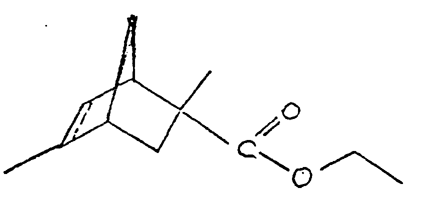 has a fruity, raspberry and seedy aroma and flavor characteristic with additional bitter flavor nuance at 0.2 ppm.

From a tobacco flavor standpoint, the resulting mixture has a sweet, green, herbaceous, dill and fruity aroma and taste both prior to and on smoking in smoking tobacco in the mainstream and in the sidestream.

### Example VII-A

Preparation of 1,3- and 3,5-dimethyl norbornane carboxylic acid esters

Reaction:

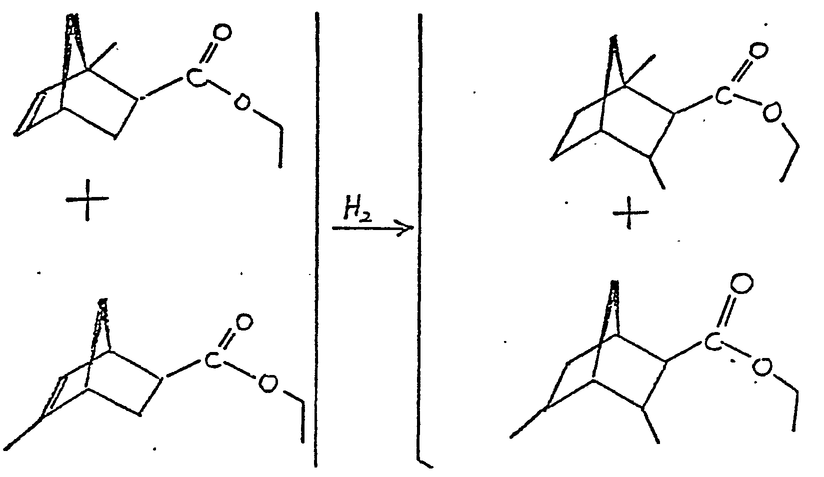

Into a 500 ml pressure bottle equipped with Parr shaker is placed 200 grams of the reaction product of Example III containing the compound having the structures: 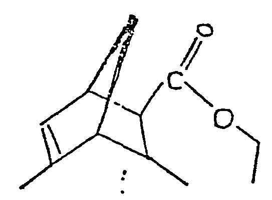 and 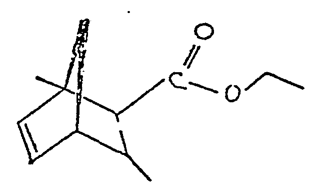 100 ml isopropyl alcohol and 5 grams of Raney nickel. The 500 ml pressure bottle is then closed and placed under hydrogen pressure. The Parr shaker is shaken while maintaining the hydrogen pressure at 50 psig for a period of 5.5 hours at a temperature of 20-30°C. The Parr shaker and pressure bottle apparatus is then opened and the reaction mass is filtered and the solvent is removed from the reaction mass. The resulting reaction product is then distilled yielding the following fractions:

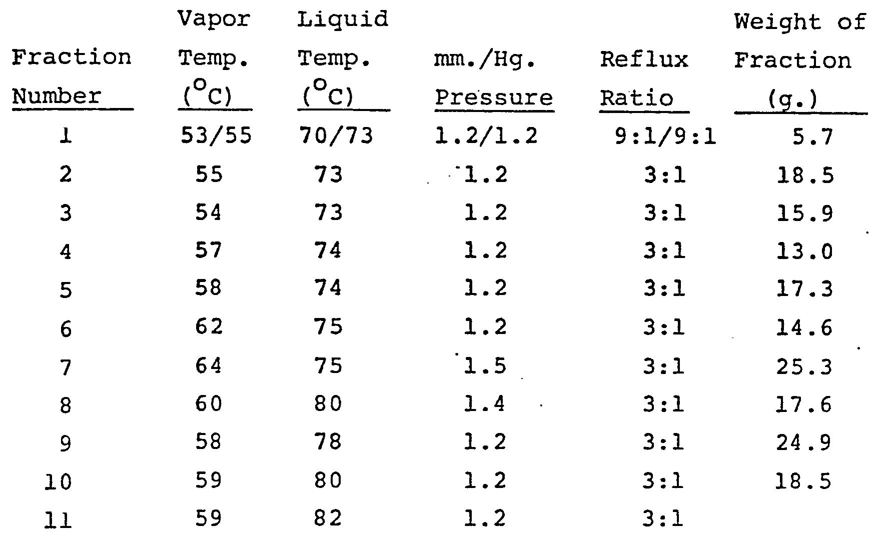

Fractions 3-8 are bulked and MNR, IR and mass spectral analysis yield the information that bulked fractions 3-8 consist of the compounds having the structures: 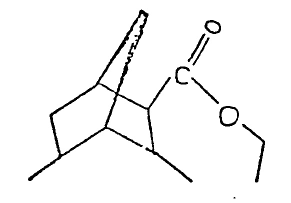 and

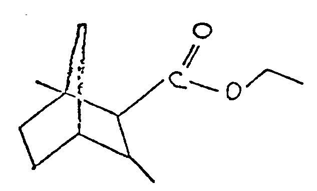

Fraction 1 of the foregoing distillation contains the pure compound having the structure:

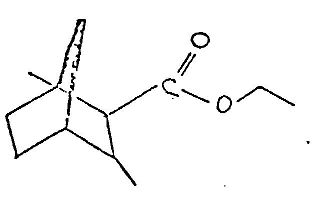

Peak 2 of the foregoing GLC analysis consists of the compound having the structure:

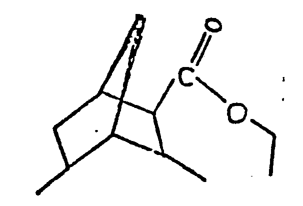

Figure 31-A is the NMR spectrum for the compound having the structure: 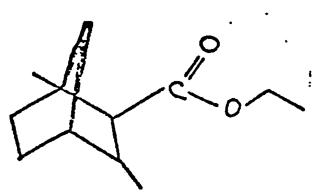 of fraction 1 of the foregoing distillation.

Figure 31-B is the NMR spectrum for the compound having the structure: 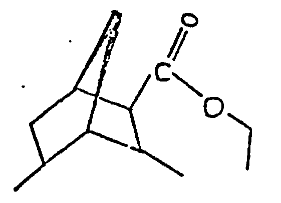 of peak 2 of the foregoing GLC profile.

Figure 32-A is the infra-red spectrum for fraction 1 of the foregoing distillation consisting of the compound having the structure:

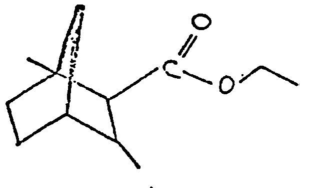

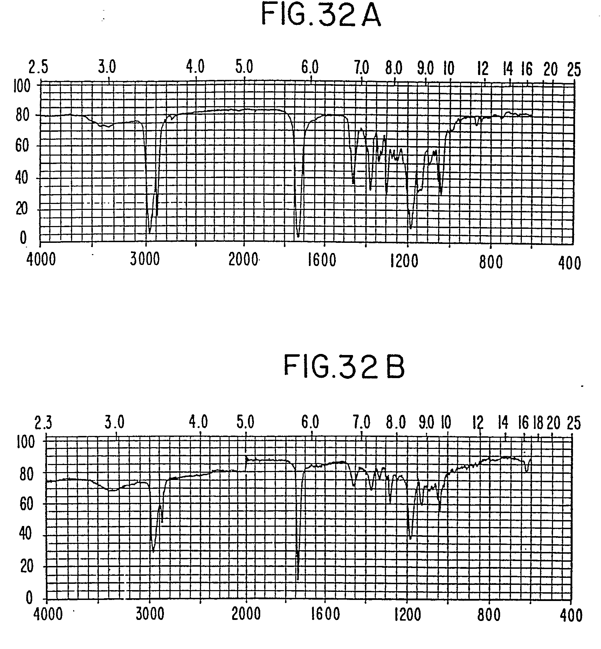

Figure 32-B is the infra-red spectrum for peak 2 of the foregoing GLC profile consisting of the compound having the structure:

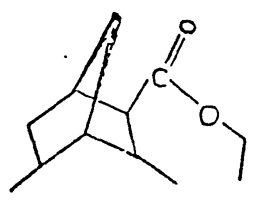

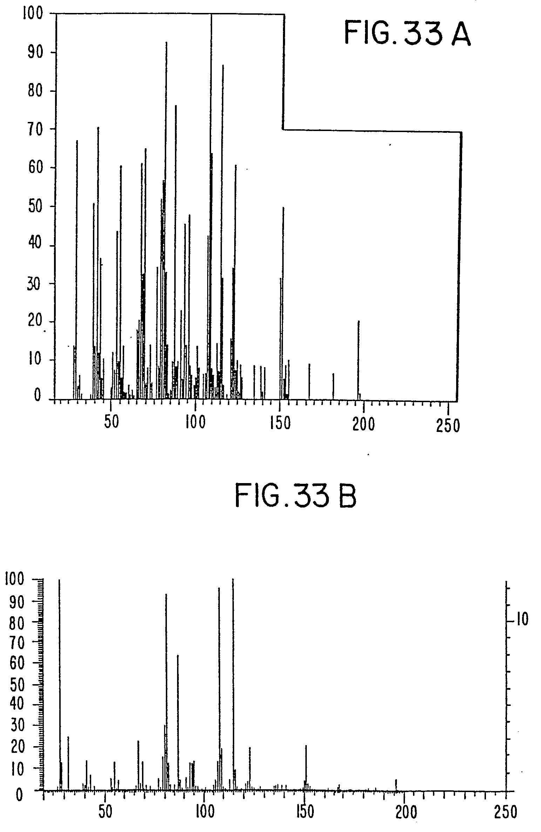

Figure 33-A is the mass spectrum for fraction 1 of the foregoing distillation consisting of the compound having the structure:

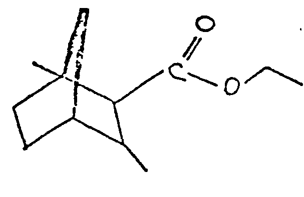

Figure 33-B is the mass spectrum for peak 2 of the foregoing GLC profile consisting of the compound having the structure:

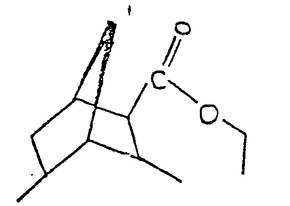

### Example VIII

The following spicy, floral and herbal type formulae are prepared:

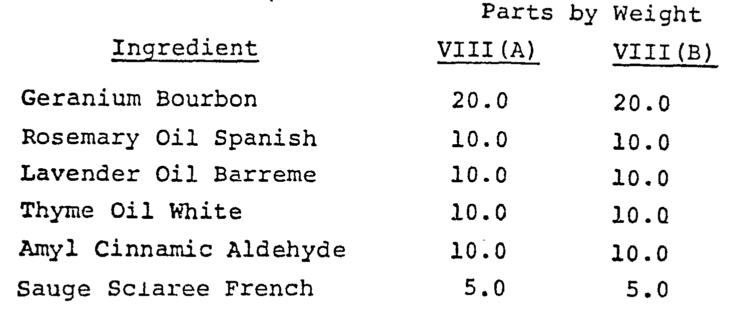

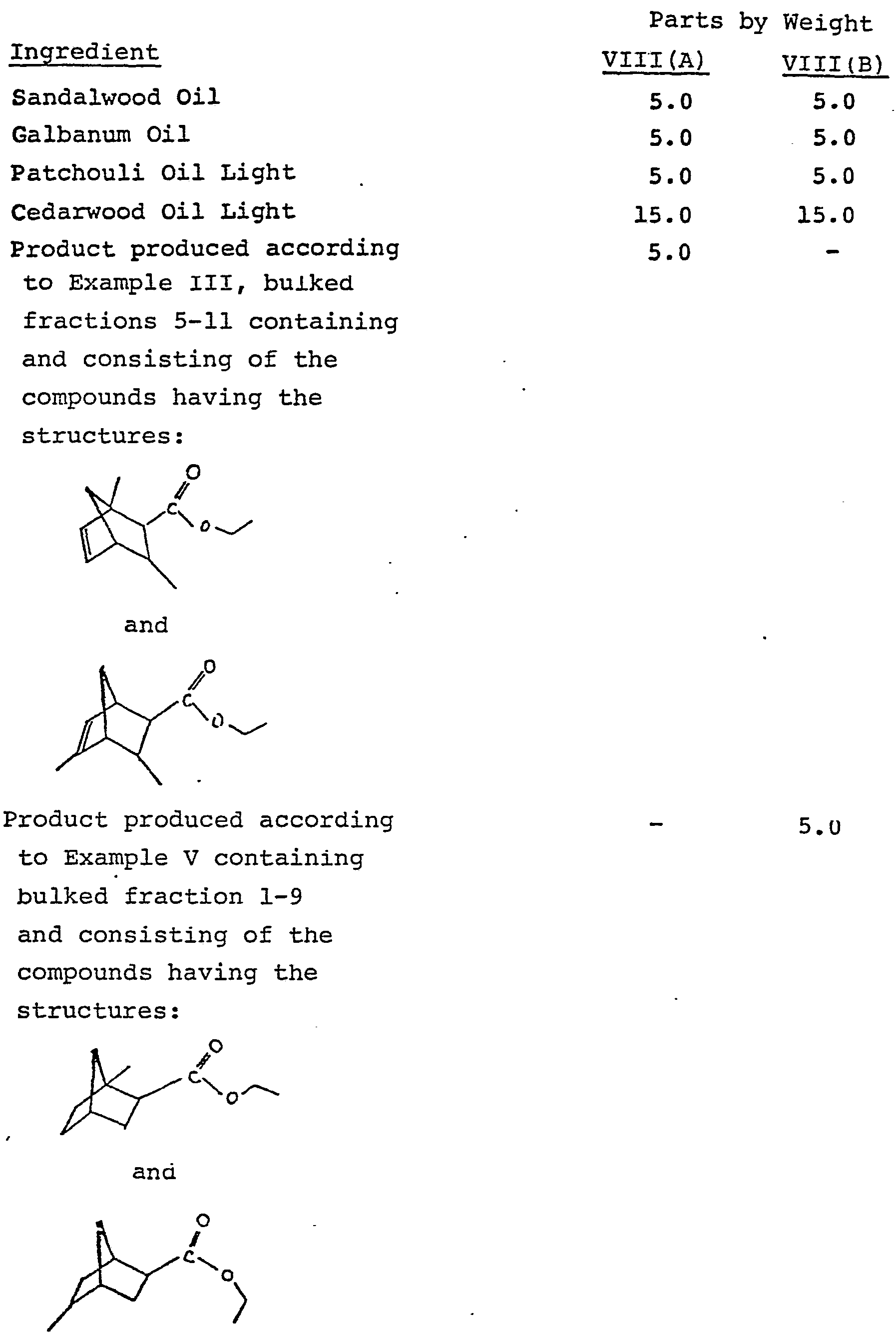

When the compositions of matter prepared according to Example III or Example V are inccrporated into the formulae at 5.0%, both compositions of matter add a pleasant fruity, tagette character to these spicy, herbal, floral type perfume formulations.

### Example IX

A jasmine formulae is produced containing the following ingredients:

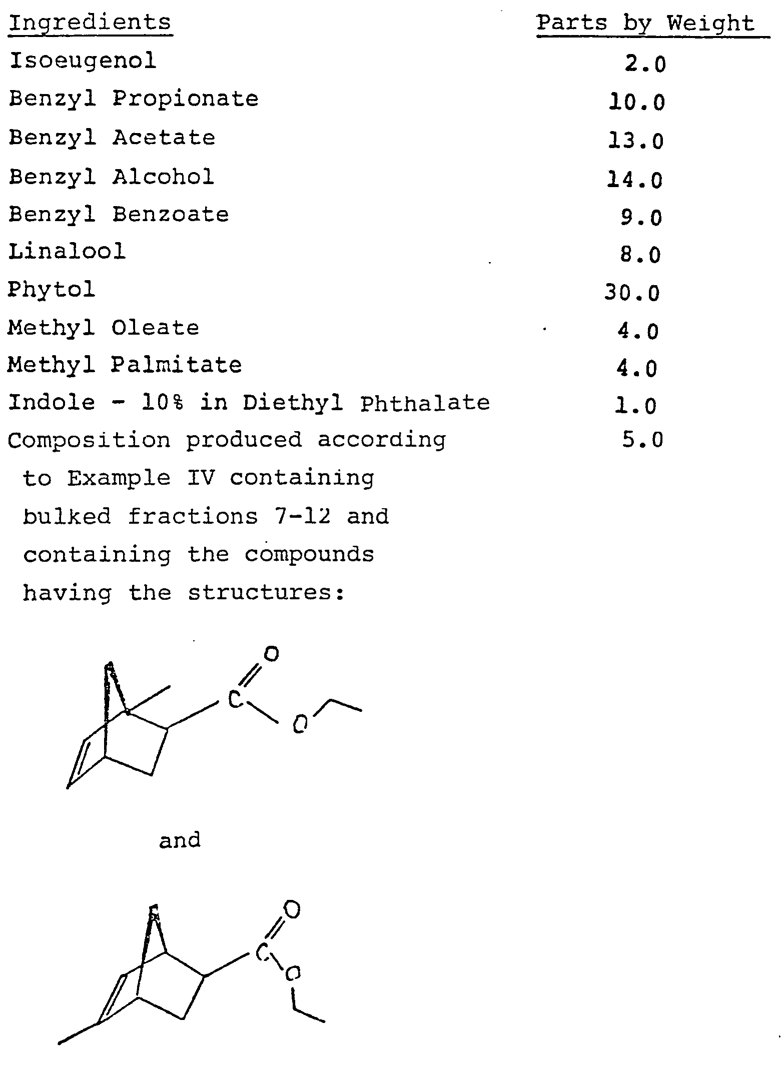

The material produced according to Example IV incorporated into the formula above at 5.0% gives rise to the pleasant fruityness aroma undertone of this jasmine formulation.

### Example X

The following sweet, floral formula is produced:

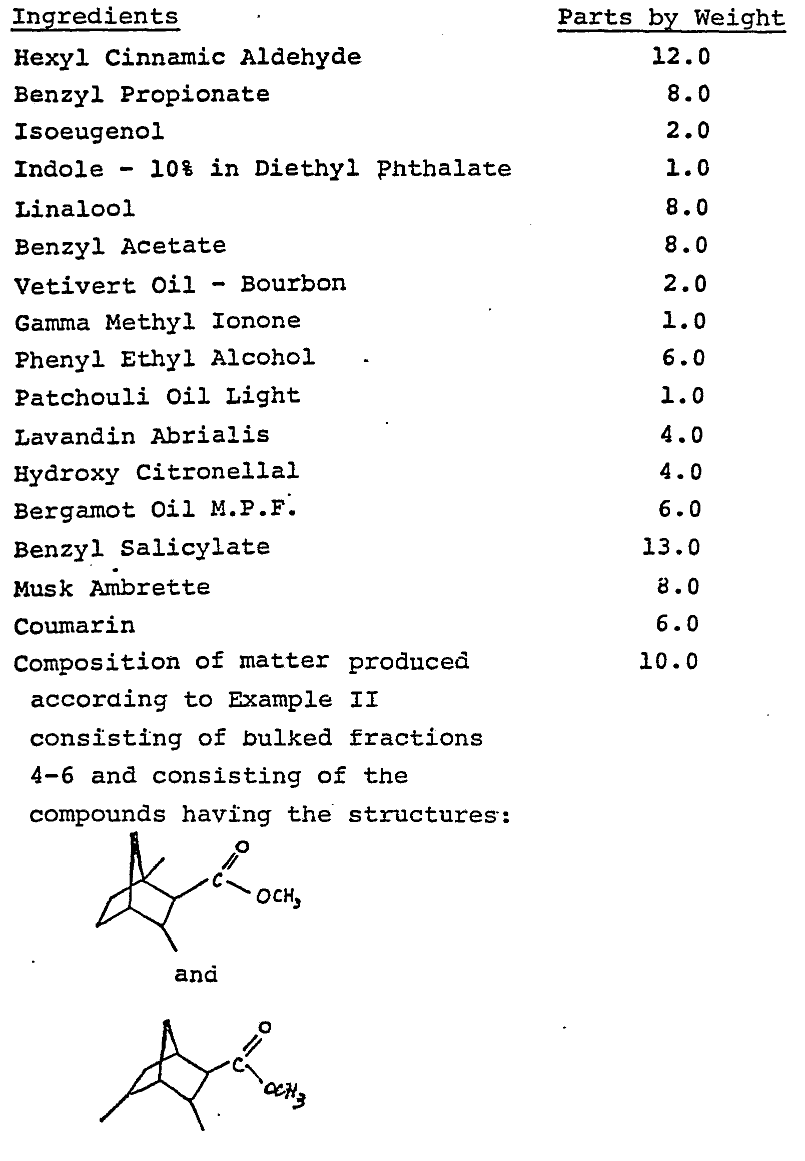

At the ₁0% level in the foregoing sweet, floral formulation the compounds having the structures: 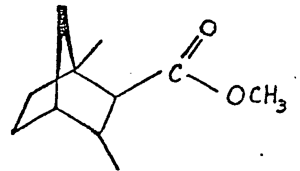 and 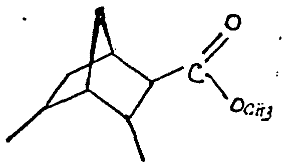 produced according to Example II contribute an intense and pleasant fruity, floralcy to this sweet, floral fragrance.

### Example XI

A stable lotion is prepared with the following formulation:

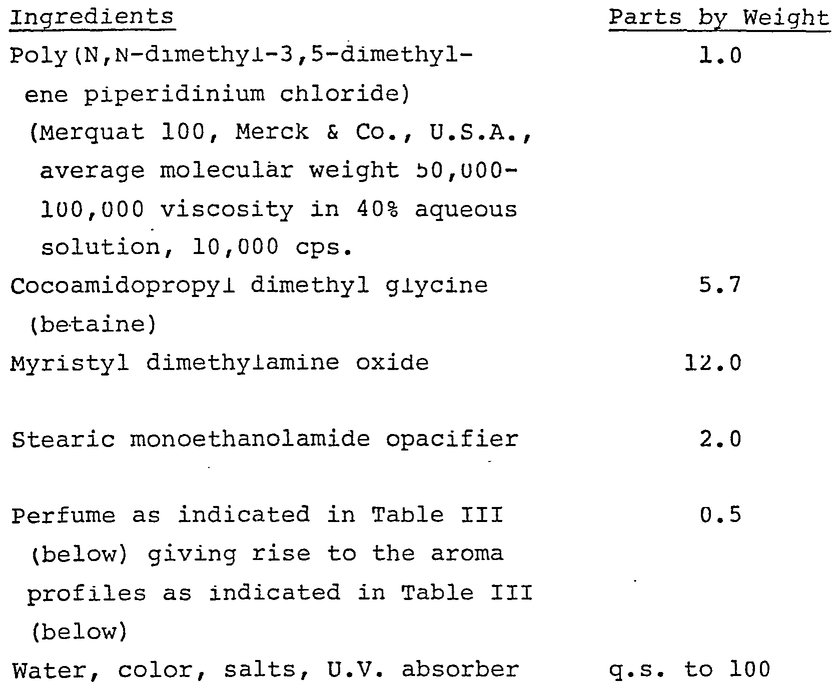

Two or three bottle capfuls of the above lotion held under the tap releasing the water into the bathtub yields a copiously foamed bubble bath with no visible precipitation flocculation, or "bathtub ring" even using hard water. Bathing in this bath is found to have a cleansing and pleasing emollient effect on the skin as described above.

When, after immersion in this bath, the body is soaped, rinsed and dried, an even better, more long-lasting emollient, moisturizing effect on the skin is observed. The foam or bubbles are substantially unaffected by the soaping step, and no precipitate, flocculate or "bathtub ring" or any bothersome film or coating on the bathtub surface is found.

The aroma produced is as set forth in Table III below:

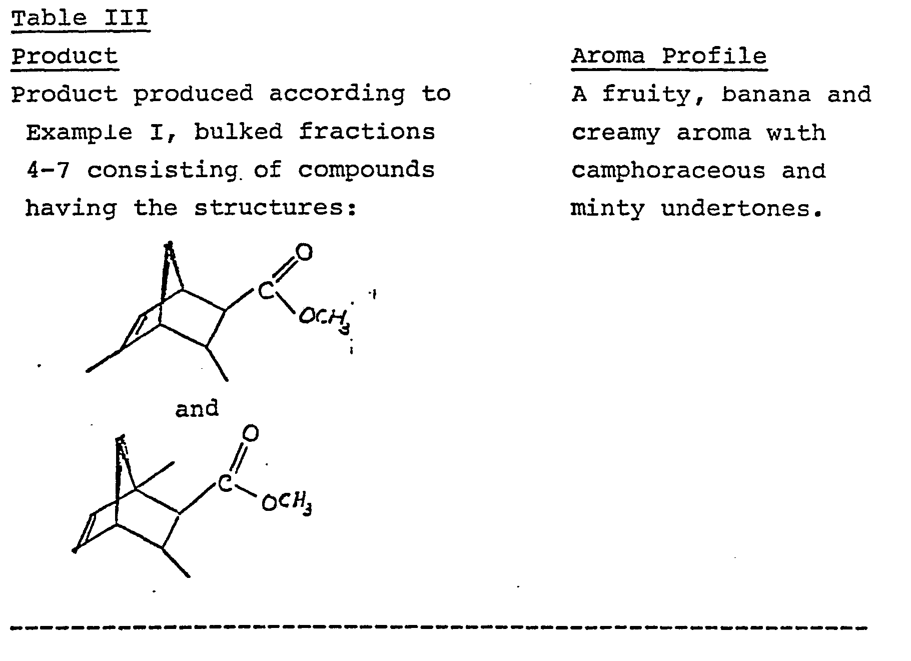

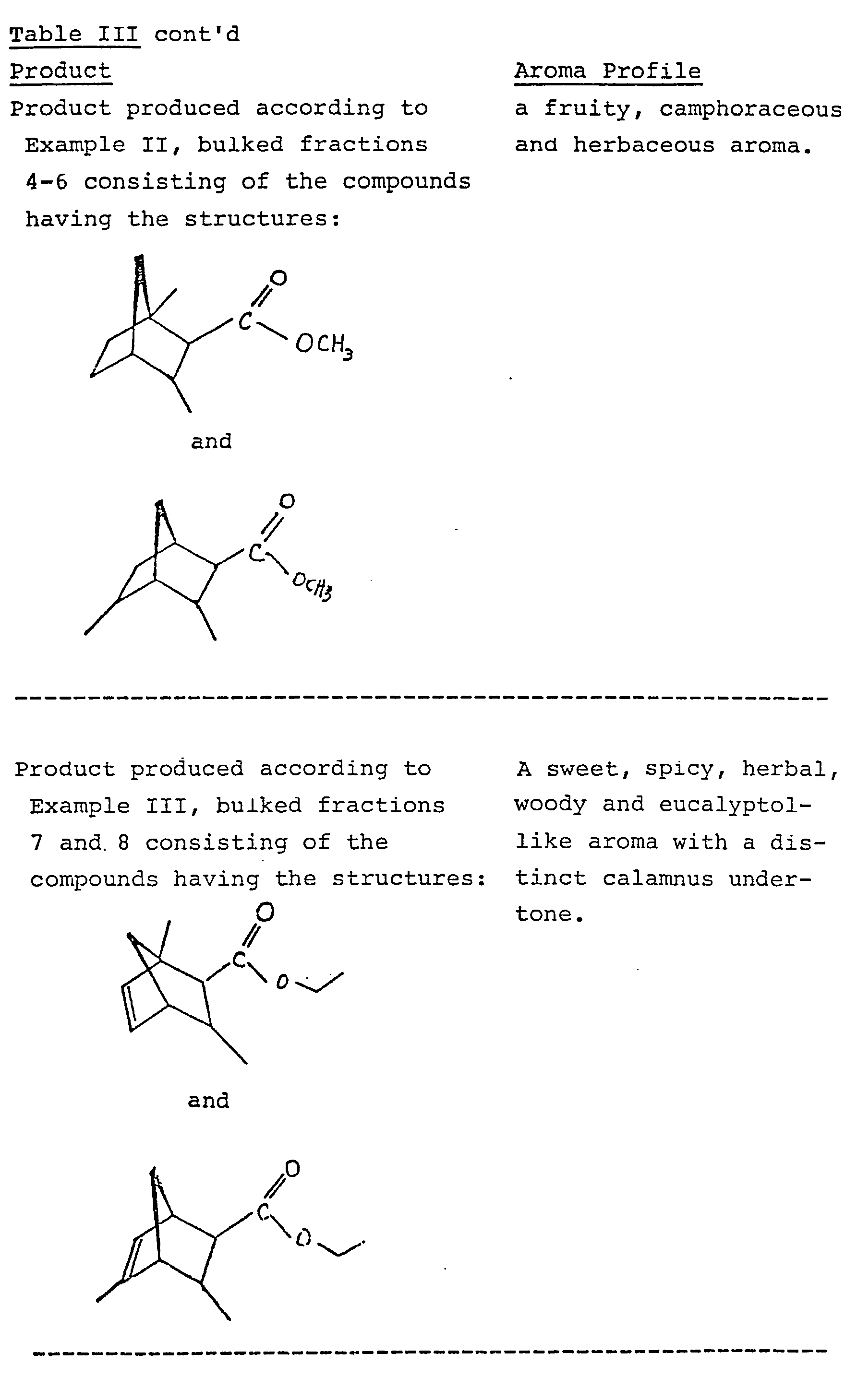

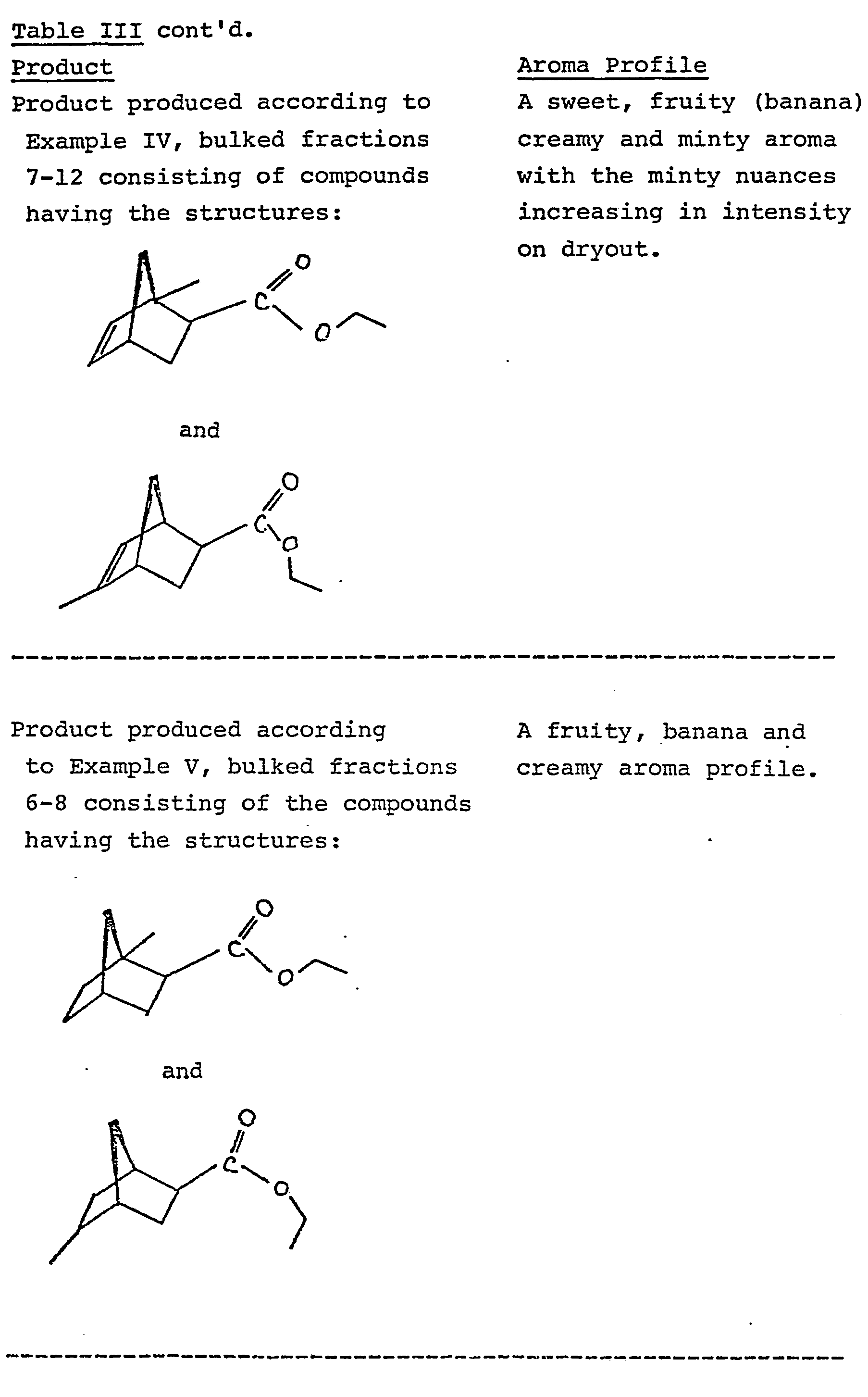

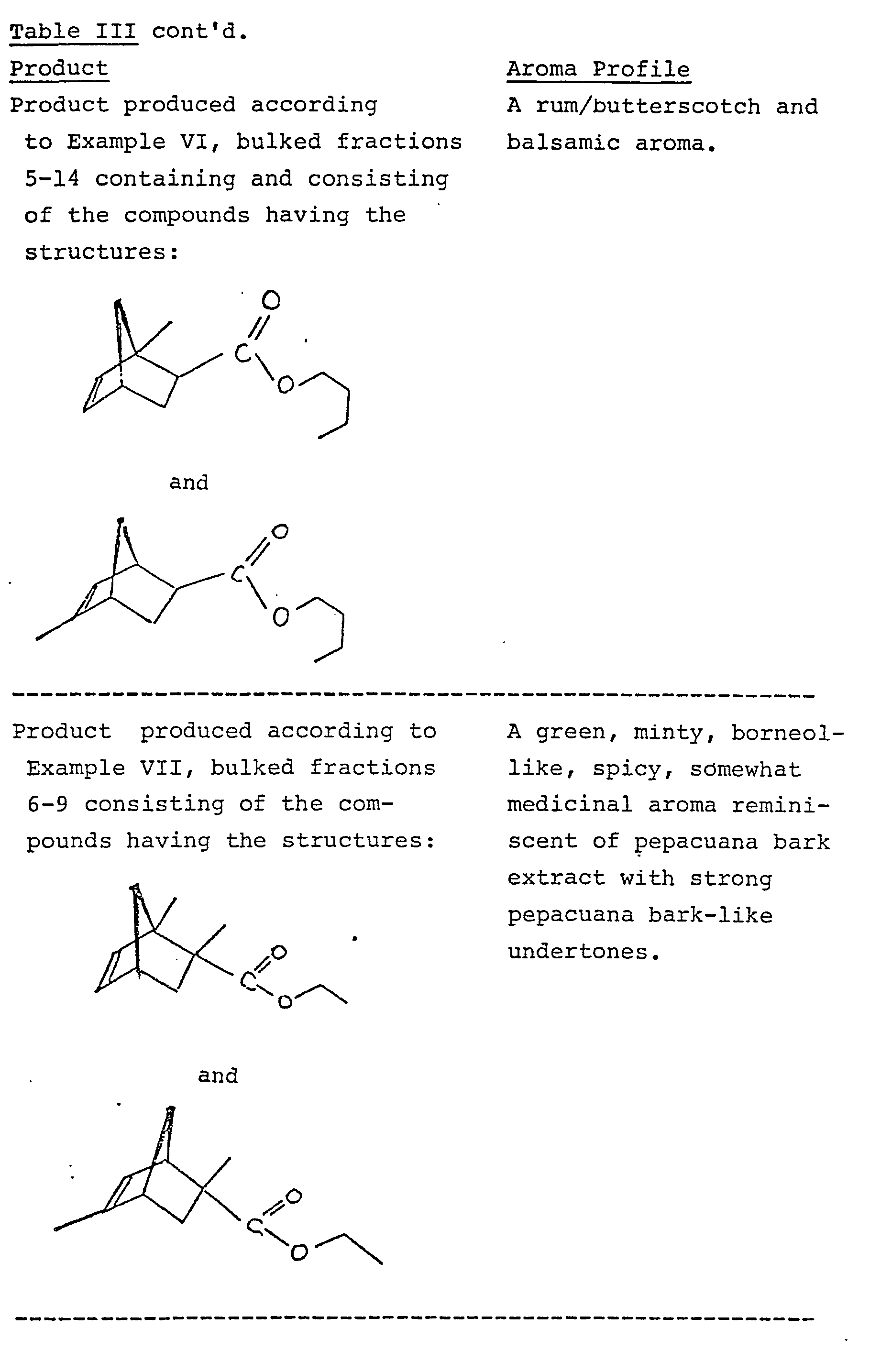

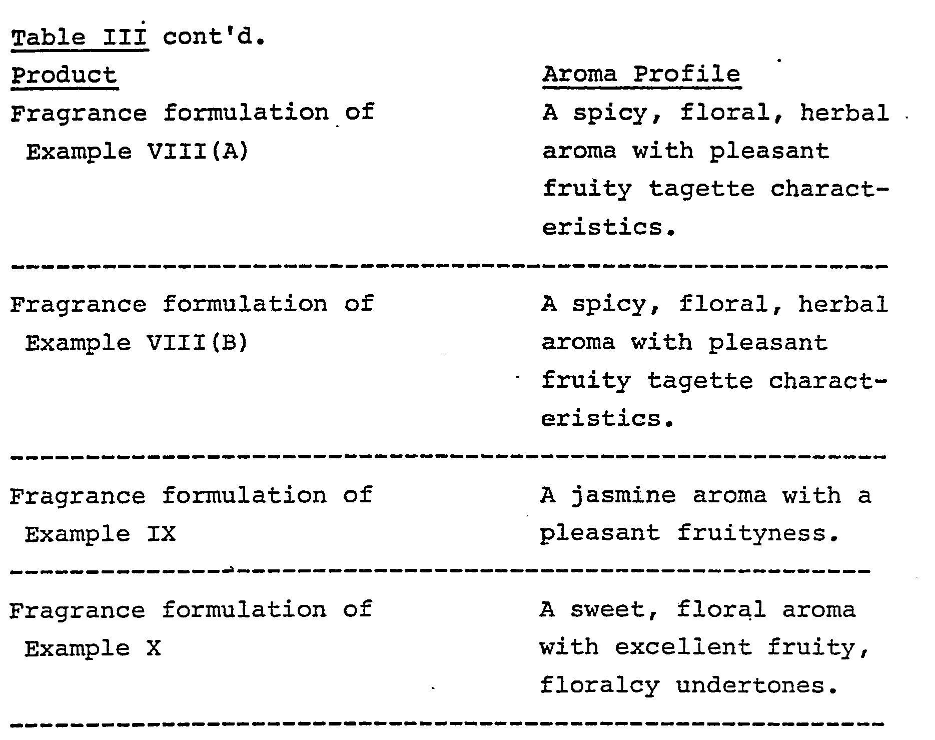

### Example XII

The following formulation is prepared with results in properties and use similar to those described in Example XI.

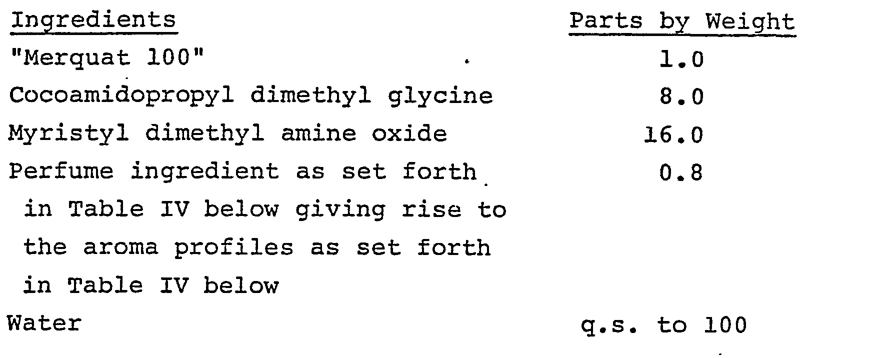

The composition is a clear liquid. Its viscosity may be increased by addition of a lauric or myristic diethanolamine or a soluble polyethylene glycol ester such as polyethylene glycol 6000. In addition, this composition may be rendered opaque by addition of stearic monoethanolamide stearate, polyethylene glcyol 600 monostearate or a glyco amido stearate such as "Cerasynt 1P".

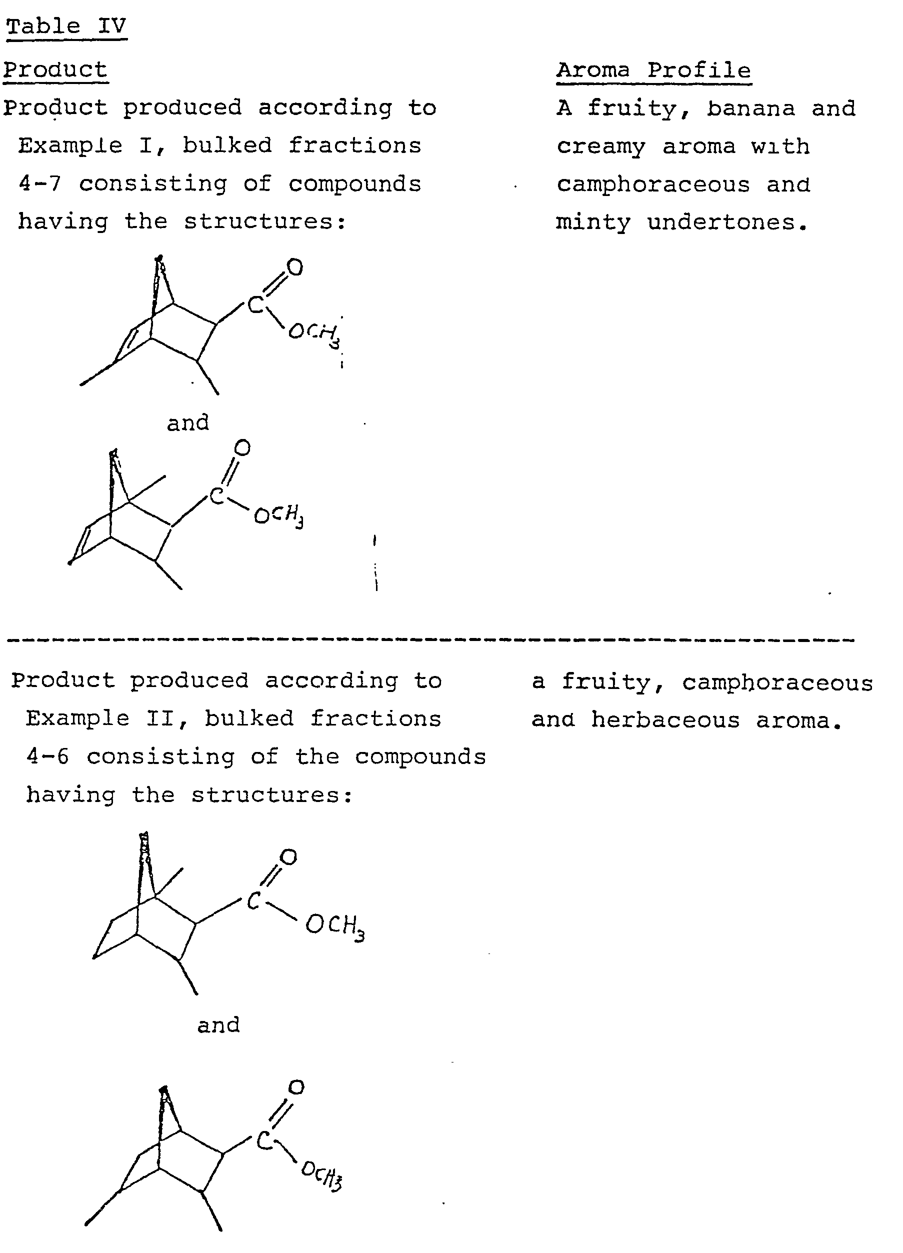

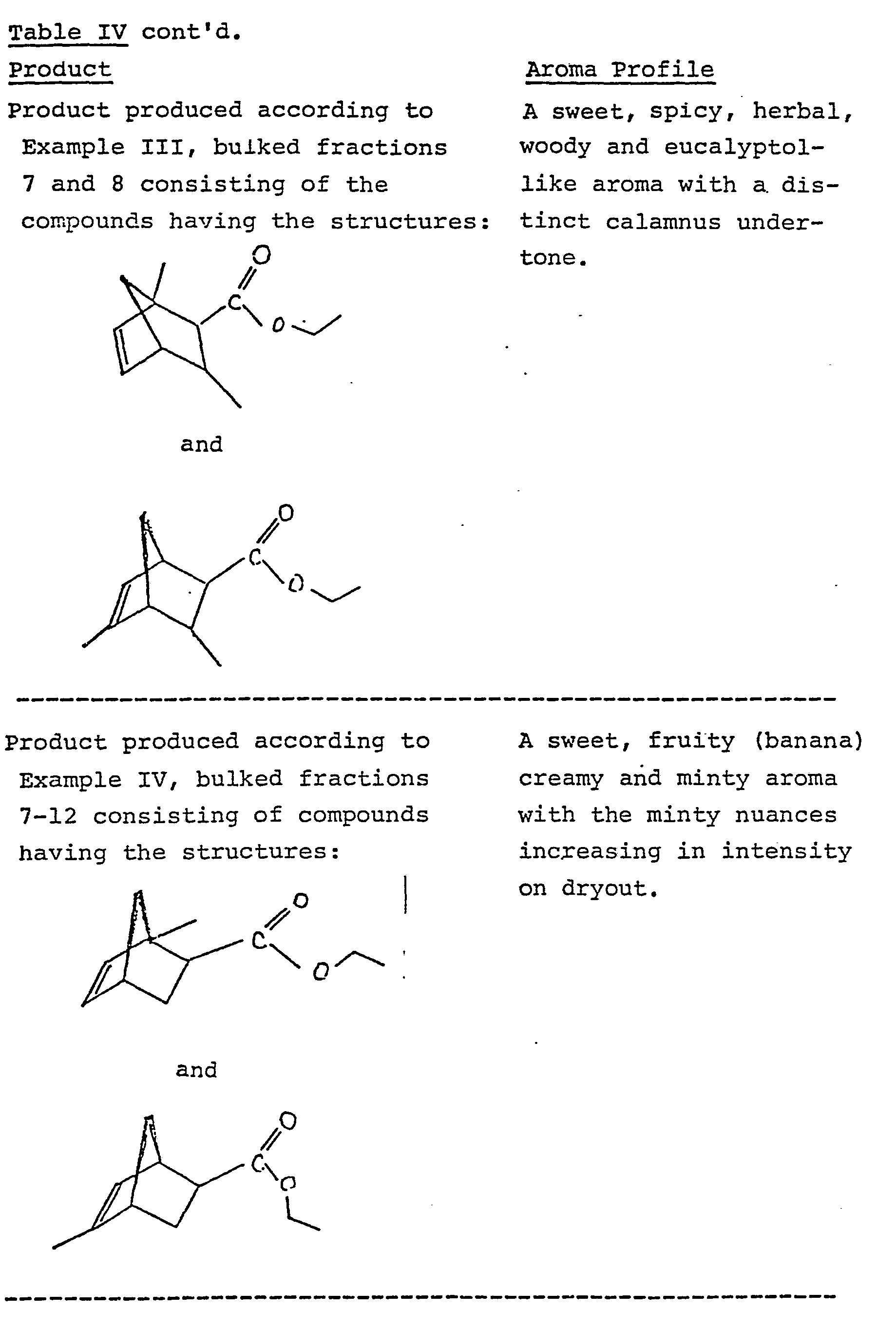

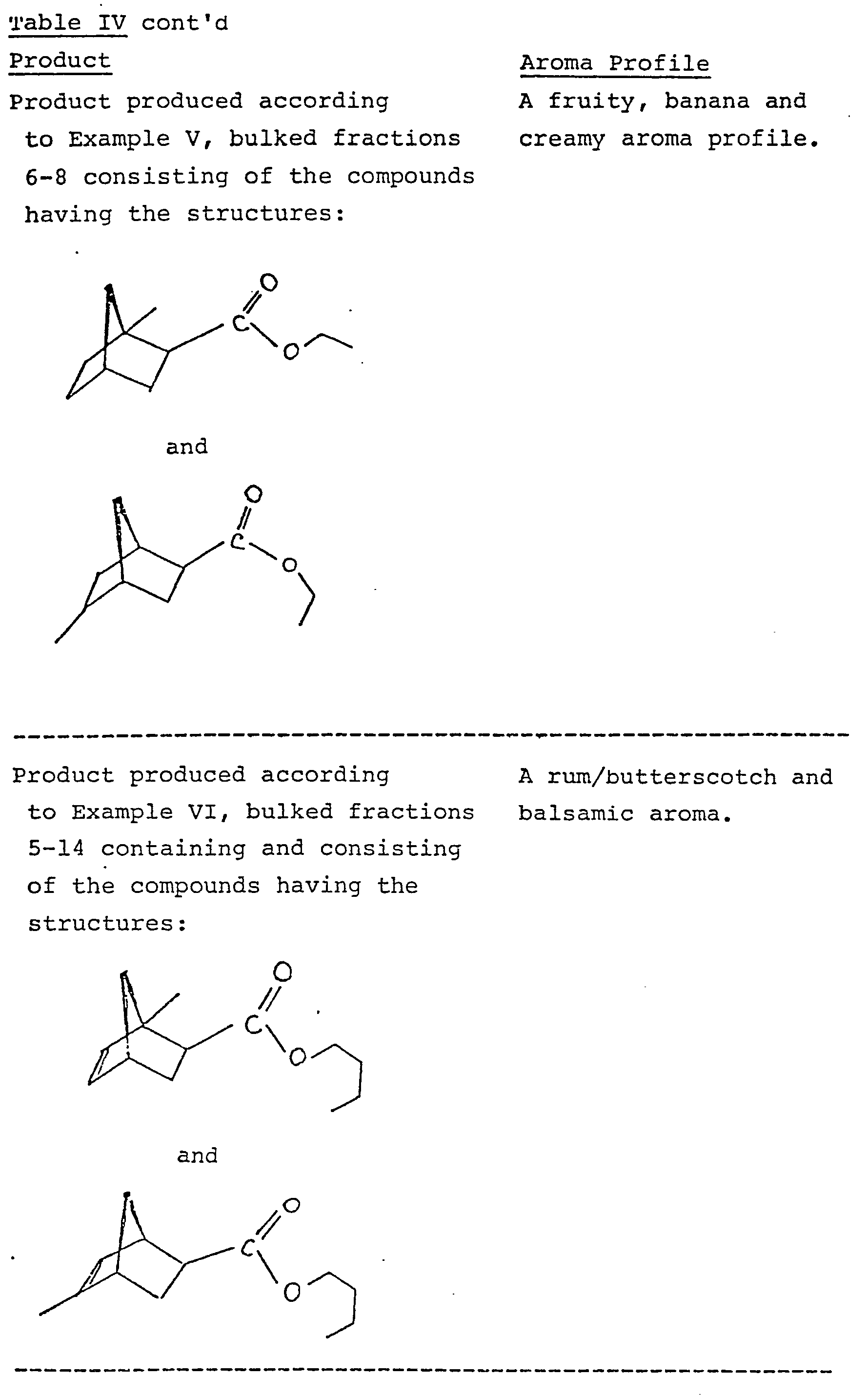

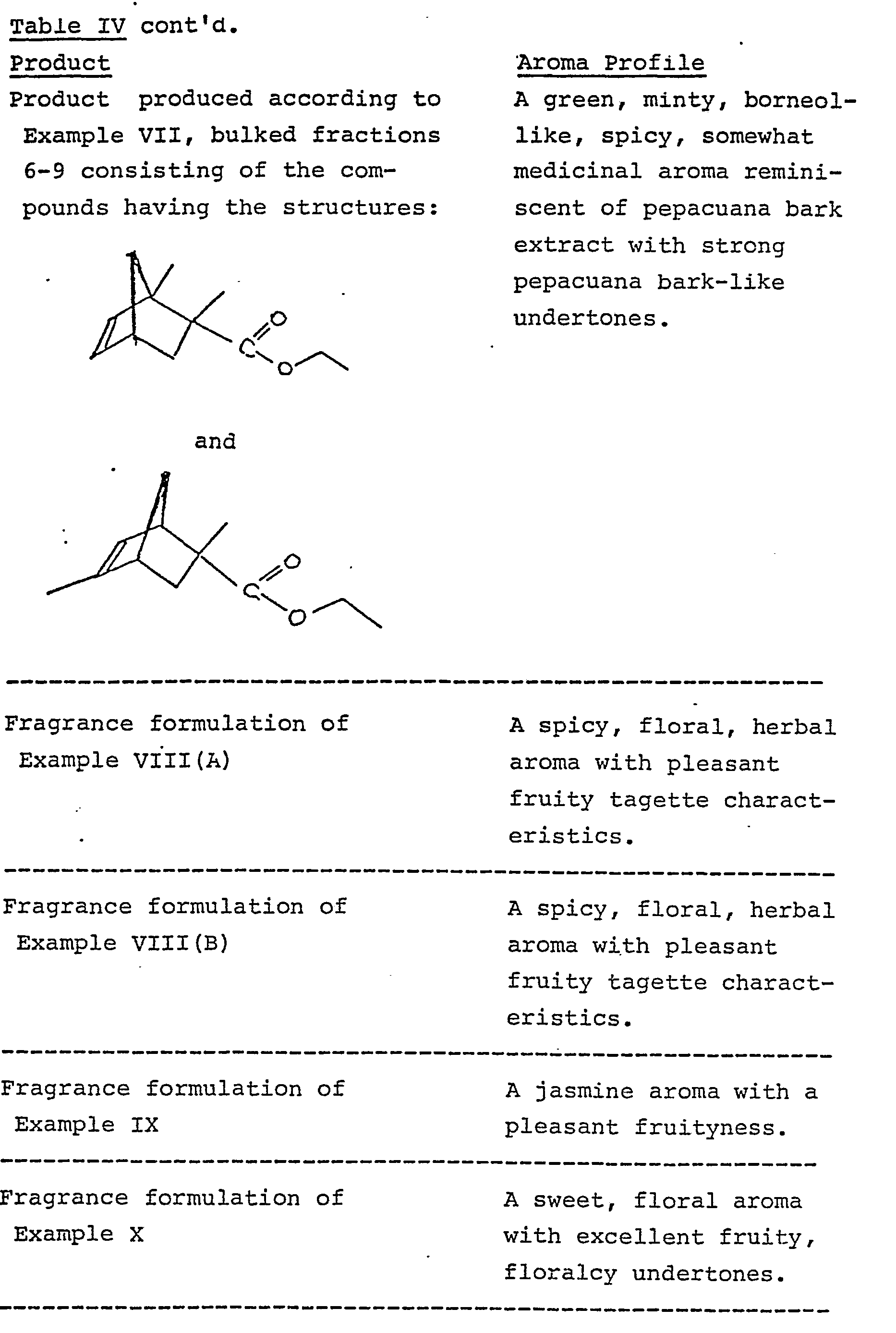

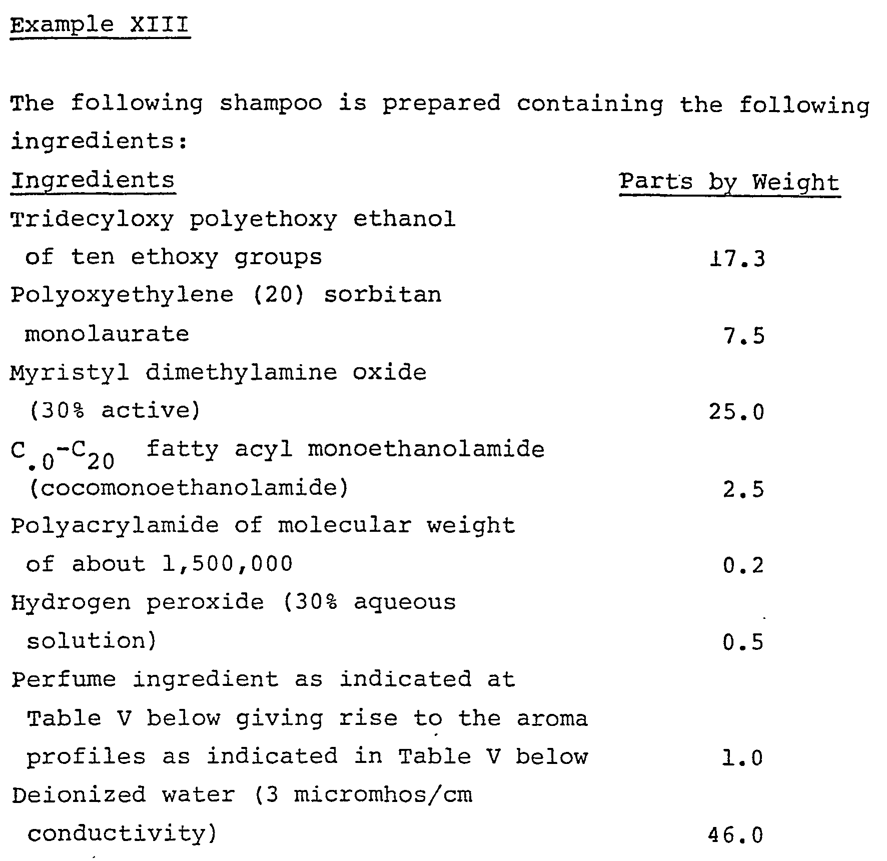

A shampoo of the above composition is made in the following manner. First, the tridecyloxy polyethoxy ethanol is added to a clean mixing tank, with the agitator on, and the amine oxide, polyoxyethylene sorbitan monolaurate and cocomonoethanolamide are added sequentially, with continued agitation. The mix is then heated to 68°C, until the cocomonoethanolamide is melted and/or dissolved. The hydrogen peroxide solution is then admixed with the mentioned nonionics and mixing is continued for about half an hour, in which the peroxide destroys any free amines or other harmful impurities that may be present. The mix is then cooled to 38°C.

In a separate mixer the polyacrylamide is gradually added to the formula weight of deionized water, with the mixer on. Addition is effected carefully and slowly (the polyacrylamide is sprinkled in) to avoid the production of "fish eyes" in the mix. After dissolving of the polyacrylamide, the solution thereof is added to the first mixing tank with agitation and is blended with the nonionics, such mixings being at room temperature. Subsequently, the perfume as indicated in Table V below giving rise to the aroma profile as set forth in Table V below is admixed with the balance of the composition and mixing is continued for another half hour.

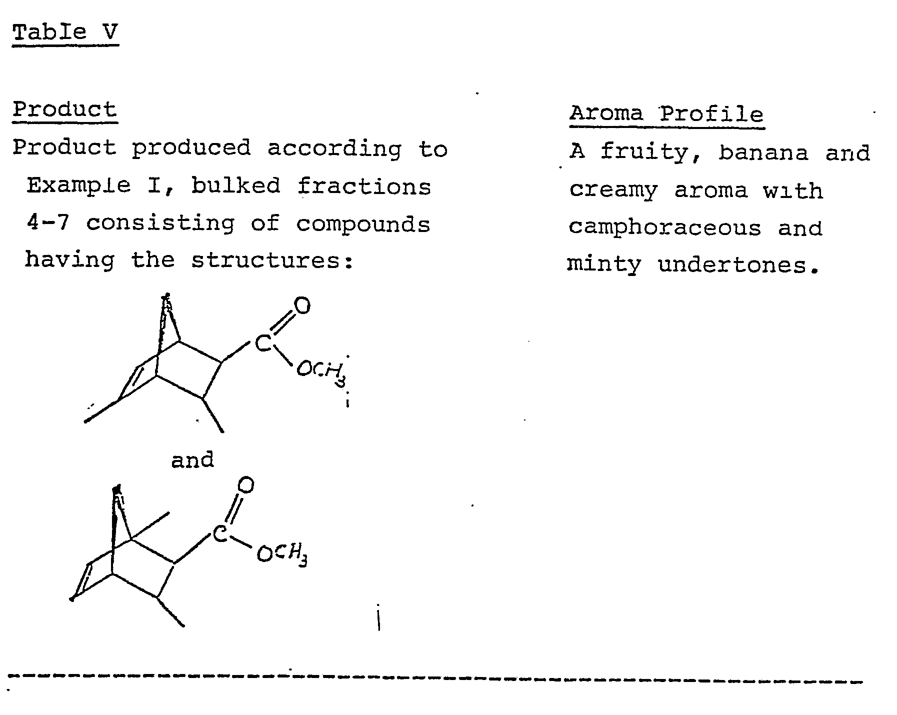

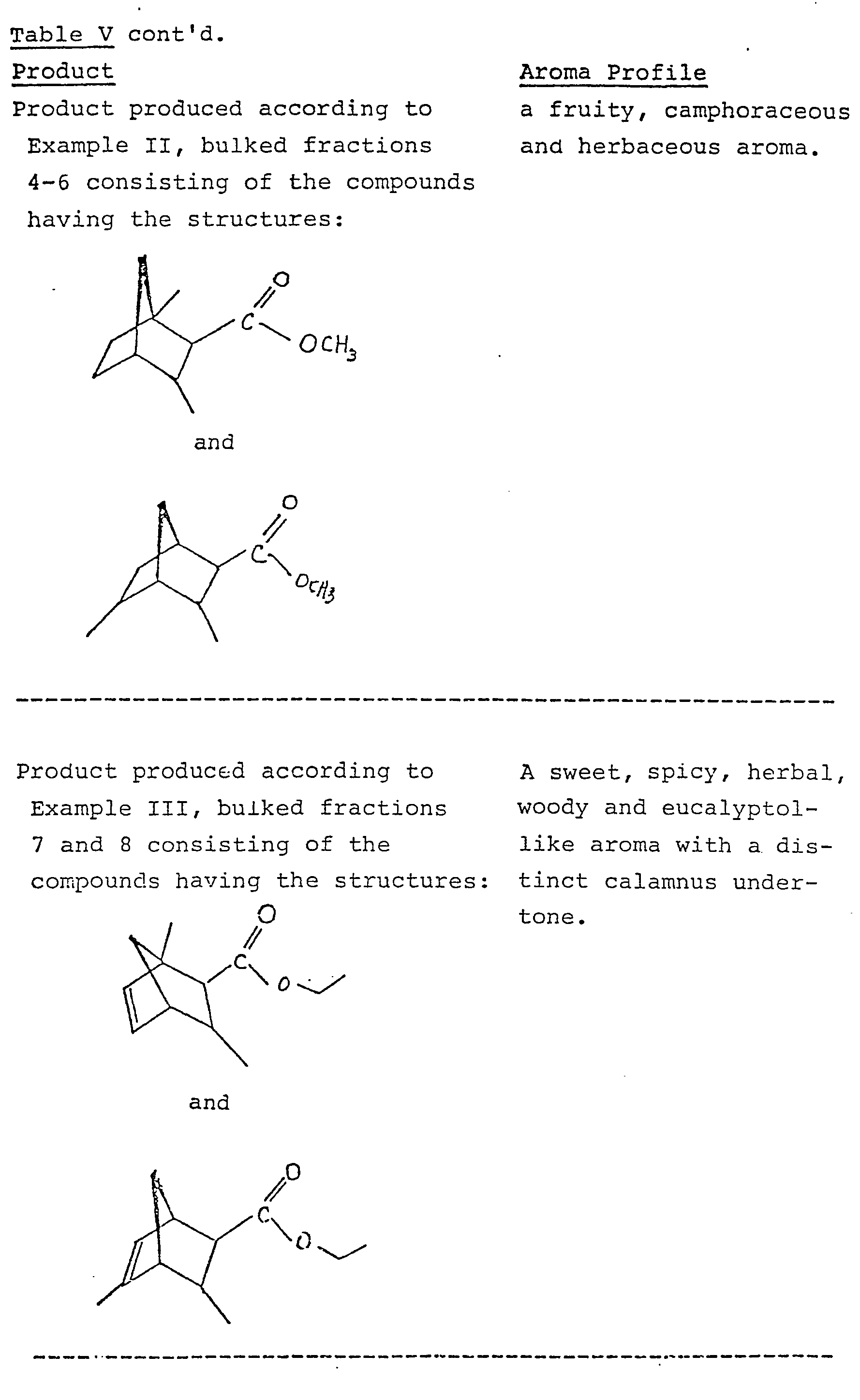

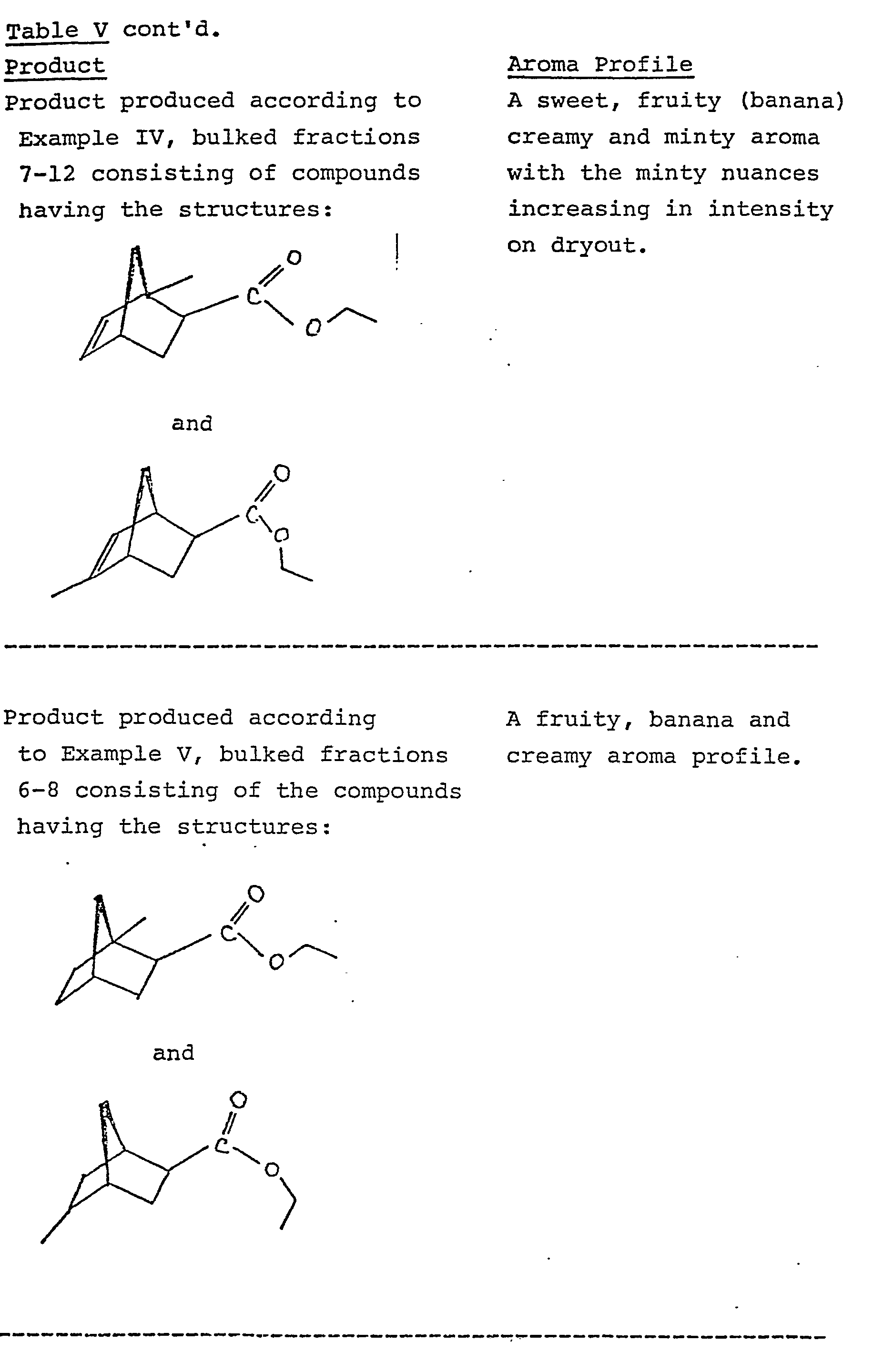

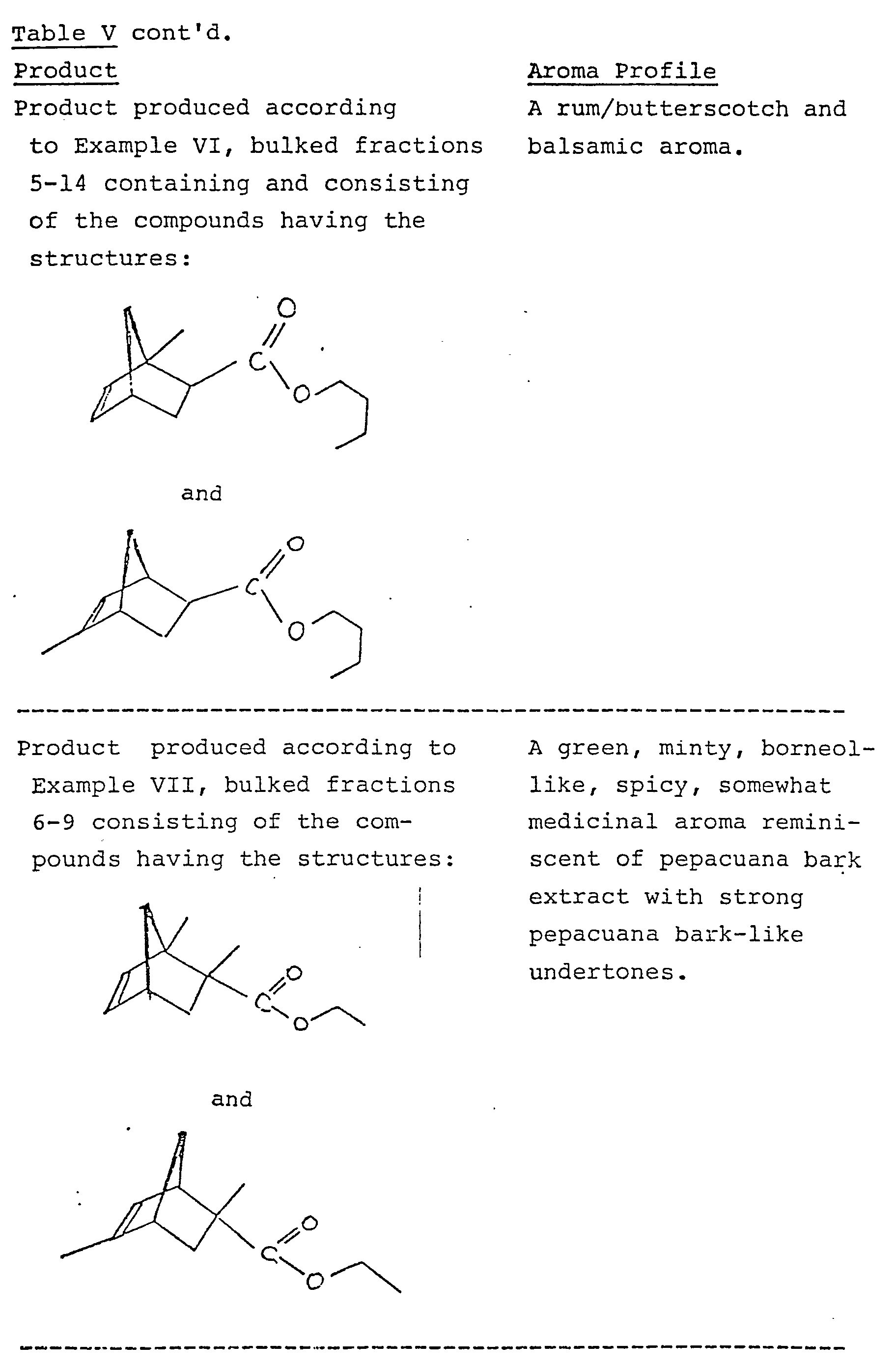

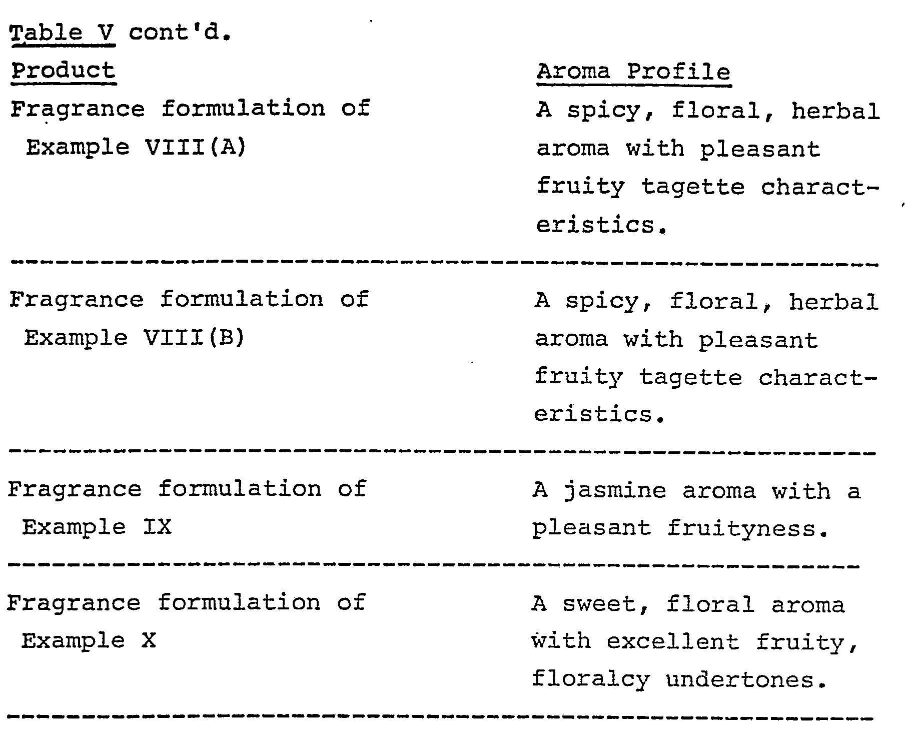

The product made is an excellent shampoo of satisfactory viscosity and aroma, foaming power, foam stability, low conductivity and good shampooing effects. The viscosity is about 1,000 centipoises at 20°C and the conductivity, using the Hach Conductivity Meter, is 750 micromhos/cm. The foaming power is 250 ml and the foam stability is 22 seconds, by the test method previously described. In com-parison, a commercial shampoo based on triethanolamine lauryl sulphate detergent has a conductivity of about 22,000 micromhos/cm, a viscosity of about 1,500 centipoises, a foaming power of about 380 ml and a foam stability of 60 seconds.

In panel evaluations of the experimental shampoo compared to a different commercial product, in actual shampooing, the experimental formula was adjudged significantly better in being less drying, producing a softer feel for the wet hair, leaving the wet hair easier to comb, being less prone to accumulate static charges (less flyaway) and having a foam of better appearance and feel. Additionally, the experimental product was judged better in almost all hair effect properties, with the control only being about equal to it in curl retention. In properties other than those mentioned, the experimental product was better in rinsability, the control was better in foam build-up rate and the foams were about equal in volume and stability.

In the shampooing described herein and in subsequent examples the human hair is washed on the head by wetting the hair with warm tap water at about 42°C, applying 15 grams of shampoo to the hair, lathering it into the hair for a minute, rinsing with warm tap water for 30 seconds, re-lathering with 7 grams of shampoo for a minute and rinsing off for 30 seconds, after which the hair is towel dried and dried further with an automatic hair dryer.

### Example XIV

### Fabric freshener composition

A fabric freshener composition is prepared as follows:

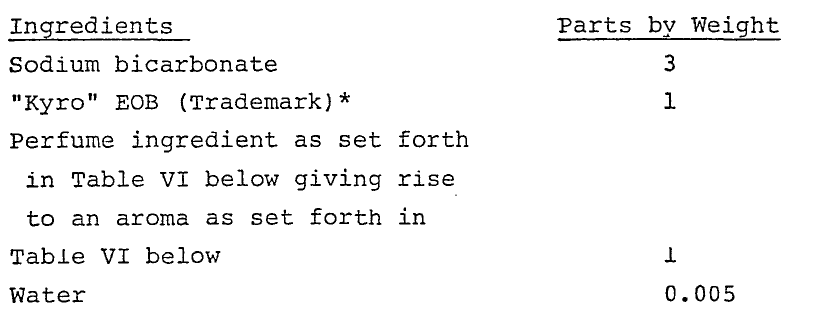

The composition of this example is prepared by simply mixing the ingredients.

The above described composition is applied to a lightly soiled and wrinkled fabric as droplets (ca. 5.0 mm avg. size) using a trigger action sprayer having a nozzle which is adjustable to provide composition droplets in the desired range. The composition is applied at a rate of about 1 gram of composition to about 10 grams of fabric.

The fabric is then placed in an automatic dryer and dried, with tumbling action, at a temperature of 60°C-80°C for a period of 15 minutes. The fabric is rendered free of wrinkles and static, and has a fresh appearance and pleasant odor profile as set forth in Table VI below. Surprisingly, the sodium bicarbonate is not visible on the refreshed fabric.

In the foregoing procedure, substantially the same results were obtained when sodium carbonate is substituted for the sodium bicarbonate.

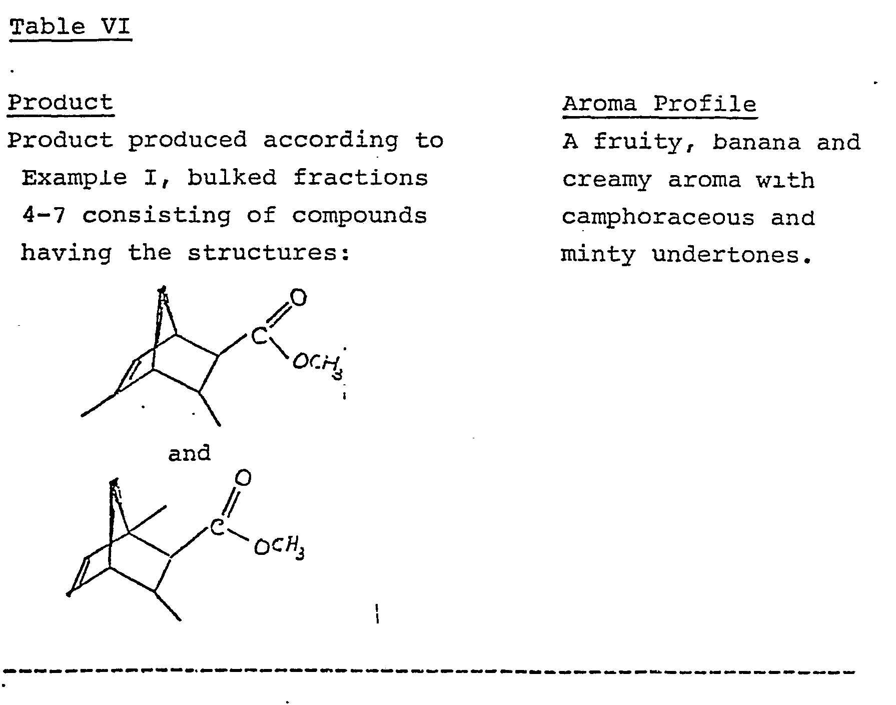

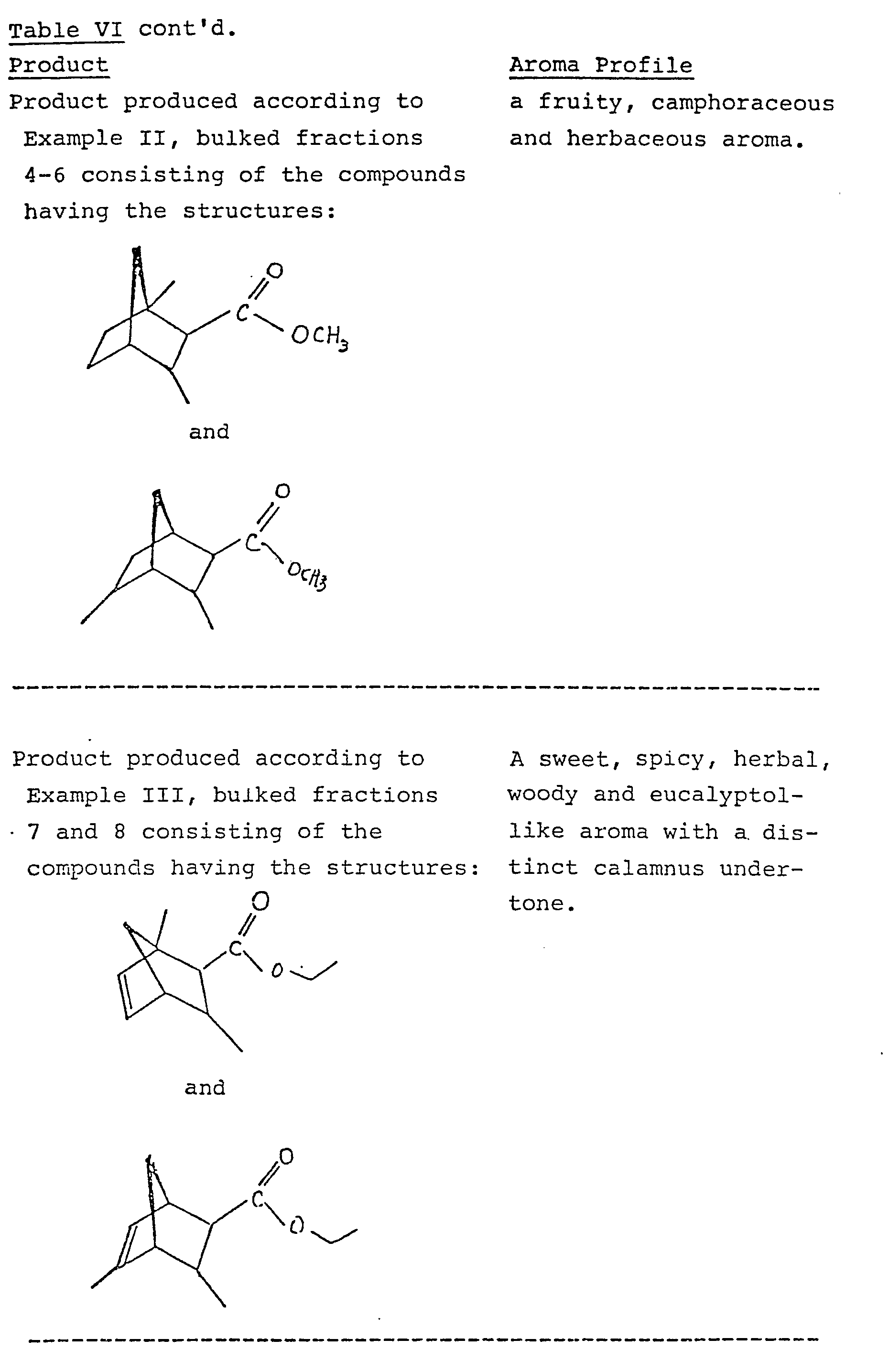

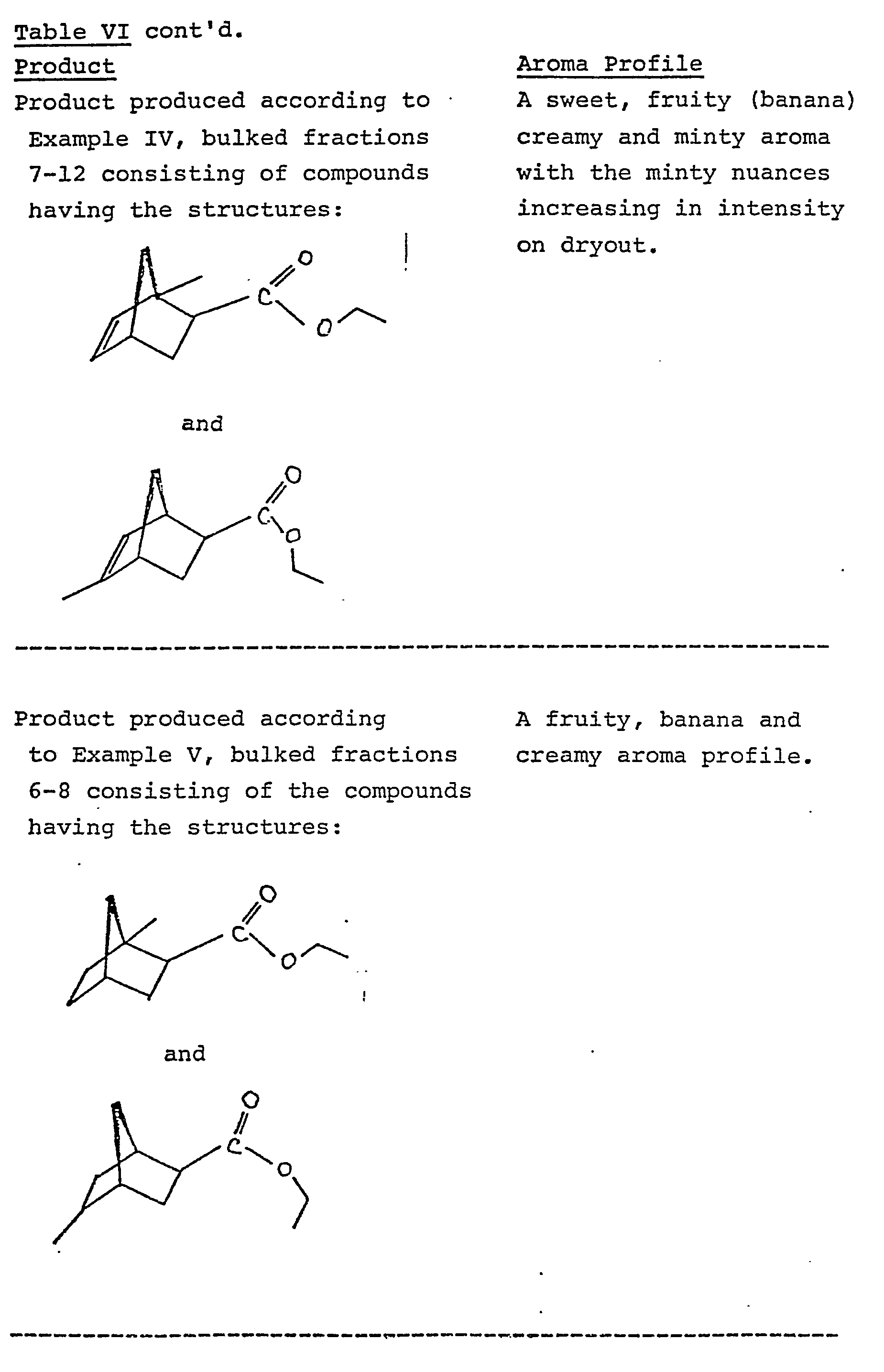

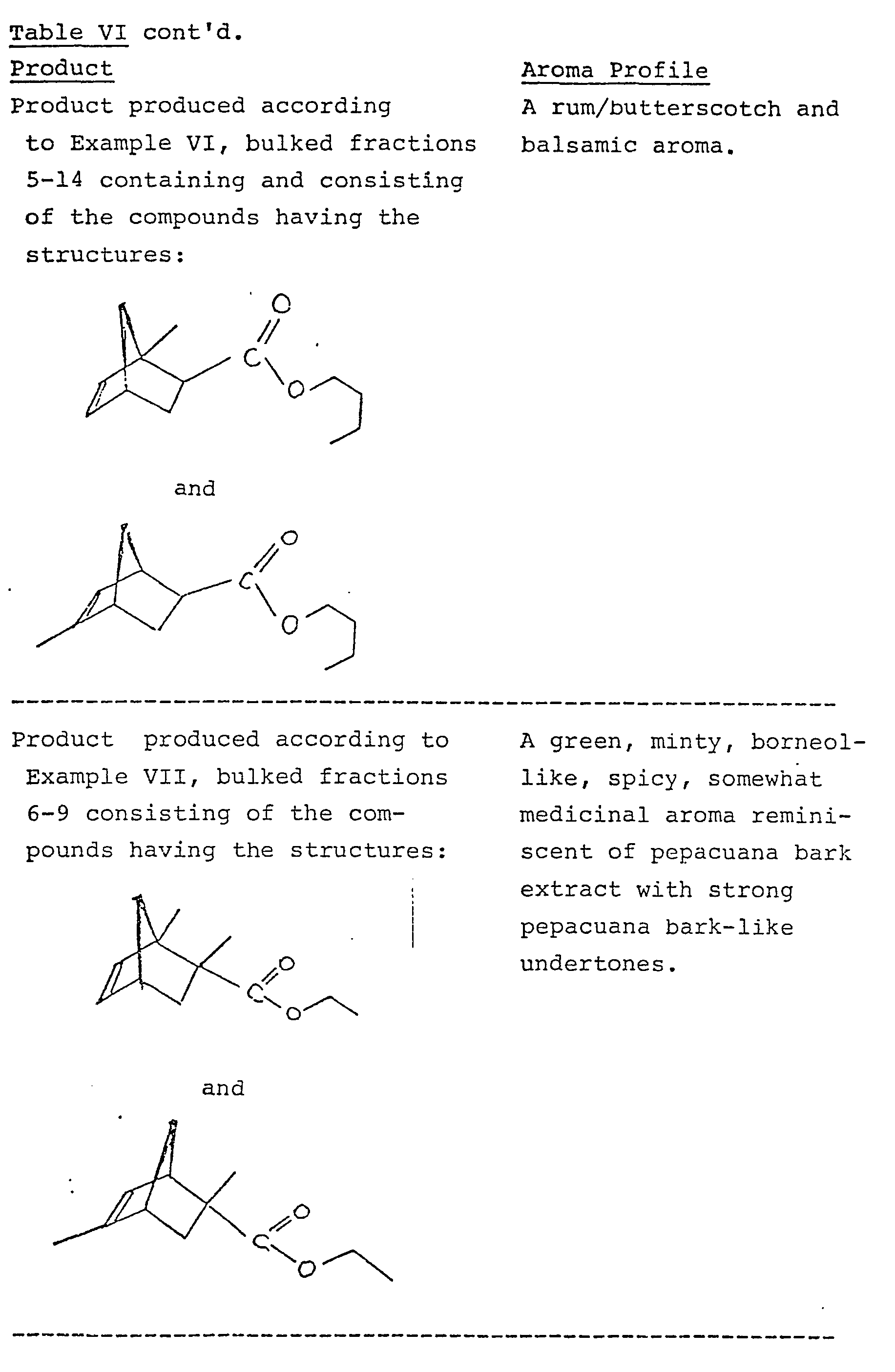

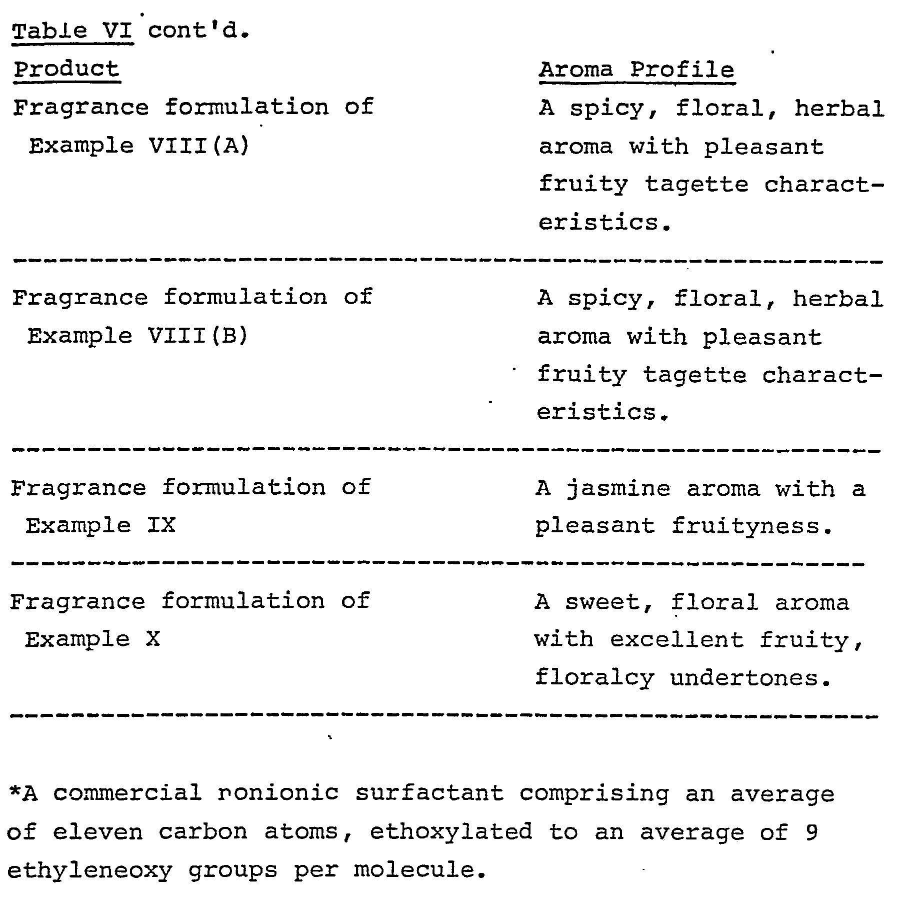

### Example XV

### Perfumed liquid detergent

Concentrated liquid detergents (lysine salts of n-dodecyl benzene sulfonic acid as more specifically described in _{U}.S. Patent No. 3,948,818, issued on April 6, 1976) with aromas as indicated in Table VII below are prepared containing 0.10%, 0.15%, 0.20%, 0.25%, 0.30%, 0.40%, 0.50% and 0.80% of the perfume ingredient as set forth in Table VII below. The detergents are prepared by adding and homogeneously mixing the appropriate quantity of perfume ingredient as set forth in Table VII below. The detergents all possess aromas as set forth in Table VII below with the intensity of each increasing with greater concentrations of the perfume ingredient as indicated in Table VII below.

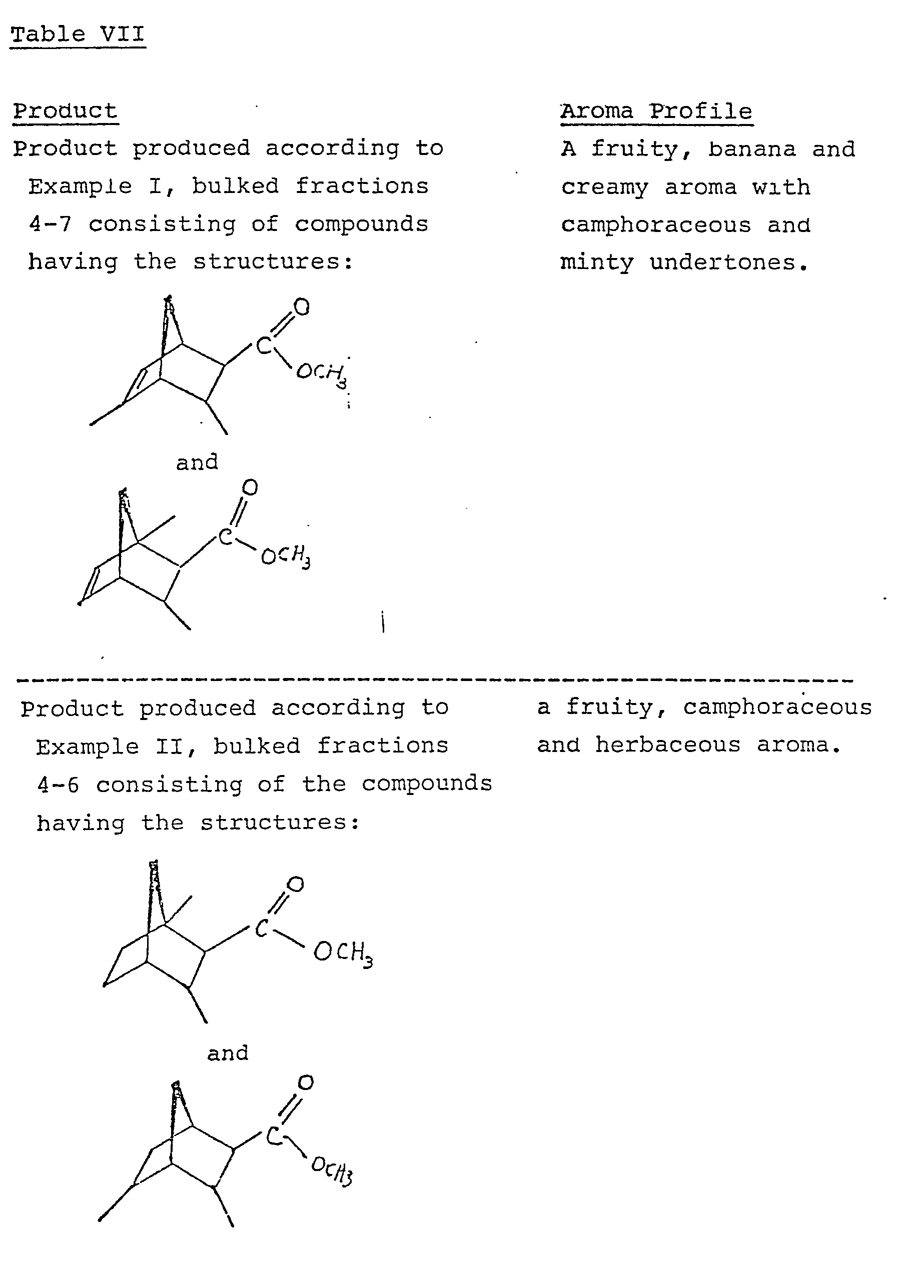

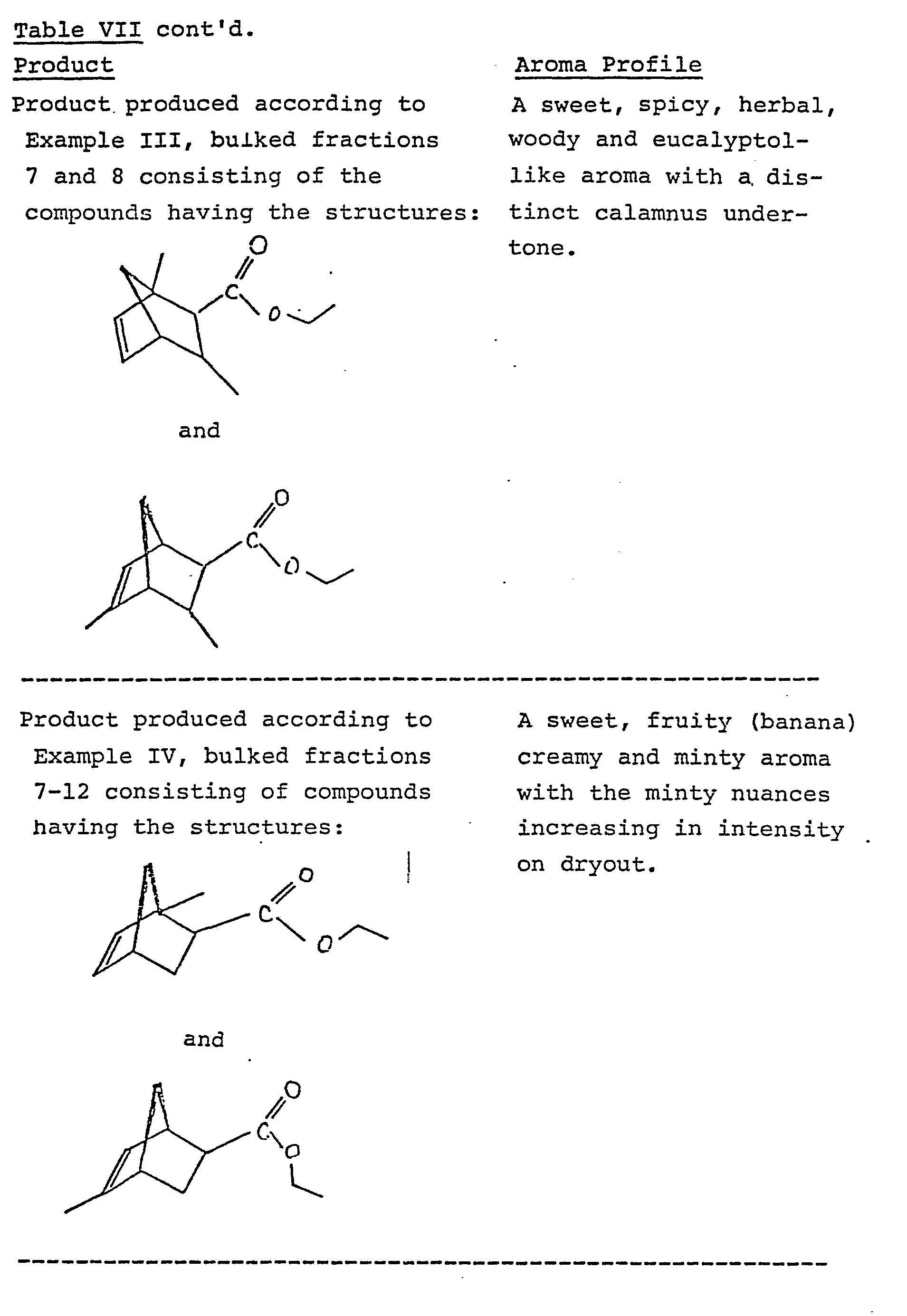

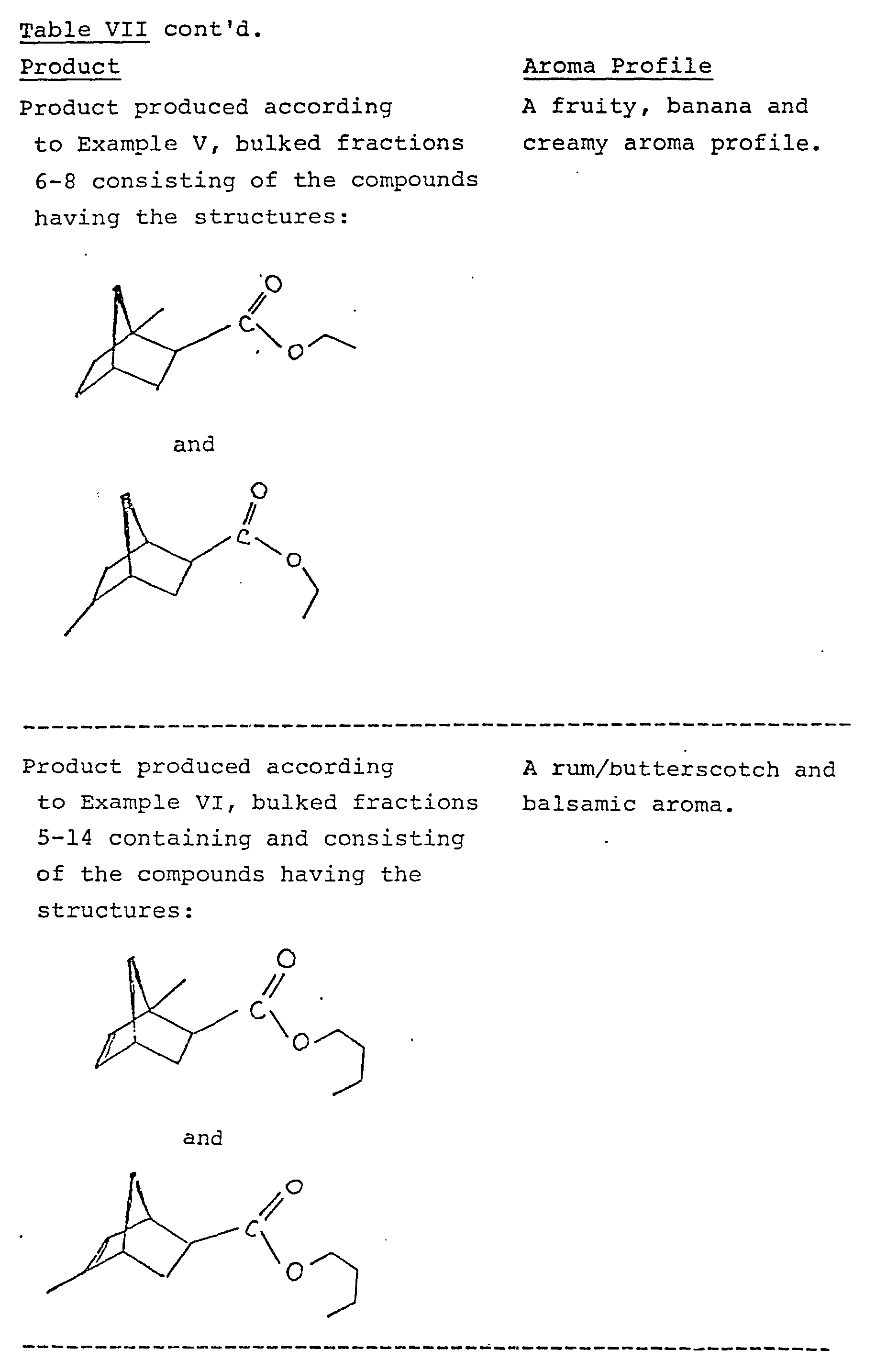

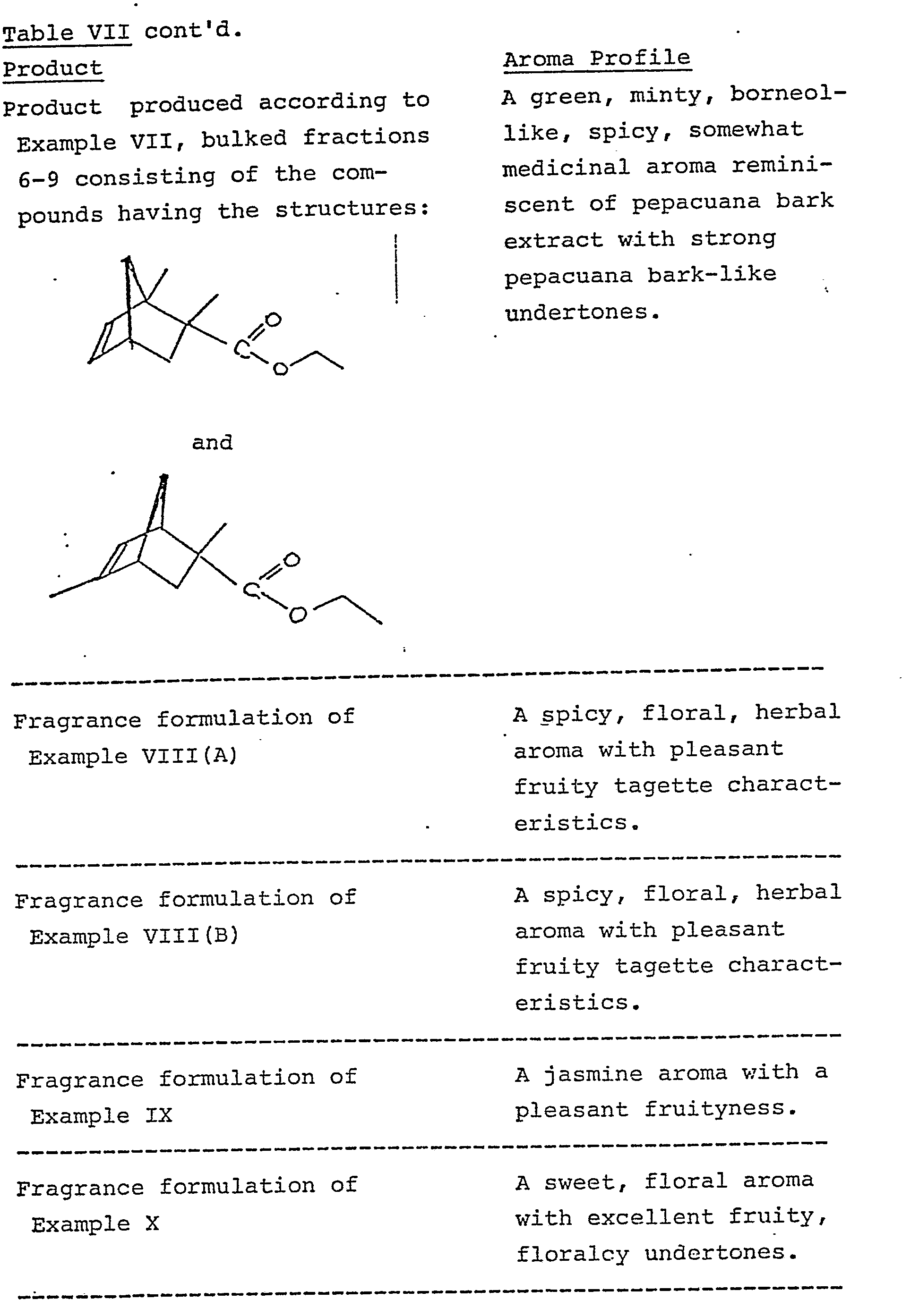

### Example XVI

### Preparation of a cologne and handkerchief perfume

The perfume ingredient as set forth in Table VIII below is incorporated into colognes at concentrations of 2.0%, 2.5%, 3.0%, 3.5%, 4.0%, 5.0% and 6.0% in 75%, 80%, 85%, 90% and 95% aqueous ethanol solutions. Distinct and definite aromas as set forth in Table VIII below are imparted to the colognes. The perfume ingredients as indicated in Table VIII below are also added to handkerchief perfumes at concentrations of 15%, 20%, 25%, 30% and 35% (in 75%, 80%, 85%, 90% and 95% aqueous ethanol) and aroma profiles as set forth in Table VIII are imparted to the handkerchief perfume.

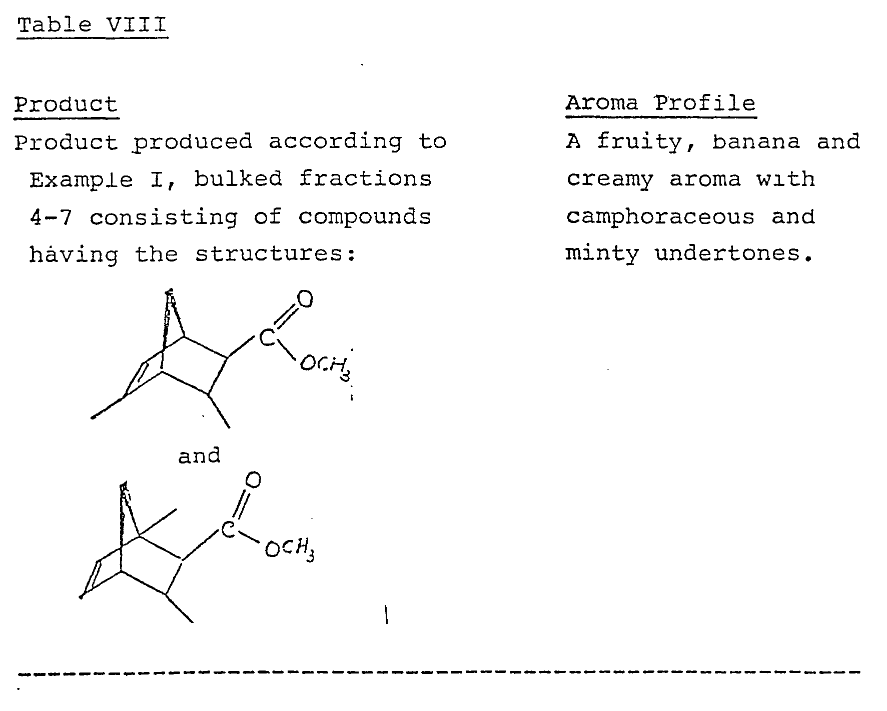

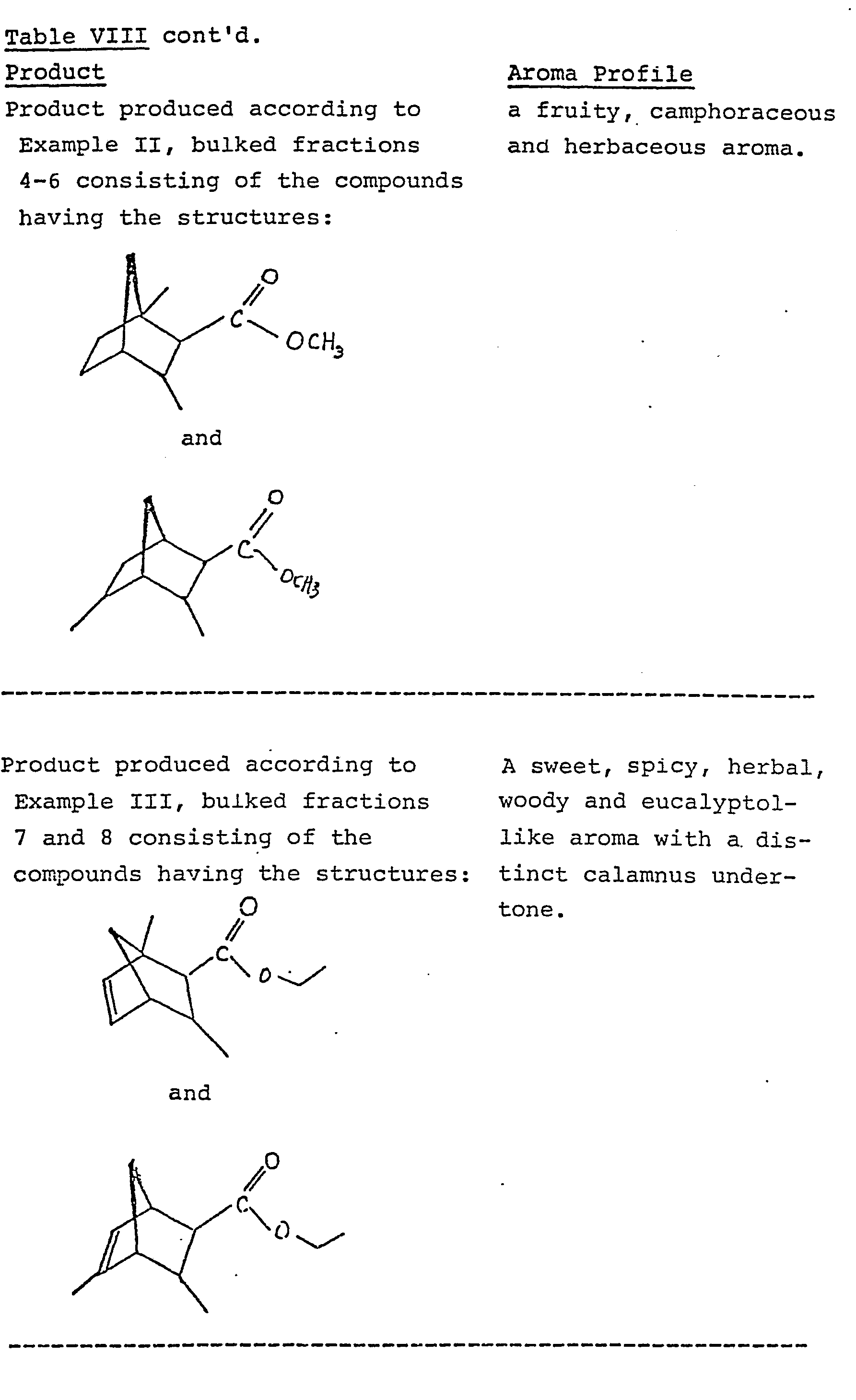

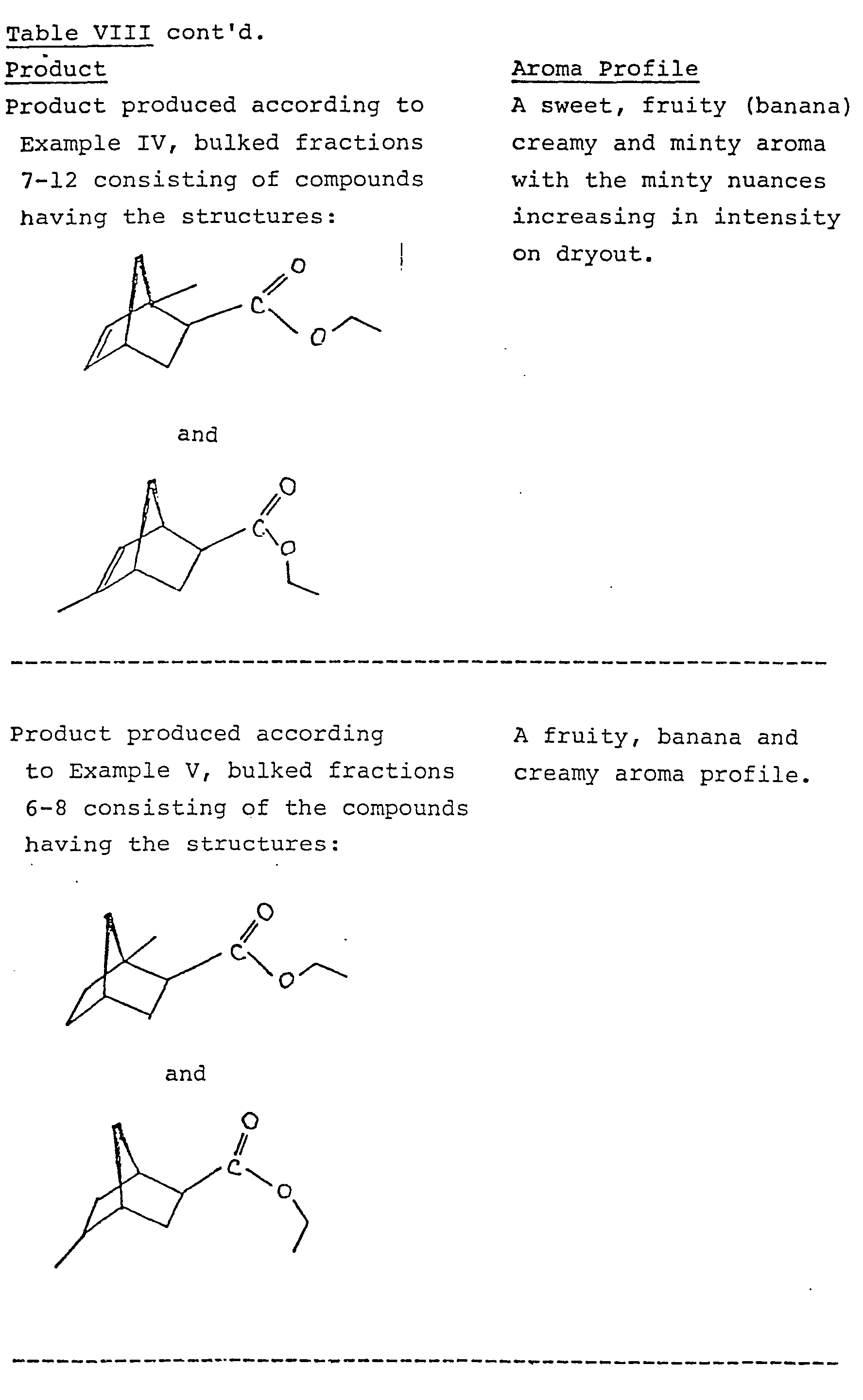

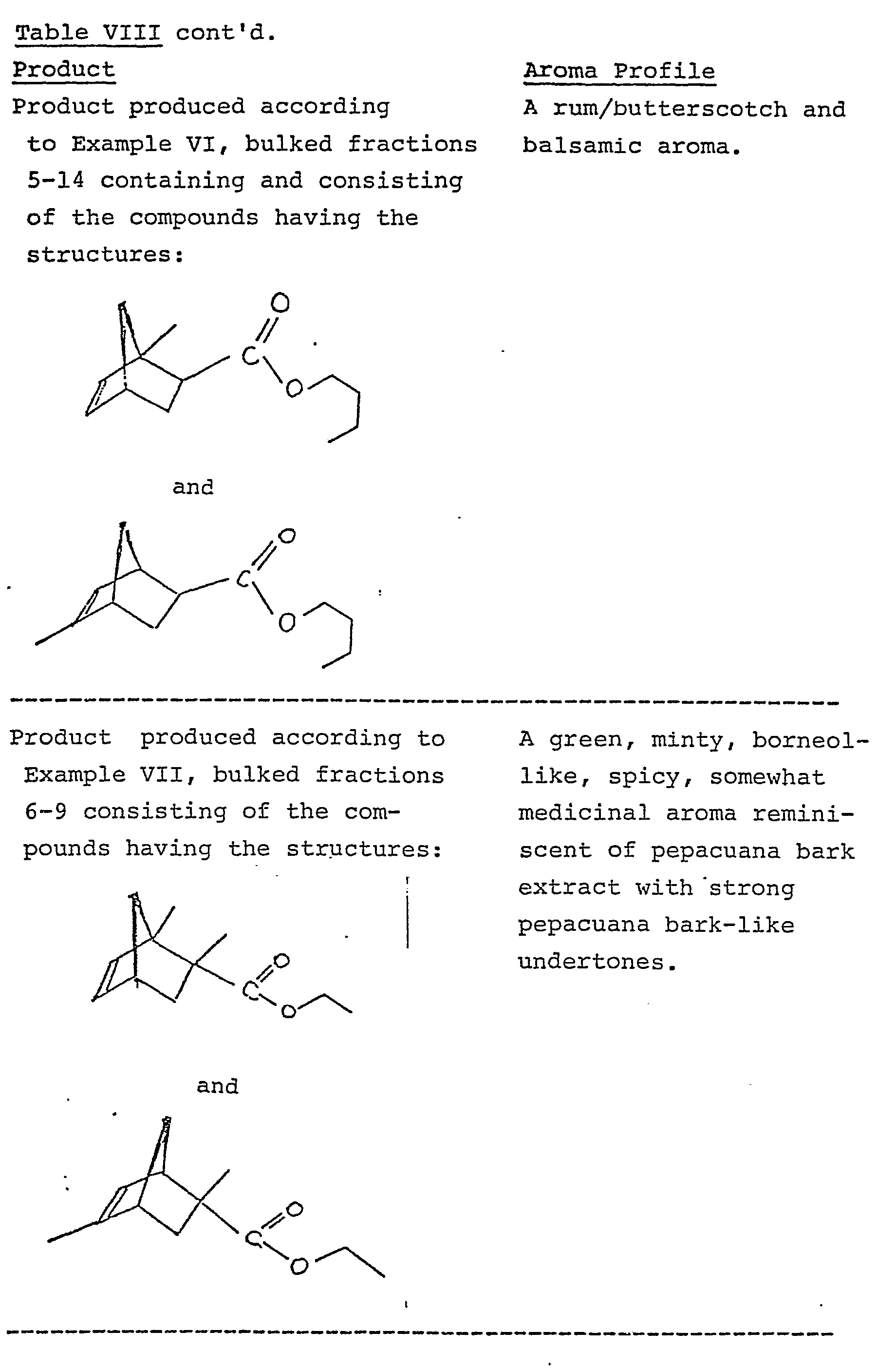

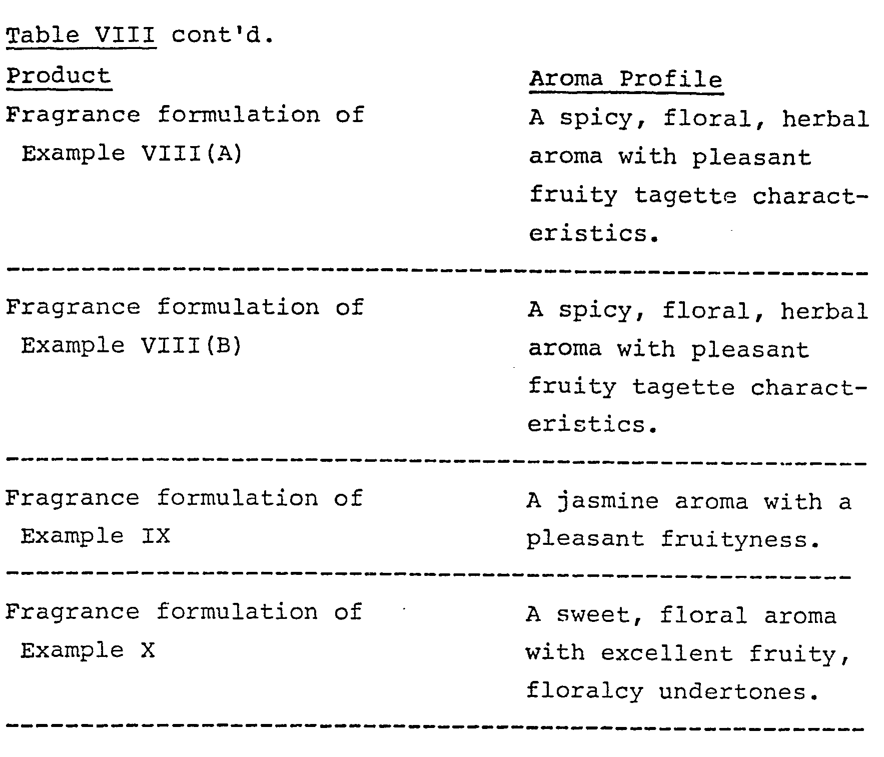

### Example XVII

### Blueberry flavor formulation

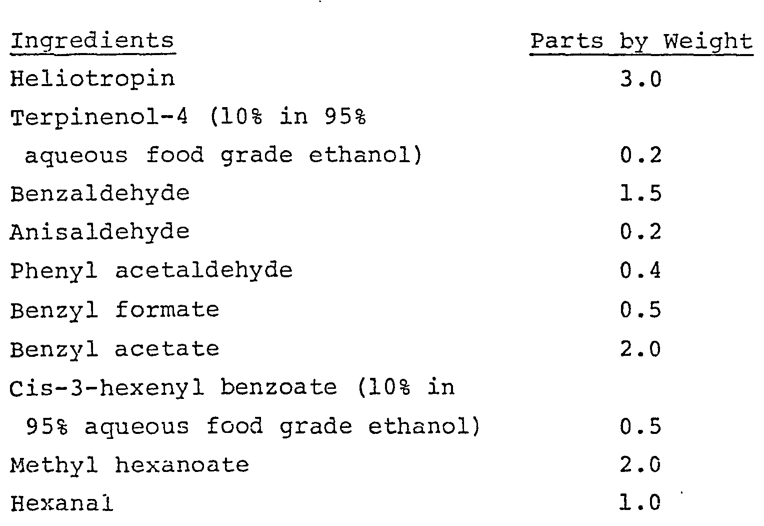

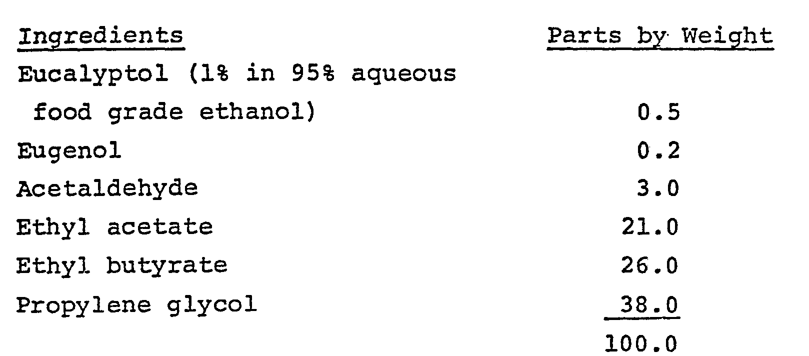

The above formulation is split into 5 portions. To the first four portions are added at the rate of 1%, the norbornane derivatives of Table IX. To the fifth portion nothing is added. All five formulations with and without said norbornyl derivatives are combined with water at the rate of 100 ppm. The flavor of the first four portions each including a norbornyl derivative prepared according to the preceding examples as indicated in Table IX below have fruity, blueberry characteristics with several other nuances as indicated in Table IX below and are all closely similar to the flavor of wild blueberries. It is therefore preferred to use the formulations containing the norbornyl derivatives as listed in Table IX below, to the basic blueberry formulation which does not contain any of said norbornyl derivatives.

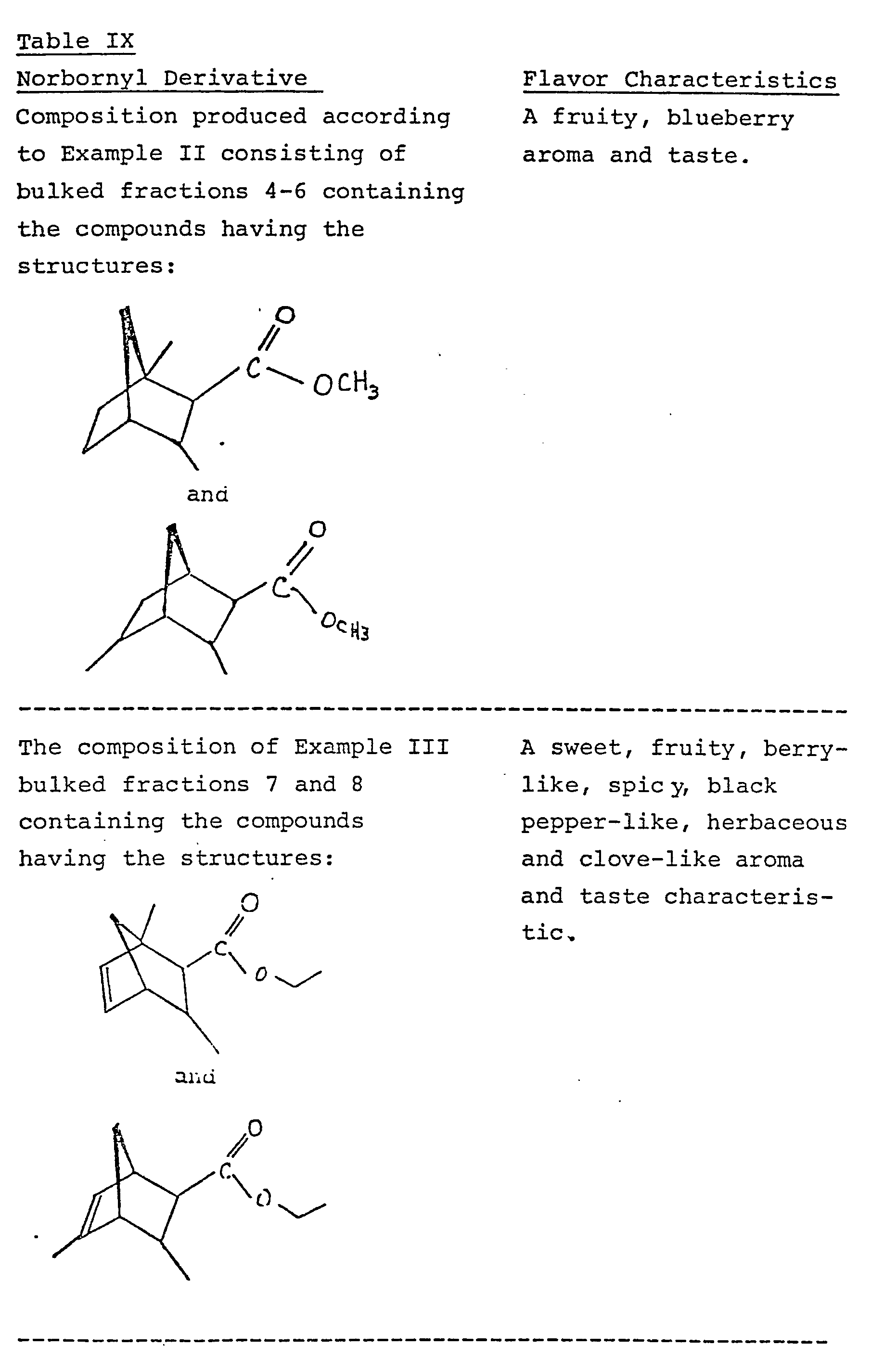

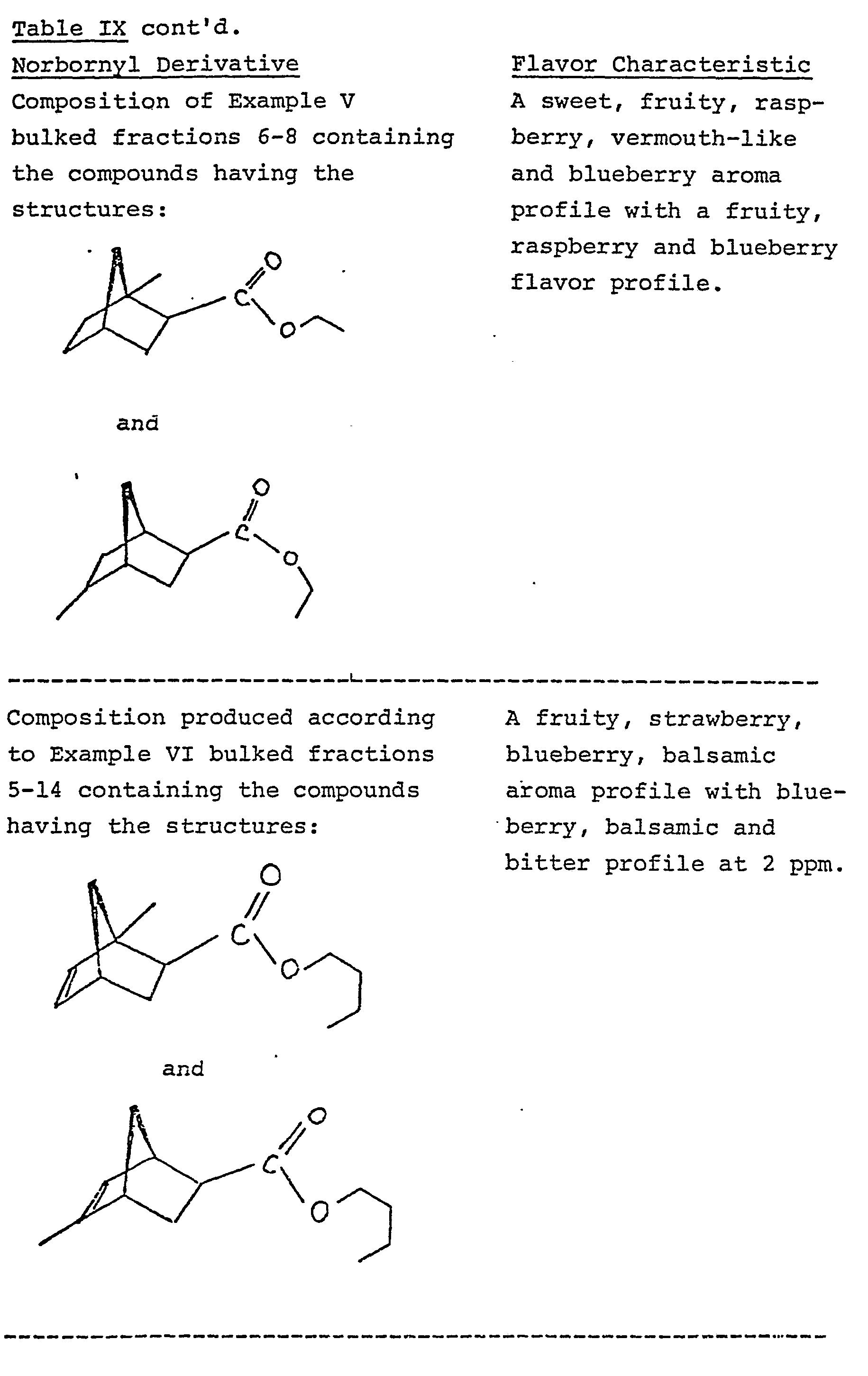

### Example XVIII

### Use of norbornane derivatives for raspberry flavors

The following basic raspberry formulation is prepared:

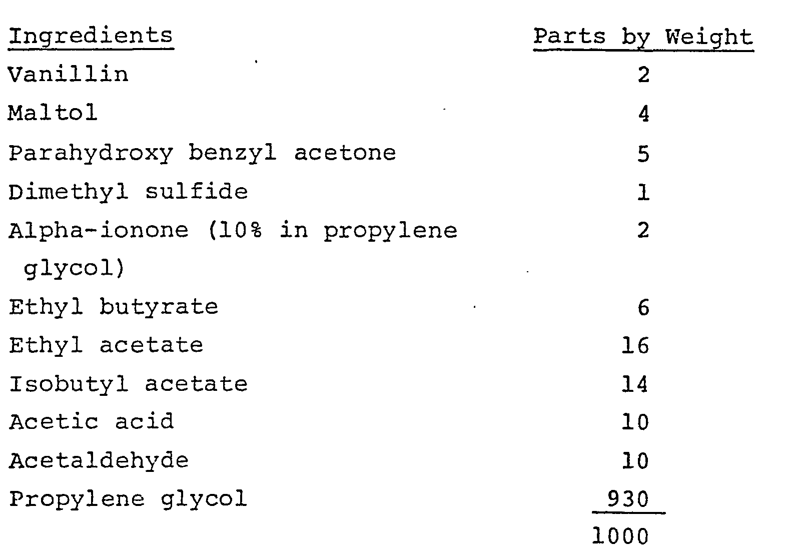

The above formulation is split into five parts. To the first four parts, at the rate of 0.3%, are added the norbornyl derivative produced according to the preceding examples, as set forth in Table X below. No additive is added to the fifth part. The five formulations are compared in water at the rate of 50 ppm. The four flavors containing the norbornyl derivatives have more ripe raspberry tastes and woody, raspberry kernel characters. They also have additional nuances as set forth in Table X below. Therefore the raspberry formulations containing the four norbornyl derivatives as additives have more natural-like and more characteristic raspberry flavors and are therefore preferred.

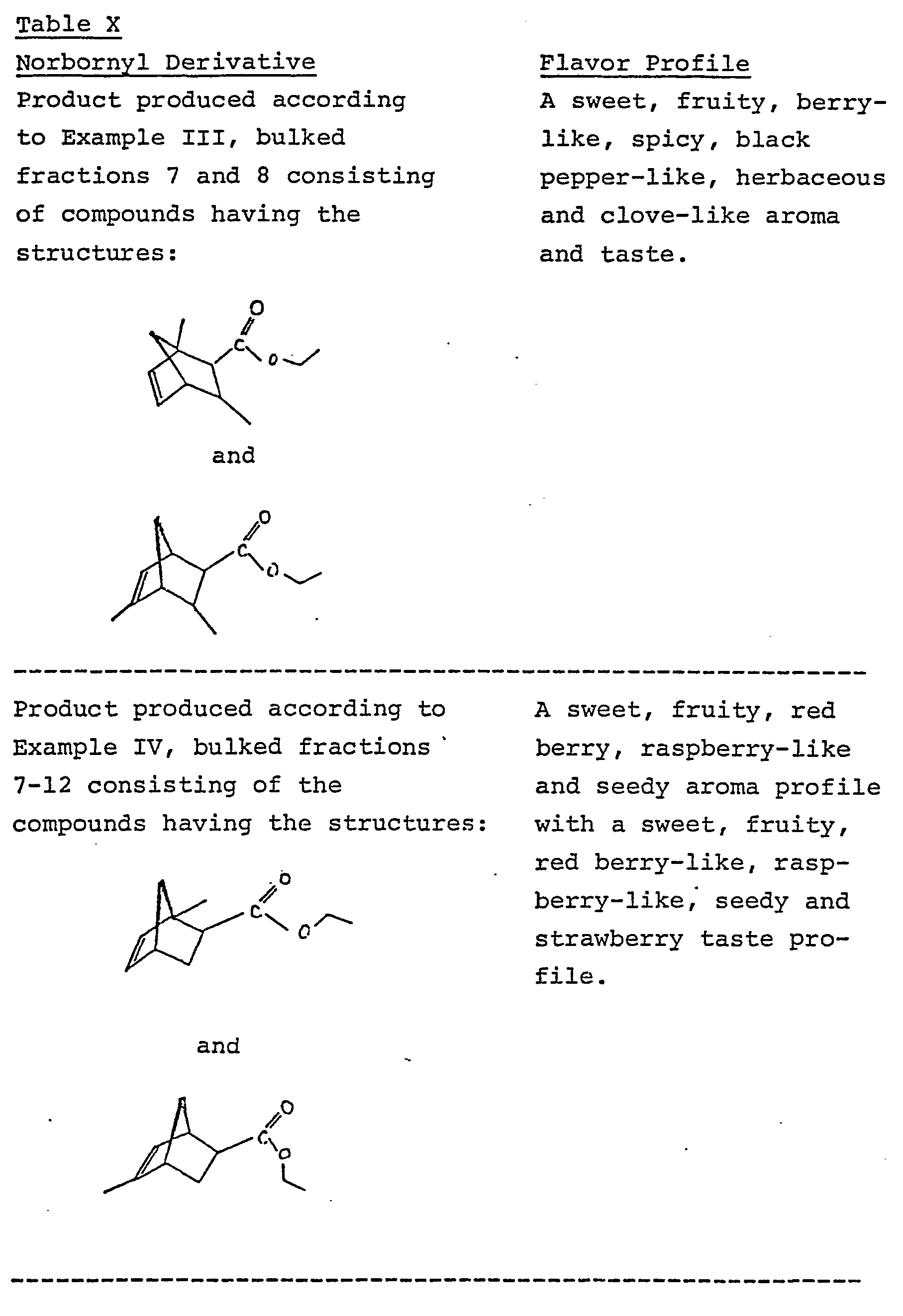

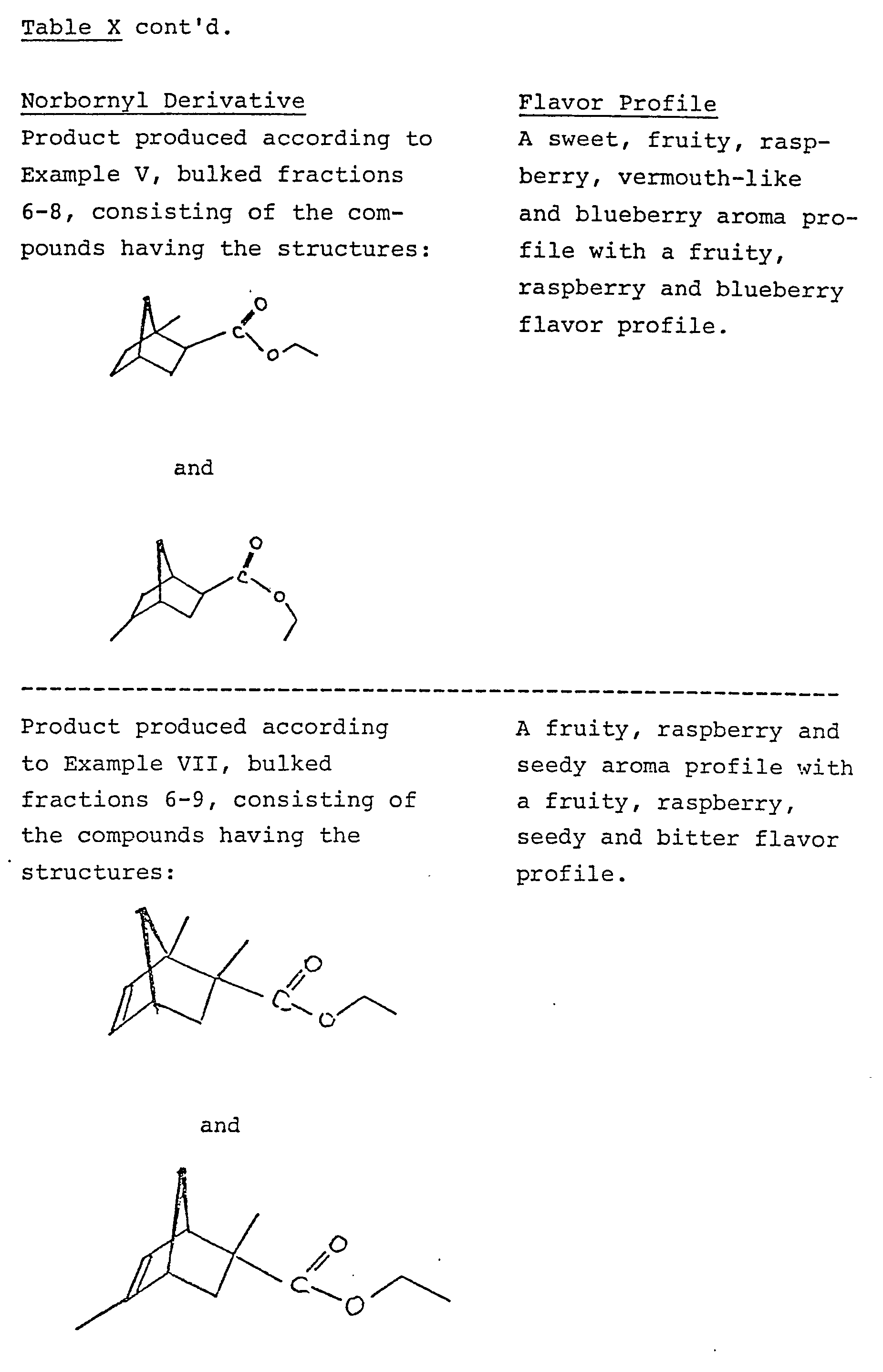

### Example XIX

### Fruited tapioca cream

The contents of Ann Page Tapioca pudding mix (ingredients: sugar, corn starch, tapioca, sodium chloride, tapioca flavor and artificial color; net weight 138 grams) is emptied into a sauce pan. Three cups of milk are added together with one beaten egg yolk previously blended therewith. The resulting mix is cooked over medium heat stirring constantly while slowly adding at the rate of 0.2%, either one of the flavor materials of Exmaple XVII (blueberry flavor) or one of the flavor materials of Example XVIII (raspberry flavor) each of the materials containing one of the norbornyl derivatives of Table IX or of Table X as the case may be. The resulting mixture after heating, is then cooled to room temperature in the saucepan. One egg white is slowly added to the resulting mixture together with three tablespoons of sugar. The resulting mixture is then blended and chilled yielding pleasantly tasting blueberry or raspberry tapioca cream deserts depending upon whether the materials of Example XVII or Example XVIII are added.

### Example XX

### Flavored instant pudding

A pudding mix (Royal Instant, net weight 3.5 ozs. produced by Standard Brands Inc., New York, N.Y. 10022) is intimately admixed with 2 cups of cold fresh whole milk.

To this mixture, at the rate of 0.3%, is added one of the blueberry flavors of Example XVII containing the norbornyl derivative as listed in Table IX or one of the raspberry flavors of Example XVIII containing one of the norbornyl derivatives as listed in Table X. The resulting mixture is blended in'a Waring blender for a period of three minutes. The resulting mixture is then cooled at 15°C for a period of five minutes. The resulting puddings have excellent natural blueberry or natural raspberry flavors as the case may be, depending on whether the blueberry flavors of Example XVII or whether the raspberry flavors of Example XVIII are used.

### Example XXI

### A. Powder flavor composition

20 grams of one of the flavor compositions of Example XVII (containing at least one of the norbornyl derivatives of Table IX) is emulsified in a solution containing 300 grams gum acacia and 700 grams water. The emulsion is spray-dried with a Bowen Lab Model Drier utilizing 260 c.f.m. of air with an inlet temperature of 500°F, an outlet temperature of 200°F, and a wheel speed of 50,000 rpm.

### B. Sustained release flavor

The following mixture is prepared:

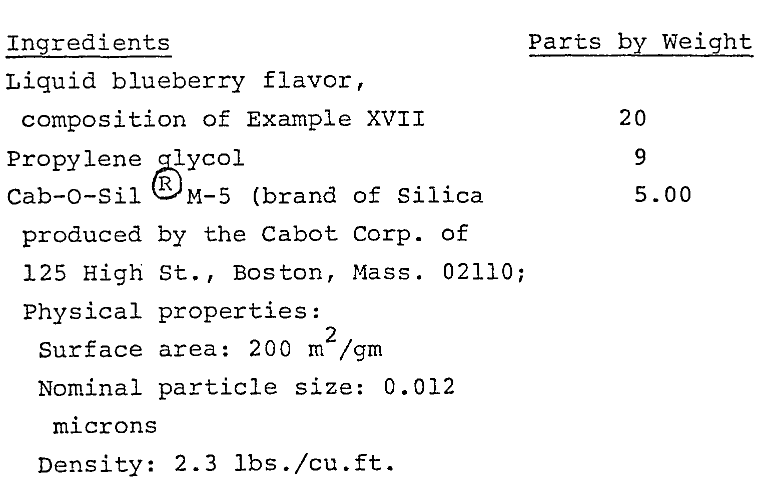

The Cab-O-Sil ®is dispersed in the liquid blueberry flavor compositions (containing the norbornyl derivatives) of Example XVII with vigorous stirring, thereby resulting in a viscous liquid. 71 parts by weight of the powder flavor composition of Part A supra, is then blended into the said viscous liquid, with stirring, at 25°C for a period of 30 minutes resulting in a dry, free flowing sustained release flavor powder.

### Example XXII

10 parts by weight of 50 Bloom pigskin gelatin is added to 90 parts by weight of water at a temperature of 150°F. The mixture is agitated until the gelatin is completely dissolved and the solution is cooled to 120°F. 20 parts by weight of one of the liquid flavor compositions of Example XVII (containing at least one of the norbornyl derivatives of Table IX) is added to the solution which is then homogenized to form an emulsion having particle size typically in the range of 2-5 microns. This material is kept at 120OF under which conditions the gelatin will not jell.

Coascervation is induced by adding, slowly and uniformly 40 parts by weight of a 20% aqueous solution of sodium sulphate. During coascervation, the gelatin molecules are deposited uniformly about each oil droplet as a nucleus.

Gelatin is effected by pouring the heated coascervate mixture into 1,000 parts by weight of 7% aqueous solution of sodium sulphate at 65°F. The resulting jelled coascervate may be filtered and washed with water at temperatures below the melting point of gelatin, to remove the salt.

Hardening of the filtered cake, in this example, is effected by washing with 200 parts by weight of 37% solution of formaldehyde in water. The cake is then washed to remove residual formaldehyde.

### Example XXIII

100 parts by weight of chicle are mixed with 4 parts by weight of the flavor prepared in accordance with Example _{XXI}. 300 parts of sucrose and 100 parts of corn syrup are added. Mixing is effected in a ribbon blender with jacketed side walls of the type manufactured by the Baker Perkins Co.

The resultant chewing gum blend is then manufactured into strips 1 inch in width and 0.1 inches in thickness. The strips are cut into lengths of 3 inches each. On chewing, the chewing gum has a pleasant, long lasting blueberry flavor.

### Example XXIV

### Chewing gum

100 parts by weight of chicle are mixed with 18 parts by weight of the flavor prepared in accordance with Example XXII. 300 parts of sucrose and 100 parts of corn syrup are then added. Mixing is effected in a ribbon blender with jacketed side walls of the type manufactured by the Baker Perkins Co.

The resulting chewing gum blend is then manufactured into strips 1 inch in width and 0.1 inches in thickness. The strips are cut into lengths of 3 inches each. On chewing the chewing gum has a pleasant, long lasting blueberry flavor.

### Example XXV

### Toothpaste formulation

The following separate groups of ingredients are prepared:

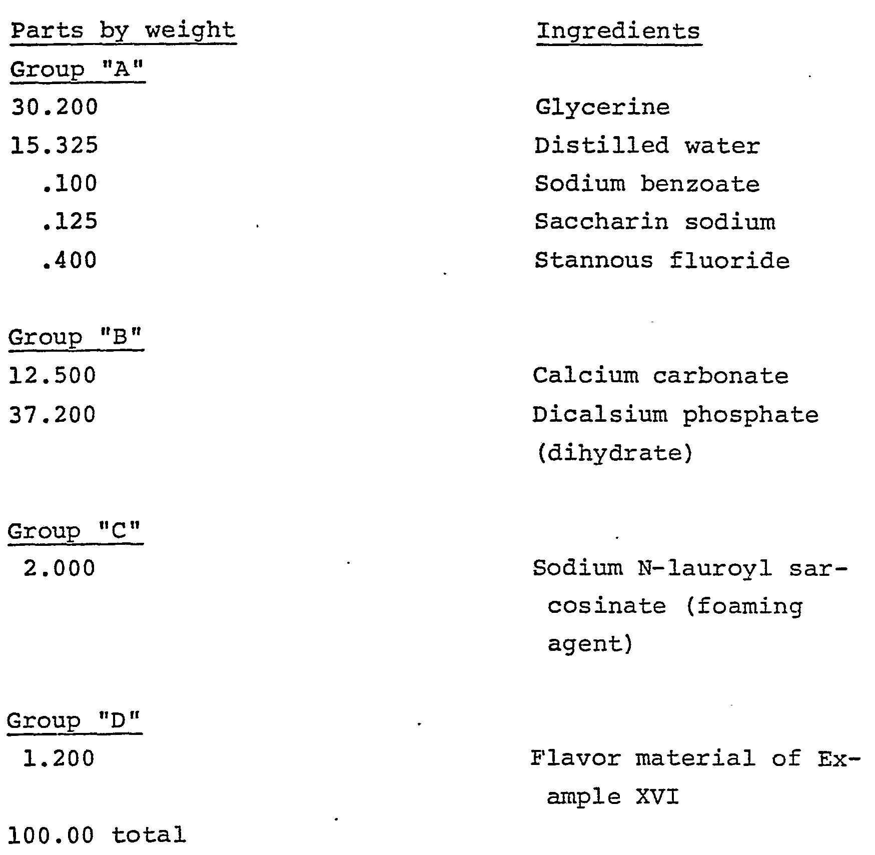

### Procedure:

1. The ingredients in Group "A" are stirred and heated in a steam jacketed kettle to 160°F.
2. Stirring is continued for an additional three to five minutes to form a homogeneous gel.
3. The powders of Group "B" are added to the gel, while mixing, until a homogeneous paste is formed.
4. With stirring, the flavor of "D" is added and lastly the sodium n-lauroyl sarcosinate.
5. The resultant slurry is then blended for one hour.

The completed paste is then transferred to a three roller mill and then homogenized, and finally tubed.

The resulting toothpaste when used in a normal toothbrush- ing procedure yields a pleasant blueberry flavor, of constant strong intensity throughout said procedure (1-1.5 minutes).

### Example XXVI

### Chewable vitamin tablets

The flavor material (containing norbornyl derivatives) of Table X of Example XVIII is added to a chewable vitamin tablet.formulation at the rate of 10 gm/Kg which chewable vitamin tablet formulation is prepared as follows:

In a Hobart mixer the following materials are blended to homogeneity:

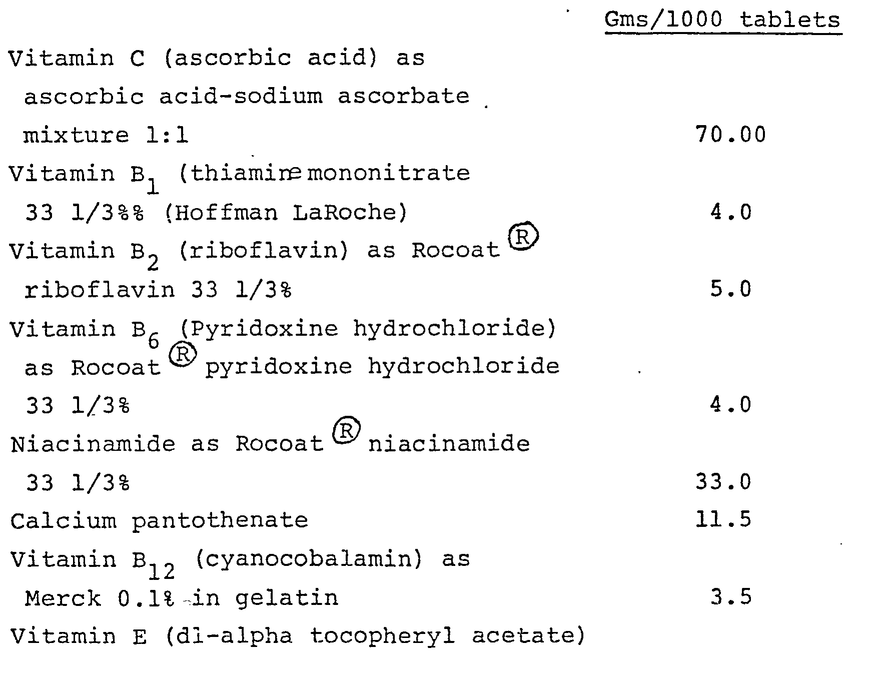

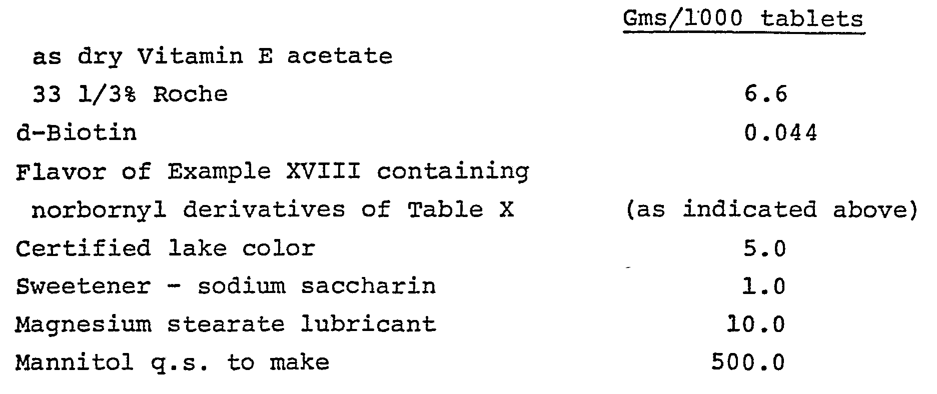

Preliminary tablets are prepared by slugging with flat- faced punches and grinding the slugs to 14 mesh. 13.5 g dry Vitamin A acetate and 0.6 g Vitamin D are then added as beadlets. The entire blend is then compressed using concave punches at 0.5 g each.

Chewing of the resultant tablets yields a pleasant long lasting, consistently strong raspberry flavor for a period of 12 minutes.

### Example XXVII

### Chewing tobacco

Onto 100 pounds of.tobacco for chewing (85% Wisconsin leaf and 15% Pennsylvania leaf) the following casing is sprayed at a rate of 30%:

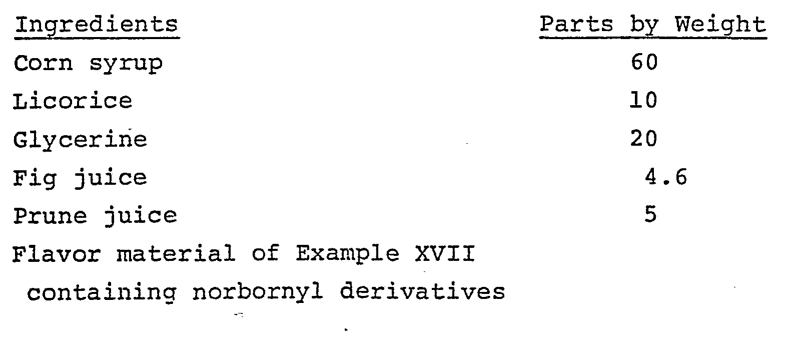

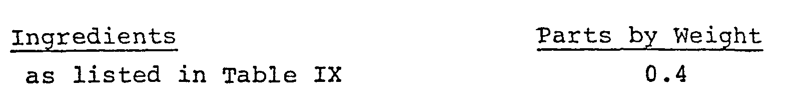

The resultant product is redried to a moisture content of 20%. On chewing, this tobacco has an excellent substantially consistent, long-lasting blueberry nuance in conjunction with the tobacco note.

### Example XXVIII

### Tobacco formulation

A tobacco mixture is produced by admixing the following ingredients:

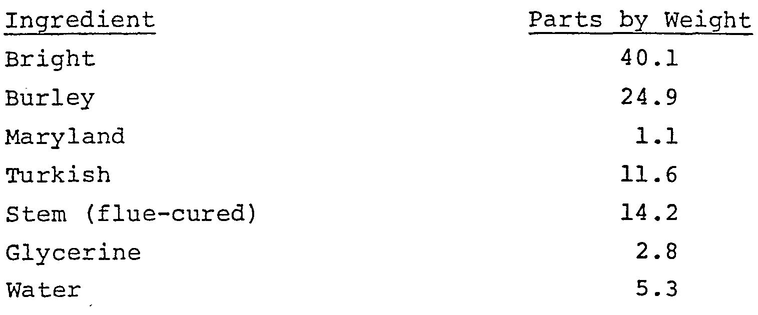

Cigarettes are prepared from this tobacco. The following flavor formulation is prepared:

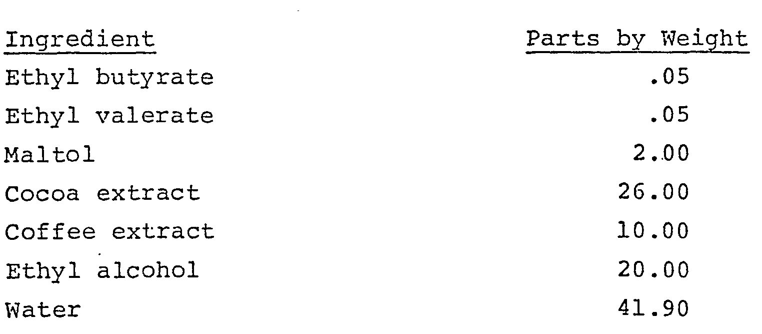

The above stated tobacco flavor formulation is applied at the rate of 0.1% to all of the cigarettes produced using the above tobacco formulation. Half of the cigarettes are then treated with 500 to 1,500 ppm of one of the following norbornyl derivatives of Table XI as follows:

### Table XI

A. Product produced according to Example II, bulked fractions 4-6 consisting of the compounds having the structures: 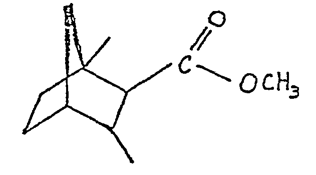 and

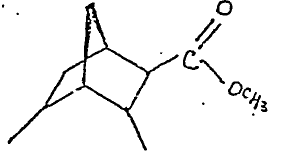

B. Composition of matter produced according to Example III bulked fractions 7 and 8 consisting of the compounds having the structures: 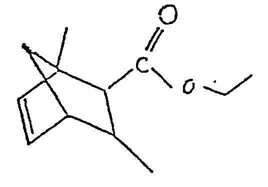 and

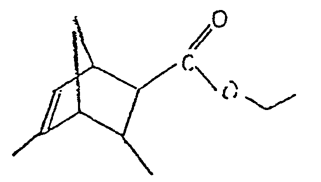

The control cigarettes not containing the norbornyl derivatives as set forth in Table XI and the experimental cigarettes which contain the norbornyl derivatives as set forth in Table XI are evaluated by paired comparison and the results are as follows:

The experimental cigarettes are found, on smoking, to have a sweeter, spicy, woody-oriental, clove-like taste with much more body and much more natural tobacco-like aroma prior to smoking and on smoking in the mainstream and the sidestream. The experimental cigarettes containing the product produced according to Example II in addition have cooling nuances. The experimental cigarettes produced containing the product produced according to Example III also have cinnamon bark nuances prior to and on smoking in the mainstream and in the sidestream.

All cigarettes are evaluated for smoke flavor with a 20 mm cellulose acetate filter.

Accordingly, it is concluded that the norbornyl derivatives set forth in Table XI produced according to the processes of Examples II and III enhance the tobacco-like taste and aroma particularly the oriental-like taste of the blended cigarette imparting to it sweet, spicy and woody-oriental tobacco-like nuances and in addition clove-like nuances.

### Example XXIX

The following is a description of a preferred embodiment of the invention as carried out using a process wherein minute capsules having a diameter in the range of 50 up to 500 microns were added to a wet web of reconstituted tobacco (weight ratio of dry web to dry capsules - 1:0.04). The capsules and binder materials (weight ratio of dry capsule to dry binder = 1:0.1) when placed among the tobacco fibers, wet them and entangle with them and clothe them, thus in effect securing and binding the capsules against migration through the sheet, thereby forming a subsident stratum. The majority of binder and associated capsules are caught in the sheet. Substantially no capsules migrate through the sheet. When the wet tobacco web is dried, the binder shrinks by loss of solvent, leaving the dried polymeric binder material, and the capsules remain in place relatively with respect to sheet thickness. The sheets containing the capsules are then shredded and used in producing smoking articles such as cigarettes. Such cigarettes are farmed using a wrapper, containing a fill of tobacco extending from one end of the wrapper to the other, and intimately admixed with the tobacco, a plurality of microcapsules each comprising an aromatic volatile synthetic clove oil flavorant. The capsules are homogeneously spaced in contiguous relationship with the tobacco such that as the burning front of the tobacco advances the length of the tobacco article, a concomitant elevation of temperature initiates consecutive rupture of the capsules (1) releasing the volatile synthetic clove oil-containing material which emanates with smoke from the smoking article into the smoker's mouth and (2) yielding a crackling sound audible to the human ear.

Tragacanth gum solution and starch solution were prepared in the following manner:

### Part A

### Concentrated tragacanth gum solution (binder)

4.5 pounds of dry tragacanth gum powder were stirred into 50 gallons of water, using a suitable mixer. Five minutes after all the powder had been added, the mixer was turned off. The tragacanth gum solution was allowed to stir for 2 hours, and then the mixer was turned on for 5 minutes. Sitting for 2 additional hours, enabled the tragacanth gum to hydrate. After five minutes, the mixer was turned off, and the'55 gallon drum was covered. Just prior to combining the tragacanth gum solution and the capsular slurry, 50 gallons of tragacanth gum solution was diluted with water to 3 percent tragacanth gum on a solids basis.

### Part B

### Starch solution

The hydrolyzed starch solution was prepared by heating a slurry of the starch at 190°F for a minimum of 15 minutes to provide a 1% by weight starch-in-water solution.

Capsule slurries were prepared in the following manner:

### Part C

### Preparation of synthetic clove oil

A mixture is prepared containing the following ingredients:

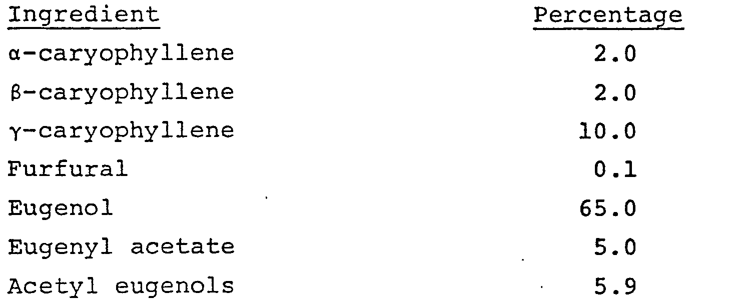

Norbornol derivatives as follows:

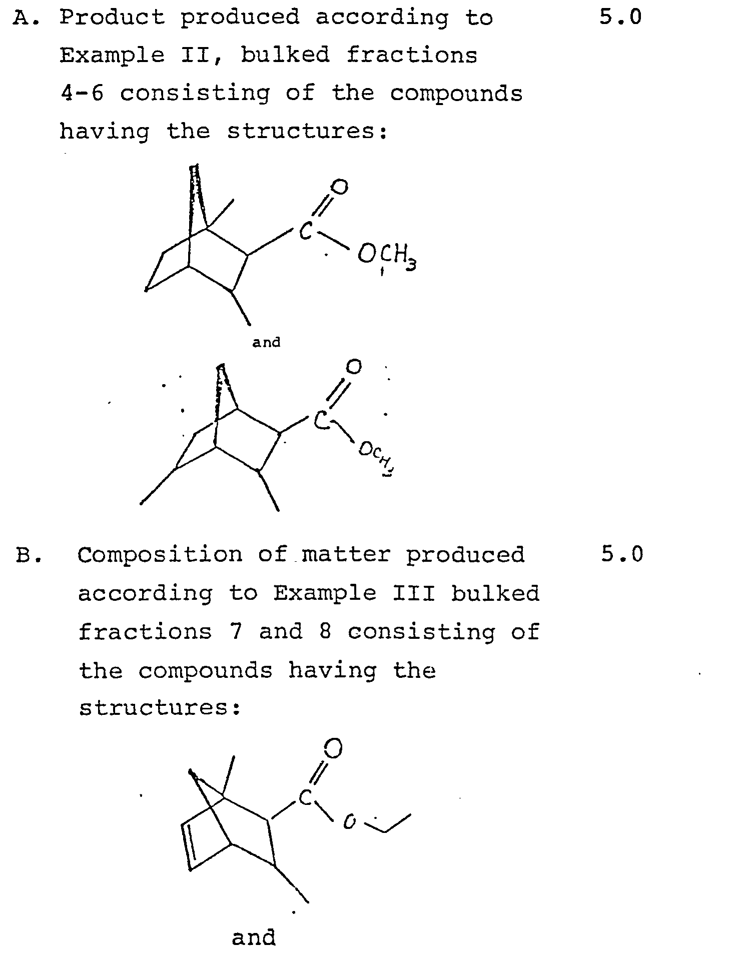

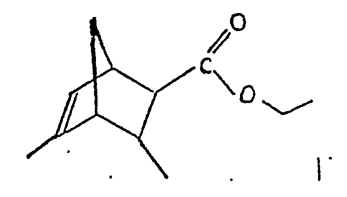

### Part D

### Preparation of capsular slurry-encapsulation of synthetic clove oil

Ten grams of gum arabic were dissolved at room temperature in 220 grams of deionized water. The mixture was agitated until the gum arabic was fully dissolved. In a separate 240 milliliter Erlenmeyer flask, 10.0 grams of modified gelatin was mixed with 220.0 grams of deionized water. The gelatin was allowed to tumefy at room temperature and also then warmed in a water bath to about 40°C with stirring so that the gelatin was dissolved.

The gelatin solution and the gum arabic solution were poured into a beaker equipped with a stirrer. A floccu- lence indicating the precipitation of the gelatin was noted. The temperature of the mixture was decreased to 35°C. The speed of the stirrer was adjusted so that it was turning only enough to keep the phases mixed. The pH of the mixture was 4.50.

Into the beaker containing the mixture of gum arabic and gelatin was poured 118.0 grams of synthetic clove oil as prepared in Part C. The speed of the stirrer was then adjusted to mix the colloids and the oil. The oil separated into droplets. Two drops of octyl alcohol were added to prevent foaming. The progress of the coacervation was monitored by means of microscopic examination.

The temperature of the mixture was lowered to room temperature, e.g., 24°C. At the higher temperature of 31°C, colloid deposition was observed on the oil droplets. At 24°C, little colloid could be observed in aqueous portions of the mixture. Deposition had ceased. Stirring was continued for 30 minutes, whereupon the reaction mixture was cooled on an ice bath to 4°C. The reaction mixture was maintained at this temperature for 200 minutes. (When hardening was desired, 1.0 milliliters of a 25% glutaraldehyde in water per gram of gelatin is added.)

The internal phase of the capsules thus formed was approximately 80-90% of the total weight of the capsules.

The capsules thus produced had diameters in the range of from 50 up to 500 microns. They were coated with the binder onto a tobacco sheet material which was shredded and used as a fill in the manufacture of a smoking article.

### Part E

### Variation of the encapsulation of synthetic clove oil

A variation of the encapsulation procedure set forth immediately supra is shown below.

The solution of gum arabic was warmed to 38°C, placed in a Waring blender and stirred. The clove oil prepared in Part C was added gradually while the speed of the blender was being increased until the size of the clove oil droplets was approximately 50-500 microns. The mixture thus formed was poured into a 1,000 milliliter beaker containing gelatin, also at 38°C, and was stirred thoroughly. The temperature was then allowed to drop to room temperature and then further decreased to a temperature of 4°C to 10°C by means of an ice bath.

It is evident that the tobacco film or filaments can be made from various types and combinations of tobacco. For instance, the tobacco sheet material can be made from relatively expensive tobacco such as Latakia in which it is highly desirous to use all waste because of the high price thereof. So, also, it may be formed of Burley or one or more scrap or waste cigarette type tobaccos and incorporated in accordance with a particular cigarette manufacture's formula as if it were natural cigarette tobacco leaves. Any desired formula can thus be maintained in accordance with the demands of a manufacturer's particular brand using one or more types of natural shredded tobacco leaves and admixed desired quantities of shredded capsule containing tobacco film material or filaments, either as a blending or flavoring medium or both or for purposes of bulking.

In the case of the manufacture of cigarettes, according to the present invention, tobacco films are shredded into strands or the film is formed directly into filaments substantially the width of the strands of natural shredded tobacco and of any desired length. In the case of cigars, the capsule containing films are used in large pieces much as long filler tobacco and forming long filler cigars. In all cases the shredded film or filaments or film used in cigarettes and cigars can be handled either manually or by machine in the same manner as natural shredded tobacco leaves or whole leaves or portions thereof. The amount of shredded capsule-containing reconstituted tobacco or pieces of this material employed-in a particular blend in cigarettes or cigars, respectively, will vary according to types of tobacco used in the sheet material and the requirements of a particular manufacture.

### Example XXX

### Preparation of a detergent composition

A total of 100 grams of detergent powder (a low phosphate content detergent composition which contains 12% by weight phosphate builder, 8% hardness mineral ion insensitive detergent, 0.9% by weight maleic anhydride-vinyl compound co-polymer and 2% alkylene oxide condensation product prepared according to Exam le IV at column XI, of U.S. Patent No. 4,000,080 issued on December 28, 1976) is intimately admixed with 0.15 grams of one of the perfume materials of Table XII below until a substantially homogeneous composition is obtained. This composition has an aroma as set forth in Table XII below which is pleasant and long lasting:

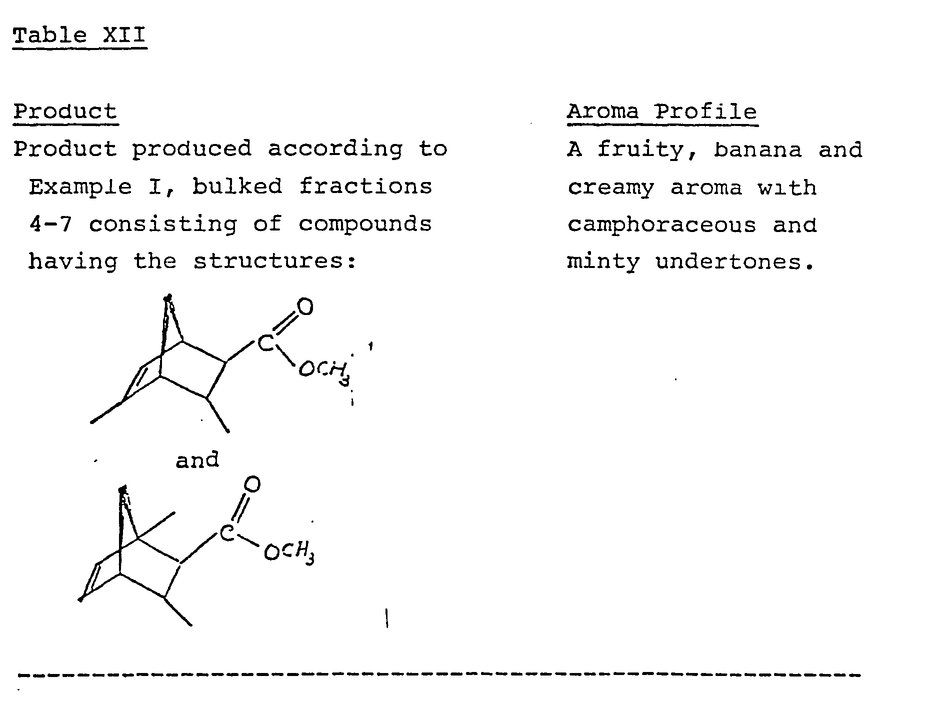

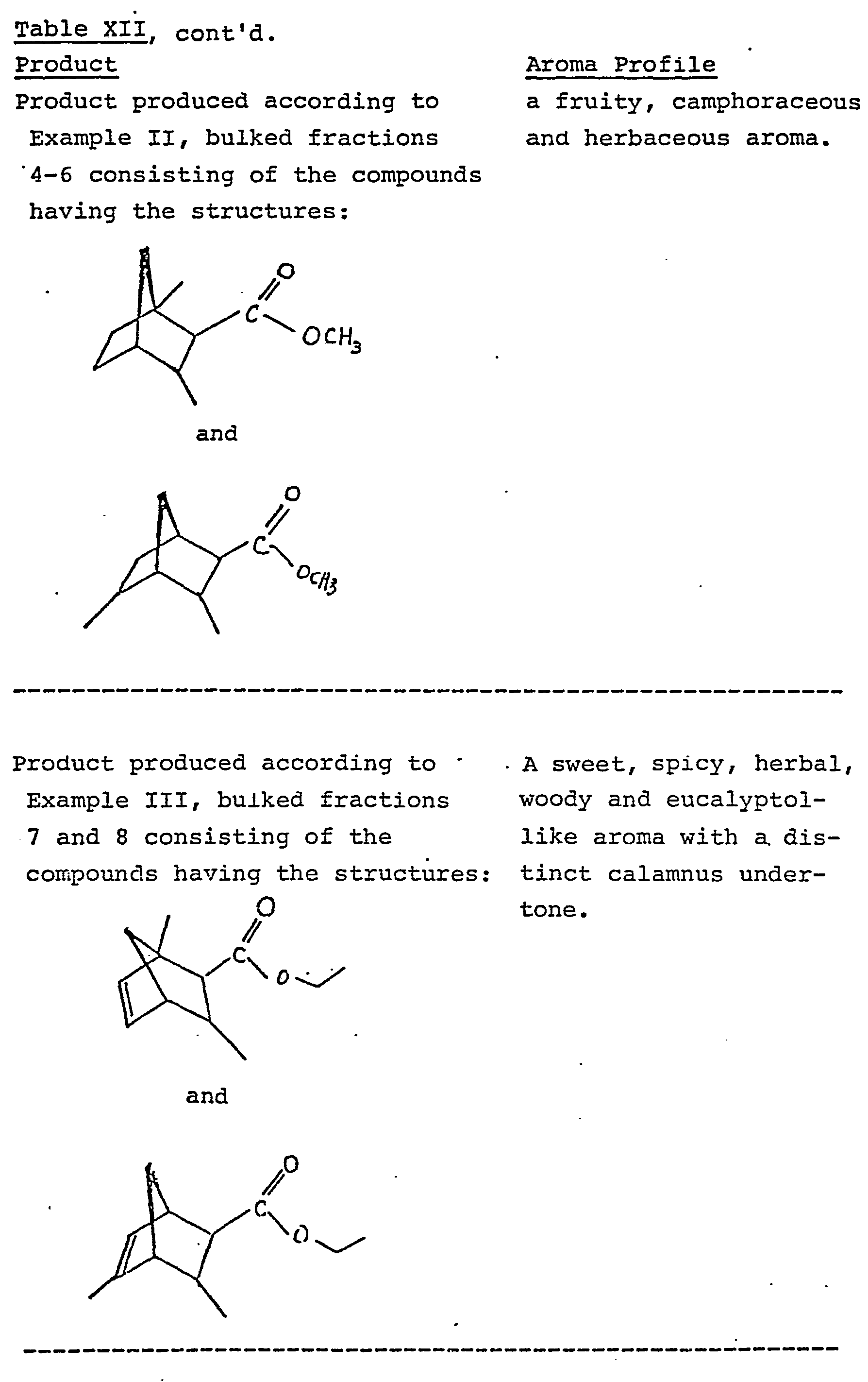

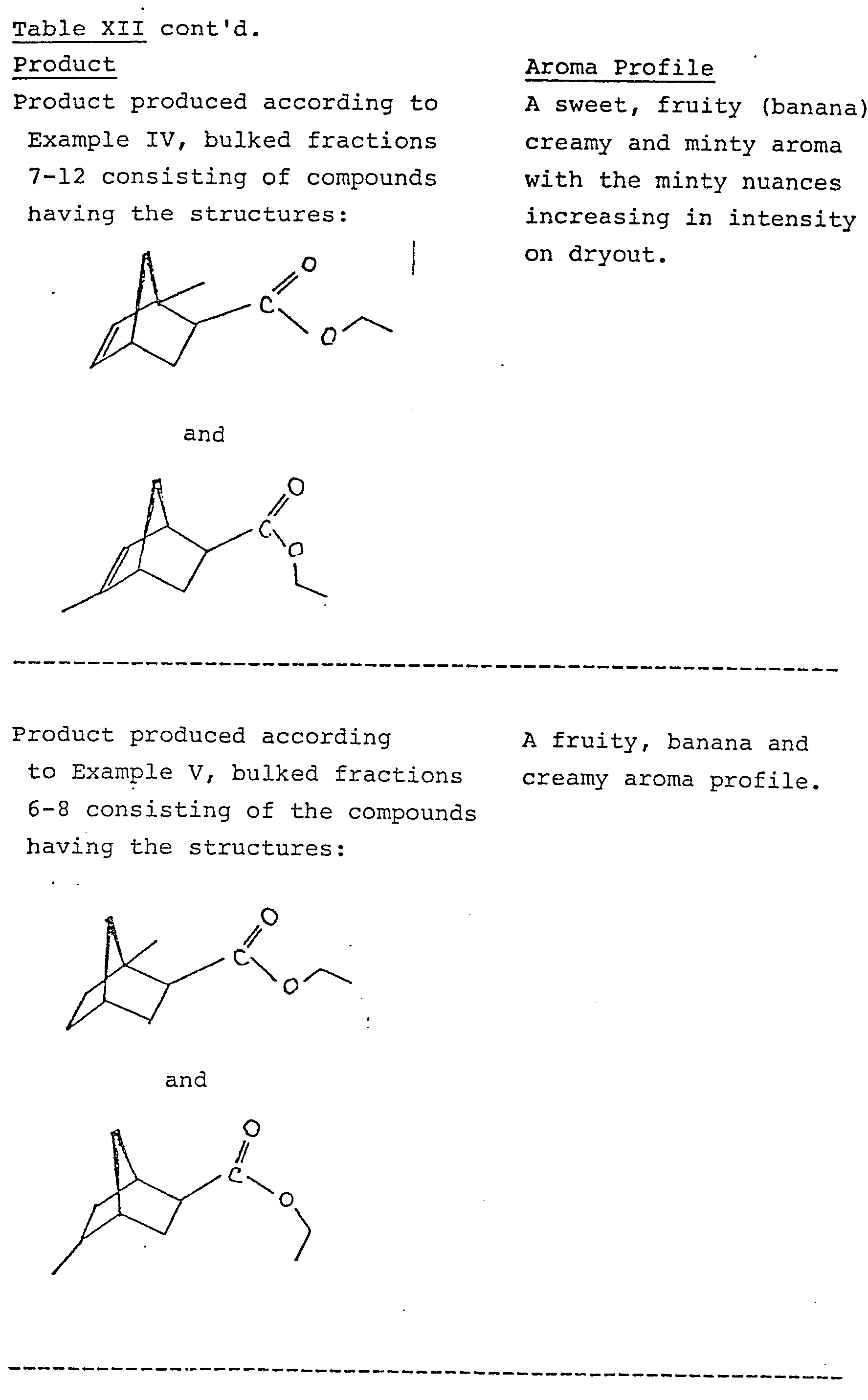

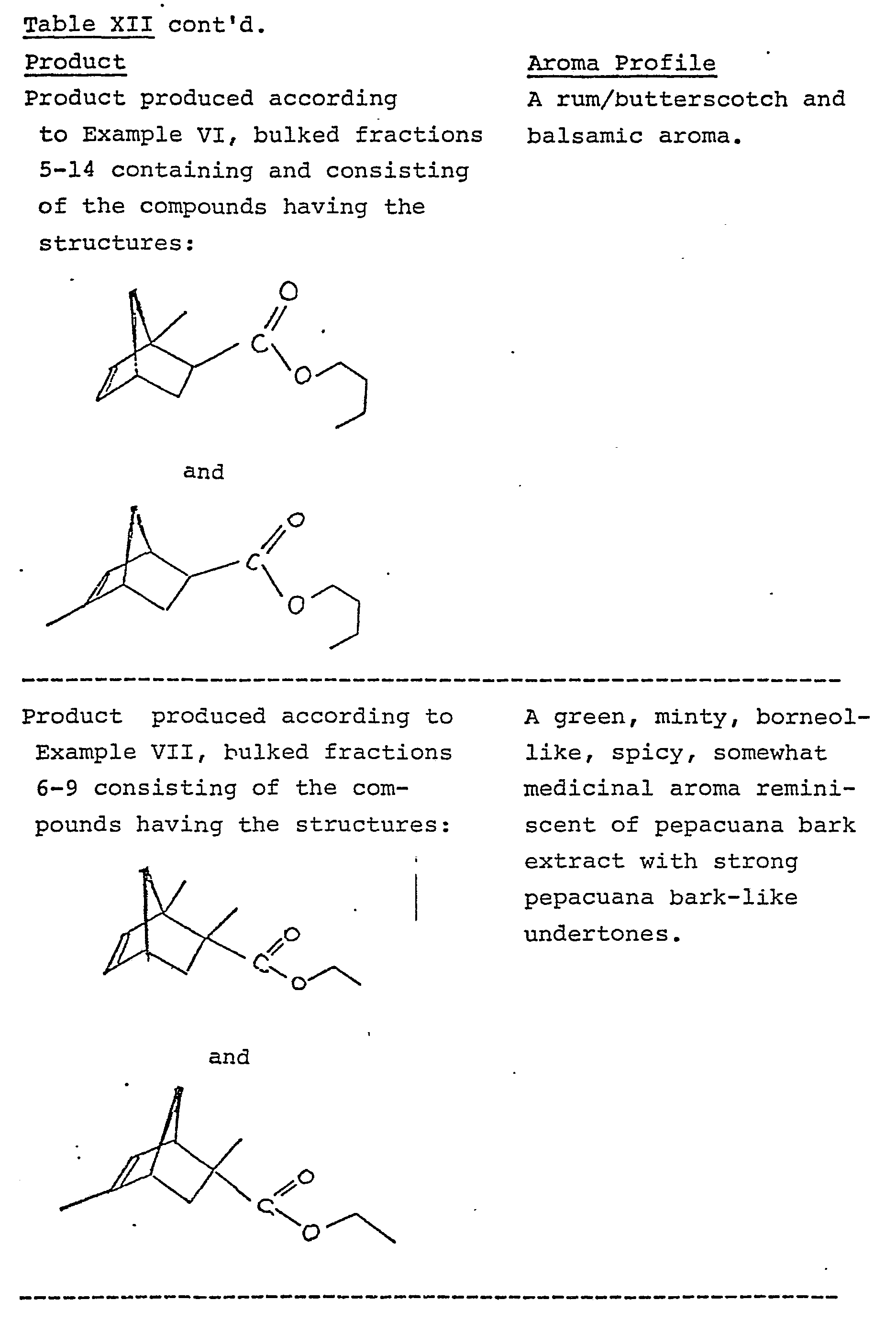

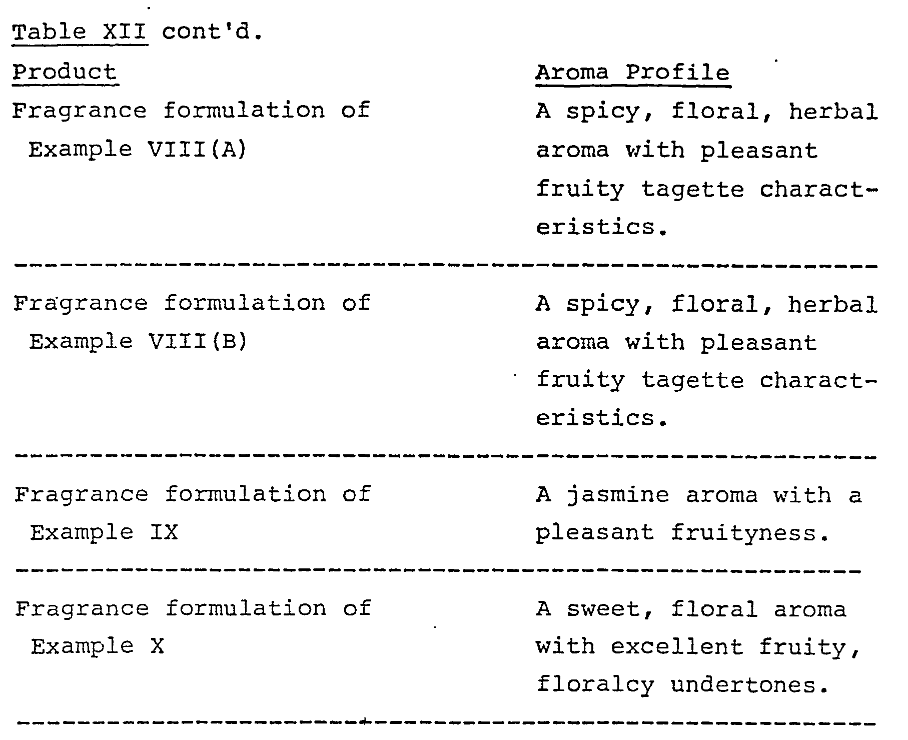

### Example XXXI

### Perfumed liquid detergents

Concentrated liquid detergents with aromas as set forth in Table XIII below are prepared containing 0.10%, 0.15% and 0.20% of the perfume ingredients set forth in Table XIII below. They are prepared by adding and homogeneously mixing the appropriate quantity of perfume ingredient set forth in Table XIII below in a liquid detergent which is a homogeneous single-phase heavy duty liquid detergent composition containing:
a. 12.5% by weight based on the free acid form of an anionic detersive surfactant;
b. 0.5% magnesium sulfate;
c. 12% by weight of an ethoxylated nonionic detersive surfactant;
d. 3% by weight of a water-soluble bis(styrylsul- fonate)biphenyl brightener; and
e. the balance of the compositon being water, prepared according to U.S. Patent No. 3,998,750 issued on December 21, 1976. the detergents all possess aromas as described in Table XIII below:

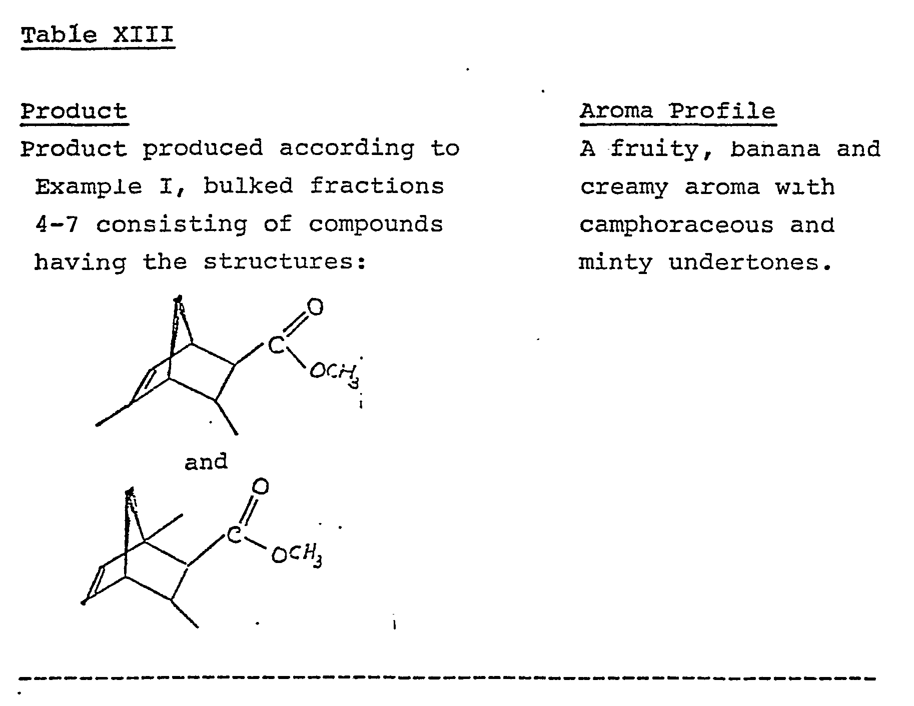

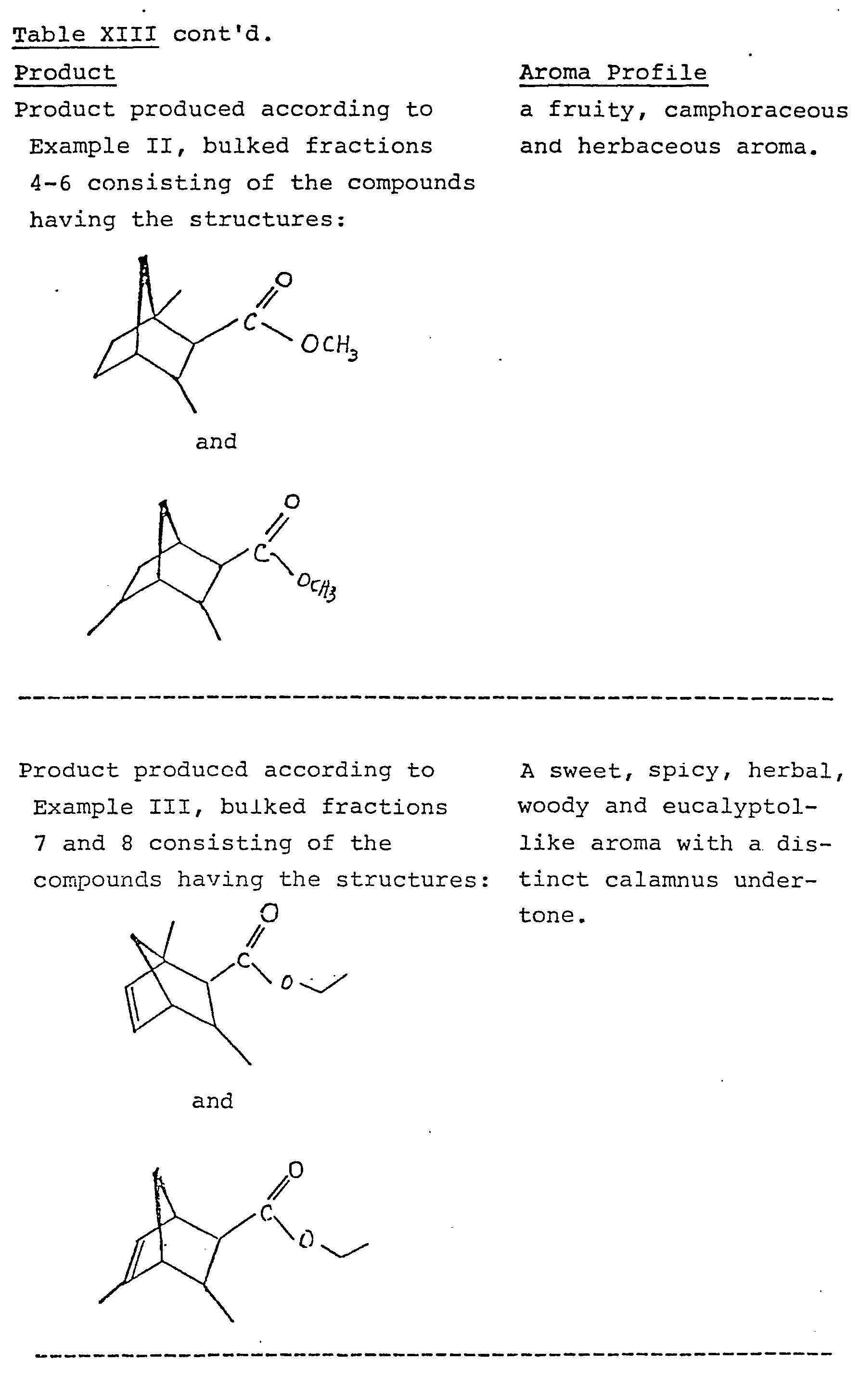

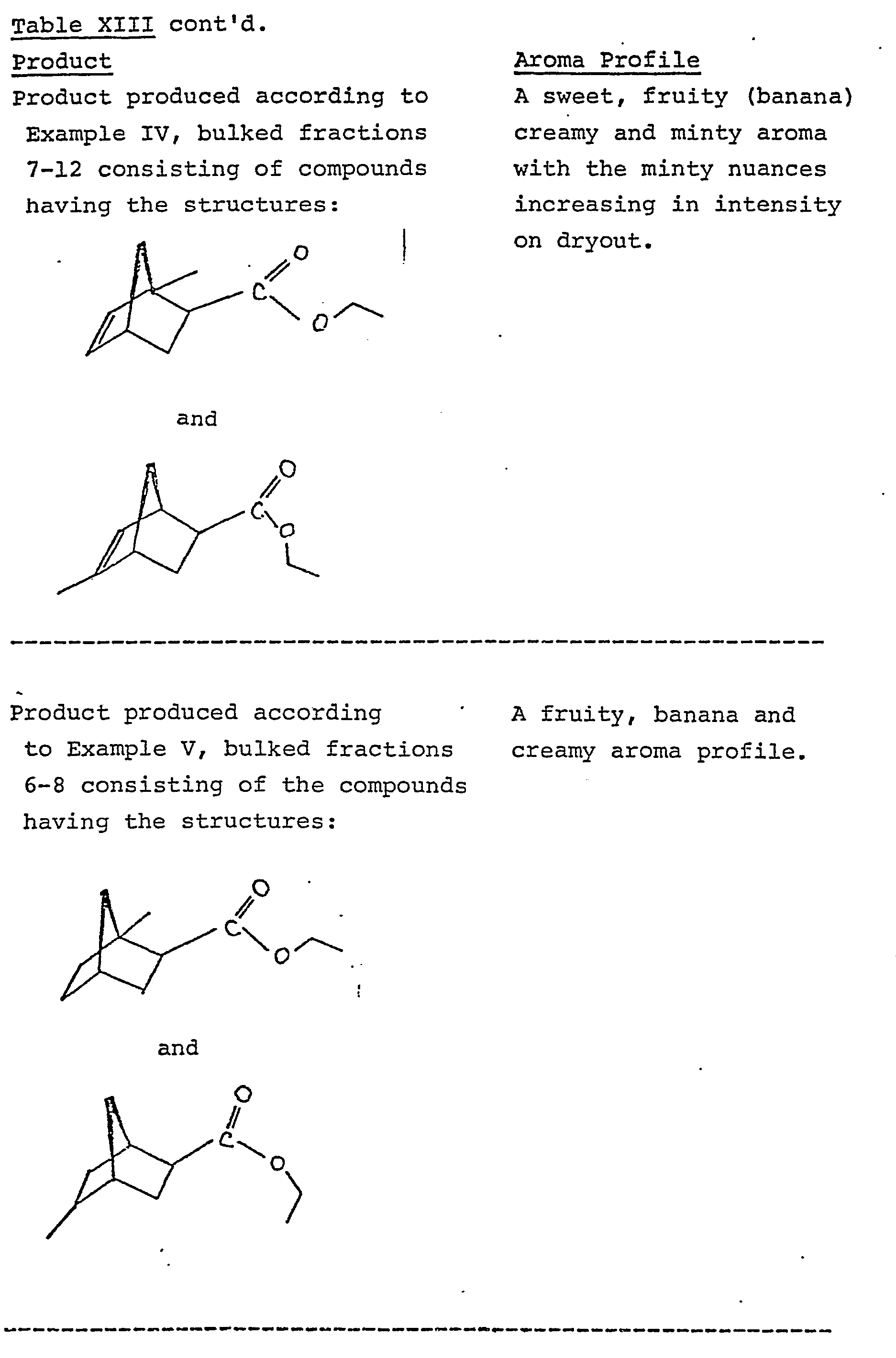

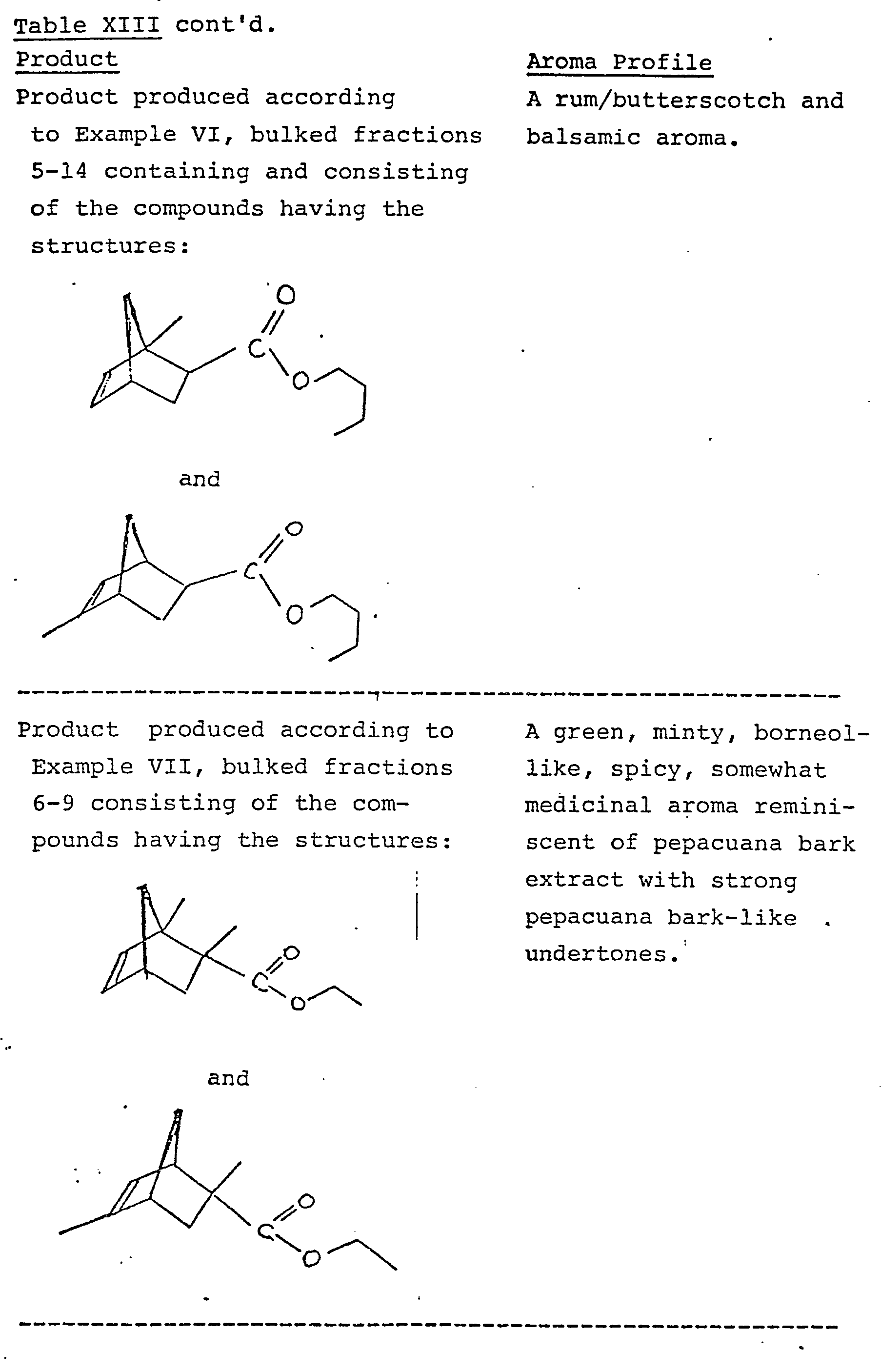

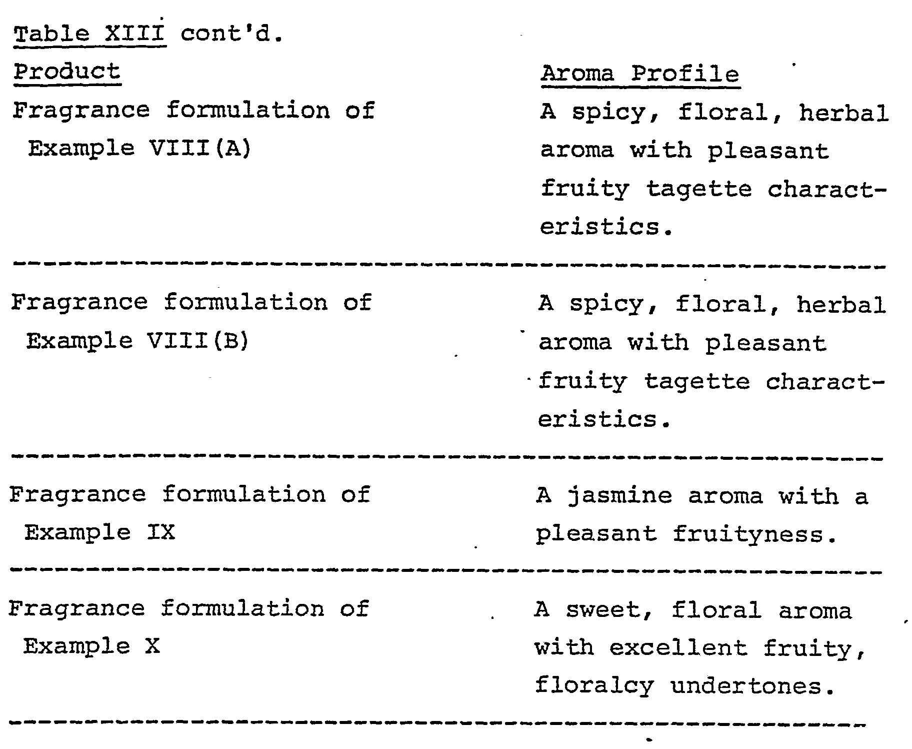

### Example XXXII

### Preparation of mixture of isopropyl esters of 5-methyl and 6-methyl-2-norbornane carboxylic acids (thermal non-catalytic Diels-Alder reaction)

Reaction:

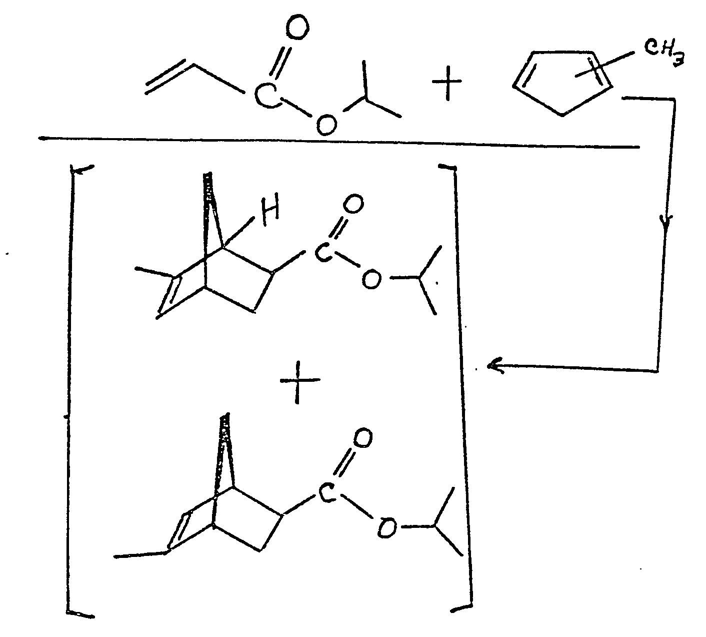

Into a 500 cc high pressure stainless steel autoclave equipped with shaker are placed 140 grams of methylcyclopentadiene dimer, 200 grams of isopropyl acrylate and 1 gram if Ionol ®. The autoclave is closed and heated to 200°C and maintained at 200°C for a period of 10 hours. At the end of the 10 hour period, the autoclave is opened and the contents cooled to room temperature.

The reaction mass is then distilled on a fractional distillation column yielding the following fractions:

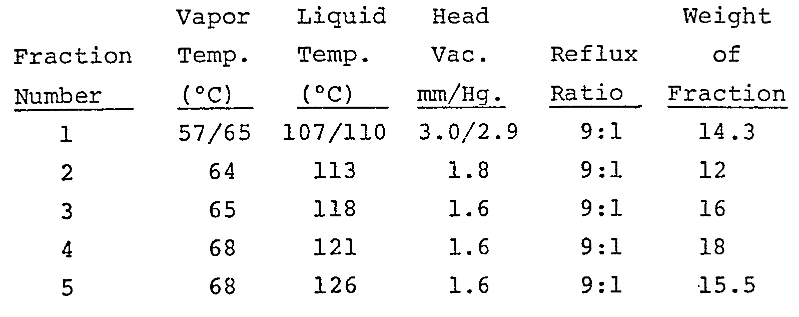

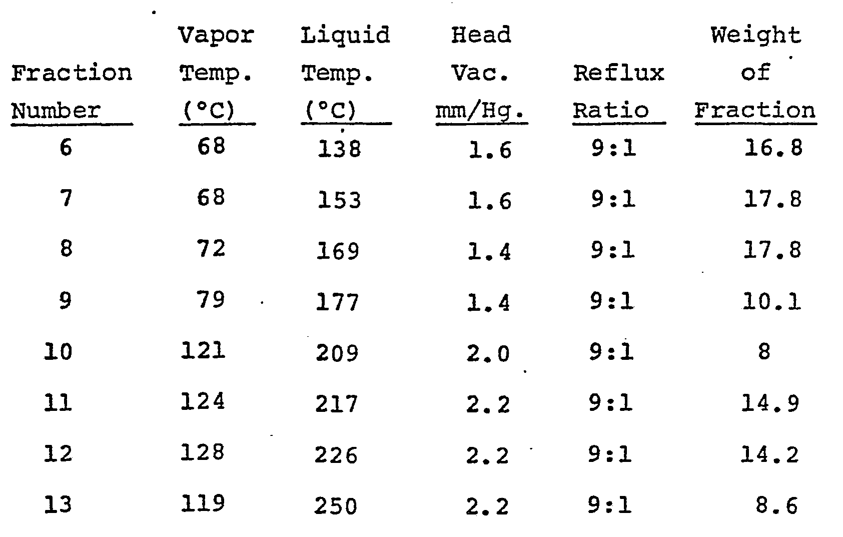

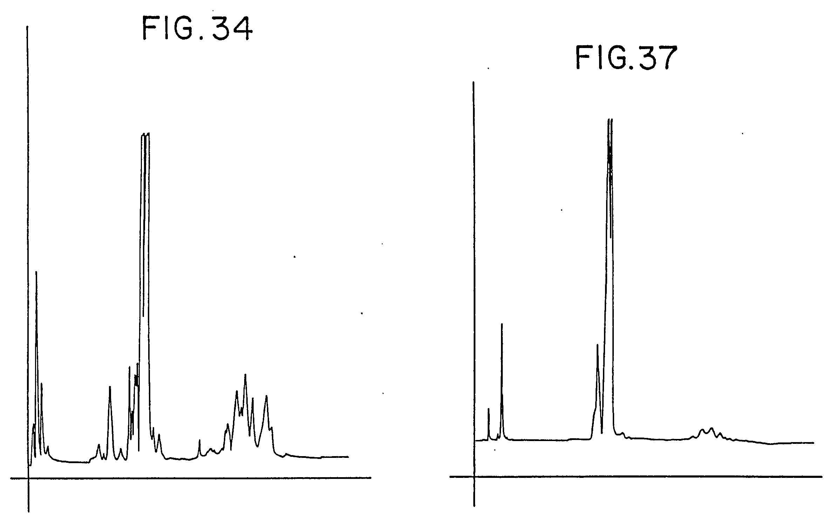

Figure 34 is the GLC profile of the reaction product prior to distillation (conditions: Carbowax column programmed at 80-220°C at 8°C per minute).

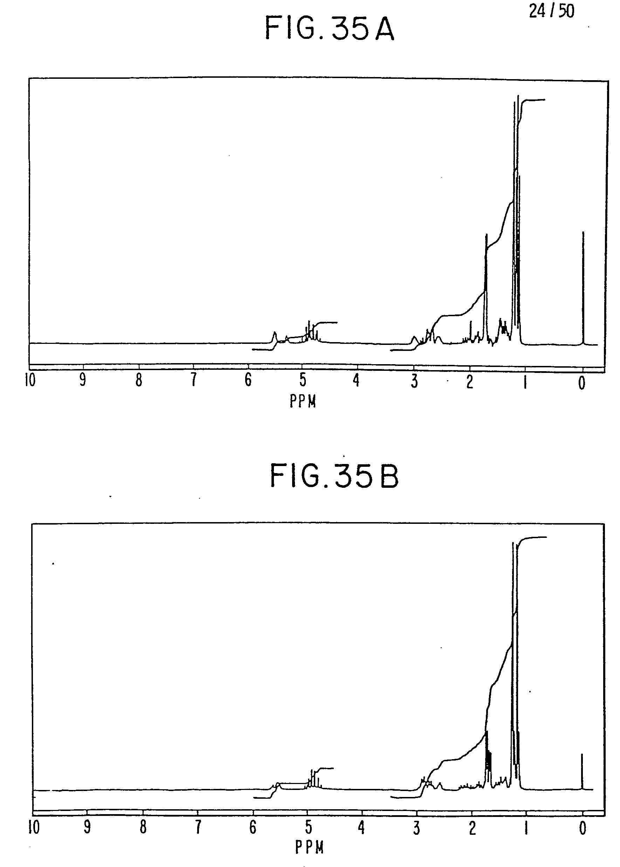

Figure 35-A is the NMR spectrum for Peak 1 of the GLC profile of Figure 34 containing the compound having the structure:

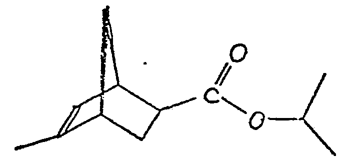

Figure 35-B is the NMR spectrum for Peak 2 of the GLC profile of Figure 34 containing the compound having the structure:

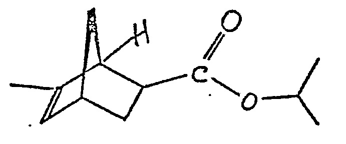

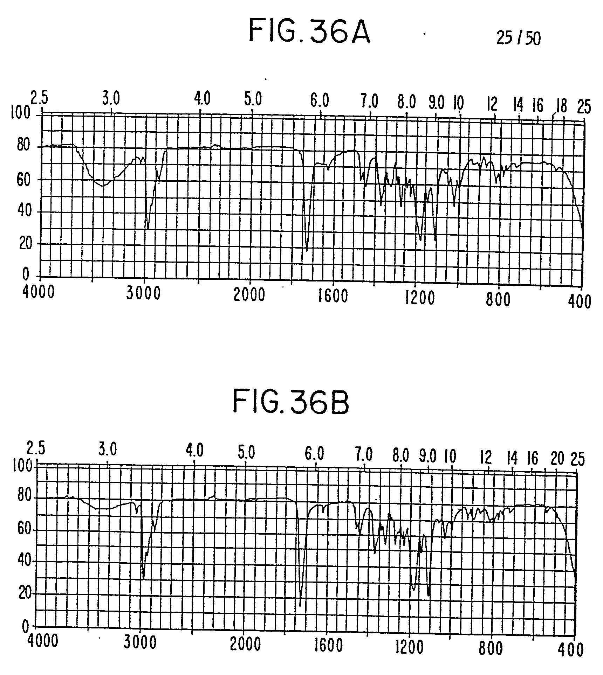

Figure 36-A is the infra-red spectrum for Peak 1 of the GL_{C} profile of Figure 34 containing the compound having the structure:

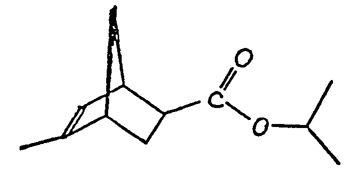

Figure 36-B is the infra-red spectrum for Peak 2 of the GLC profile of Figure 34 containing the compound having the structure:

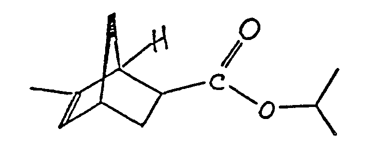

### Example XXXIII

### Preparation of ethyl ester of 1-; 4-; 5-; and 6-methyl-2-norbornane-carboxylic acids (non-catalytic thermal Diels-Alder reaction)

Reaction:

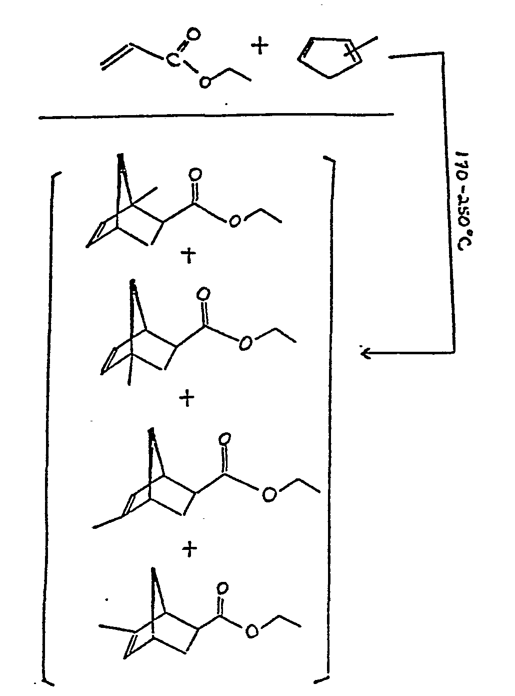

Into a 1 liter autoclave is placed 400 grams of methylcyclopentadiene dimer and 500 grams of ethyl acrylate. The autoclave is closed and the contents are heated with stirring to a temperature of 200°C and maintained at that temperature for a period of 7 hours. The autoclave pressure is 25 psig. At the end of the 7 hour period, the autoclave is depressurized and opened and the contents cooled to room temperature.

The contents of the autoclave are then distilled on a fractional distillation column yielding the following fractions:

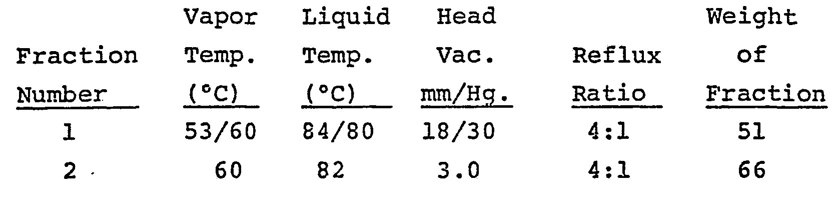

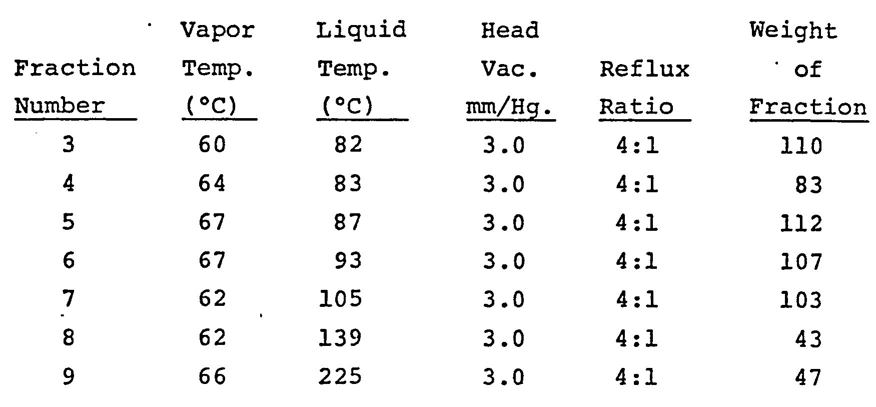

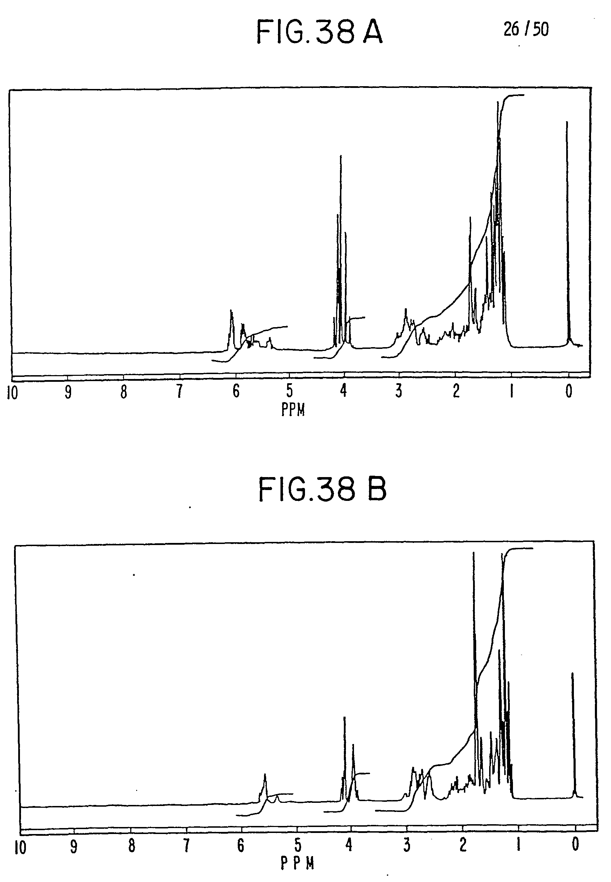

Figure 37 is the GLC profile of the reaction product prior to distillation. Conditions: programmed at 80-220°C at 8°C per minute: SE-30 column.

Figure 38-A is the NMR spectrum for Fraction 1, Peak 1 of the distillation product of the reaction product of Example XXXIII containing the compounds having the structures: . 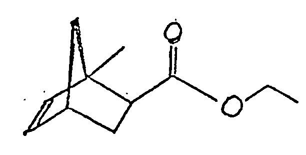 and

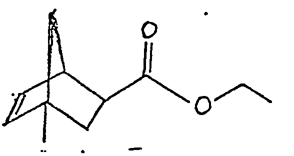

Figure 38-B is the NMR spectrum for Fraction 8, Peak 2 of the distillation product of the reacticn product of Example XXXIII containing the compounds having the structures: 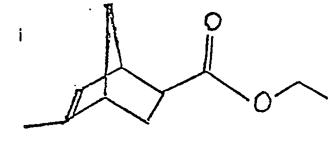 and

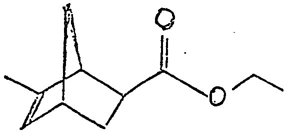

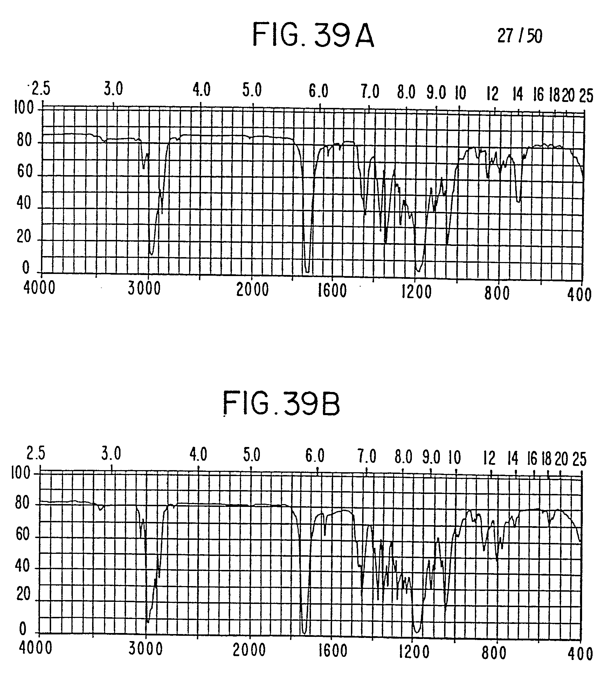

Figure 39-A is the infra-red spectrum for Fraction 1, Peak 1 of the distillation product of the reaction product of Example XXXIII containing the compounds having the structures: 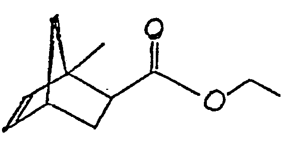 and

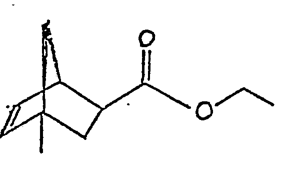

Figure 39-B is the infra-red spectrum for Fraction 8, Peak 2 of the distillation product of the reaction product of Example XXXIII containing the compounds having the structures: 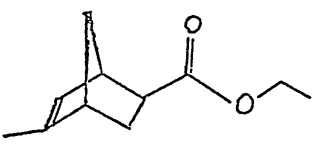 and

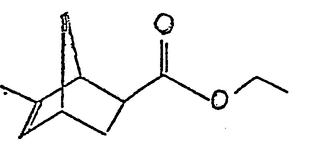

### Example XXXIV

Preparation of 1-; 4-; 5-; and 6-methyl norbornane carboxylic acid ethyl esters

Reaction:

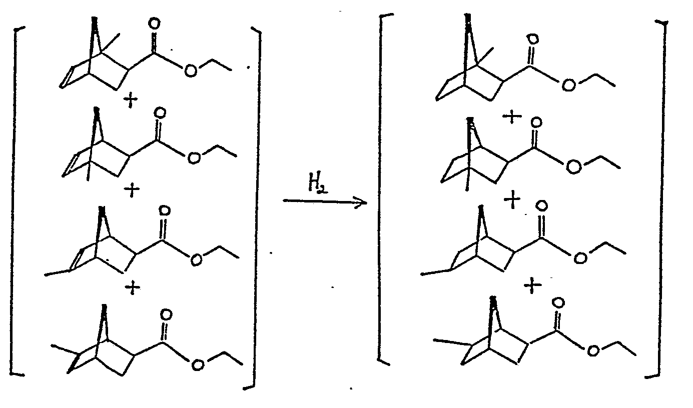

Into a high pressure bottle equipped with Parr shaker is placed 200 grams of the methyl cyclopentadiene-ethyl acrylate reaction product produced according to Example XXXIII; 50 ml isopropyl alcohol and 5 grams of Raney Nickel catalyst. The contents are purged with hydrogen and the bottle is then pressurized to 50 psig. While maintaining the hydrogen pressure at between 20 and 50 psig over a period of 13 hours, the reaction mass is shaken using the Parr shaker. At the end of the 13 hour period, the pressure bottle is opened and the reaction mass is distilled on a 12 inch Goodloe column yielding the following fractions:

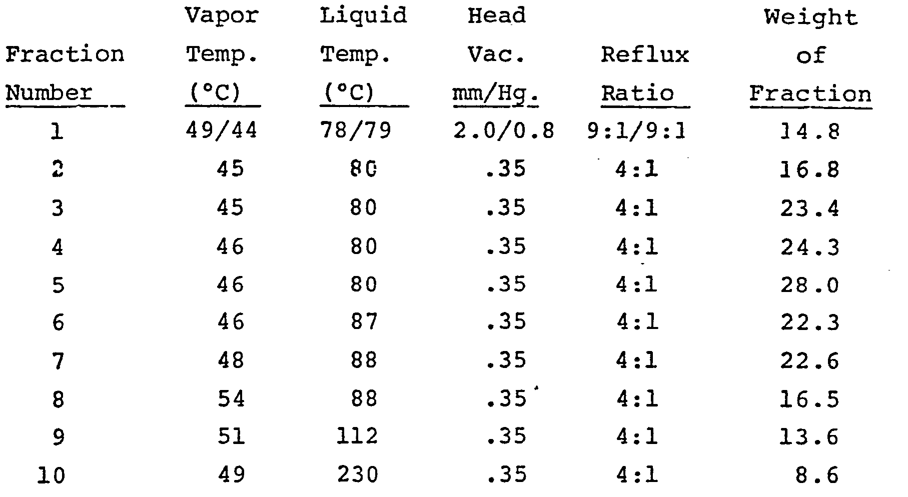

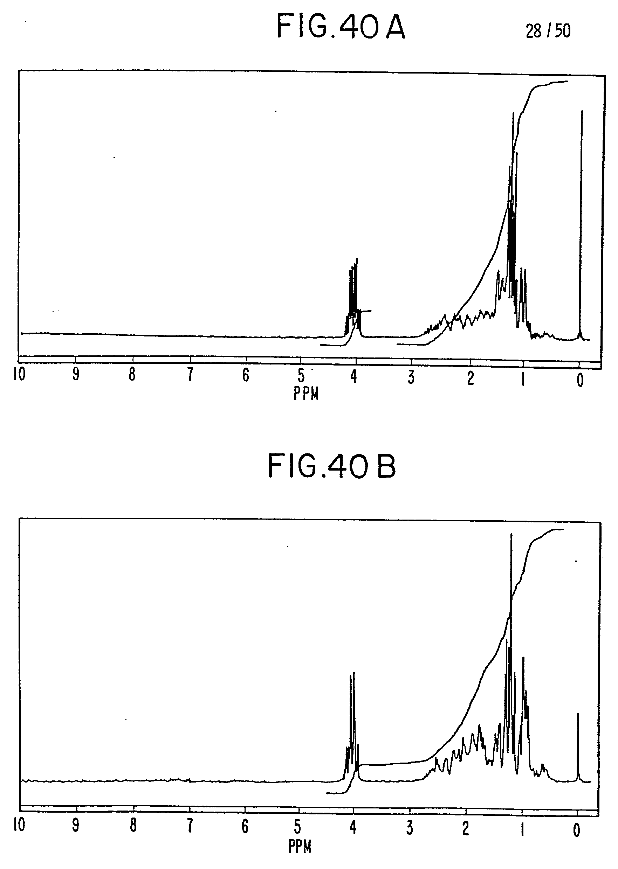

Figure 40-A is the NMR spectrum for Fract-on 2, Peak 1 of the distillation product of the reaction product of Example XXXIV containing the compounds having the structures: 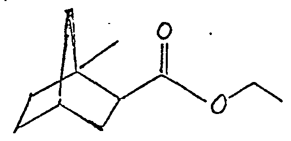 and

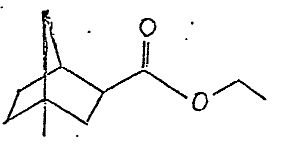

Figure 40-B is the NMR spectrum for Fraction 10, Peak 2 of the distillation product of the reaction product of Example XXXIV containing the compounds having the structures: 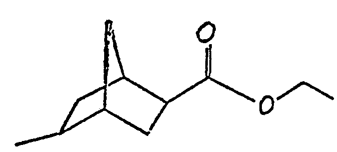 and

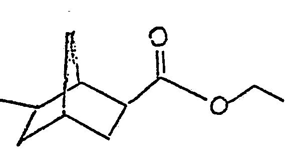

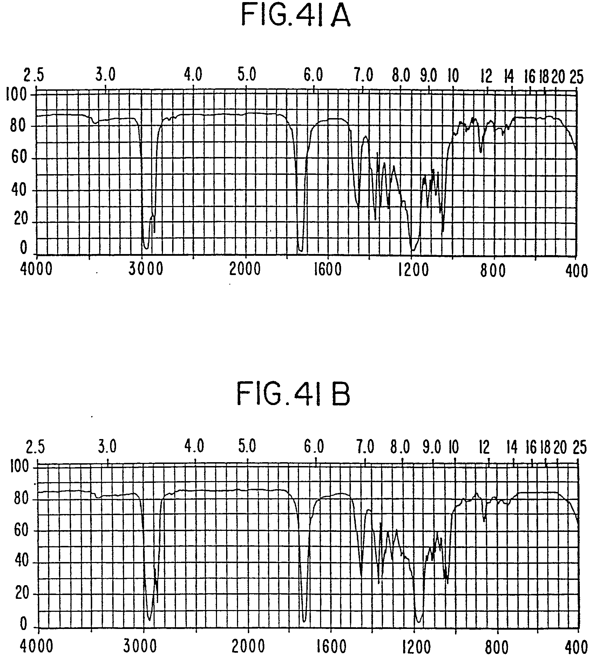

Figure 41-A is the infra-red spectrum for Fraction 2, Peak 1 of the distillation product of the reaction product of Example XXXIV containing the compounds having the structures: 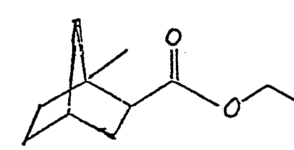 and

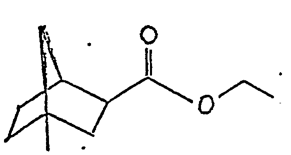

Figure 41-B is the infra-red spectrum for Fraction 10, Peak 2 of the distillation product of the reaction product of Example XXXIV containing the compounds having the structures: 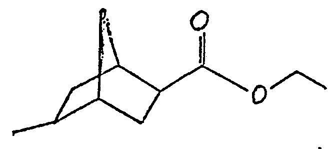 and

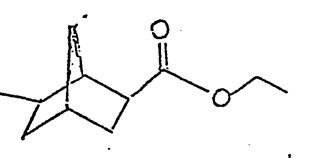

### Example XXXV

### Preparation of 3,5- and 3,6-dimethyl-2-norbornene carboxylic acid ethyl esters

Reaction:

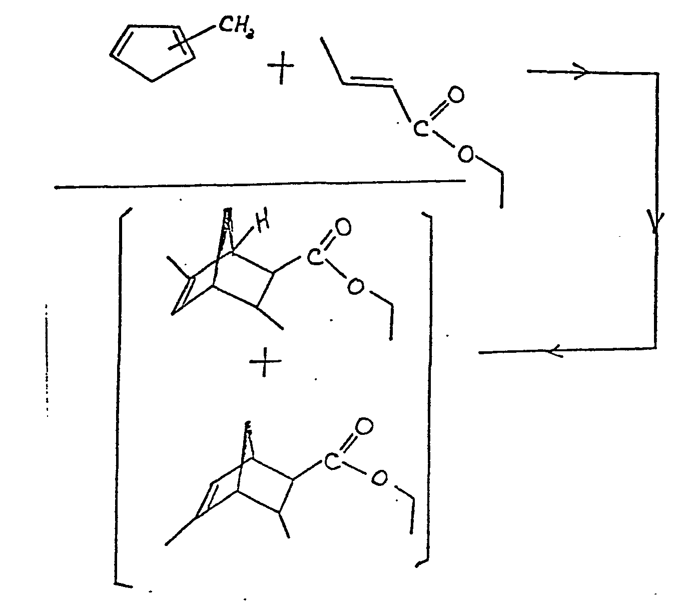

Into a 500 cc autoclave equipped for high pressure reactions is placed 175 grams of methyl cyclopentadiene dimer; 250 grams of ethyl crotonate and 1 gram of Ionol ®. The autoclave is closed and with stirring heated to 200°C and maintained at 200°C under pressure for a period of 10 hours. The contents of the autoclave are then cooled to room temperature and the autoclave is opened. The autoclave is then emptied of its contents and the contents are then distilled in an 18" silver column with Goodloe packing yielding the following fractions:

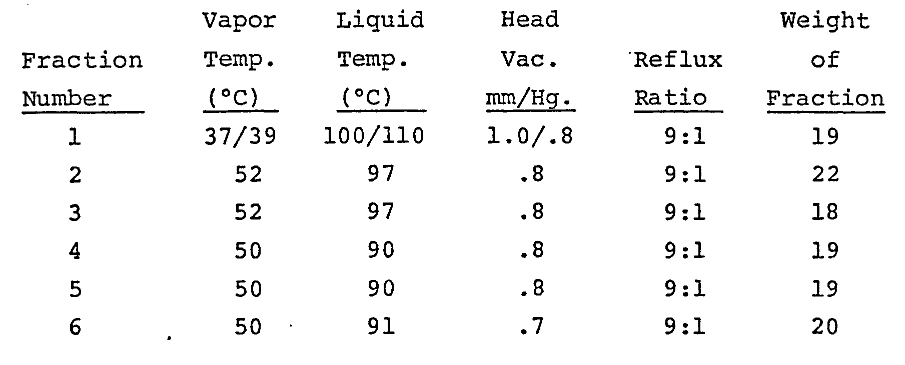

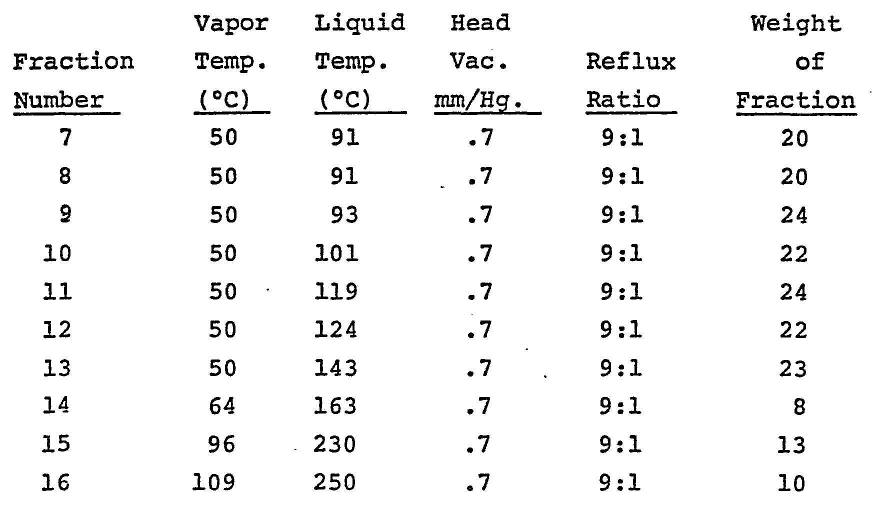

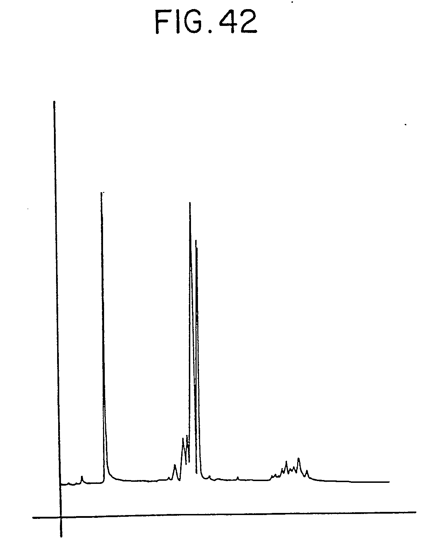

Figure 42 is the GLC profile of the reaction product prior to distillation.

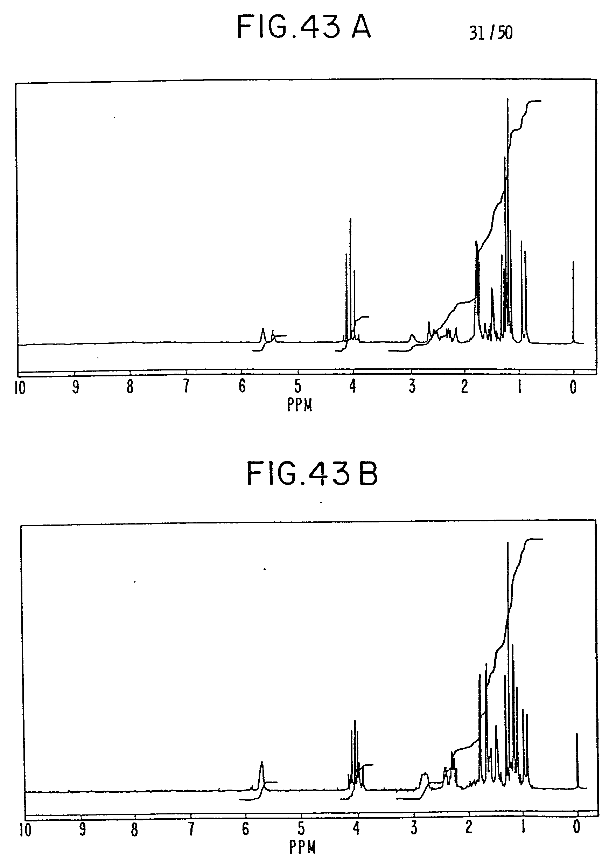

Figure 43-A is the NMR spectrum for Peak 1 of the GL_{C} profile of Figure 42 of the reaction product of Example XXXV containing the compound having the structure:

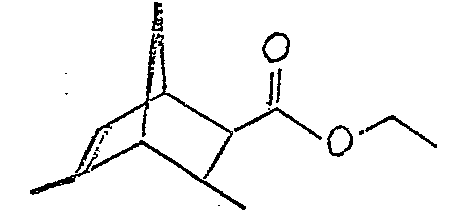

Figure 43-B is the NMR spectrum for Peak 2 of the GLC profile of Figure 42 for the reaction product of Example XXXV containing the compound having the structure:

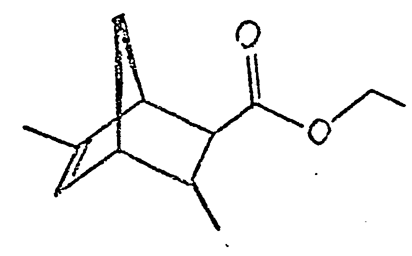

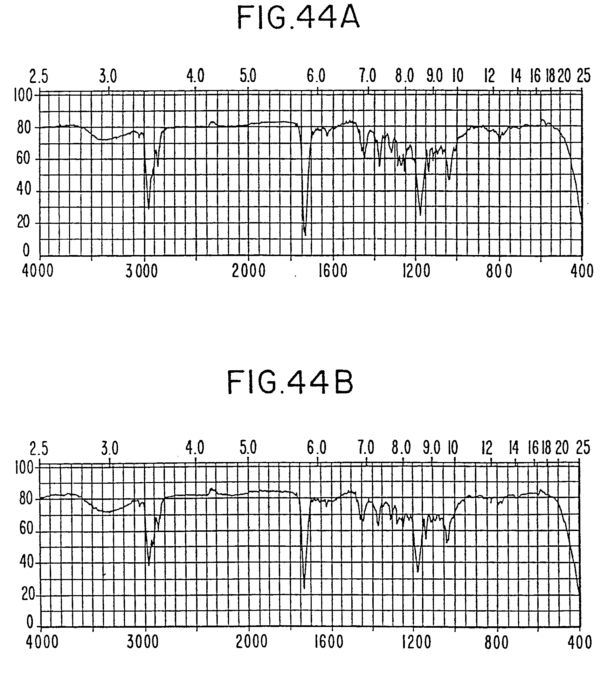

Figure 44-A is the infra-red spectrum for Peak 1 of the GLC profile of Figure 42 of the reaction product of Example XXXV containing the compound having the structure:

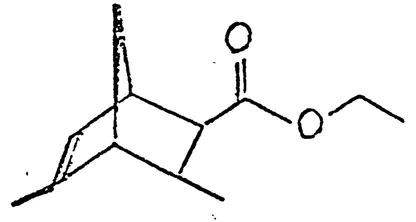

Figure 44-B is the infra-red spectrum for Peak 2 of the GLC profile of Figure 42 of the reaction product of Example XXXV containing the compound having the structure:

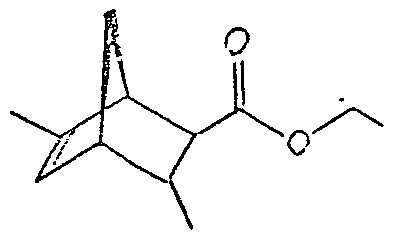

### Example XXXV(A)

### Preparation of a detergent composition

A total of 100 grams of detergent powder (a low phosphate content detergent composition which contains 12% by weight phosphate builder, 8% hardness mineral ion insensitive detergent, 0.9% by weight maleic anhydride-vinyl compound co-polymer and 2% alkylene oxide condensation product prepared according to Example IV at column XI, of U.S. Patent No. 4,000,080 issued on December 28, 1976) is intimately admixed with 0.15 grams of one of the perfume materials of Table XIV below until a substantially homogeneous composition is obtained. This composition has an aroma as set forth in Table XIV below which is pleasant and long-lasting:

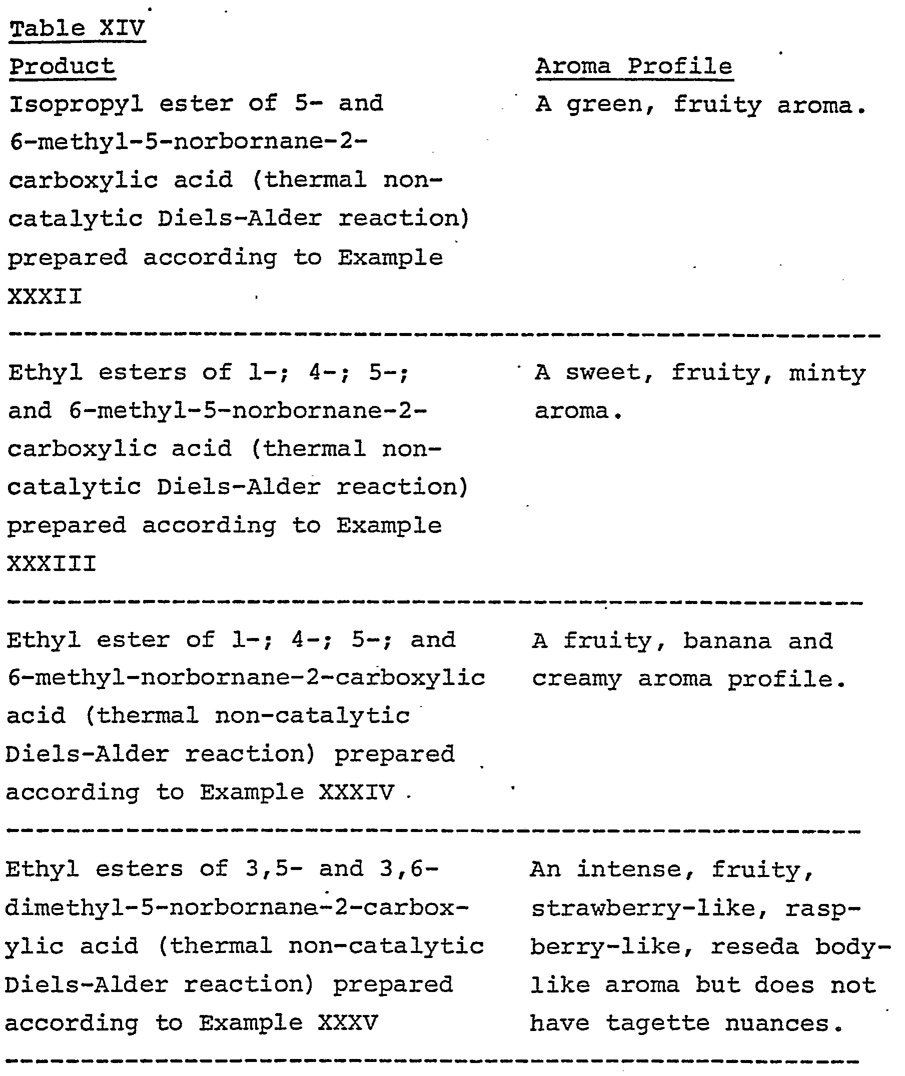

### Example XXXV(B)

### Perfumed liquid detergents

Concentrated liquid detergents with aromas as set forth in Table XV below are prepared containing 0.10%, 0.15% and 0.20% of the perfume ingredients set forth in Table XV below. They are prepared by adding and homogeneously mixing the appropriate quantity of perfume ingredient set forth in Table XV below in a liquid detergent which is a homogeneous single-phase heavy duty liquid detergent composition containing:
a. 12.5% by weight based on the free acid form of an anionic detersive surfactant;
b. 0.5% magnesium sulfate;
c. 12% by weight of an ethoxylated nonionic detersive surfactant;
d. 3% by weight of a water-soluble bis(styrylsulfon- ate)-biphenyl birghtener; and
e. the balance of the composition being water, prepared according to U.S. Patent No. 3,998,750 issued on December 21, 1976. The detergents all possess aromas as described in Table XV below:

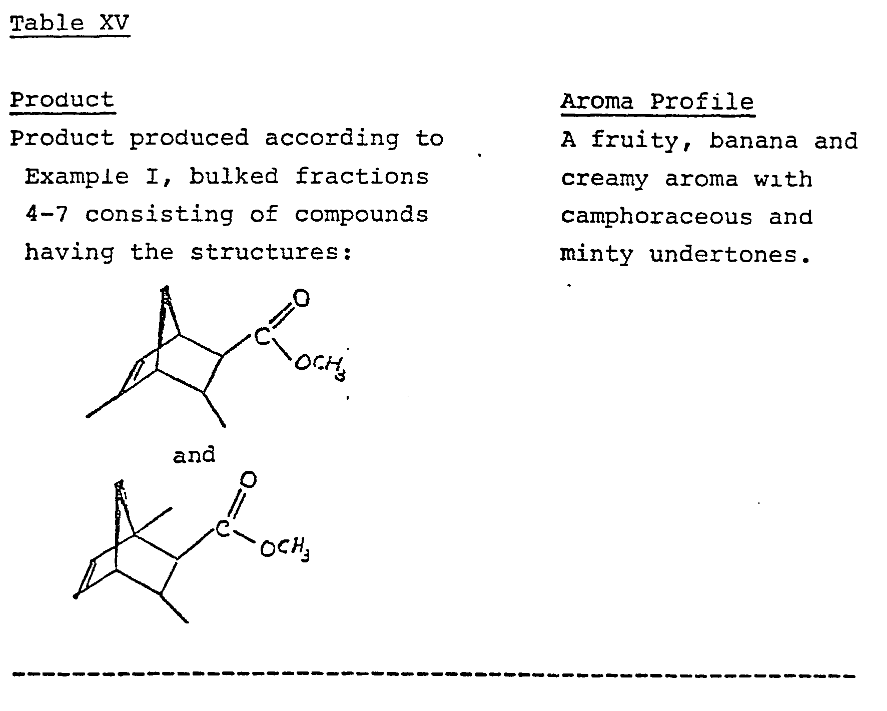

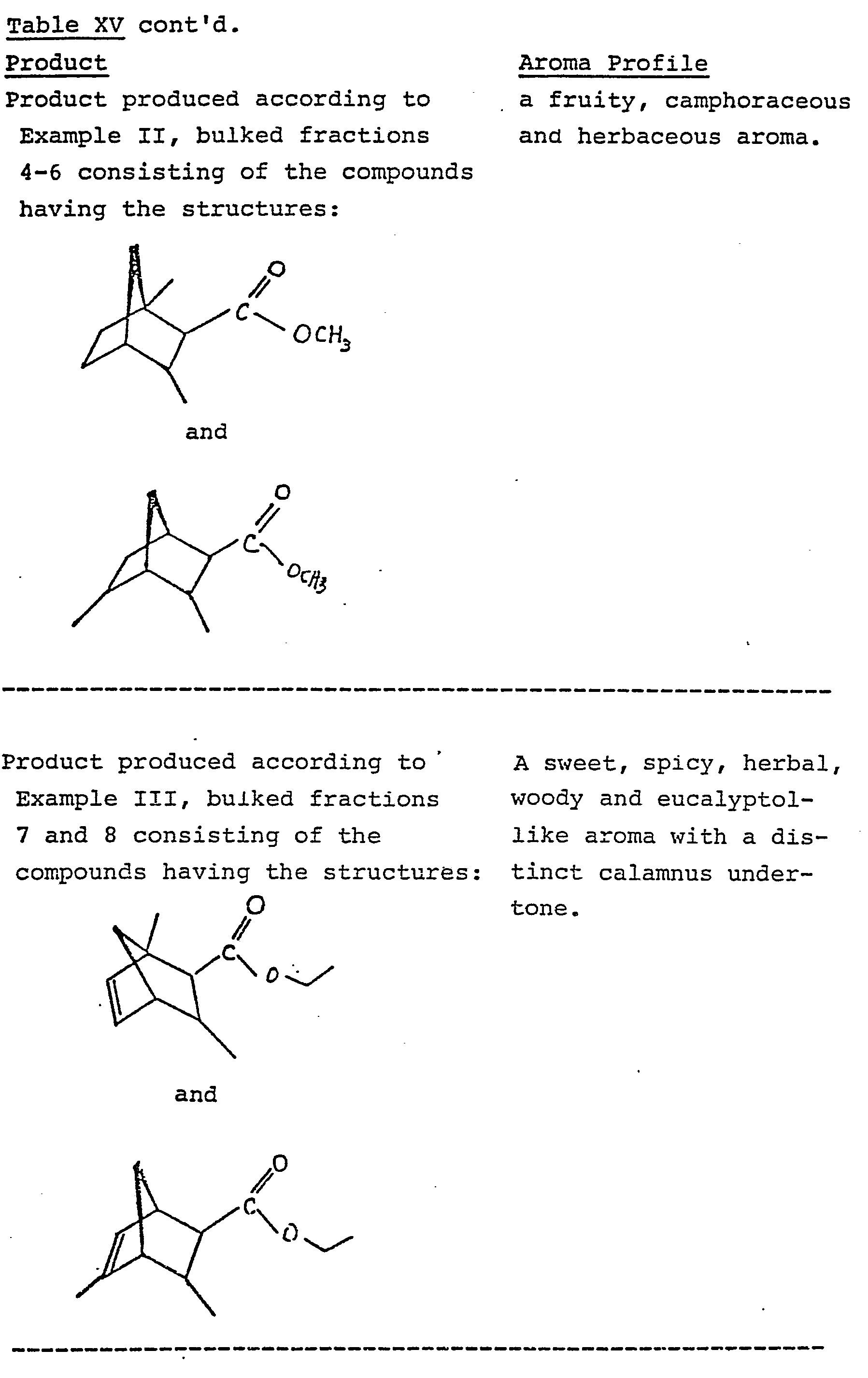

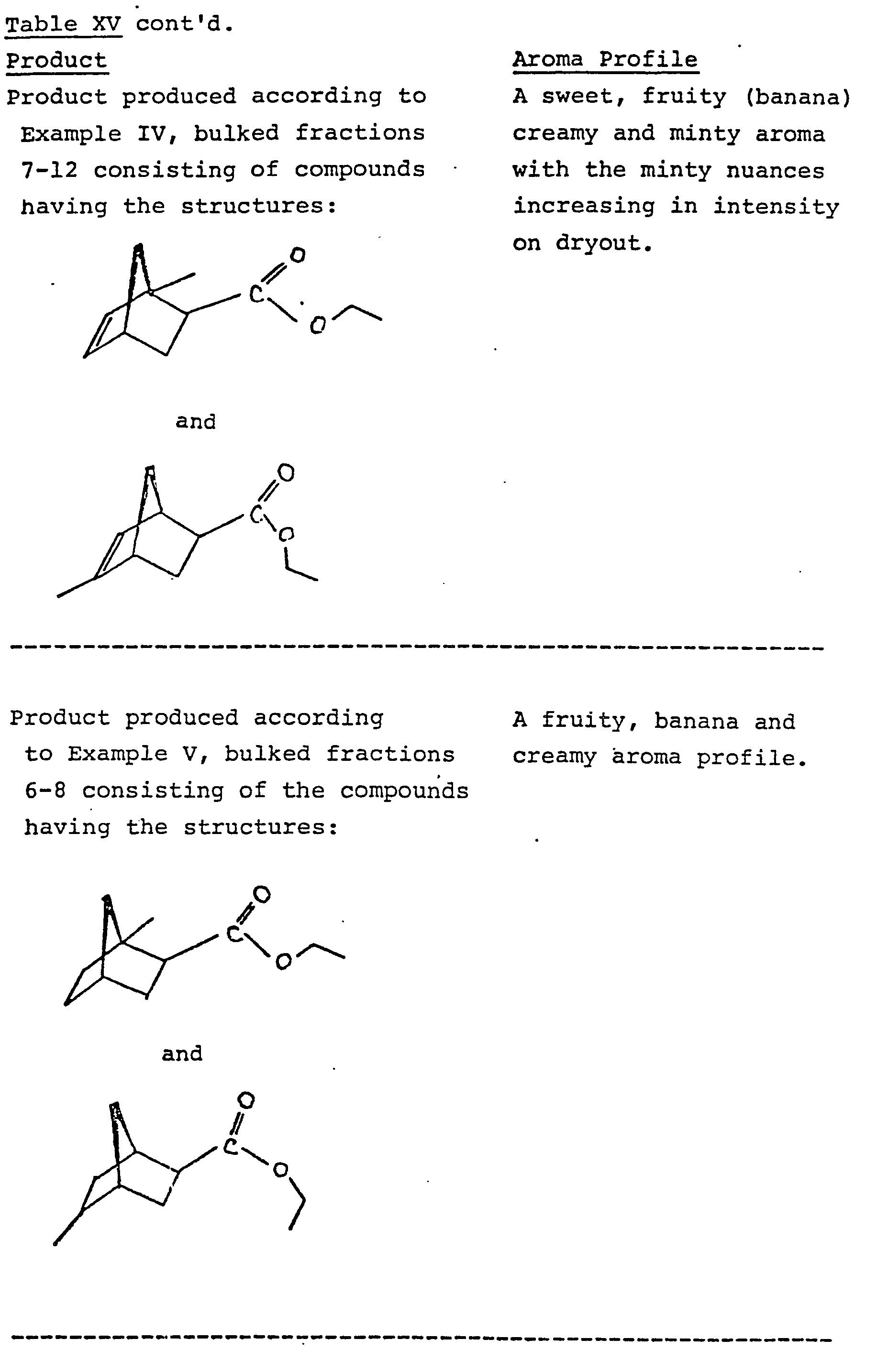

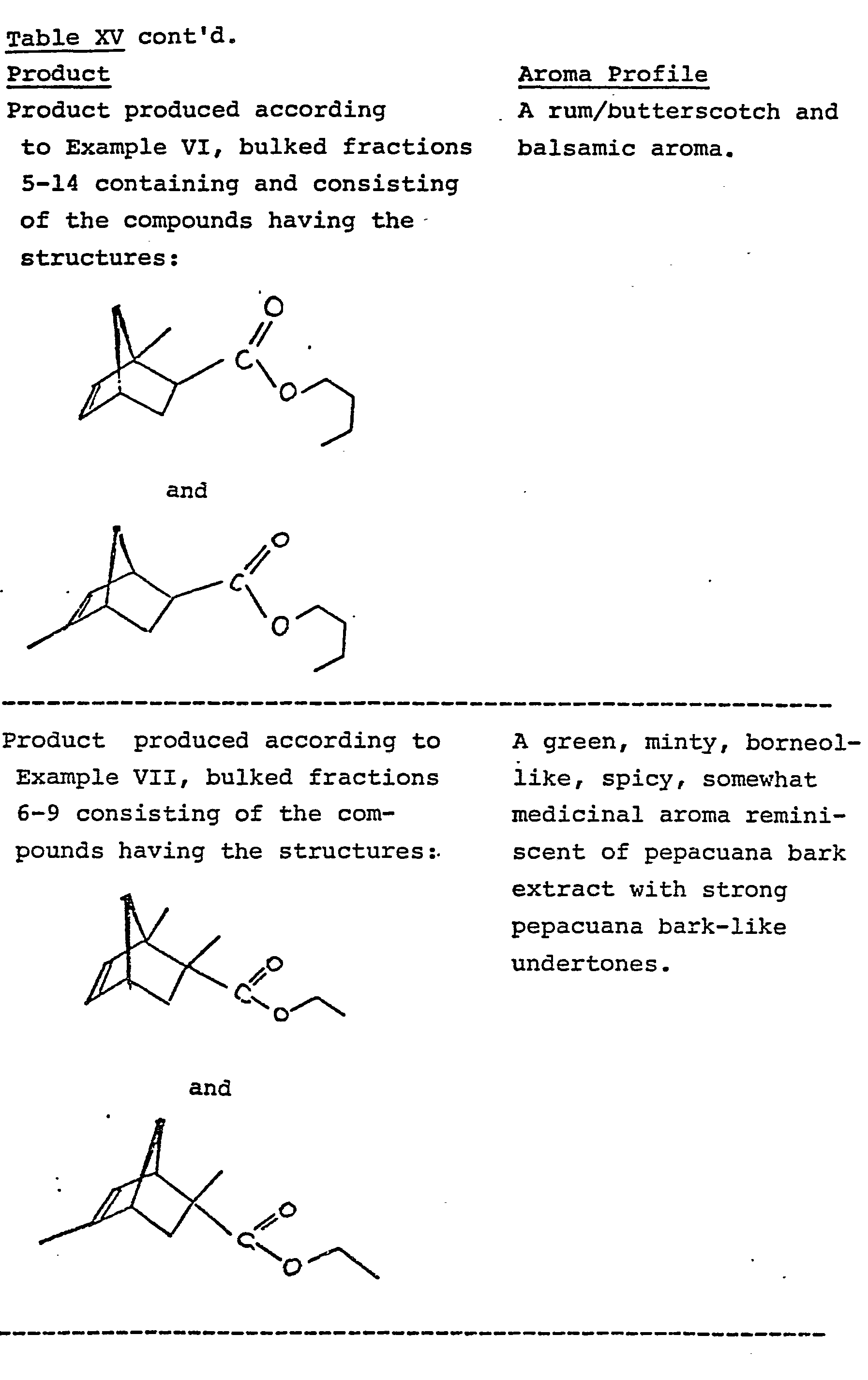

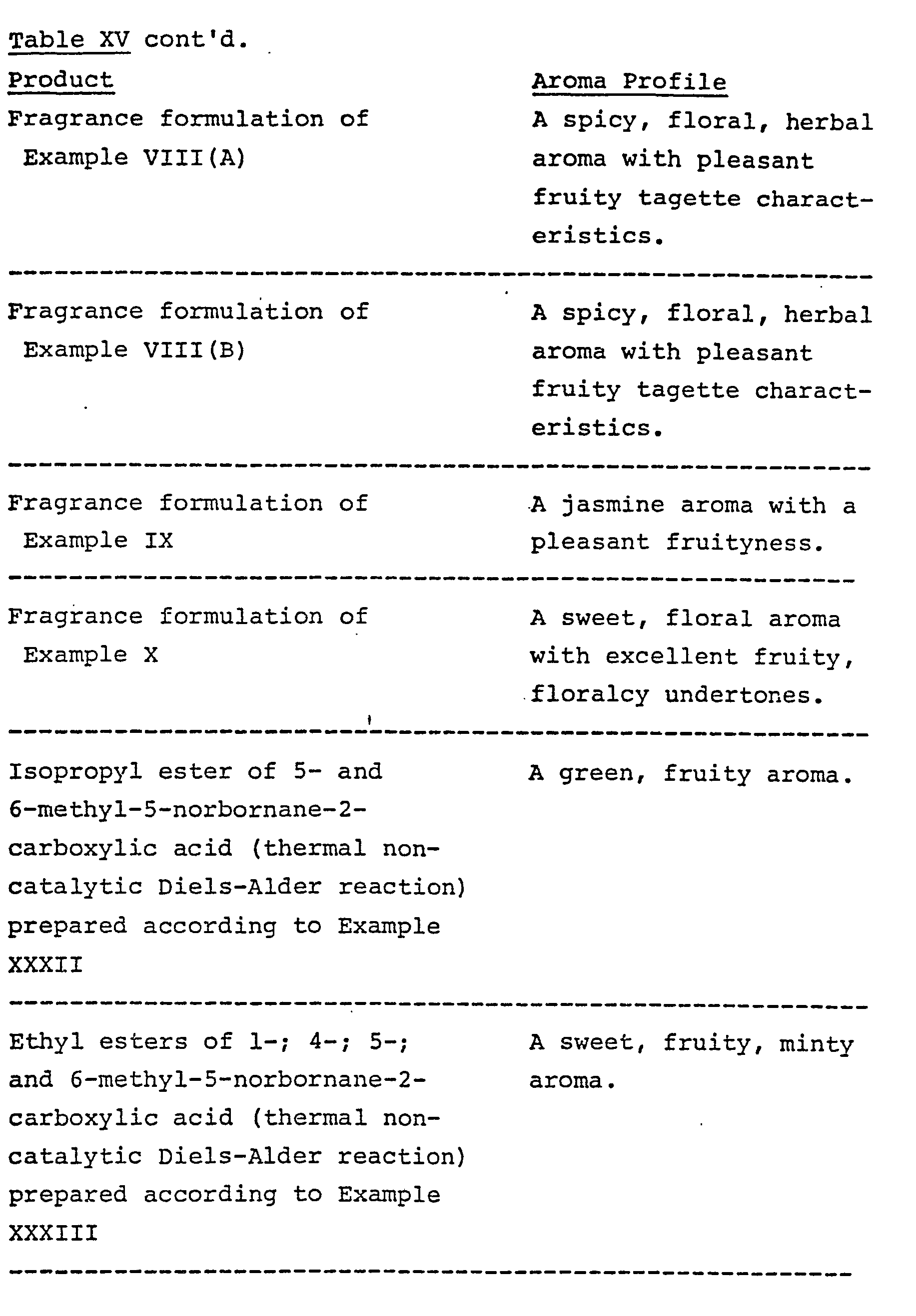

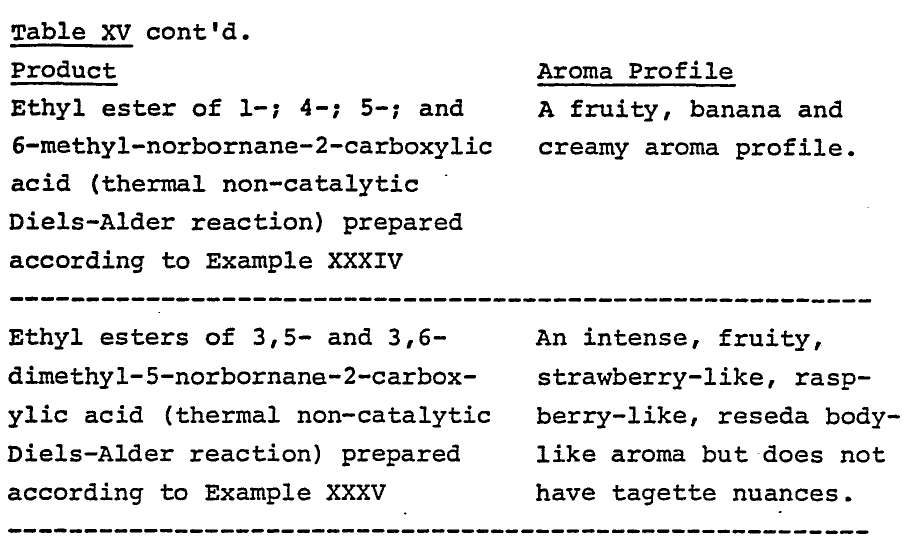

### Example XXXVI

### Preparation of 1,3 and 3,5-dimethyl-5-norbornene carboxaldehyde reaction

Reaction:

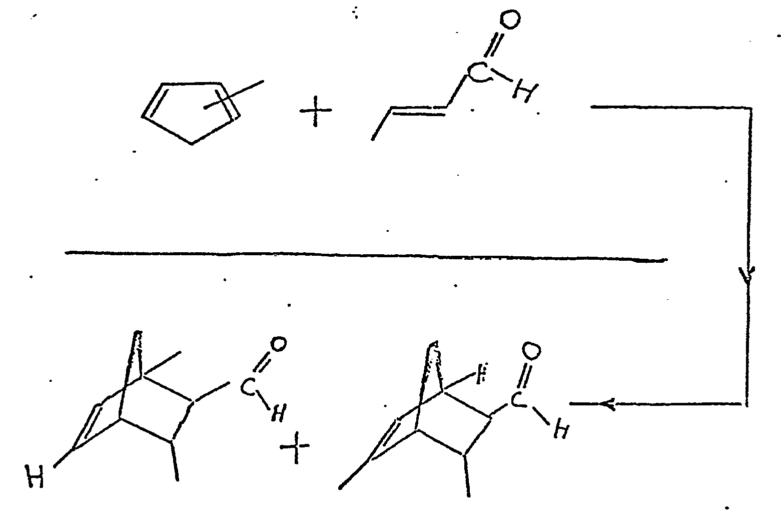

Into a 3 liter Morton flask equipped with stirrer, thermometer, condenser, bidwell, trap, addition funnel, heating mantle and cooling bath is placed one liter of toluene and 500 grams of 85% crotonaldehyde. the resulting mixture is heated to reflux to remove the water and the resultant material is then cooled to 20°-25°C over a period of 20 minutes while maintaining the mixture temperature at 23°C, 51 grams of ethyl aluminum dichloride is added. While maintaining the reaction temperature over a range of 22°-25°C over a period of twenty minutes, 400 grams of a 50% solution of l-methyl-l,3-cyclopentadiene and 2-methyl-1,3-cyclopentadiene (produced according to Example A) in toluene is added to the reaction mass. At the end of another twenty-five minutes, while maintaining the reaction temperature at 22°C, GLC analysis indicates that the reaction is complete.

The reaction mass is then poured into one liter of hydrochloric acid. the reaction mass now exists in two phases; an aqueous phase and an organic phase. The aqueous phase is extracted with 500 ml of toluene and the organic phase and toluene extracts are combined. The resulting organic material is washed as follows:
A - one 1-liter water portion;
B - one 1-liter saturated sodium bicarbonate portion;
C - two 1-liter portions of water,

and resulting mixture has a pH of 6. The solvent is then stripped off under vacuum and then fractionally distilled on a 12" Goodloe column yielding the following fractions:

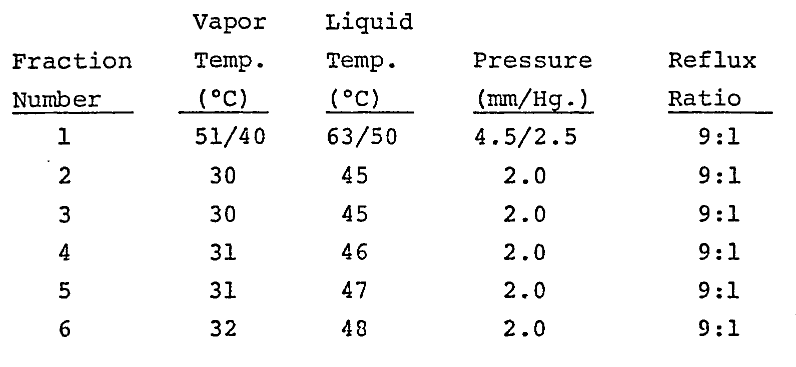

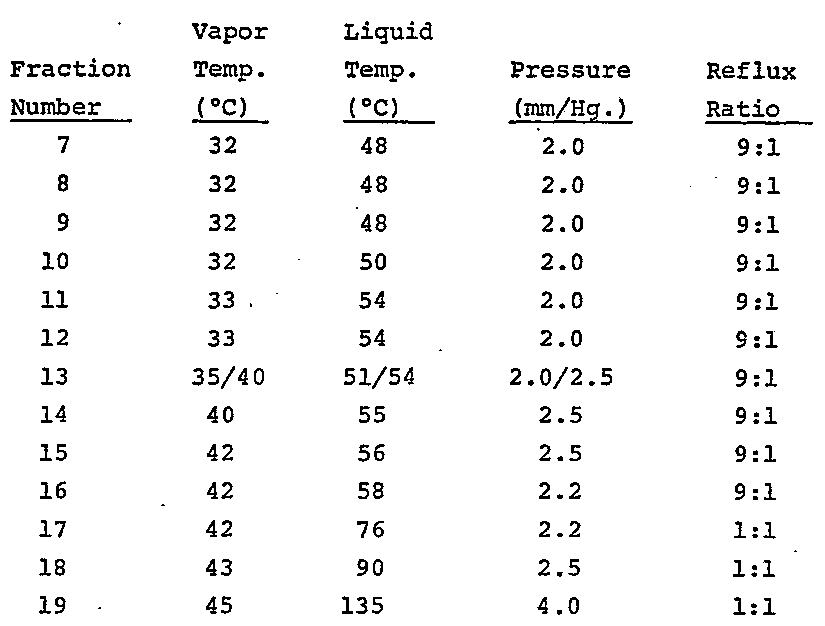

NMR, IR and mass spectral analysis yield the information that the resulting distillate consists of the compounds having the structures: 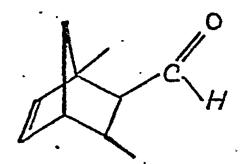 and

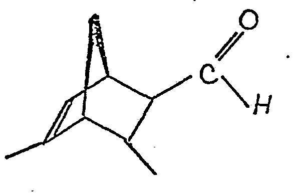

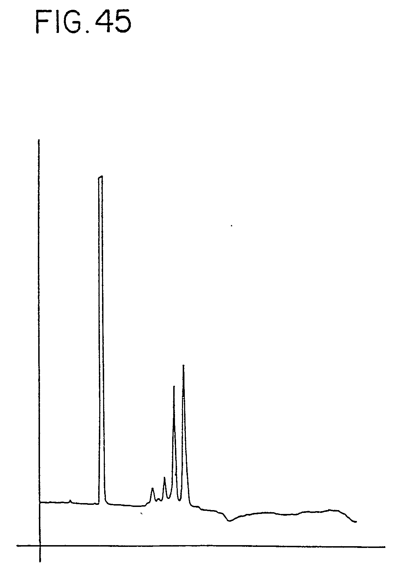

Figure 45 represents the GLC profile of the reaction product after thirty minutes of reaction.

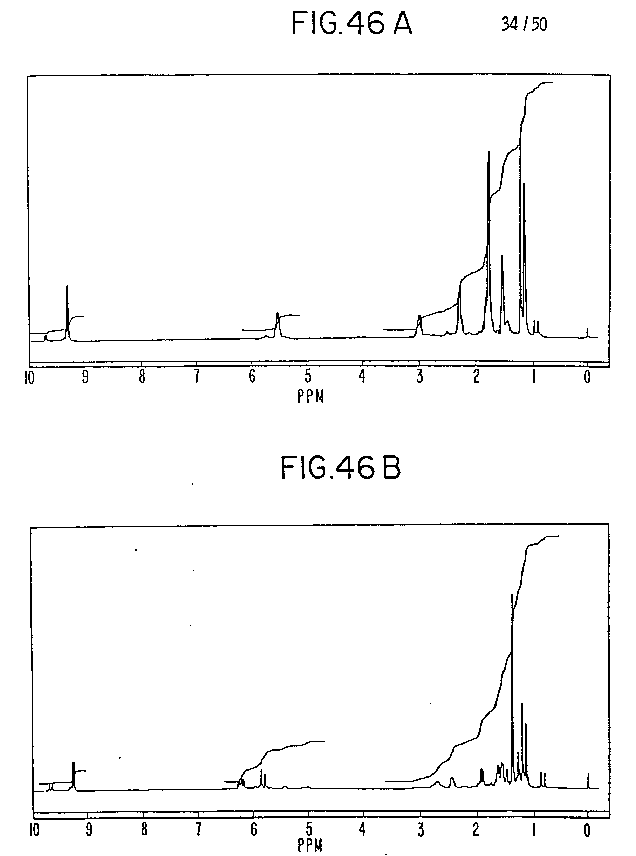

Figure 46A represents the NMR spectrum for fraction 19 of the foregoing distillation product of the reaction product of this example consisting of the compound having the structure:

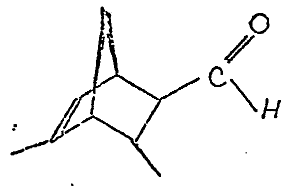

Figure 46B represents the NMR spectrum for fraction 3 of the distillation product of the foregoing distillation consisting of the compound having the structure:

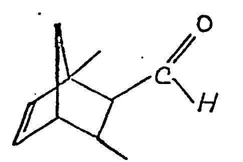

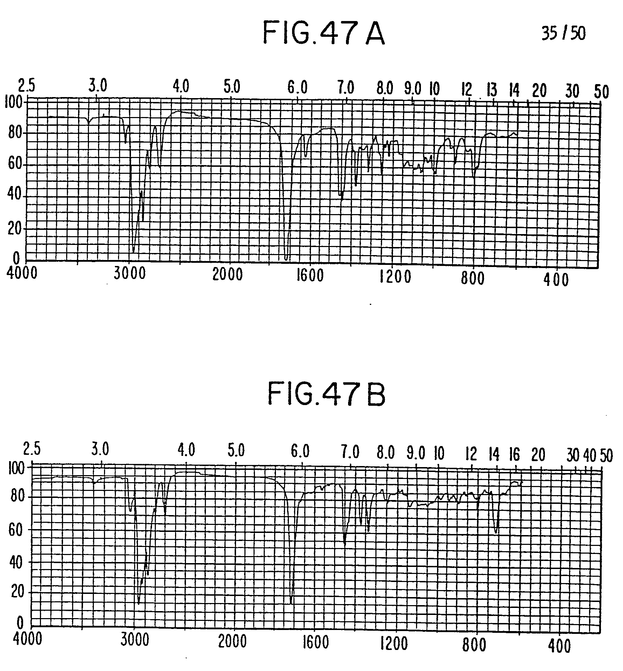

Figure 47A represents the infra-red spectrum for fraction 19 of the foregoing distillation consisting of the compound having the structure:

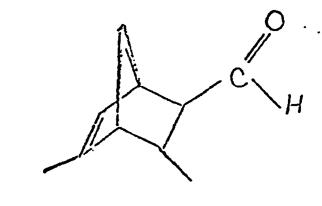

Figure 47B represents the infra-red spectrum for fraction 3 of the foregoing distillation consisting of the compound having the structure:

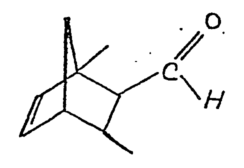

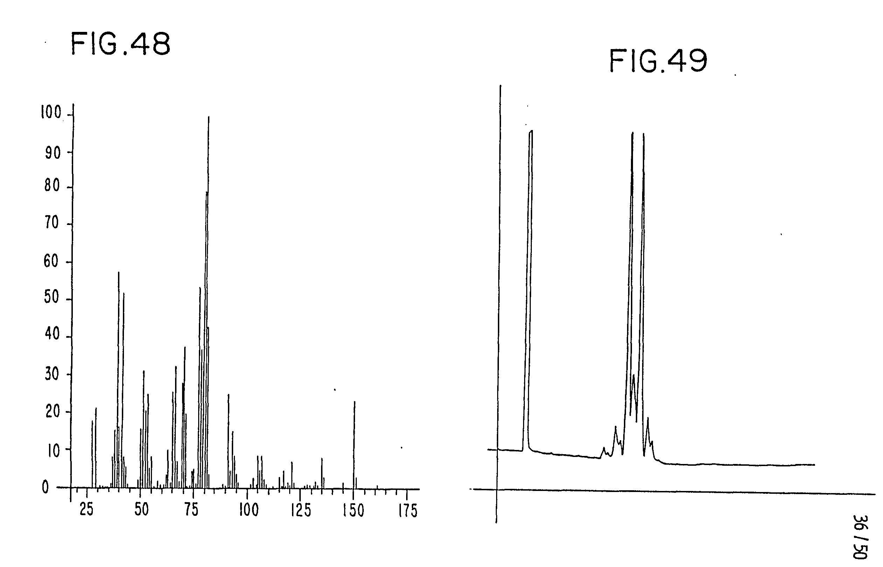

Figure 48 represents the mass spectrum of the reaction product consisting of the compounds having the structures: 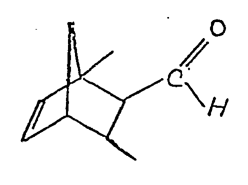 and

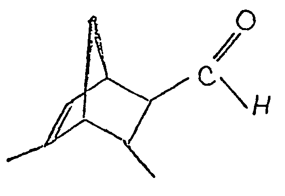

### Example XXXVII

### Preparation of 1,3- and 3,5- dimethyl-5-norbornane carboxaldehyde

Reaction:

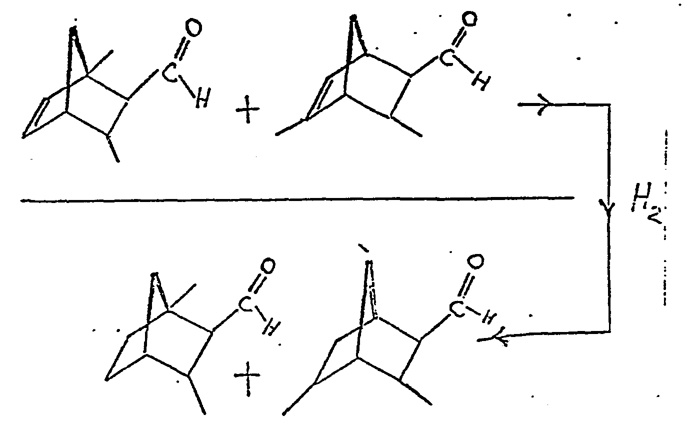

Into a 500 ml pressure bottle on a Parr shaker is placed 150 grams of the mixture of 1,3- and 3,5-dimethyl-5-norbornane carboxaldehyde prepared according to Example XXXVI (bulked fractions 1-19) and 100 ml isopropyl alcohol and 1 gram of 5% (weight) palladium on carbon.

The 500 ml pressure bottle is purged six times with hydrogen and then closed and pressurized to 50 lbs./psig with hydrogen. Reaction is continued while maintaining the pressure at 50 psig until hydrogen absorption ceases (5.5 hours). A total pressured drop of 71 psig of hydrogen is observed from the hydrogen reservoir. The resulting reaction mass is complete as shown by NMR analysis. The resulting solution is then filtered after opening the Parr shaker and pressure bottle. The solvent is stripped off and accrued material is distilled to yield 122 grams (80% theoretical yield) of a product consisting of compounds having the structures: 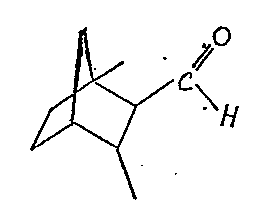 and 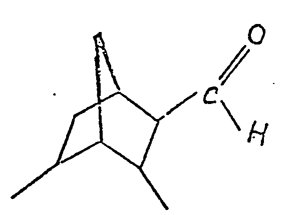 the resulting product is then distilled on a 3 foot spinning band column to yield the following fractions:

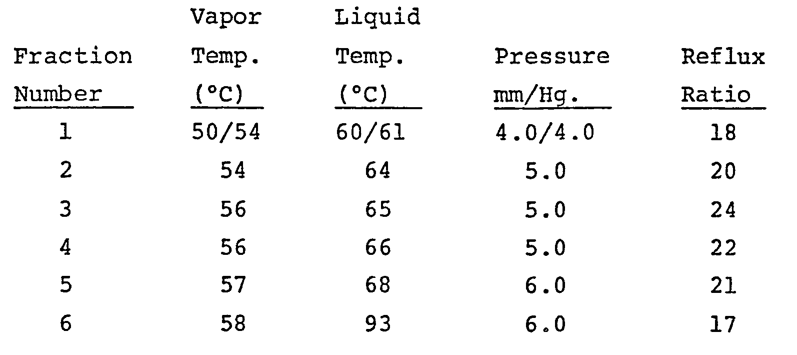

Figure 49 represents the GLC profile for the crude reaction mass.

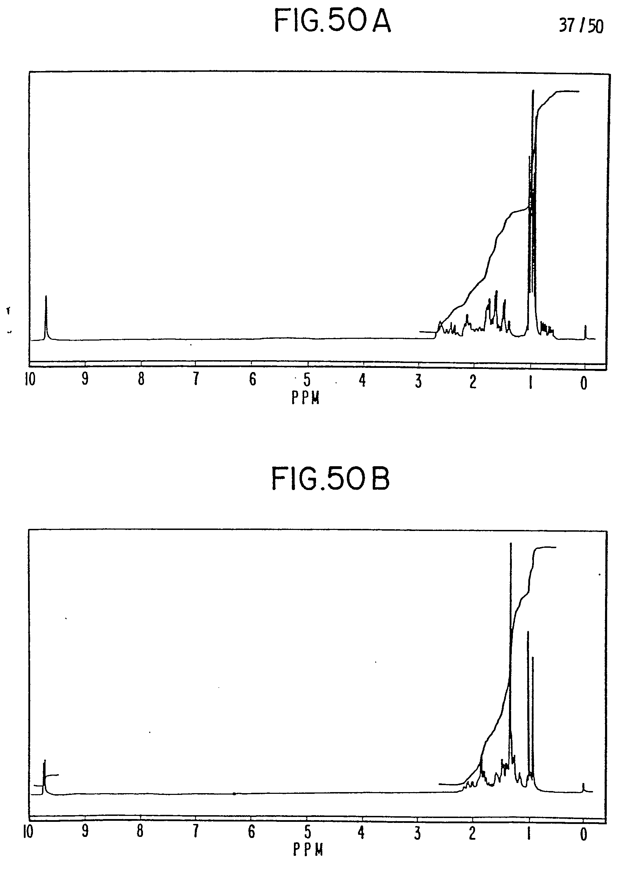

Figure 50A represents the NMR spectrum for peak 2 of the foregoing GLC profile consisting of the compound having the structure:

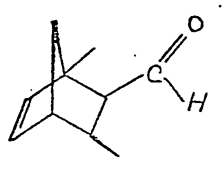

Figure 50B represents the NMR spectrum for peak 1 of the foregoing GLC profile consisting of the compound having the structure:

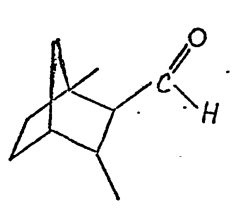

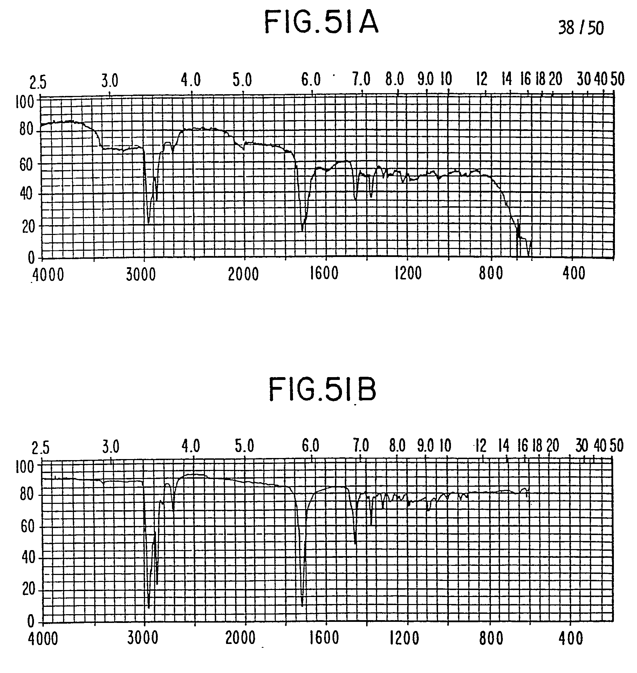

Figure 51A represents the infra-red spectrum for peak 2 of the foregoing GLC profile consisting of the compound having the structure:

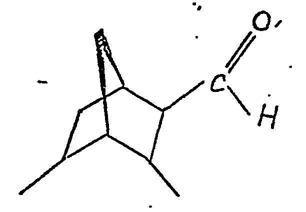

Figure 51B represents the infra-red spectrum for peak 1 of the foregoing GLC profile consisting of the compound having the structure:

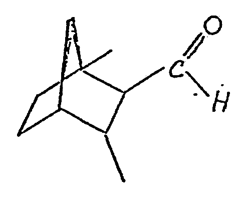

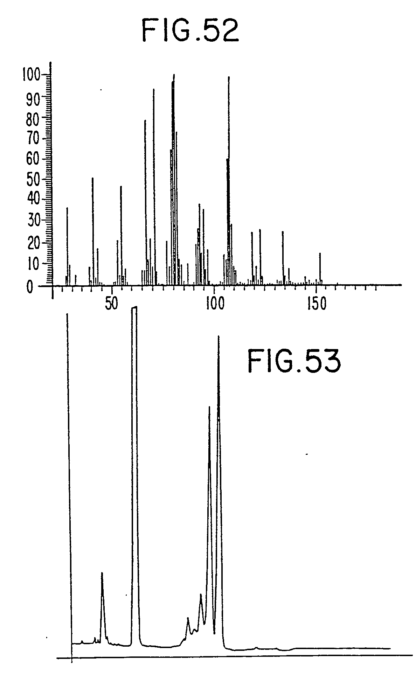

Figure 52 represents the mass spectrum of the mixture of the foregoing distillation containing the compounds having the structures: 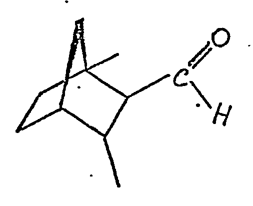 and

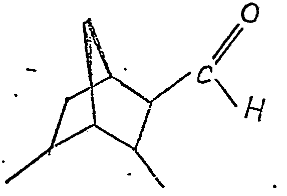

Both fractions 3 and 4 have a green, cut grass and minty aroma with fruity (apple) and herbaceous undertones.

Both fractions 3 and 4 have a eucalyptus, camphoraceous, blueberry-like, patchouli-like and fruity aroma profile and a camphoraceous, blueberry and patchouli-like flavor profile insofar as their use for foodstuffs is concerned. This causes them to be useful for blueberry and raspberry flavored foodstuffs.

### Example XXXVIII

### Preparation of 1- and 5-methyl-5-norbornene carboxaldehyde

Reaction:

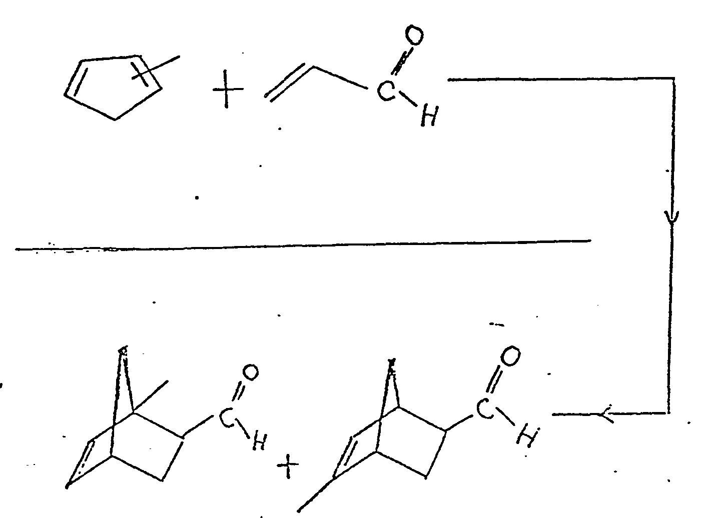

Into a 3-liter flask equipped with stirrer, thermometer, addition funnel, condenser, nitrogen purge, and cooling bath is charged 500 ml toluene and 50 grams of a 25_{%} solution of ethyl aluminum dichloride. The resulting mixture is cooled to 20°C and over a period of one hour while maintaining the temperature between 20° and 25°C, 280 grams of acrolein is added.

370 grams of 1-methyl-1,3-cyclopentadiene and 2-methyl-l, 3-cyclopentadiene produced according to Example A is mixed with 500 ml toluene. The resulting mixture is added over a period of two hours while maintaining the reaction temperature of 20°-22°C to the reaction mass. The resulting reaction mass is then stirred at 21°-22°C for a period of one hour. At the end of this period of time GLC analysis indicates that the reaction is complete.

The resulting reaction mass is poured into 1 kilogram of a 10% sodium chloride solution. The resulting mixture exists in two phases; an aqueous phase and an organic phase. The aqueous phase is removed and the organis phase is washed as follows:
A - one 250 ml portion of 10% hydrochloric acid solution;
B - one 1 kilogram portion of 10% sodium chloride solution;
C - one 1 kilogram portion of 10% sod um hydroxide solution;
D - one 1 kilogram portion of 10% sodium chloride solution;
E - one 1 kilogram portion of 20%. sodium chloride solution.

The toluene is stripped off of the reaction mass under reduced pressure.

Triethanolamine and 32 grams of Primol ® and 0.5 grams of Ionox ® and 0.5 grams of calcium carbonate is then added to the reaction mass and the reaction mass is distilled on a 12" Goodloe column yielding the following fractions:

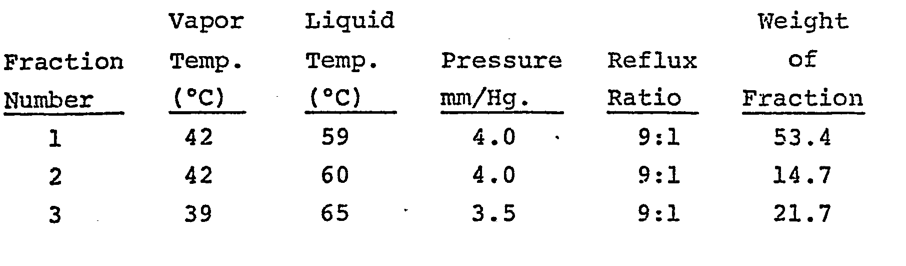

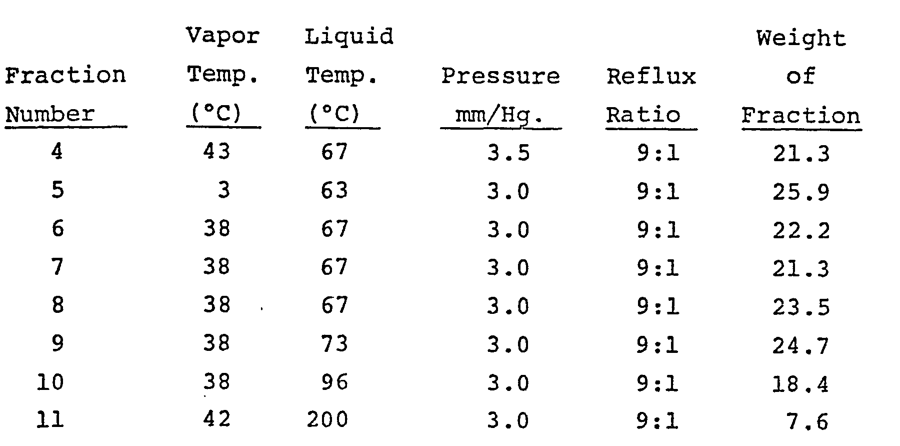

NMR, IR and mass spectral analysis yield the information that the reaction product consists of the two compounds having the structures: 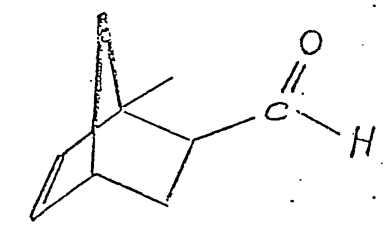 and

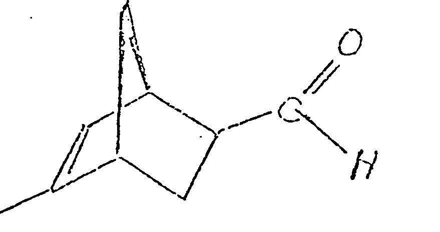

Figure 53 represents the GLC profile after one hour of reaction.

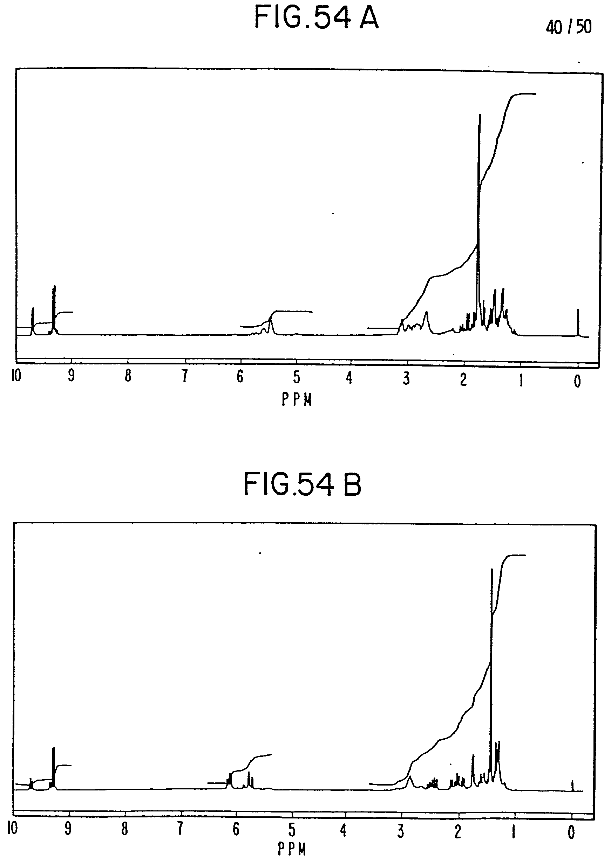

Figure 54A represents NMR spectrum for fraction 10 of the foregoing distillation, consisting of the compound having the structure:

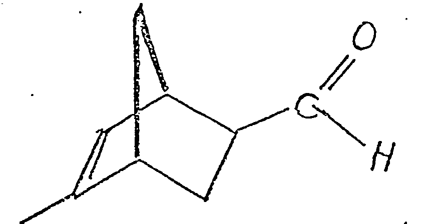

Figure 54B represents the NMR spectrum for fraction 3 of the foregoing distillation, consisting of the compound having the structure:

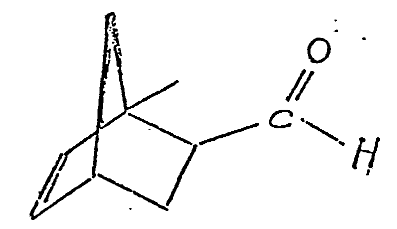

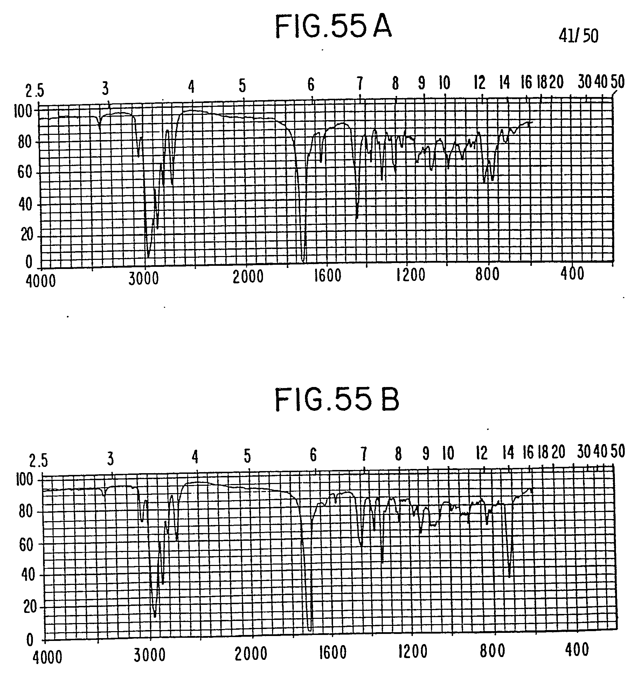

Figure 55A represents the infra-red spectrum for fraction 10 ofthe:foregoing distillation, consisting of the compound having the structure:

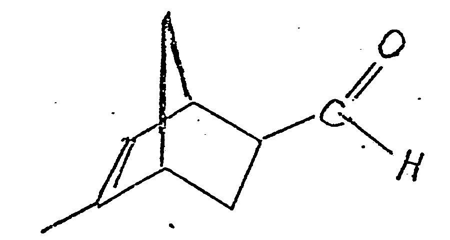

Figure 55B represents the infra-red spectrum for fraction 3 of the foregoing distillation, consisting of the compound having the.structure:

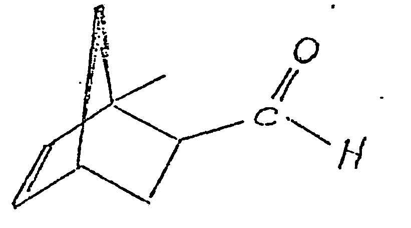

### Example XXXIX

### Basic raspberry formulation

The following basic raspberry formulation is prepared:

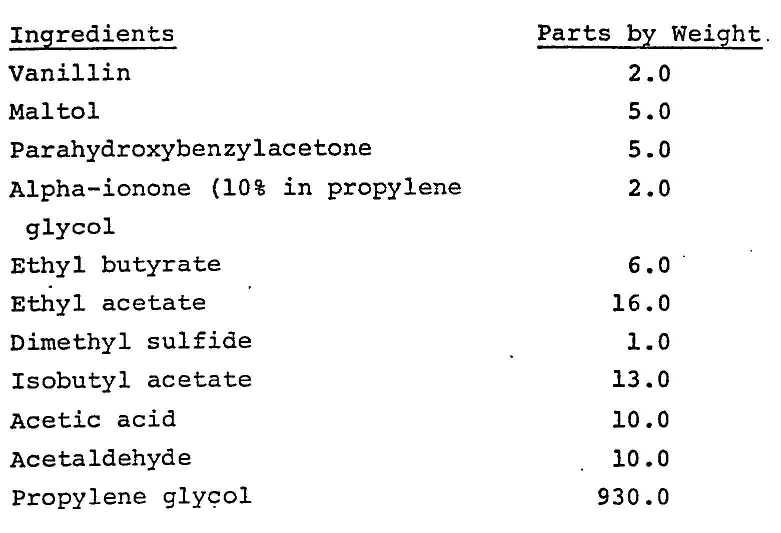

To a first portion of this basic formulation, a mixture of compounds having the structures: 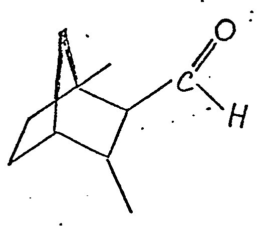 and 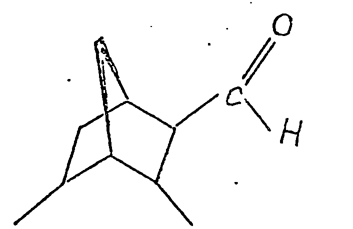 (produced according to Example XXXVII) has been added at the rate of 1%. Nothing is added to a second portion of this formulation. Both flavors with and without the compounds having the structures: 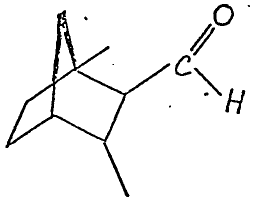 and 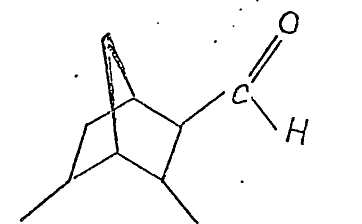 are compared at the rate of 100 parts per million by a bench panel of experts.

The flavor containing the compounds having the structures: 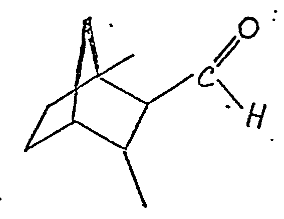 and 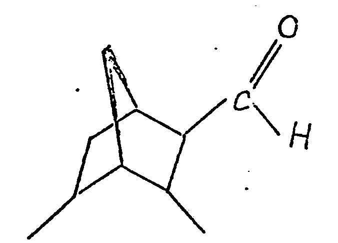 (bulked fractions 3 and 4 of Example XXXVII) is considered to have a more raspberry kernel, more seedy herbaceous more natural character in both aroma and taste. The flavor is therefore preferred as more true to the taste of natural raspberries.

### Example XL

The following mixture is prepared:

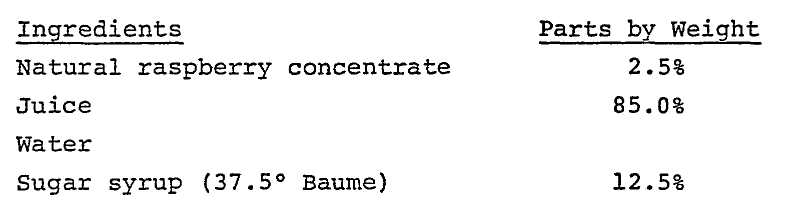

The ripened raspberry and seedy, raspberry kernel note of this raspberry juice is imparted in increased strength by addition of a mixture of compounds having the structures: 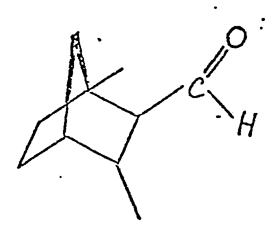 and 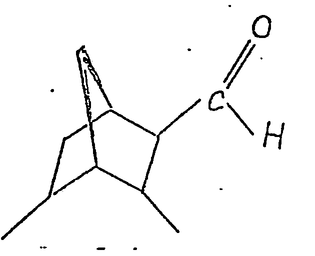 prepared according to Example XXXVII at the rate of from 0.02 ppm up to 10 ppm.

### Example XLI

To the raspberry formulation as set forth in Example IV, the mixture of compounds having the structures: 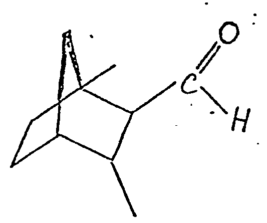 and 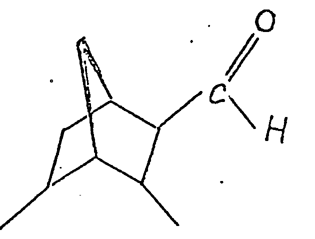 prepared according to Example XXXVII (bulked fractions 3 and 4) is added at the rate of 0.2%, this material is then called the "test composition". The raspberry formulation without the compounds having the structures: 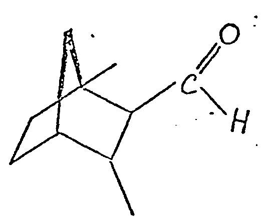 and 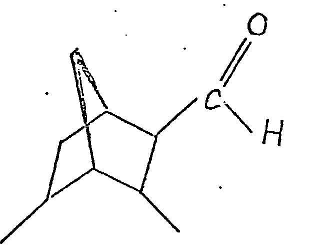 is called the "control composition".

The test and control compositions are added to the food products described hereinafter in the proportions shown for 10 kilograms of material to be flavored:

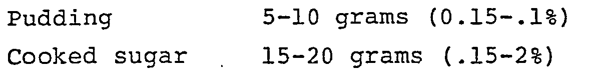

Cooked sigar - 100 ml of sugar syrup (prepared by dissolving 1 kilogram of sucrose in 600 ml of water) and 20 grams of glucose are mixed together and slowly heated to 145°C. The flavor is added and the mass is allowed to cool and harden.

Pudding - To 500 ml of warmed milk are added with stirring a mixture of 60 grams sucrose and 3 grams of pectin. The mixture is boiled for a few seconds and the flavor is added. The mixture is allowed to cool.

The finished foodstuff samples are tested by a panel of trained persons who express their views about the flavor of the samples. All members of the panel prefer the test samples having a more distinguished ripened raspberry aroma with taste of the ripe raspberries and its seedy kernel note.

An improved effect occurs when a mixture of 25:25:50 of 2-(4-hydroxy-4-methylpentyl)-norbornadiene:compound having the structure: 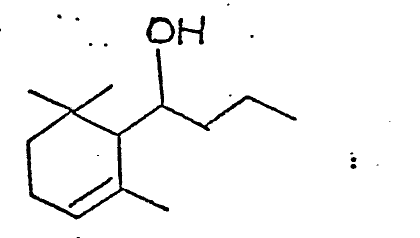 mixture of compounds produced according to Example XXXVII (bulked fractions 3 and 4) having the structures: 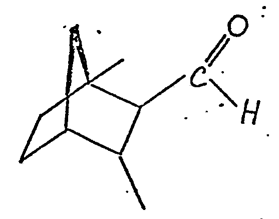 . and 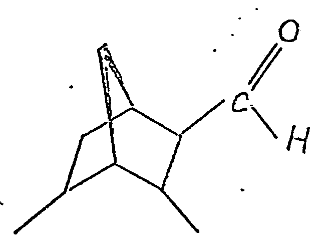 at the rate of 0.02 parts per million up to about 10 parts per million.

### Example XLII

### A. Powder flavor composition

20 grams of the flavor composition of Example XXXIX containing the compounds having the structures: 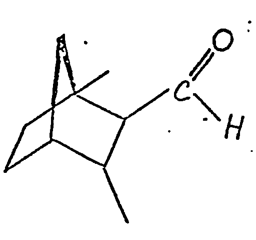 and 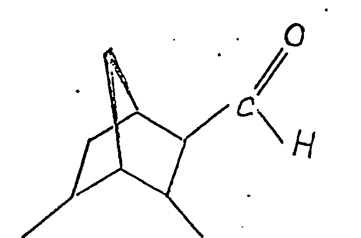 prepared according to Example XXXVII (bulked fractions 3 and 4) is emulsified in a solution containing 300 grams gum acacia and 700 grams water. The emulsion is spray-dried with a Bowen Lab Model Dryer utilizing 260 c.f.m. of air with an inlet temperature of 500°F, an outlet temperature of 250°F, and a wheel speed of 50,000 rpm.

### B. Sustained release flavor

The following mixture is prepared:

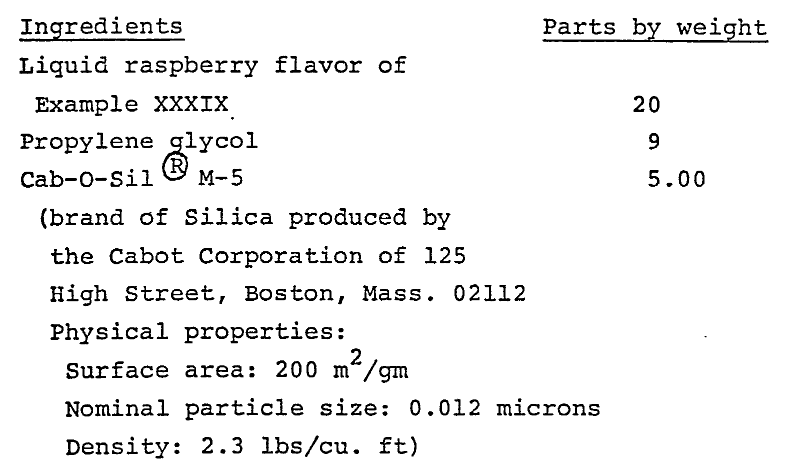

The Cab-O-Sil ®is dispersed in the liquid raspberry flavor composition of Example XXXIX with vigorous stirring, thereby resulting in a viscous liquid. 71 parts by weight of the powder flavor composition of Part A, supra, is then blended into the said viscous liquid, with stirring at 25°_{C} for a period of thirty minutes resulting in a dry, free flowing sustained release raspberry flavor powder.

### Example XLIII

10 parts by weight of 50 Bloom pigskin gelatin is added to 90 parts by weight of water at a temperature of 150°F. The mixture is agitated until the gelatin is completely dissolved and the solution is cooled to 120°F. 20 parts by weight of the liquid raspberry flavor composition of Example XXXIX is added to the solution which is then homogenized to form an emulsion having particle size typically in the range of 2-5 microns. This material is kept at 120°F under which conditions the gelatin will not gel.

Coacervation is induced by adding, slowly and uniformly, 40 parts by weight of a 20% aqueous solution of sodium sulphate. During coacervation the gelatin molecules are deposited uniformly about each oil droplet as a nucleus.

Gelatin is effected by pouring the heated coacervate mixture into 1,000 parts by weight of 7% aqueous solution of sodium sulphate at 65°F. The resulting gelled coacervate may be filtered and washed with water at temperatures below the melting point of gelatin, to remove the salt.

### Example XLIV

### Chewing gum .

100 parts by weight of chicle are mixed with 4 parts by weight of the flavor prepared in accordance with Example XLII. 300 parts of sucrose and 100 parts of corn syrup are added. Mixing is effected in a ribbon blender with jacketed side walls of the type manufactured by the Baker Perkins Co.

The resultant chewing gum blend is then manufactured into strips 1 inch in width and 0.1 inches in thickness. The strips are cut into lengths of 3 inches each. On chewing, the chewing gum has a pleasant, long lasting raspberry flavor.

### Example XLV

### Chewing gum

100 parts by weight of chicle are mixed with 18 parts by weight of the flavor prepared in accordance with Example XLIII. 300 parts of sucrose and 100 parts of corn syrup are added. Mixing is effected in a ribbon blender with jacketed walls of the type manufactured by the Baker Perkins Co.

The resultant chewing gum blend is then manufactured into strips 1 inch in width and 0.1 inches in thickness. The strips are cut into lengths of 3 inches each. On chewing, the gum has a pleasant, long lasting raspberry flavor.

### Example XLVI

### Toothpaste formulation

The following separate groups of ingredients are prepared:

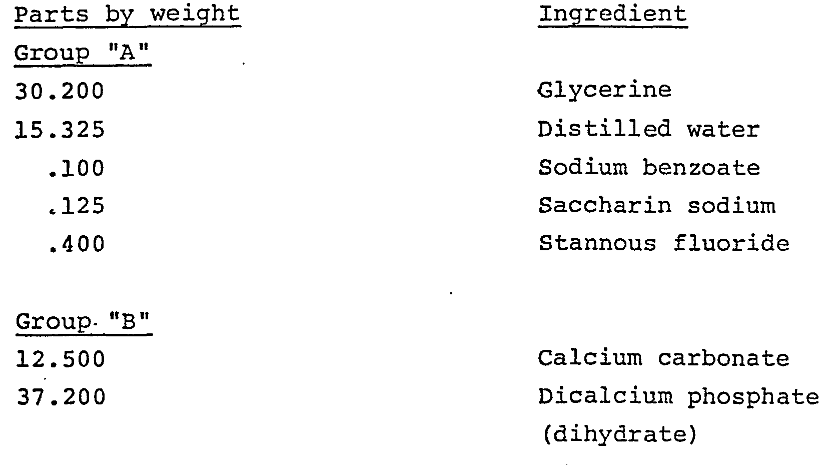

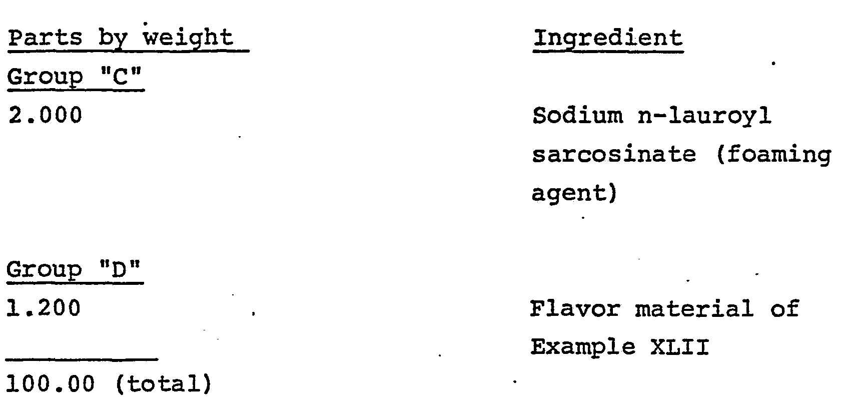

### Procedure:

1. The ingredients in Group "A" are stireed and heated in a steam jacketed kettle to 160°F.
2. Stirring is continued for an additional three to five minutes to form a homogeneous gel.
3. The powders of Group "B" are added to the gel, while mixing, until a homogeneous paste is formed.
4. With stirring, the flavor of "D" is added and lastly, the sodium n-lauroyl sarcosinate.
5. The resultant slurry is then blended for one hour.

The completed paste is then transferred to a three roller mill and then homogenized, and finally tubed.

The resulting toothpaste, when used in a normal tooth- brushing procedure yields a pleasant raspberry flavor, of constant strong intensity throughout said procedure (1-1.5 minutes).

### Example XLVII

### Chewable vitamin tablets

The flavor material produced according to the process of Example XLII is added to a chewable vitamin tablet formulation at the rate of 10 grams/kilogram which chewable vitamin tablet formulation is prepared as follows:

In a Hobart mixer the following materials are blended to homogeneity:

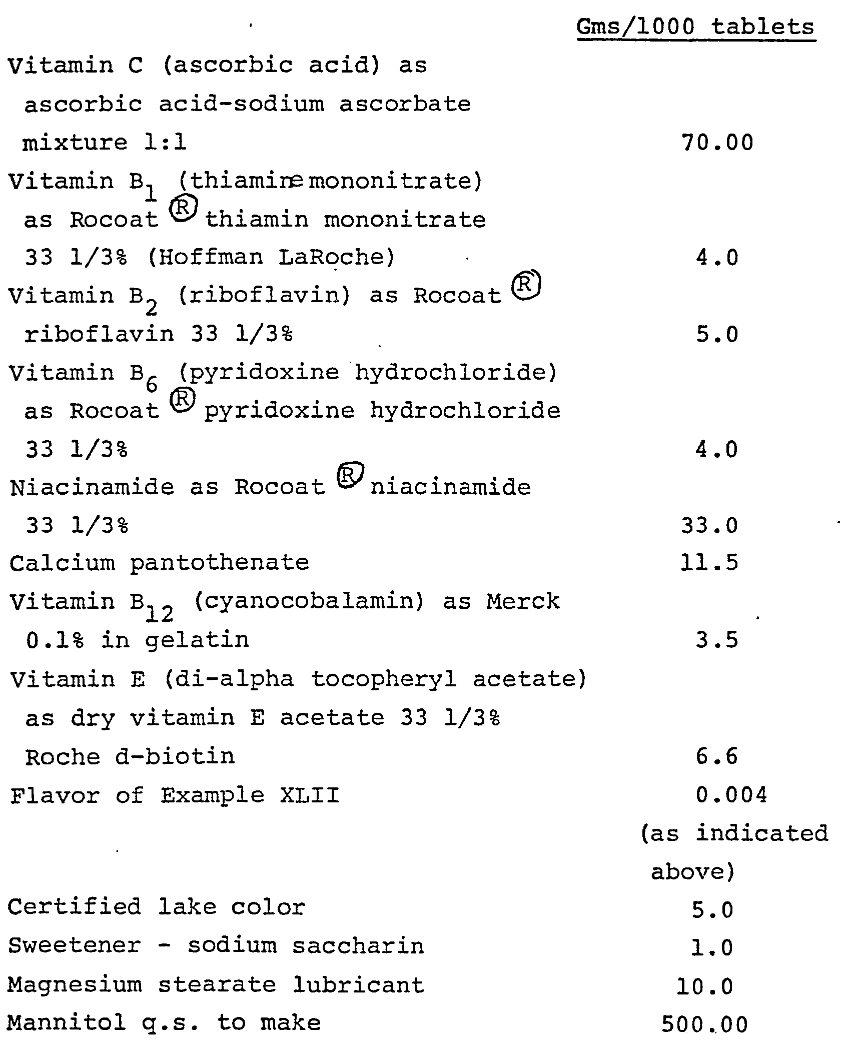

Preliminary tablets are prepared by slugging with flat- faced punches and grinding the slugs to 14 mesh. 13.5 grams dry vitamin A acetate and 0.6 grams vitamin D are then added as beadlets. The entire blend is then compressed using concave punches at 0.5 grams each.

Chewing of the resultant tablets yields a pleasant, long-lasting, consistently strong raspberry flavor for a period of 12 minutes.

### Example XLVIII

### Chewing tobacco

Onto 100 pounds of tobacco for chewing (85% Wisconsin leaf and 15% Pennsylvania leaf) the following casing is sprayed at a rate of 30%:

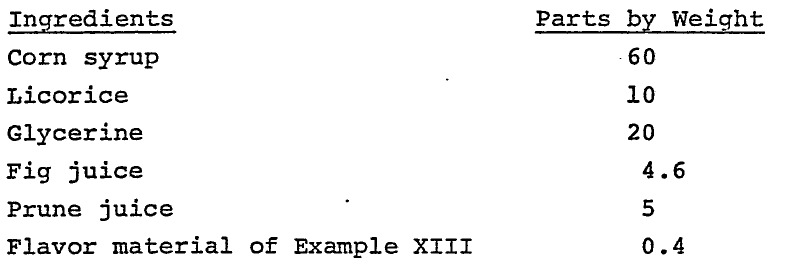

The resultant product is redried to a moisture content of 20%. On chewing, this tobacco has an excellent substantially consistent, long lasting, licorice/raspberry flavor profile in conjunction with the tobacco note.

### Example XLIX

Herbal fragrance produced using product prepared according to Example XXXVII the 1,3- and 3,5-dimethyl-5-norbornane carboxaldehyde

The following mixture is prepared:

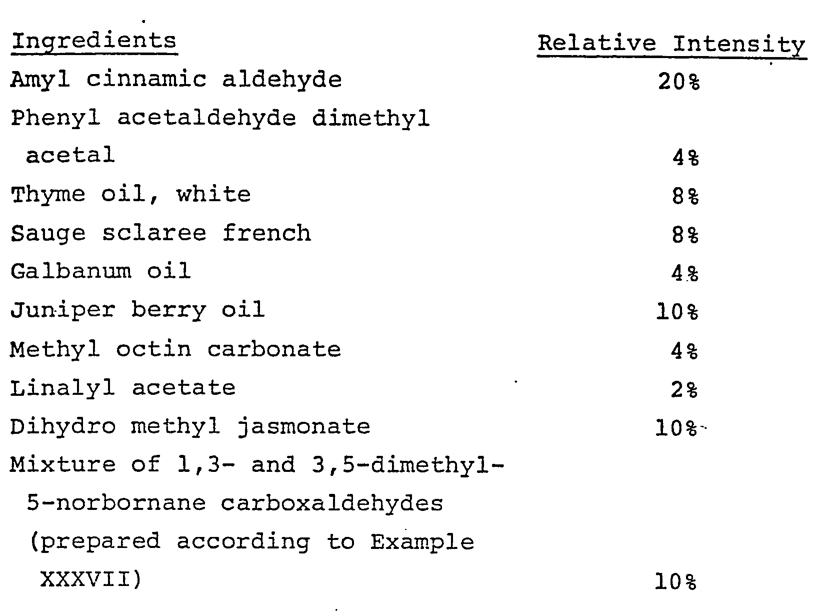

The mixture 1,3- and 3,5-dimethyl-2-norbornane carboxaldehydes prepared according to Example XXXVII (bulked fractions 3 and 4) adds a strong green, cut grass and minty aroma with fruity herbaceous undertones to this herbal fragrance formulation causing it to be more "natural-like".

### Example L

### Preparation of a detergent composition

A granular detergent composition is prepared in accordance with Example IX of Canadian Patent 1,004,566 containing the following ingredients:

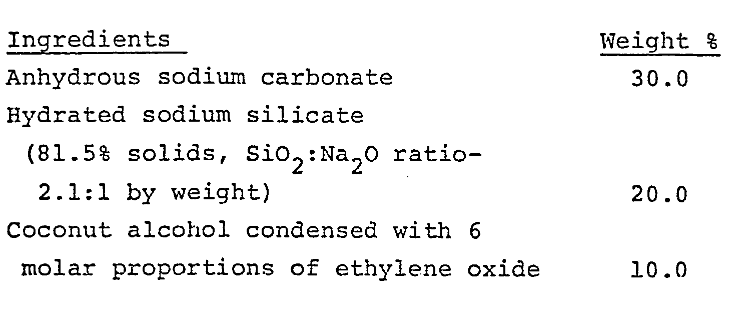

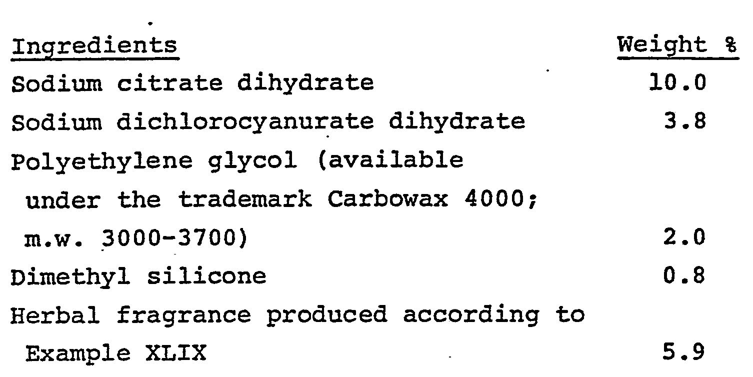

This composition has an excellent herbaceous aroma with minty, cut grass nuances.

### Example LI

### Preparation of a detergent composition

A detergent is prepared from the following ingredients according to Example I of Canadian Patent No. 1,007,948:

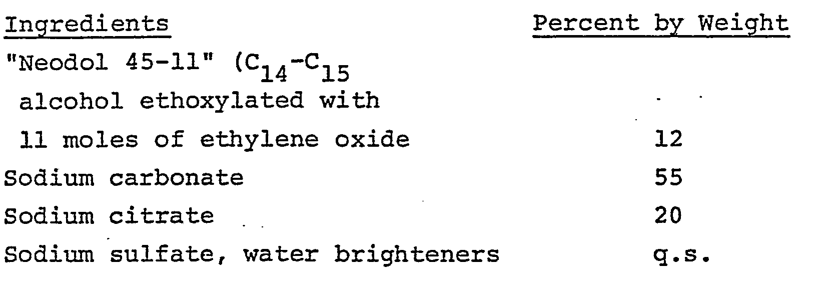

This detergent is a "phosphate free" detergent. A total of 100 grams of this detergent is admixed with 0.15 grams of the mixture of compounds according to Example XXXVII (bulked fractions 3 and 4) having the structures: 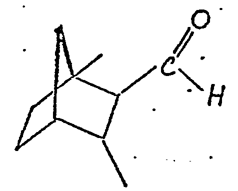 and

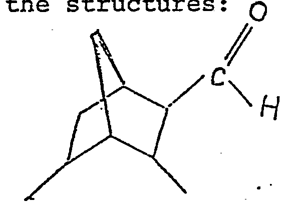

The resulting detergent has an excellent green cut grass and minty aroma with fruity and herbaceous undertones.

### Example LII

### Cologne and handkerchief perfume

One of the materials as set forth in Table XVI below is incorporated into colognes at concentrations of 1.5%, 2.0%, 2.5_{%}, 3.0%, 3.5% and 4.0% in 70%, 75%, 80%, 85%, 90% and 95_{%} aqueous ethanol and into handkerchief perfumes at concentrations of 15%, 20%, 25% and 30% (in 75%, 80%, 85%, 90% and 95% aqueous ethanol). In each of the cases distinct and definitive strong green, cut grass, minty, herbal fragrances with fruity undertones are imparted to the colognes and to the handkerchief perfumes:

### Table XVI

A - Mixture of compounds prepared according to Example XXXVII (bulked fractions 3 and 4) having the structures: 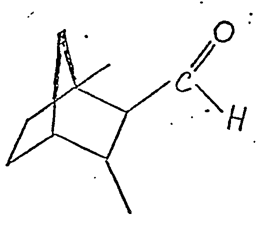 and 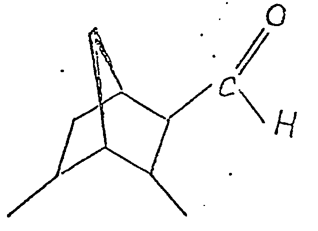
_{B} - Compound prepared according to Example XXXVII having the structure: 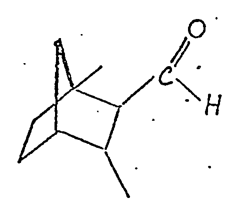
C - Compound prepared according to Example XXXVII having the structure: 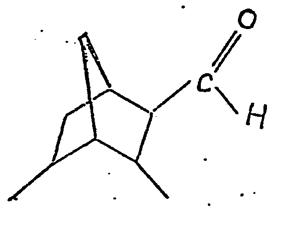
_{D} - Fragrance prepared according to Example XLIX.

### Example LIII

### Liquid detergent composition

Concentrated liquid detergents with rich herbal nuances and minty undertones are prepared according to U.K. Patent 1,526,942 containing 0.05%, 0.1%, 0.15% and 0.20% of the materials of Table XVII set forth below:

### Table XVII

A - Mixture of compounds prepared according to Example XXXVII (bulked fractions 3 and 4) having the structures: 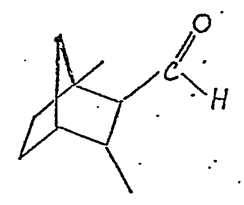 and 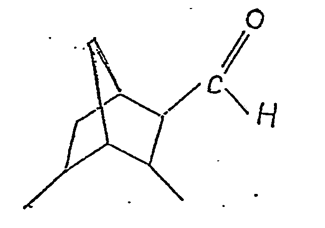
B - Compounds prepared according to Example XXXVII having the structure: 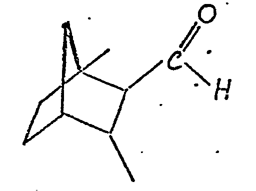
C - Compound prepared according to Example XXXVII having the structure: 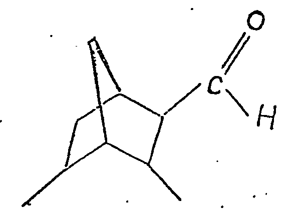
D - Fragrance prepared according to Example XLIX.

They are prepared by adding and homogeneously admixing the appropriate quantity of substance of Table XVII in liquid detergent. The resulting detergents all possess intense herbal, green, cut grass-like and minty nuances with fruity undertones. The specific fragrances are each added to heavy duty liquid detergents formulated according to Example I on page 7 of United Kingdom Patent 1,526,942 thusly:

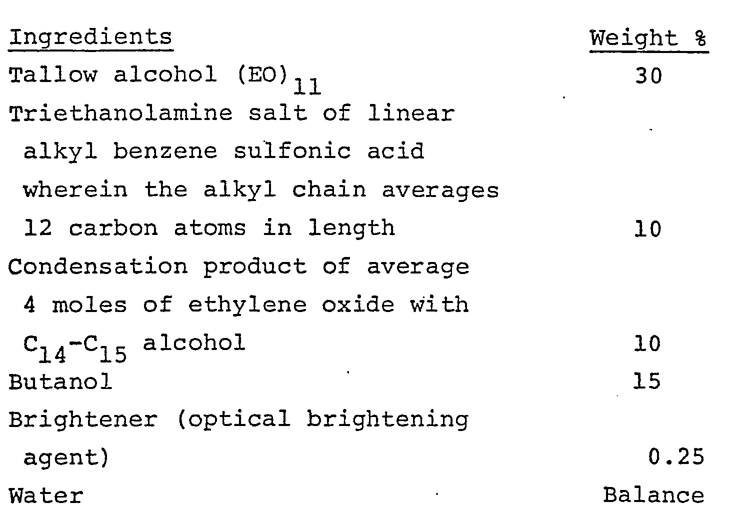

Each of the compositions prepared with each of the substances of Table XVII is stable and provides excellent fabric cleaning when used either full strength as a pretreatment or through the wash detergency at a level of 6,000 ppm. In addition the above composition gives superior cleaning of greasy cosmetic stains and furthermore, on cleaning the aroma of the detergent has a faint but distinct herbal character without any "chemical" nuances.

### Example LIV

### Fabric conditioning substance

According to Example I of U.K. Patent 1,544,863, a pre- soaking/wash additive composition of the following, formulation is prepared:

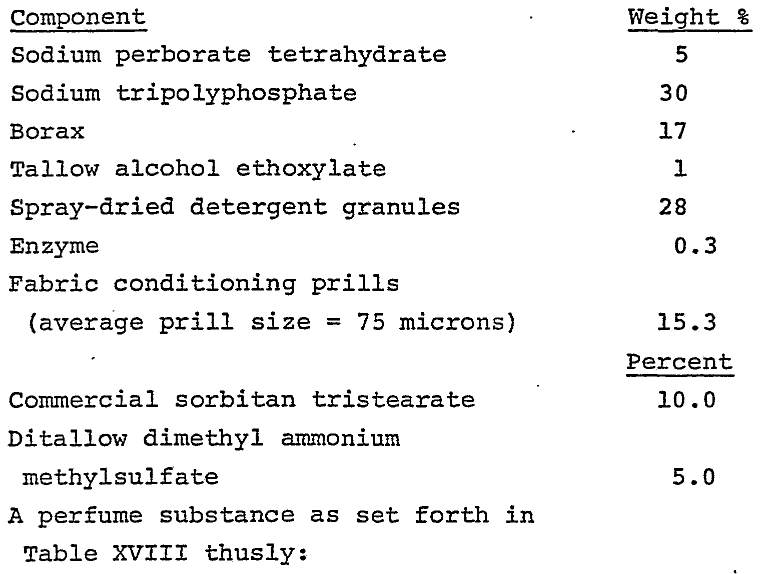

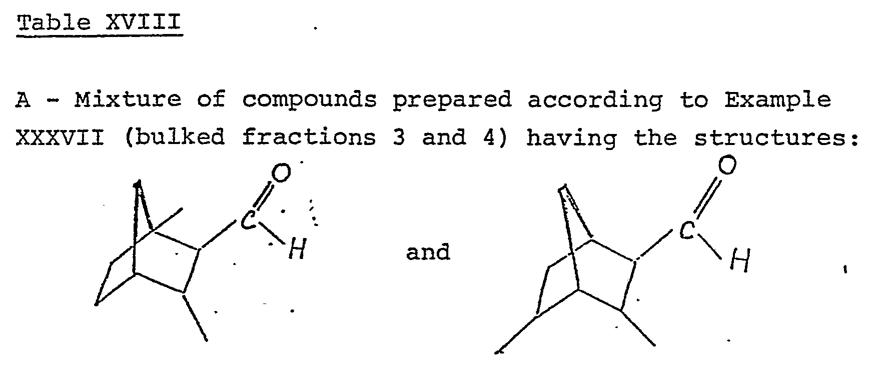

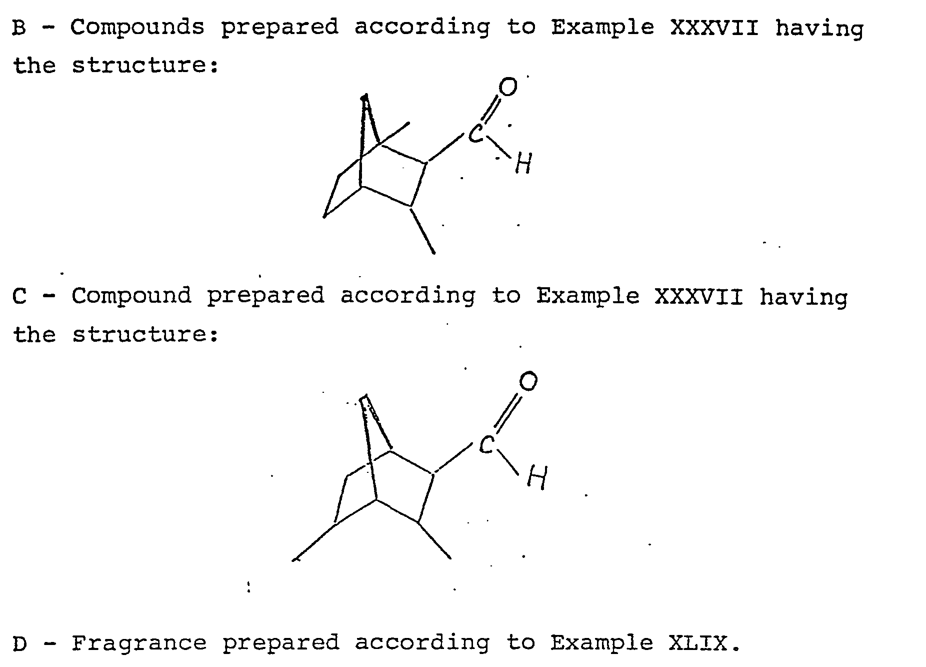

The composition is prepared by dry mixing the requisite granular ingredients until a homogeneous granular product having a melting or softening point of from 35°-58° is secured.

The composition is (a half cup) added to a conventional 17-19 gallon automatic washing machine along with soiled articles of clothing and one cup of a commercial anionic detergent product. Thereafter the articles are laundered in the machine in a conventional manner for ten minutes in wash water of 40°C. The clothing articles are then rinsed, spin dried in the automatic washing machine and are thereafter placed:in an automatic laundry dryer. The dryer is operated at an average temperature of 50°C for a period of 40 minutes. Upon removal of the fabrics from the dryer, clothing articles are perceived to have a soft feel and a noticeable herbaceous, perfume aroma from the wash additive composition perfume.

### Example LV

### Preparation of 1- and 5-methyl-5-norbornene-2-carboxylic acid amides (catalytic Diels-Alder reaction) (catalytic)

Reaction:

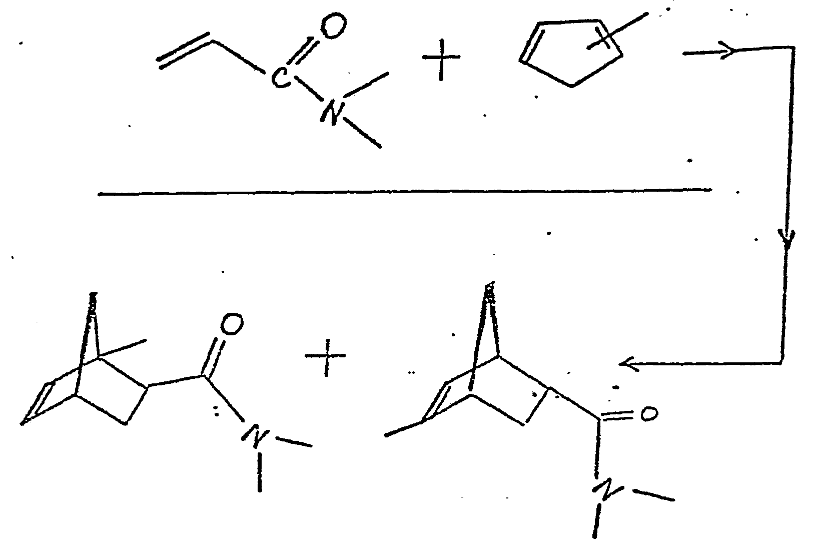

Into a 1 liter reaction flask, equipped with thermometer, stirrer, condenser, addition funnel, cooling bath and nitrogen purge are placed 200 grams of N,N-dimethyl acrylamide and 300 ml toluene. While maintaining the reaction temperature at 20-25°C over a period of twenty minutes, 50 grams of 25% ethyl aluminum dichloride is added dropwise to the reaction mass. After the addition of the ethyl aluminum dichloride is complete, over a period of twenty-five minutes while maintaining the reaction temperature at 20-25°C, 325 grams of a 50% solution of methyl cyclopentadiene in toluene is added to the reaction mass dropwise. After the addition of the methyl cyclopentadiene solution, the reaction mass is then quenched by pouring ' same into 500 ml of a 10% sodium chloride solution. The aqueous phase and the organic phase are separated and the aqueous phase is extracted with one 200 ml portion of toluene. The organic phases are combined and washed with two 500 ml portions of 10% sodium chloride solution. The solvent is then stripped off of the organic phase and the reaction mass is distilled yielding the following fractions:

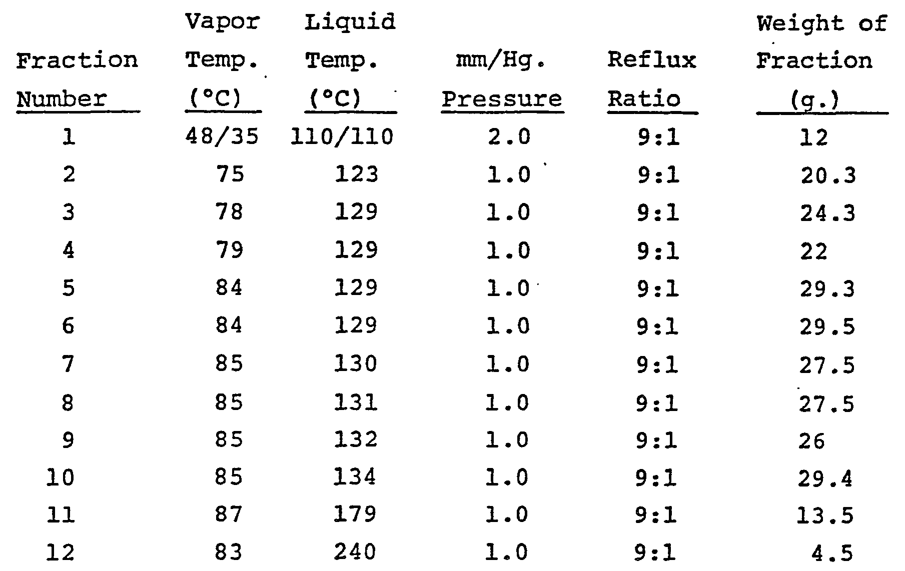

after adding to the reaction mixture 9 grams of Primol ®, 0.5 grams of Ionox ® and 5 grams of calcium carbonate.

The resulting reaction product contains compounds having the structures: 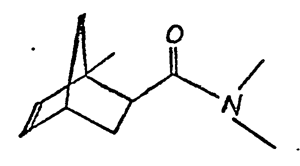 and 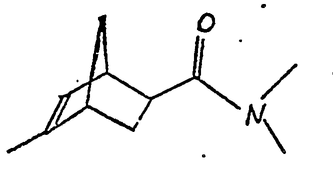 as confirmed by NMR mass spectral and IR analyses.

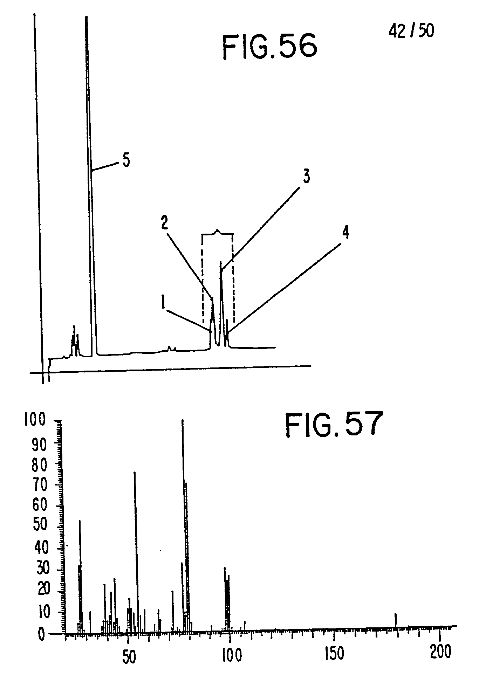

Figure 56 represents the GLC profile for the reaction product of Example LV wherein peaks 1, 2, 3 and 4 represent peaks for compounds having the structures: 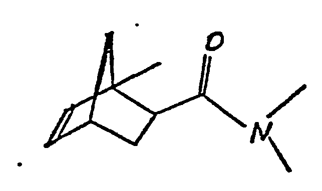 and 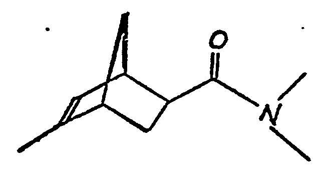
Peak 1 of said Figure 56 represents the compound having the structure: 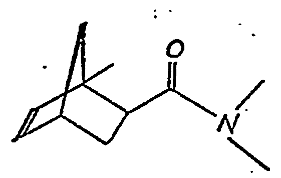
Peak 2 of Figure 56 also represents the compound having the structure: 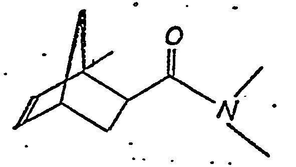
Peak 3 of said Figure 56 represents the compound having the structure: 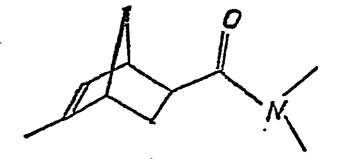
Peak 4 of said Figure 56 represents the compound having the structure: 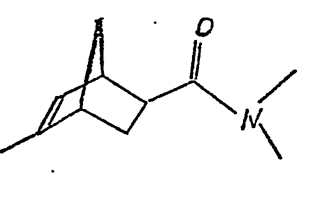
Peak 5 of said Figure .56 represents the solvent peak on said G_{L}C profile. The ratios of Peak l:Peak 2:Peak _{3:} Peak 4:Peak 5 are:
   14.76:14.96:15.77:16.22:3.90
Figure 57 represents the mass spectrum for the reaction product of Example LV which contains the compounds having the structures: 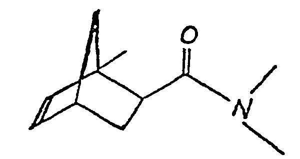 and 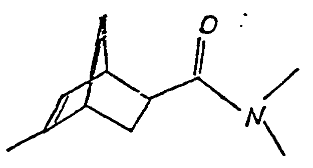

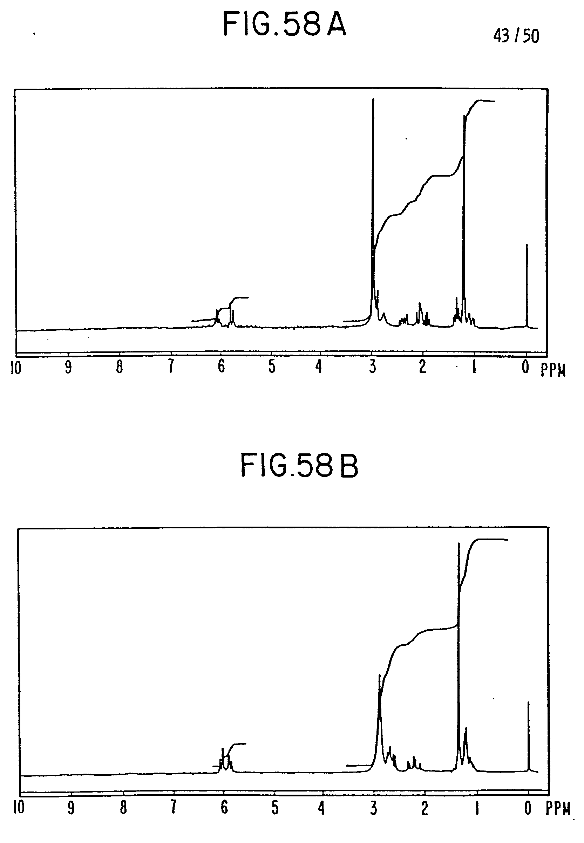

Figure 58A represents the NMr spectrum for peak 1 of the GLC profile of the reaction product of Example LV which represents the compound having the structure: 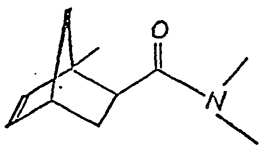
Figure 58B represents the NMR spectrum for peak 2 of the GLC profile of the reaction product of Example LV which represents the compound having the structure: 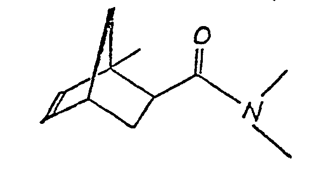

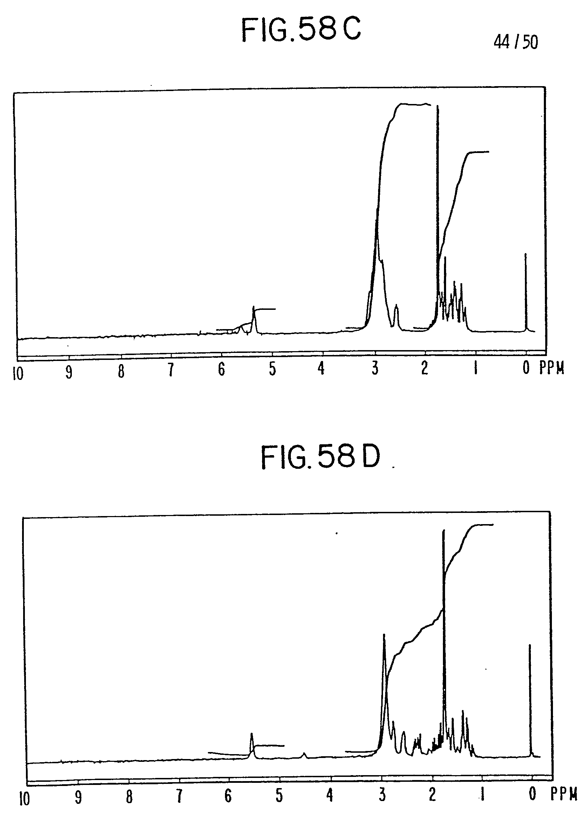

Figure 58C represents the NMR spectrum for peak 3 of the GLC profile of the reaction product of Example LV and represents the compound having the structure: 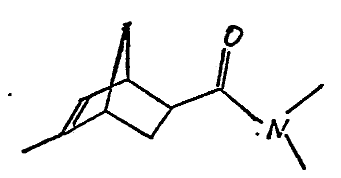
Figure 58D represents the NMR spectrum for peak 4 of the G_{LC} profile of the reaction product of Example LV and represents the compound having the structure: 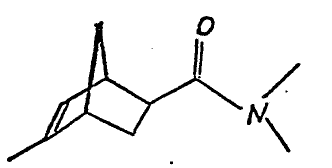

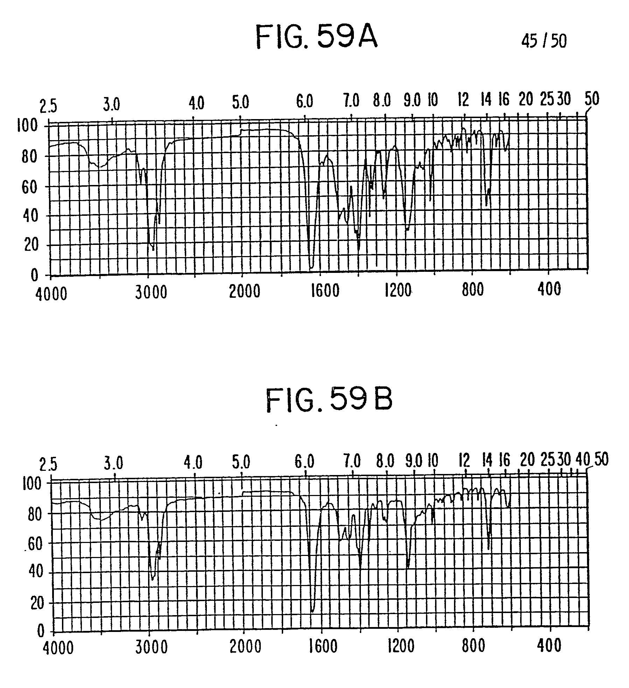

Figure 59A represents the infra-red spectrum for peak 1 of the GLC profile of the reaction product of Example LV which represents the compound having the structure: 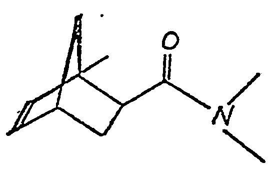
Figure 59B represents the infra-red spectrum for peak 2 of the GLC profile of the reaction product of Example LV and represents the compound having the structure: 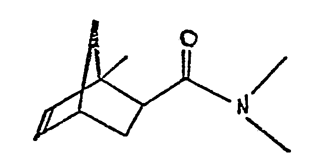

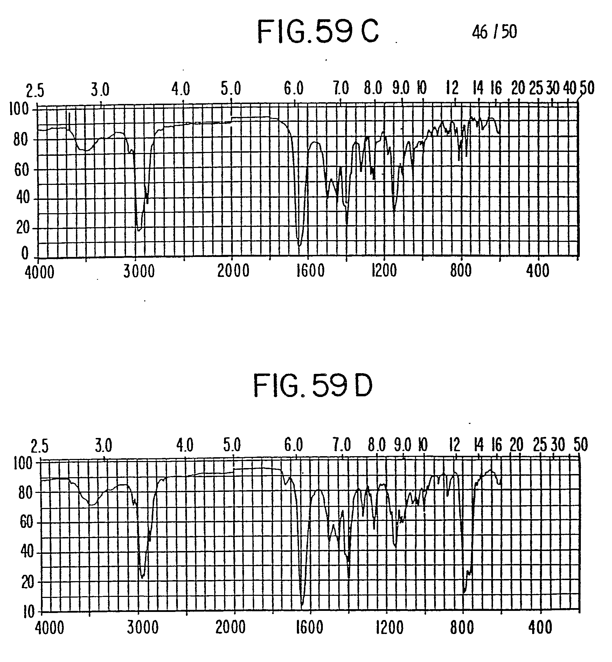

Figure 59C represents the infra-red spectrum for peak ₃ of the GLC profile of the reaction product of Example LV and represents the compound having.the structure: 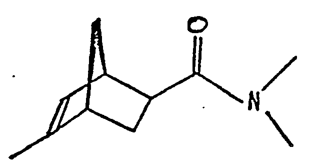
Figure 59D represents the infra-red spectrum for peak 4 of the GLC profile of the reaction product of Example LV and represents the compound having the structure:

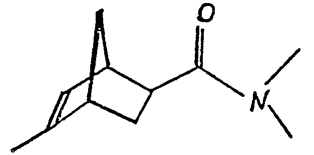

### Example LVI

### Preparation of 1- and 5-methyl-5-norbornane-2-carboxylic acid amides

Reaction:

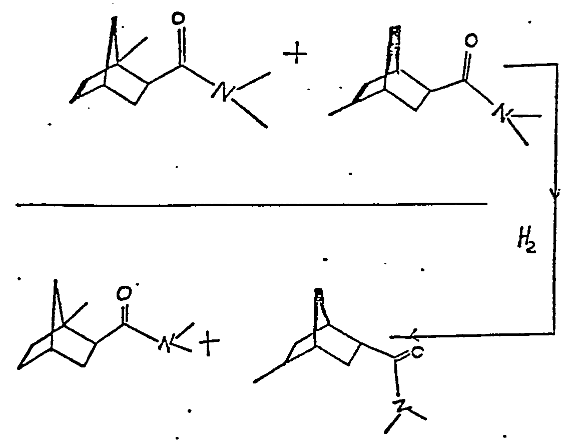

Into a 500 ml pressure bottle equipped with Parr shaker is placed 200 grams of the reaction product of Example LV containing the compounds having the structures: 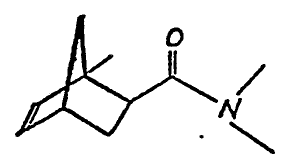 and 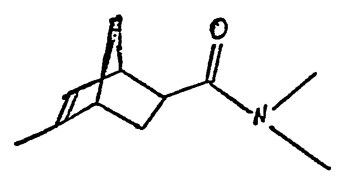 4 grams of Raney nickel and 50 ml of isopropyl alcohol. The reaction mixture is pressurized to 50 psig after the pressure bottle is closed and the reaction mass is purged five times with hydrogen in order to remove any air. Over a period of nine hours, while maintaining the reaction pressure at 25-50 psig, hydrogen gas is added to the reaction mass (total up-take 91 psig hydrogen).

At the end of the reaction, the pressure bottle is opened and the catalyst is filtered and the solvent is stripped off. The reaction product is then distilled on a 12 inch Silver Mirror Goodloe column yielding the following fractions:

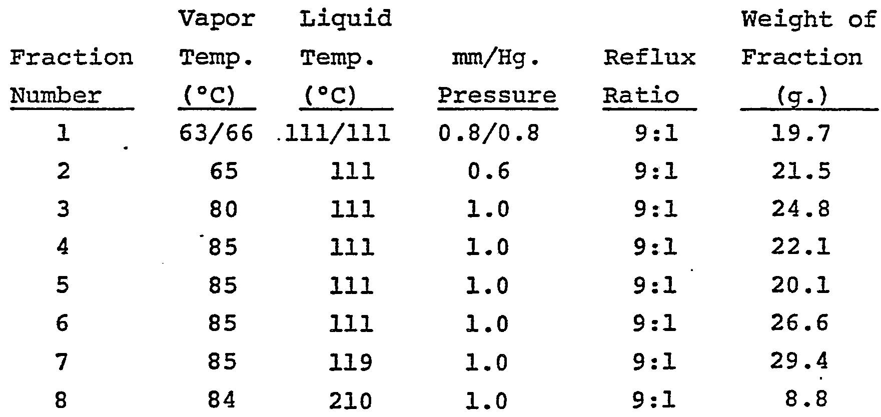

NMR, IR and mass spectral analysis yield the information that the resulting product contains the compounds having the structures: 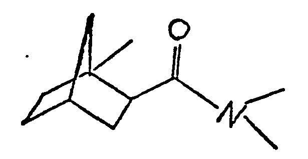 and

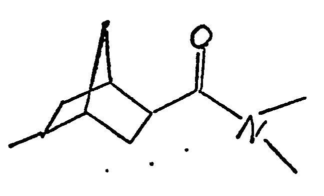

The distillation as set forth above is carried out with 6 grams of Primol ® and 0.5 grams of Ionox ®.

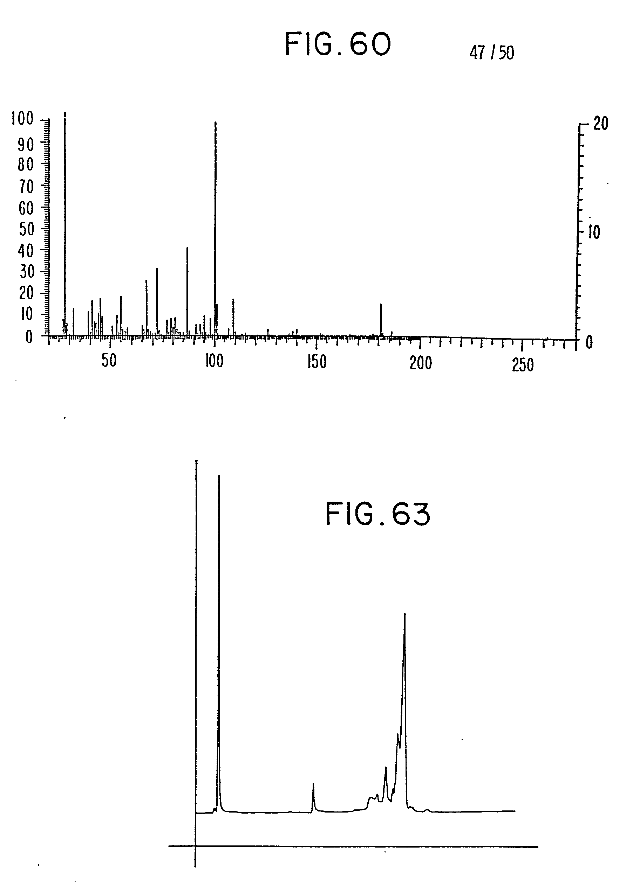

Figure 60 is the mass spectrum for the reaction product produced according to this example containing the compounds having the structures: 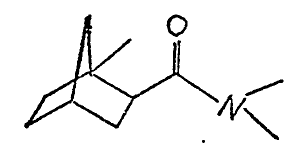 and 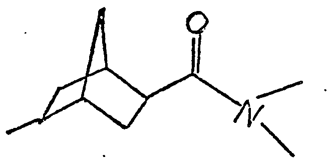 I

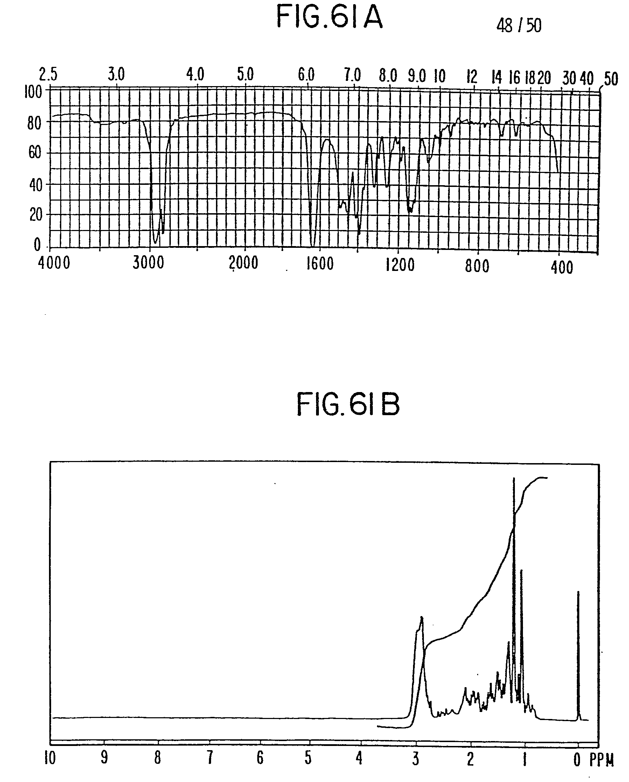

Figure 61A represents the infra-red spectrum for fraction 1 of the distillation product of the reaction product of Example LVI which consists of the compound having the structure:

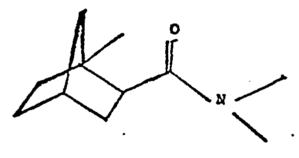

Figure 61B represents the NMR spectrum for fraction 1 of the distillation product of the reaction product of Example LVI which consists of the compound having the structure:

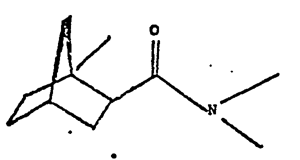

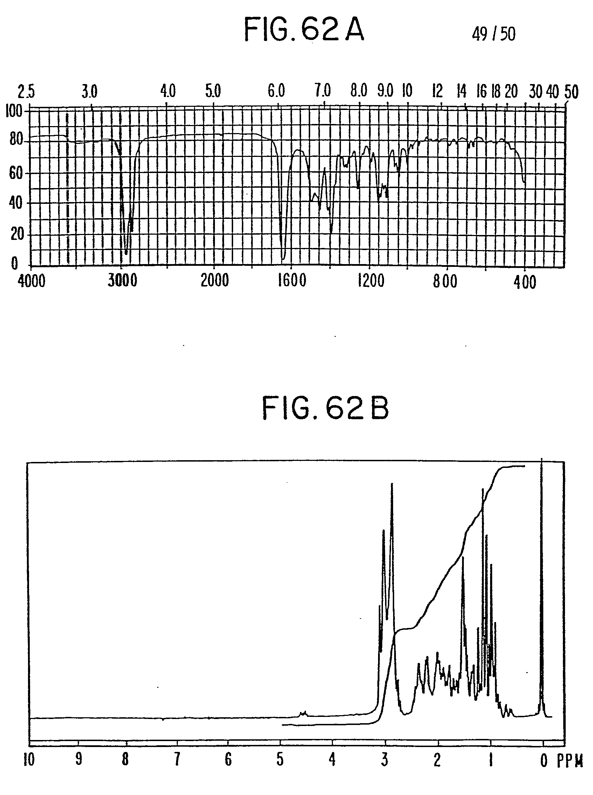

Figure 62A represents the infra-red spectrum for fraction 8 of the distillation product of the reaction product of Example LVI which consists of the compound having the structure:

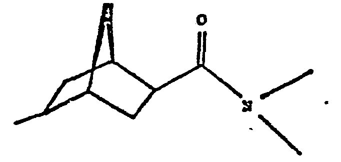

Figure 62B represents the NMR spectrum for fraction 8 of the distillation product of the reaction product of Example LVI which consists of the compound having the structure:

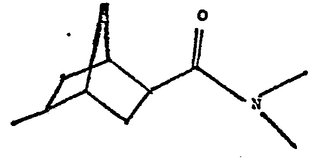

### Example LVII

### Preparation of N,N,5-trimethyl-5-norbornene-endd-2-carboxamide (thermal reaction)

Reaction:

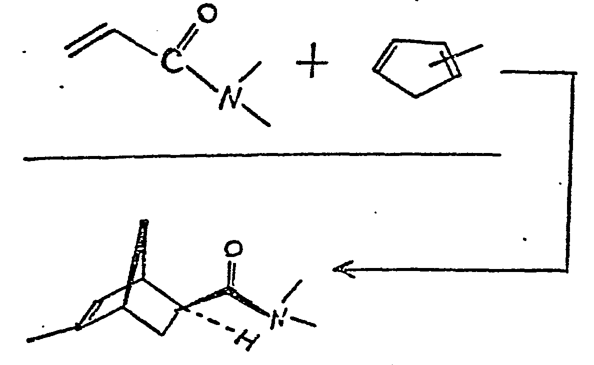

Into a 1000 cc autoclave equipped for pressure reactions, is placed 104 grams of methylcyclopentadiene dimer and 250 grams of N,N-dimethyl acrylamide. The autoclave is closed and placed in a Parr shaker. The contents of the autoclave are heated to 200°C while maintaining the pressure at 125-145 psig and with shaking maintained at that temperature and pressure for a period of 7 hours.

At the end of the 7 hour period, the contents of the autoclave are cooled and the autoclave is opened. The reaction mass is then removed from the autoclave and distilled using a micro distillation apparatus yielding the following fractions:

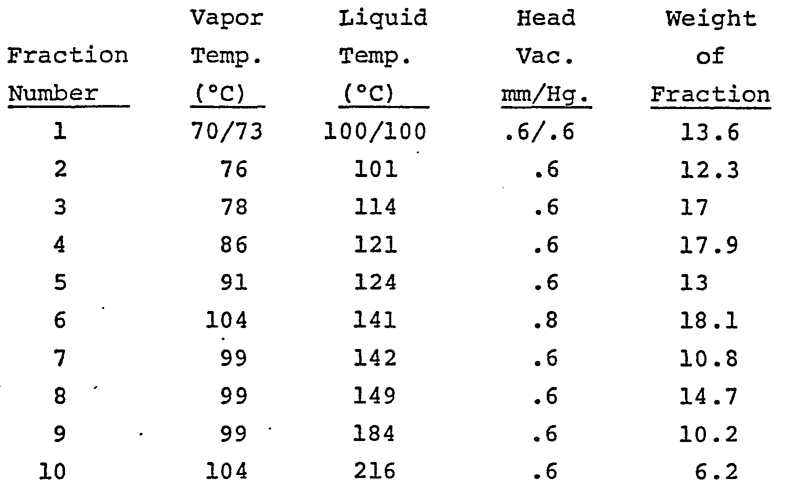

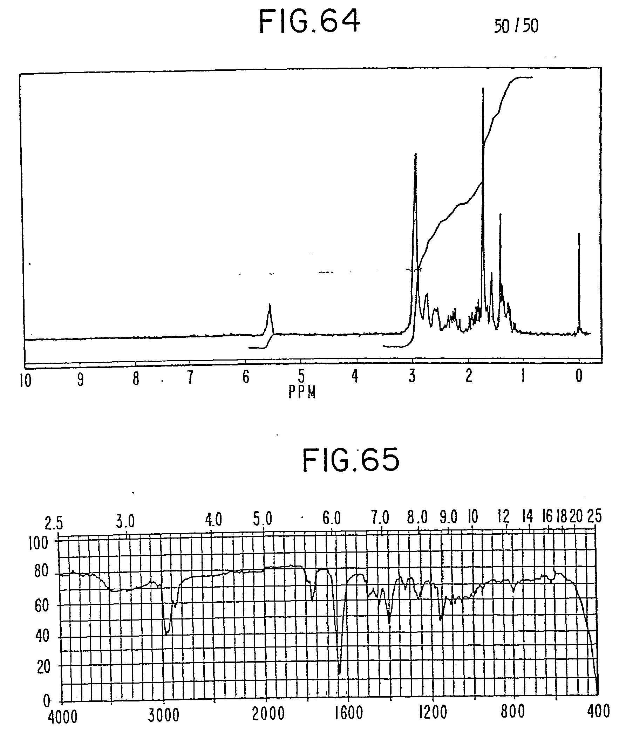

The GLC profile prior to distillation is set forth in Figure 63. The GLC conditions are: Carbowax column programmed at 80-220°C at 8°C per minute. NMR and IR analysis confirm that the product produced has the structure:

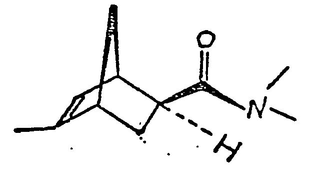

Figure 64 is the 1MR spectrum for the compound having the structure:

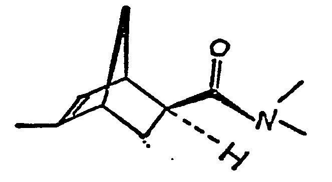

Figure 65 is the infra-red spectrum for the compound having the structure:

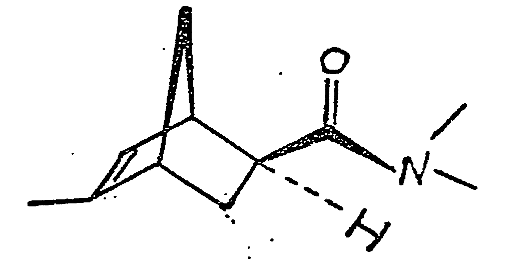

The resulting compound has an excellent fruity aroma with a castoreum-like undertone. The material tested for its organoleptic properties results from the bulking of fractions 3-8 of the foregoing distillation.

### Example LVIII

The following minty, floral and herbal type formula is prepared:

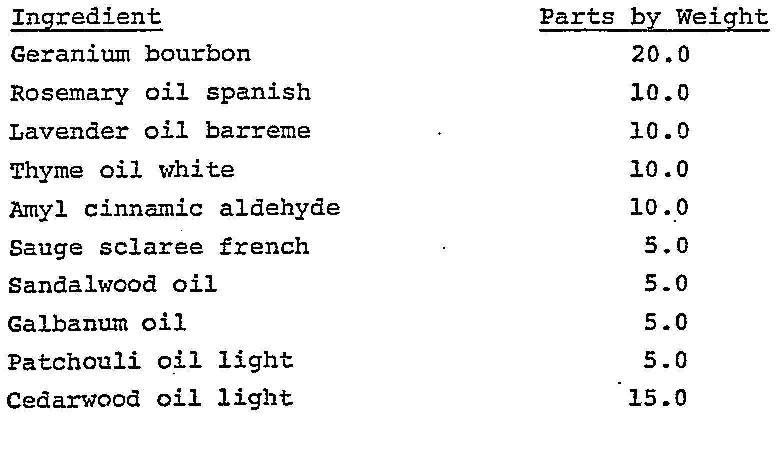

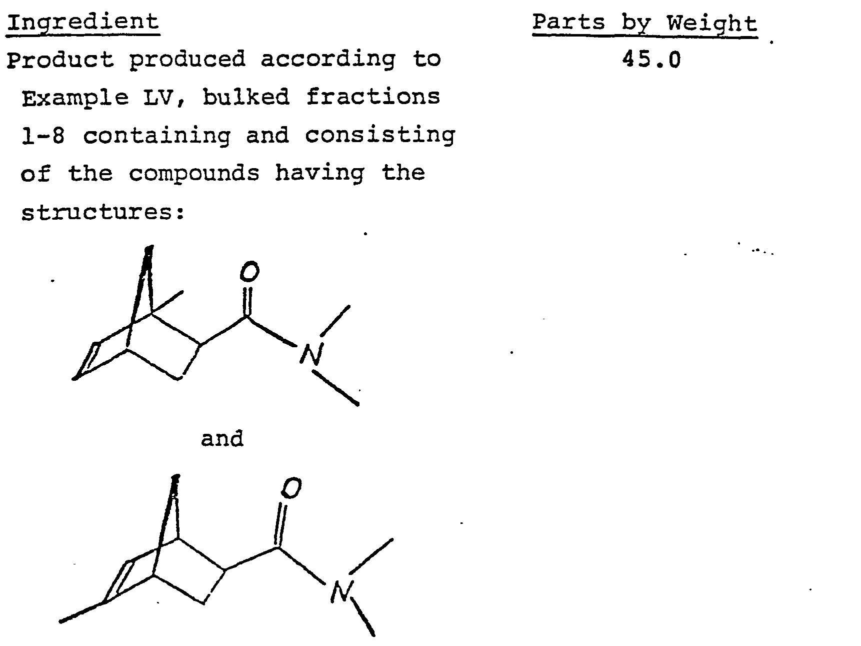

When the composition of matter prepared according to Example LV is incorporated into the formula at 45.0%, the composition of matter has added to it a pleasant, herbaceous, spearmint, spicy, powdery, floral, basil, caraway and anise aroma to the already spicy, herbal and floral type formulation.

### Example LIX

The following minty, floral and herbal type formula is prepared:

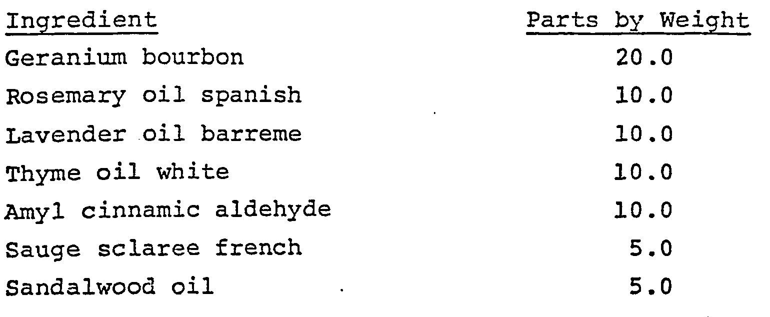

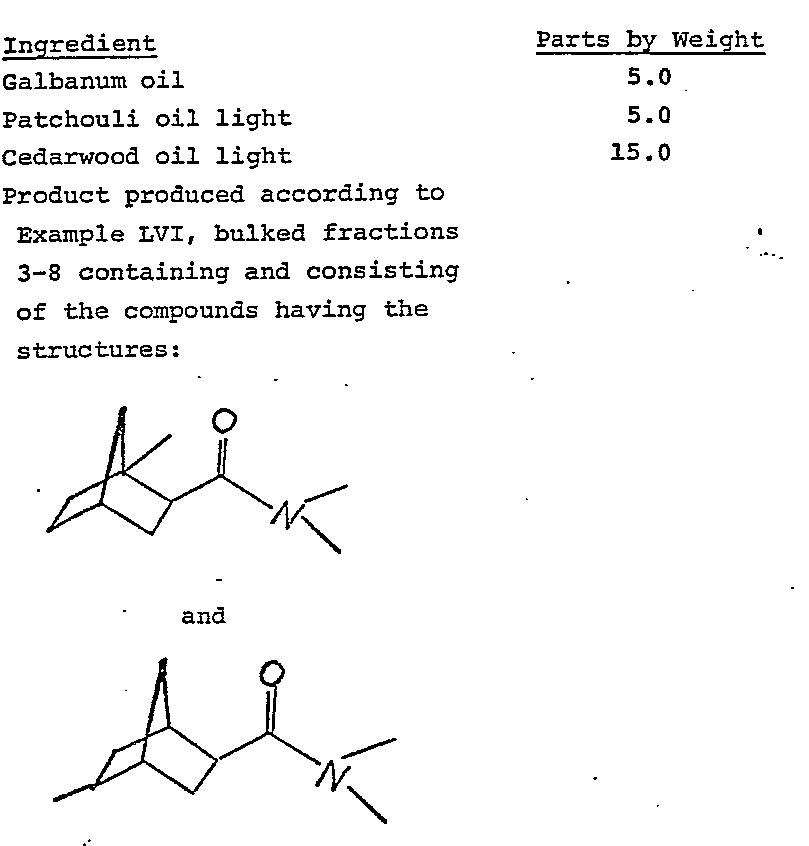

When the composition of matter prepared according to Example LVI is incorporated into the formula at 45.0%, the composition of matter has added to it a pleasant, peppery, herbaceous, spearmint, caraway aroma with basil and aniseed undertones to this already spicy, herbal and floral type formulation.

### Example LX

A stable lotion is prepared with the following formulations:

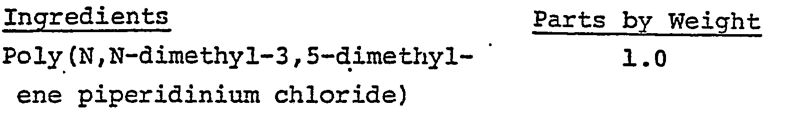

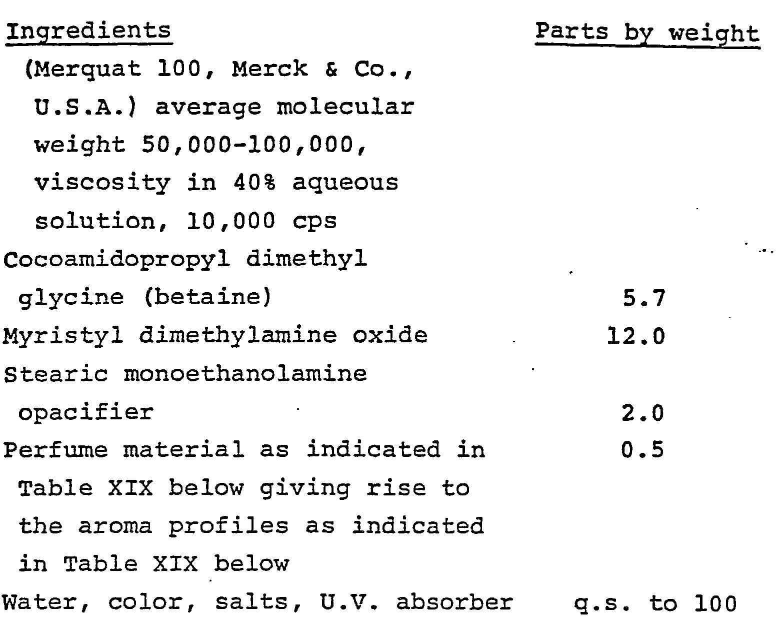

Two or three bottle capfulls of the above lotion held under the tap releasing thewater into the bathtub yields a copiously foamed bubble bath with no visible precipitation, flocculation or "bathtub ring" even using hard water. Bathing in this bath is found to have a cleansing and pleasing emollient effect on the skin as described above.

When, after immersion in this bath, the body is soaped, rinsed and dried, an even better, more lasting emollient moisturizing effect on the skin is observed. The foam or bubbles are substantially unaffected by the soaping step, and no precipitate, flocculate or "bathtub ring" or any bothersome film or coating on the bathtub surface is found.

The aroma produced is as set forth in Table XIX below:

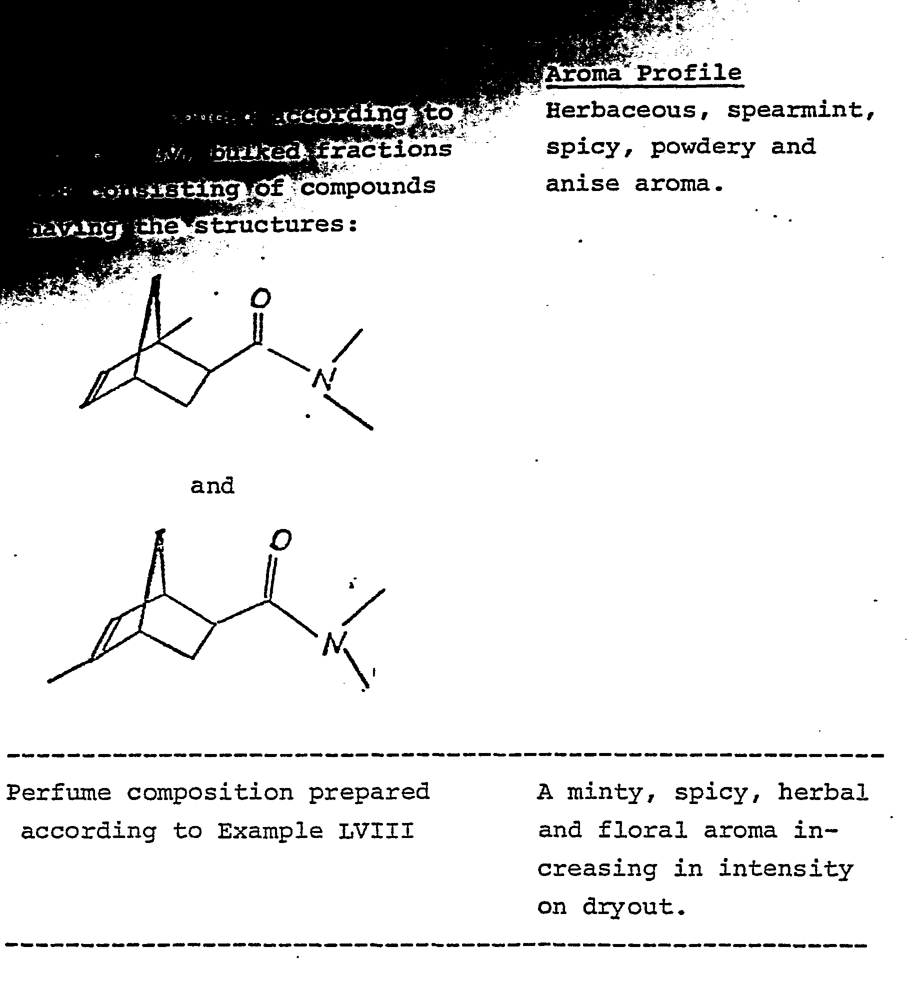

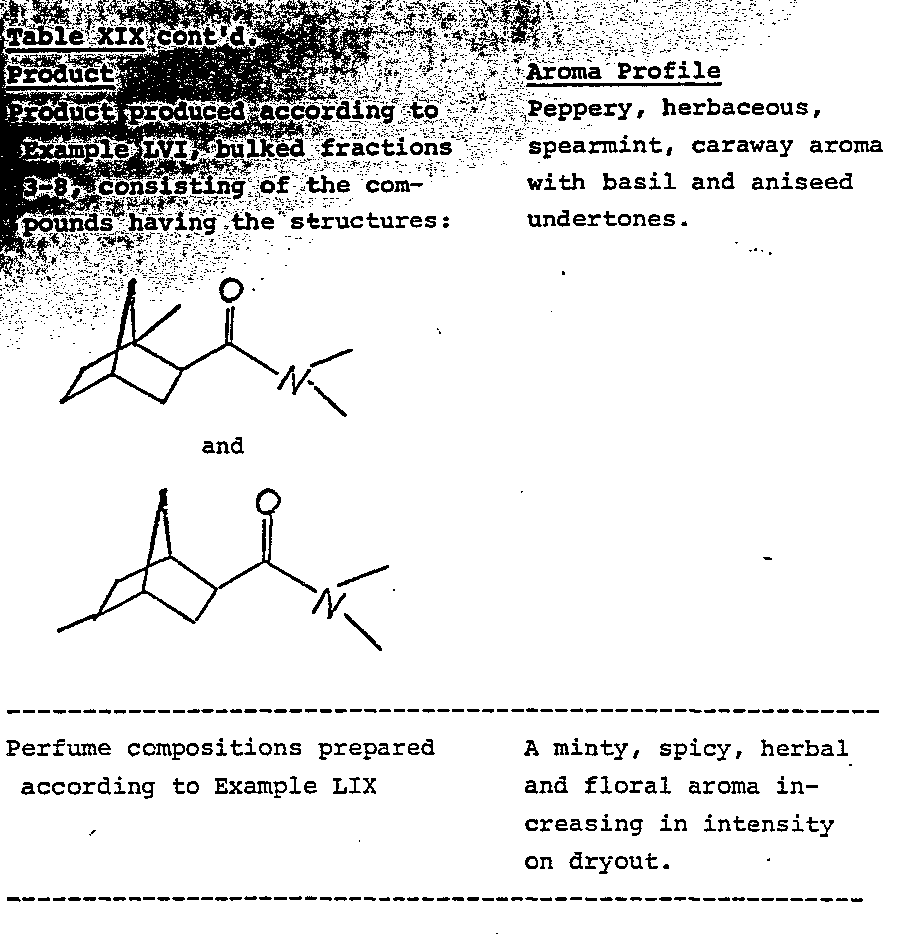

### Example LXI

The following formulation is prepared with results and properties and use similar to those described in Example LVI.

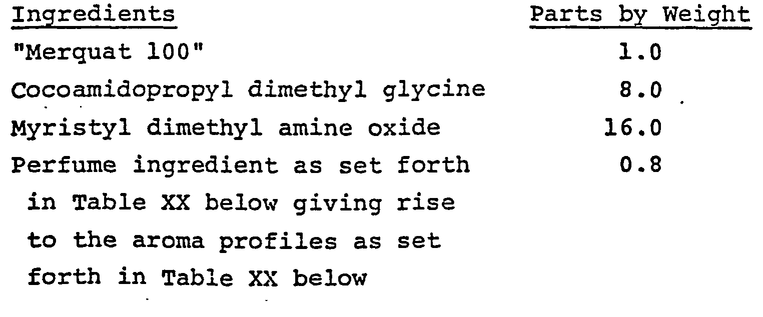

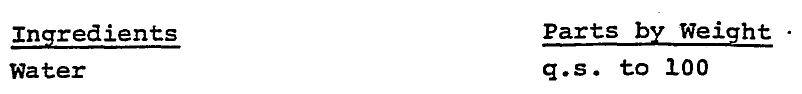

The composition is a clear liquid. Its viscosity may be increased by addition of a lauric or myristic diethanol- aminde or a soluble polyethylene glcyol ester such as polyethylene glycol 6000. In addition, this composition may be rendered opaque by addition of stearic monoethanolamide stearate, polyethylene glycol 600 monostearate or a glycoamido stearate such as "Cerasynt 1P".

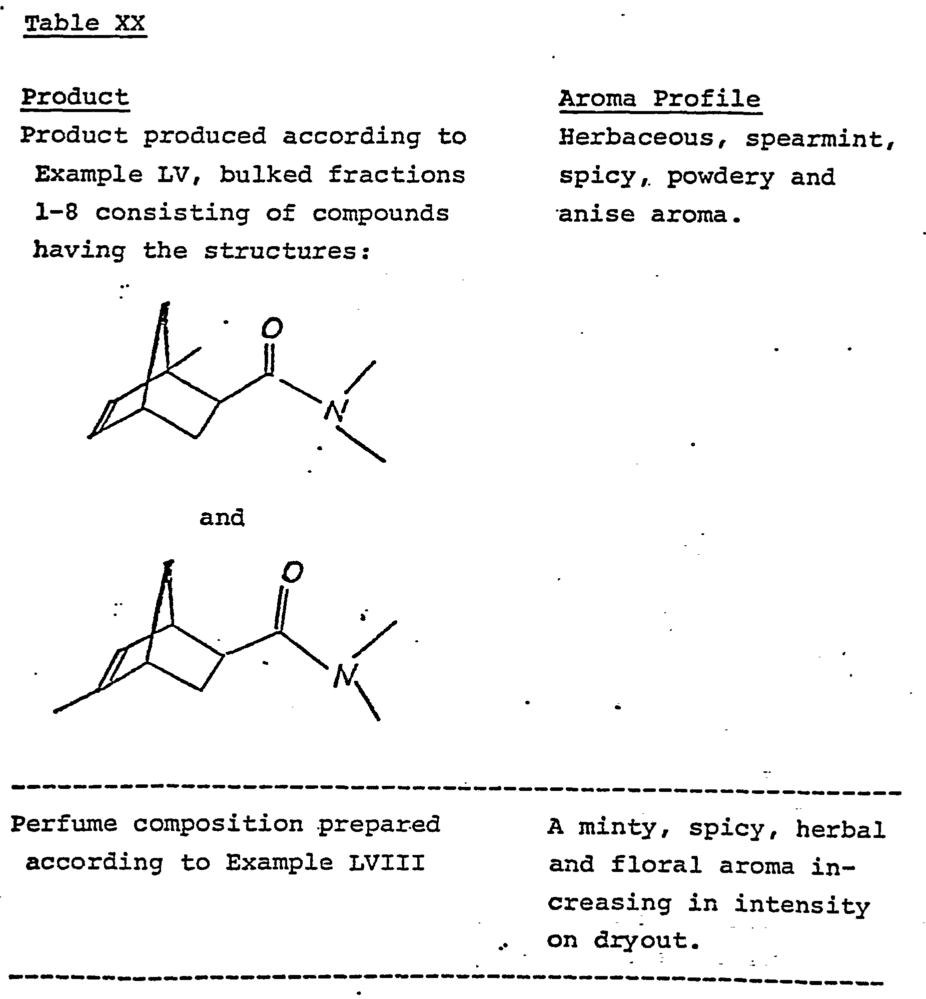

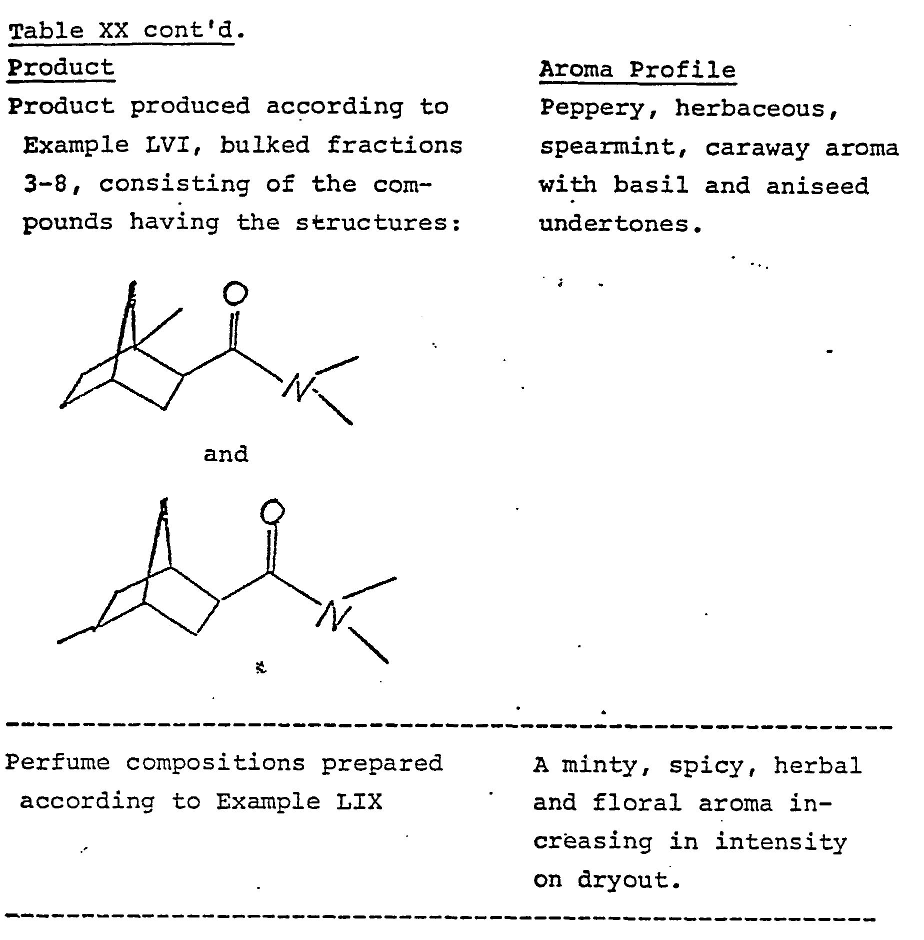

### Example LXII

The following shampoo is prepared containing the following ingredients:

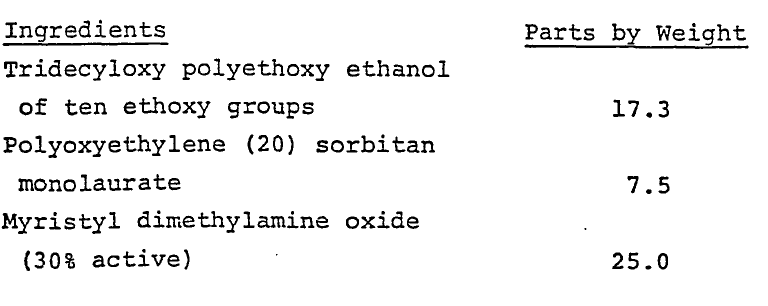

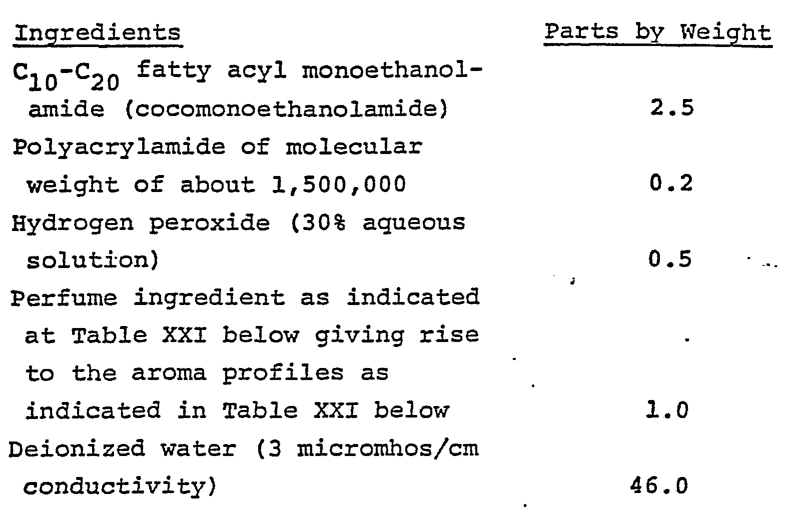

A shampoo of the above composition is made in the following manner. First, the tridecyloxy polyethoxy ethanol is added to a clean mixing,tank, with the agitator on, and the amine oxide, polyoxyethylene sorbitan monolaurate and cocomonoethanolamine are added sequentially, with continued agitation. the mix is then heated to 68°C, until the cocomonoethanolamide is melted and/or dissolved-. The hydrogen peroxide solution is then admixed with the mentioned nonionics and mixing is continued for about half an hour, in which the peroxide destroys any free amines or other harmful impurities that may be present. The mix is then cooled to 38°C.

In a separate mixer the polyacrylamide is gradually added to the formula weight of deionized water, with the mixer on. Addition is effected carefully and slowly (the polyacrylamide is sprinkled in) to avoid the production of "fish eyes" in the mix. After dissolving of the polyacrylamide the solution thereof is added to the first mixing tank with agitation and is blended with the nonionics, such mixings being at room temperature. Subsequently, the perfume as indicated in Table XXI below giving rise to the aroma profile as set forth in Table XXI below is admixed with the balance of the composition and mixing is continued for another half hour.

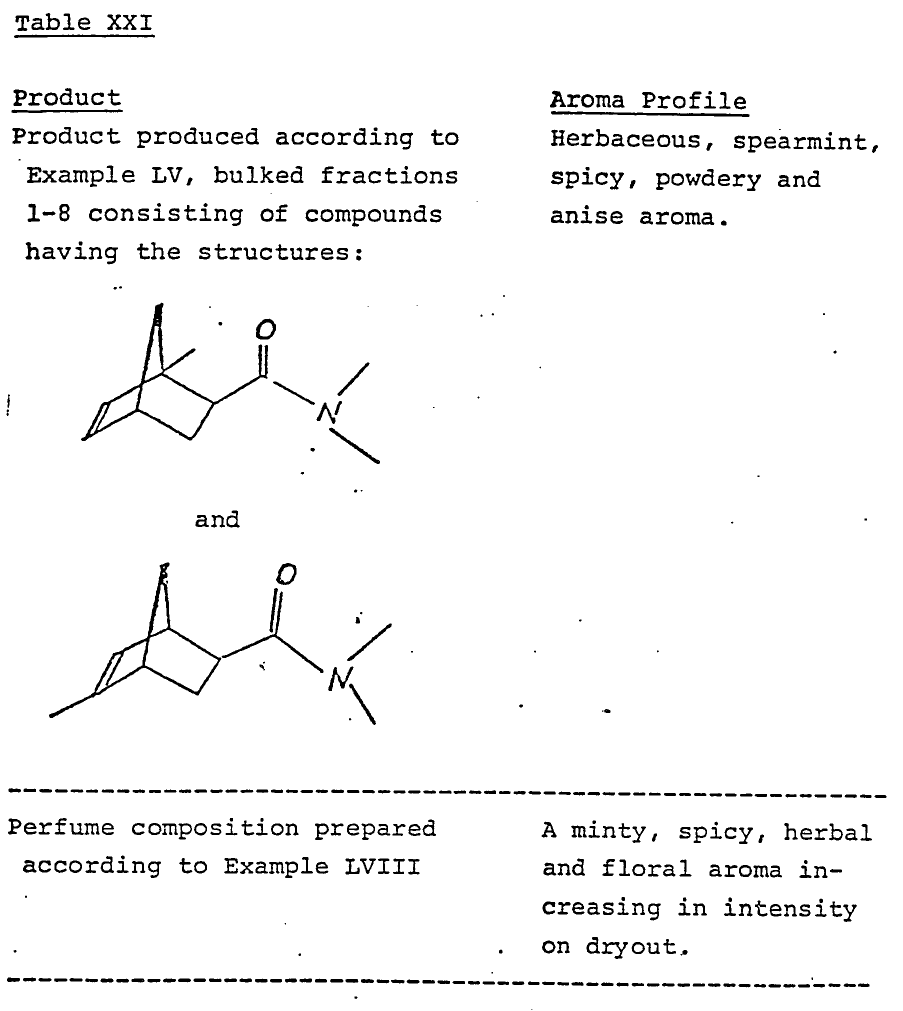

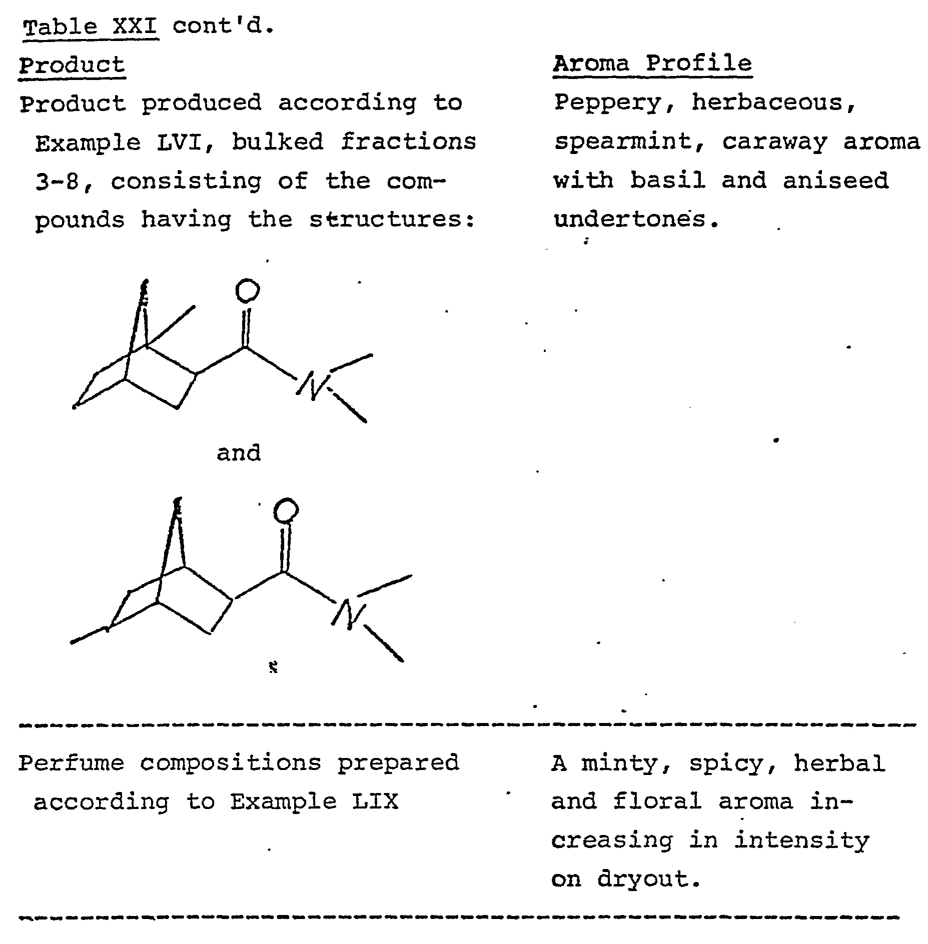

The product made is an excellent shampoo of satisfactory viscosity and aroma, foaming power, foam stability, low conductivity and good shampooing effects. The viscosity is about 1,000 centipoises at 20°C and the conductivity, using the Hach Conductivity Meter, is 750 microhos/cm. The foaming power is 250 ml and the foam stability is 22 seconds, by the test method previously described. In comparison, a commercial shampoo based on triethanolamine lauryl sulphate detergent has a conductivity of about 22,000 micromhos/cm, a viscosity of about 1,500 centipoises, a foaming power of about 380 ml and a foam stability of 60 seconds.

In panel evaluations of the experimental shampoo compared to a different commercial product, in actual shampooing, the experimental formula was adjudged significantly better in being less drying, producing a softer feel for the wet hair, leaving the wet hair easier to comb, being less prone to accumulate static charges (less flyaway) and having a foam of better appearance and feel. Additionally, the experimental product was judged better in almost all hair effect properties, with the control only being about equal to it in curl retention. In properties other than those mentioned, the experimental product was better in rinsability, the control was better in foam build-up rate and the foams were about equal in volume and stability.

In the shampooing described herein and in subsequent examples, the human hair is washed on the head by wetting the hair with warm tap water at about 42°C, applying 15 grams of shampoo to the hair, lathering it into the hair for a minute, rinsing with warm tap water for 30 seconds, re-lathering with 7 grams of shampoo for a minute and rinsing off for 30 secones, after which the hair is towel dried and dried further with an automatic hair dryer.

### Example LXIII

### Fabric freshener composition

A fabric freshener composition is prepared as follows:

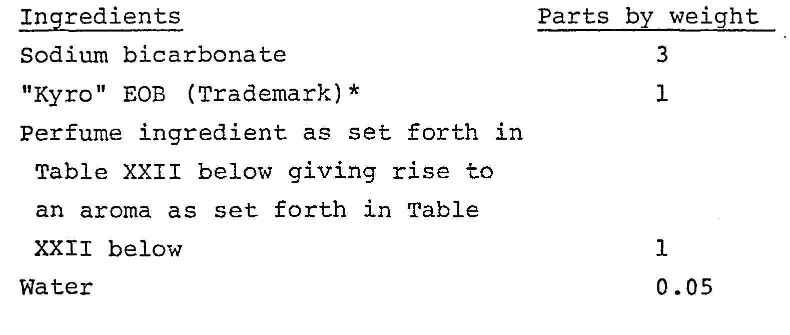

The composition of this example is prepared by simply mixing the ingredients.

The above described composition is applied to a lightly soiled and wrinkled fabric as droplets (ca. 5.0 mm avg. size) using a trigger action sprayer having a nozzle which is adjustable to provide composition droplets in the desired range. The composition is applied at a rate of about 1 gram of composition to about 10 grams of fabric.

The fabric is then placed in an automatic dryer and dried with tumbling action, at a temperature of 60°C-80°C for a period of 15 minutes. The fabric is rendered free of wrinkles and static, and has a fresh appearance and pleasant odor profile as set forth in Table XXII below.

^{*}A commercial nonionic surfactant comprising an average of eleven carbon atoms, ethoxylated to an average of 9 ethyleneoxy groups per molecule.

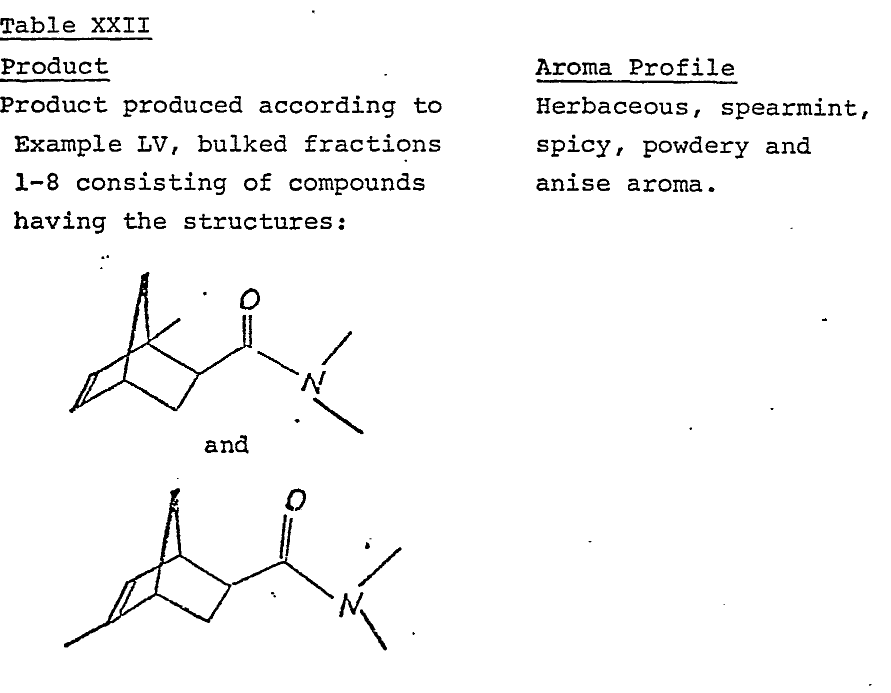

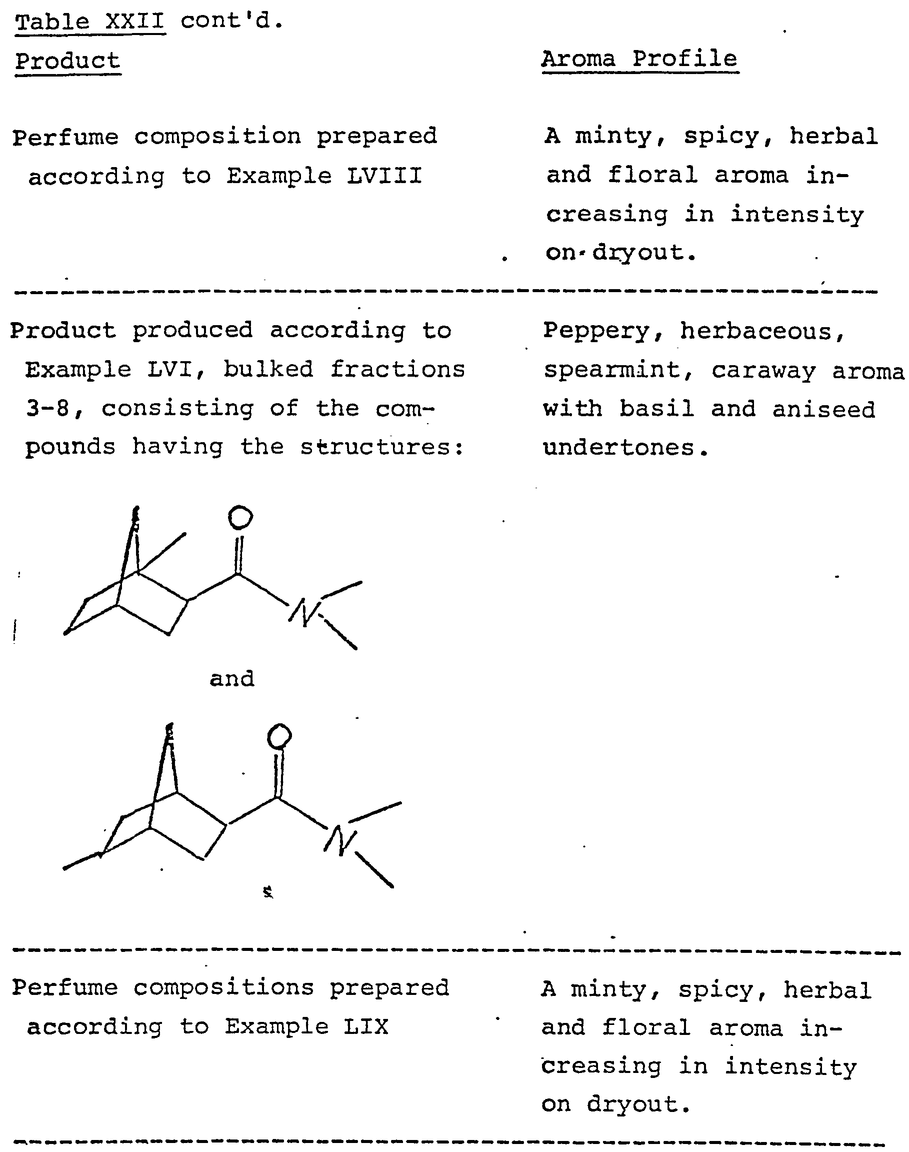

### Example LXIV

### Perfumed liquid detergent

Concentrated liquid detergents (lysine salts of n-dodecyl benzene sulfonic acid as more specifically described in _{U}.S. Patent No. 3,948,818, issued on April 6, 1976) with aromas as indicated in Table XXIII below are prepared containing 0.10%, 0.15%, 0.20%, 0.25%, 0-30%, 0.40%, 0.50% and 0.80% of the perfume ingredient as set forth in Table XXIII below. The detergents are prepared by adding and homogeneously mixing the appropriate quantity of perfume ingredient as set forth in Table XXIII below. The detergents all possess aromas as set forth in Table XXIII below with the intensity of each increasing with greater concentrations of the perfume ingredient as indicated in Table XXIII below.

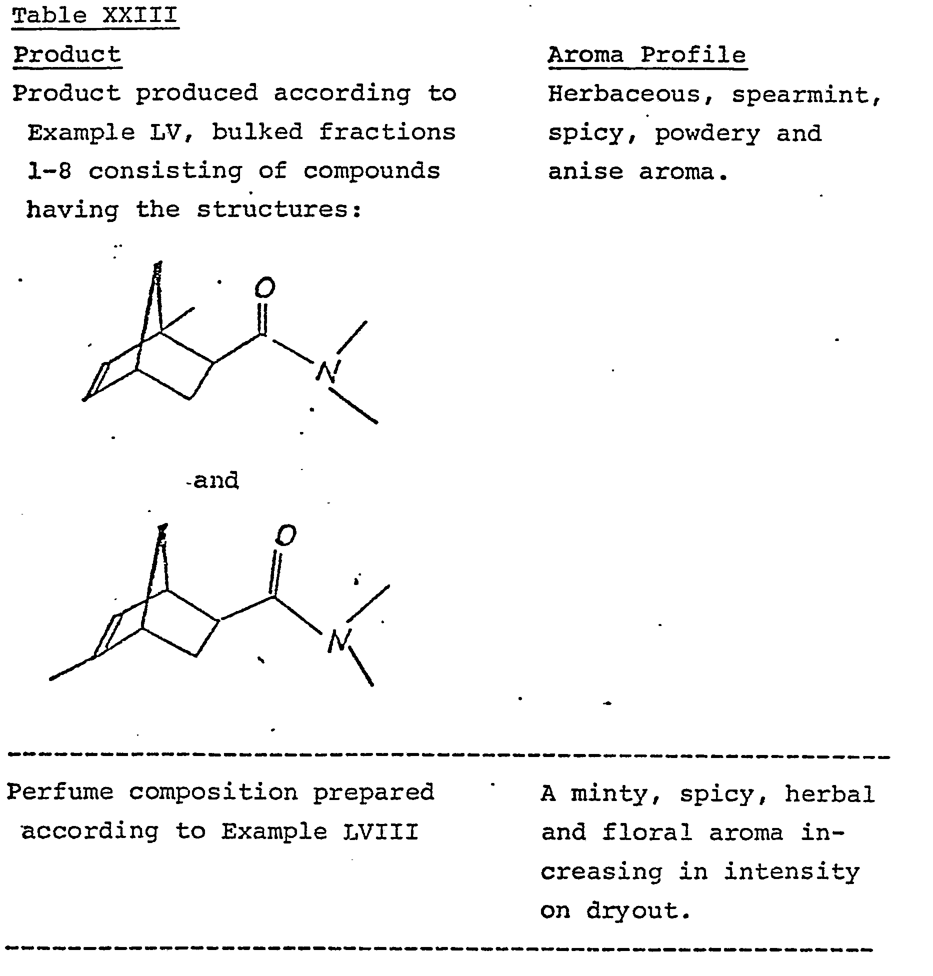

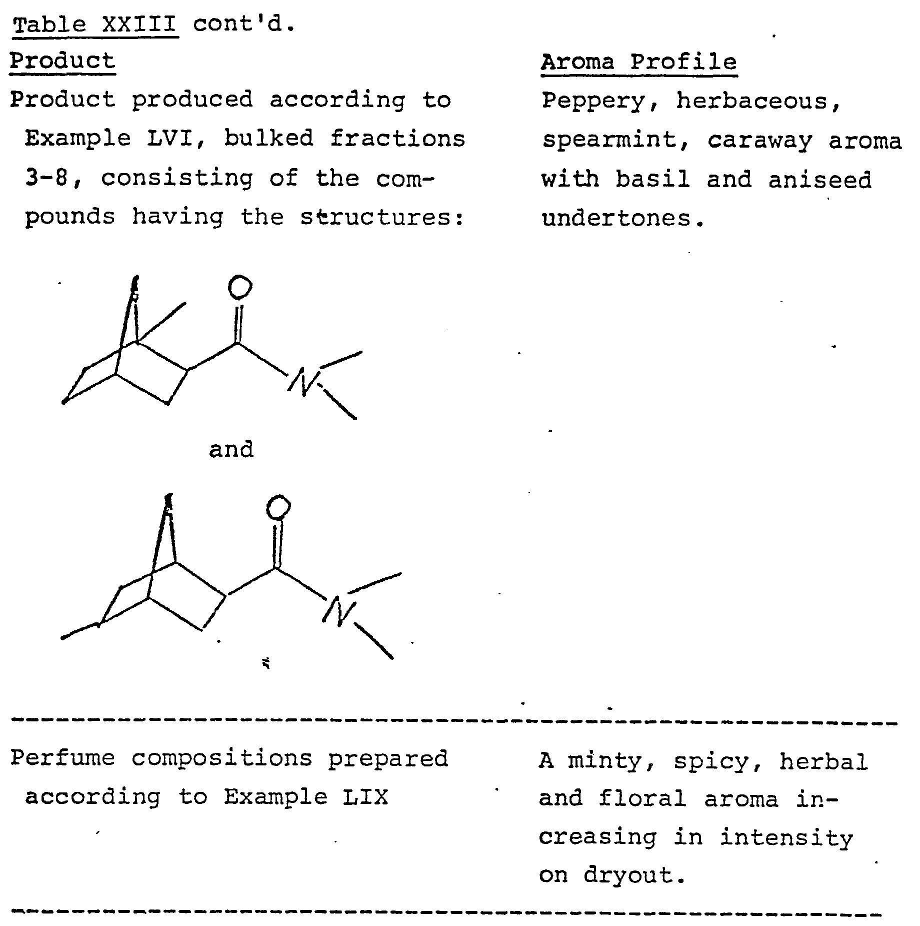

### Example LXV

### Preparation of a cologne and handkerchief perfume

The perfume ingredient as set forth in Table XXIV below is incorporated into colognes at concentrations of 2.0%, 2.5%, 3.0%, 3.5%, 4.0%, 5.0% and 6.0% in 75%, 80%, 90% and 95% aqueous ethanol solutions. Distinct and definite aromas as set forth in Table XXIV below are imparted to the colognes. The perfume ingredients as indicated in Table XXIV below are also added to handkerchief perfumes at concentrations of 15%, 20%, 25%, 30% and 35% (in 75%, 80%, 90% and 95% aqueous ethanol) and aroma profiles as set forth in Table XXIV below are imparted to the handkerchief perfume.

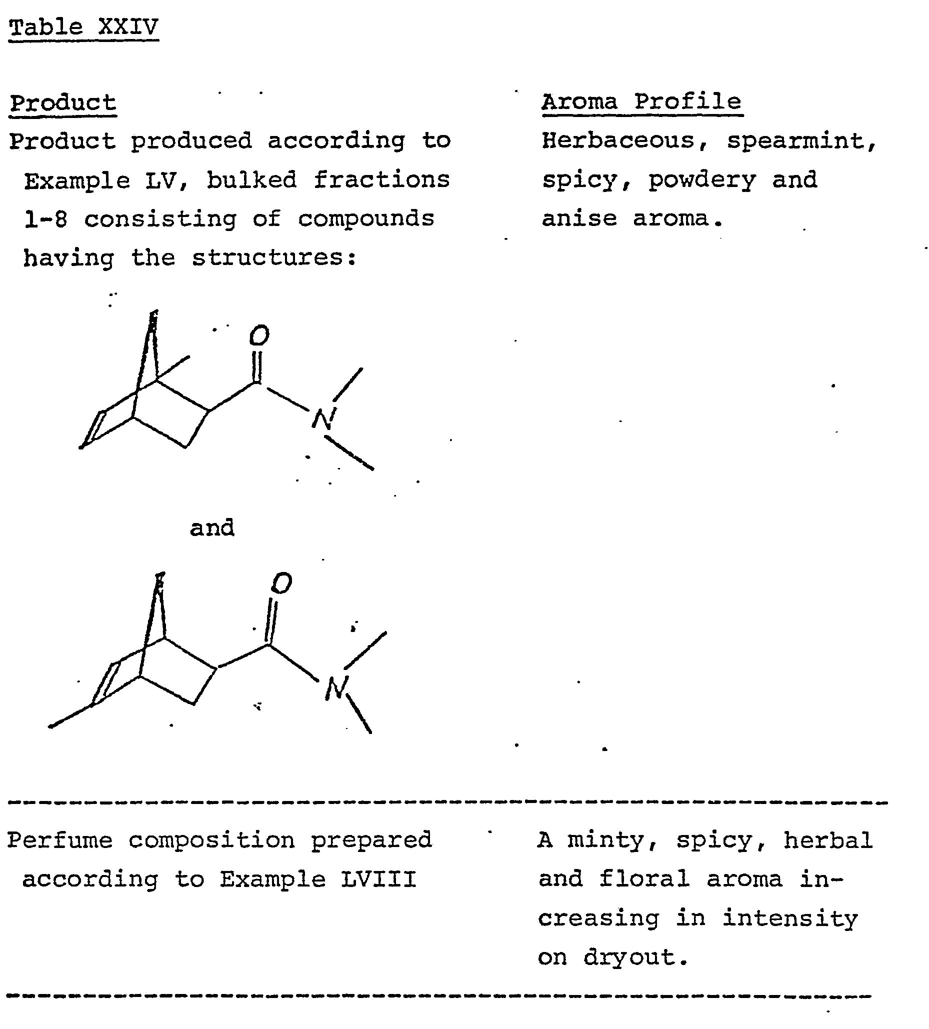

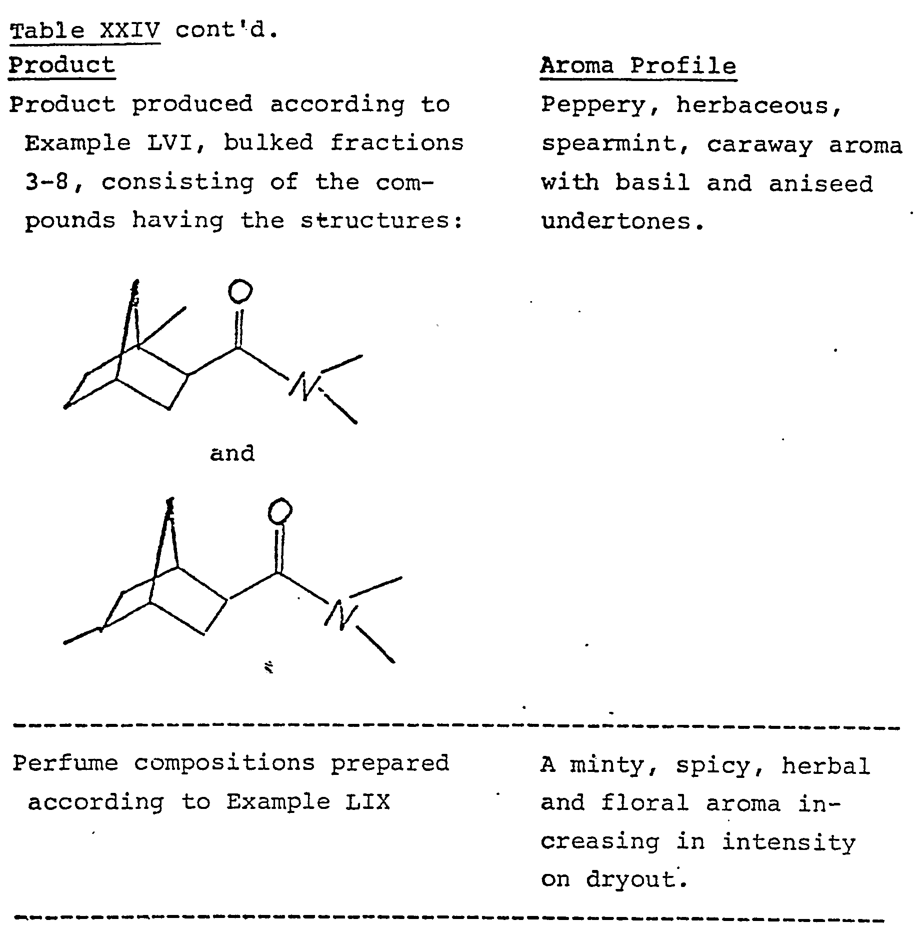

### Example LXVI

### Grapefruit formulation

The following formulation is prepared:

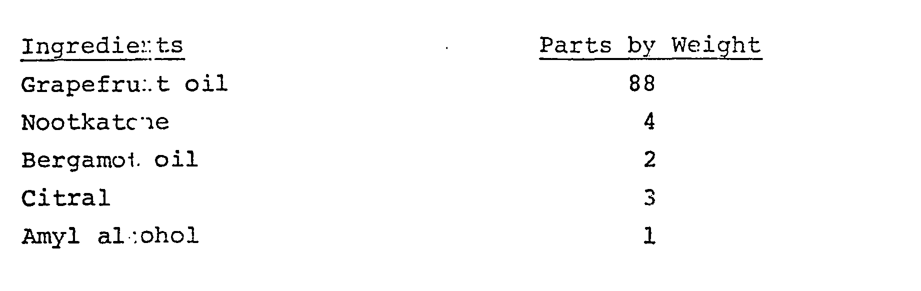

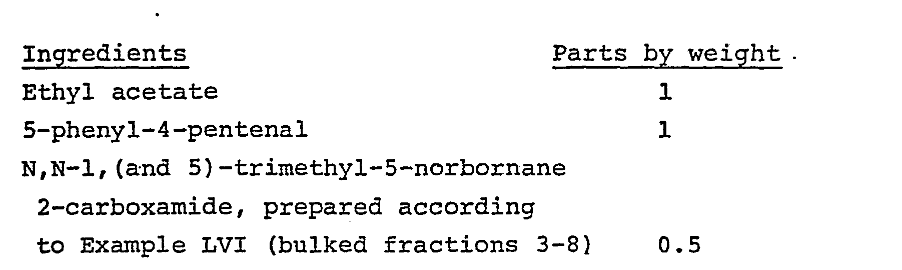

When the above grapefruit formulation is added to water at the rate of 1%, an excellent grapefruit drink is prepared. The N,N-l(and 5)-trimethyl-5-norbornane 2-carboxamide prepared according to Example LVI (bulked fractions 3-8) gives an intense sweet, anise note to the instant formulation, thereby rendering it more desirable. Additional quantities of the N,N-l(and 5)-trimethyl-5-norbornane 2-carboxamides prepared according to Example LVI, (e.g., one additional part by weight) can replace the nootkatone in the grapefruit formulation.

The foregoing grapefruit formulation can also be enhanced by one or more of the following materials at one part by weight:
4-mercapto-5-nonanone (prepared according to U.S. Patent 4,064,278)
4-mercapto-5-nonanol (prepared according to U.S.,Patent 4,064,278)
3-mercapto-2,6-dimethyl-4-heptanone prepared according to U.S. Patent 4,064,278)
2-mercapto-3-pentanone (prepared according to U.S. Patent 4,064,278)
3-mercapto-4-heptanol (prepared according to U.S. Patent 4,064,278)

Thus, our invention is also intended to cover the mixtures of alpha-oxy (oxo) mercaptans and N,N-l(and 5)-trimethyl-5-norbornane 2-carboxamides and the corresponding unsaturated norbornane derivative(s) in ratios of 0.01 parts by weight alpha-oxy (oxo) mercaptan:1 part by weight norbornane derivative(s) up to 0.01 parts by weight norbornane derivative(s):l part by weight alpha-oxy (oxo) mercaptan of U.S. Patent 4,064,278.

### Example LXVII

### A. Powder flavor composition

20 grams of the flavor composition of Example LXVI containing the compounds having the structures: 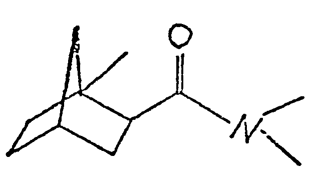 and 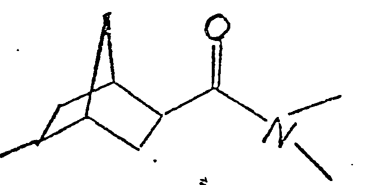 prepared according to Example LVI (bulked fractions 3 and 8) is emulsified in a solution containing 300 grams gum acacia and 700 grams water. The emulsion is spray-dried with a Bowen Lab Model Dryer utilizing 260 c.f.m. of air with an inlet temperature of 500°F, an outlet temperature of 200°F, and a wheel speed of 50,000 rpm.

### B. Sustained release flavor

The following mixture is prepared:

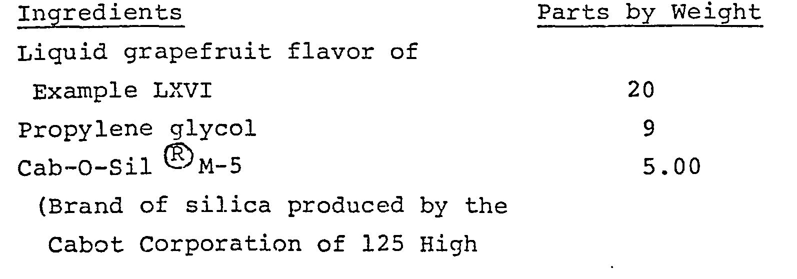

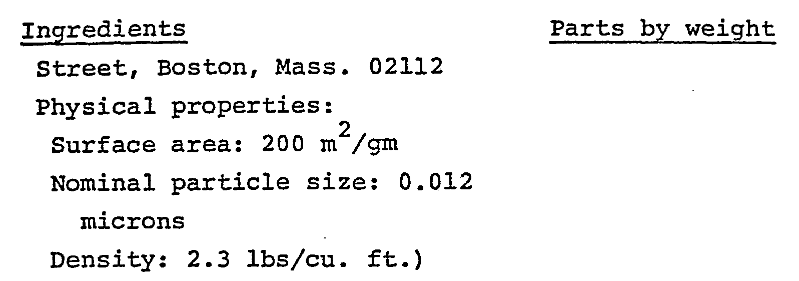

The Cab-O-Sil ®is dispersed in the liquid grapefruit flavor composition of Example LXVI with vigorous stirring, thereby resulting in a viscous liquid. 71 parts by weight of the powder flavor composition of Part A, supra, is then blended into the said viscous liquid, with stirring at 25°C for a period of thirty minutes, thereby resulting in a dry, free-flowing sustained release grapefruit flavor powder.

### Example LXVIII

10 parts by weidht of 5- Bloom pigskin gelatin is added to 90 parts by weight of water at a temperature of 150°F. The mixture is agitated until the gelatin is completely dissolved and the-solution is cooled to 120°F. 20 parts by weight of the liquid grapefruit flavor composition of Example LXVI is added to the solution which is then homogenized to form an emulsion having particle size typically in the range of 2-5 microns. This material is kept at 120°F under which conditions the gelatin will not gel.

Coacervation is induced by adding, slowly and uniformly, 40 parts by weight of a 20% aqueous solution of sodium sulphate. During coacervation, the gelatin molecules are deposited uniformly about each oil droplet as a nucleus.

Gelation is effected by pouring the heated coacervate mixture into 1,000 parts by weight of 7% aqueous solution of sodium sulphate at 65°F. The resulting gelled coacervate may be filtered and washed with water at temperatures below the melting point of gelatin, to remove the salt.

### Example LXIX

### Chewing gum

100 parts by weight of chicle are mixed with 4 parts by weight of the flavor prepared in accordance with Example _{L}XVII. 300 parts of sucrose and 100 parts of corn syrup are added. Mixing is effected in a ribbon blender with jacketed side walls of the type manufactured by the Baker Perkins Co.

The resultant chewing gum blend is then manufactured into strips 1 inch in width and 0.1 inches in thickness. The strips are cut into lengths of 3 inches each. On chewing, the chewing gum has a pleasant, long lasting grapefruit flavor.

### Example LXX

### Chewing gum

100 parts by weight of chicle are mixed with 18 parts by weight of the flavor prepared in accordance with Example LXVIII. 300 parts of sucrose and 100 parts of corn syrup are added. Mixing is effected in a ribbon blender with jacketed walls of the type manufactured by the Baker Perkins Co.

The resultant chewing gum blend is then manufactured into strips 1 inch in width and 0.1 inches in thickness. The strips are cut int.o lengths of 3 inches each. On chewing, the chewing gum hz.s a pleasant, long lasting grapefruit flavor.

### Example LXXI

### Toothpaste formulation

The following separate groups of ingredients are prepared:

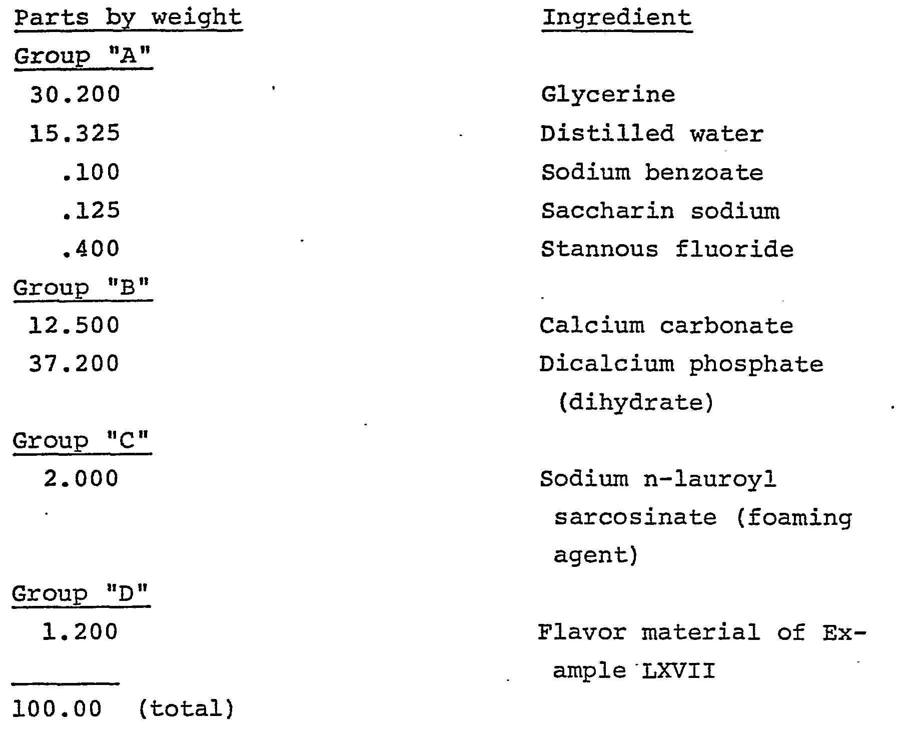

### Procedure:

1. The ingredients in Group "A" are stirred and heated in a steam jacketed kettle to 160°F.
2. Stirring is continued for an additional three to five minutes to form a homogeneous gel.
3. The powders of Group "B" are added to the gel, while mixing, until a homogeneous paste is formed.
4. With stirring, the flavor of "D" is added and lastly the sodium n-lauroyl sarcosinate.
5. The resultant slurry is then blended for one hour.

The completed paste is then transferred to a three roller mill and then homogenized, and finally tubed.

The resulting toothpaste, when used in a normal tooth- brushing procedure, yields a pleasant grapefruit flavor, of constant strong intensity throughout said procedure (1-1.5 minutes) .

### Example LXXII

### Chewable vitamin tablets

The flavor material produced according to the process of Example LXVII is added to a chewable vitamin tablet formulation at the rate of 10 grams/kilogram which chewable vitamin tablet formulation is prepared as follows:

In a Hobart Mixer the following materials are blended to homogeneity:

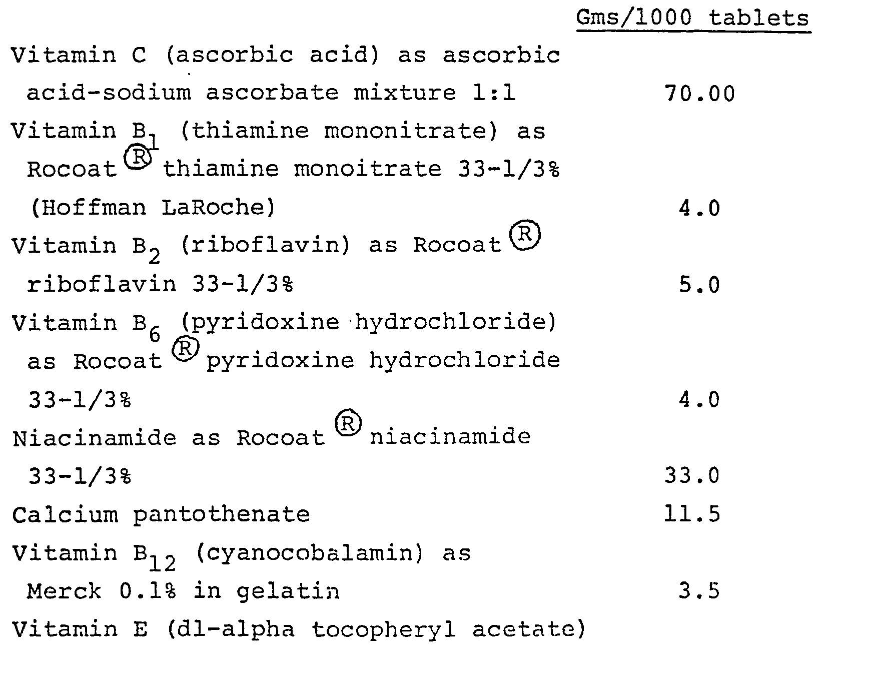

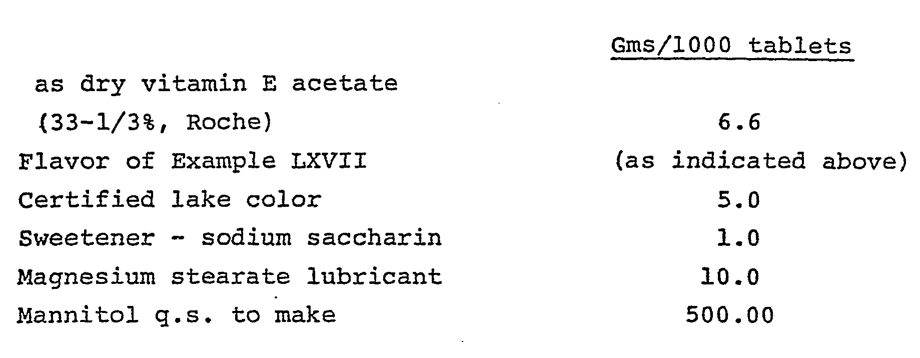

Preliminary tablets are prepared by slugging with flat- faced punches and grinding the slugs to 14 mesh. 13.5 grams dry vitamin A acetate and 0.6 grams vitamin D are then added as beadlets. The entire blend is then compressed using concave punches at 0.5 grams each.

Chewing of the resultant tablets yields a pleasant, long-lasting, consistently strong grapefruit flavor for a period of 12 minutes.

### Example LXXIII

### Chewing tobacco

Onto 100 pounds of tobacco for chewing (85% Wisconsin leaf and 15% Pennsylvania leaf) the following casing is sprayed at a rate of 30%:

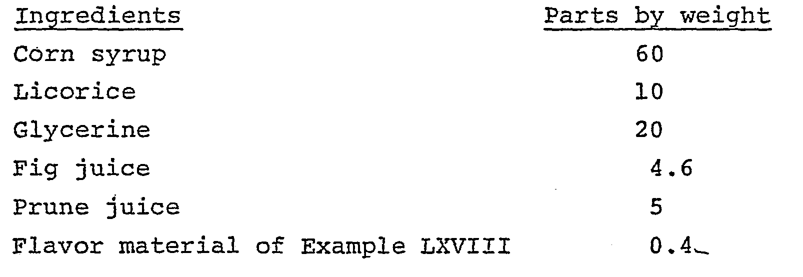

The resultant product is redried to a moisture content of 20%. On chewing, this tobacco has an excellent substantially consistent, long lasting, licorice/grapefruit flavor profile in conjunction with the tobacco note.

### Example LXXIV

### Tobacco formulation

A tobacco mixture is produced by admixing the following ingredients:

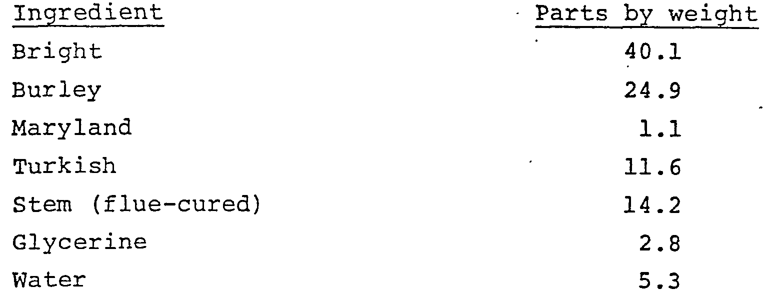

Cigarettes are prepared from this tobacco. The following flavor formulation is prepared:

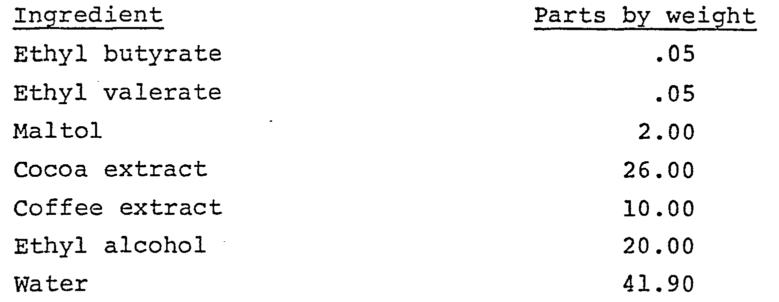

The above stated tobacco flavor formulation is applied at the rate of 0.1% to all of the cigarettes produced using the above tobacco formulation. Half of the cigarettes are then treated with 500 to 1,500 ppm of the N,N-l(and 5)-trimethyl-5-norbornane-2-carboxamide mixture produced according to Example LV.

The control cigarettes not containing the norbornyl derivative and the experimental cigarettes which contain the norbornyl derivative are evaluated by paired comparison and the results are as follows:

The experimental cigarettes are found, on smoking, to have a sweeter, fruity, more natural tobacco-like aroma prior to smoking and on smoking in the main stream and the side stream. The experimental cigarettes containing the product produced according to Example LV are basically more burley tobacco-like.

All cigarettes are evaluated for smoke flavor with a 20 mm cellulose acetate filter.

### Example LXXV

The following minty, floral and herbal type formula is prepared:

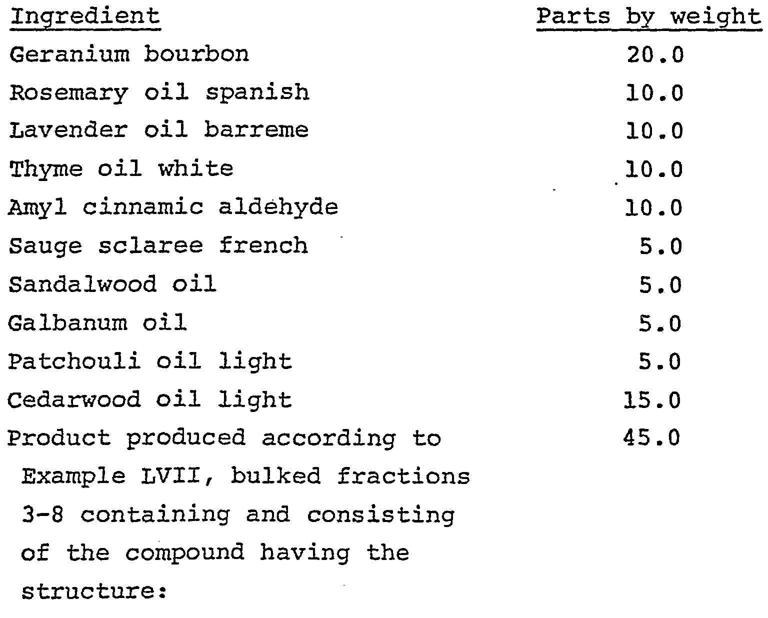

### Ingredient Parts by Weight

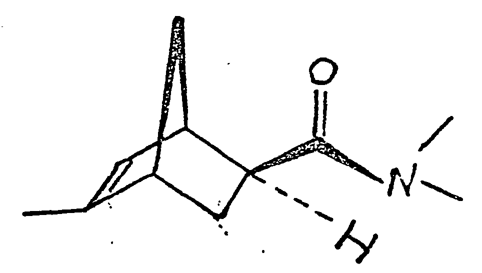

When the composition of matter prepared according to Example LVII is incorporated into the formula at 45.0%, the composition of matter has added to it a pleasant fruity, castoreum-like, herbaceous, spearmint, spicy, powdery, floral, basil, caraway and anise aroma to the already spicy, herbal and floral type formulation.

### Example LXXVI f

### Preparation of a cologne and handkerchief perfume

The perfume ingredient as set forth in Table XXV below is incorporated into colognes at concentrations of 2.0%-, 2.5_{%}, 3.0%, 3.5%, 4.0%, 5.0% and 6.0% in 75%, 80%, 90% and 95% aqueous ethanol solutions. Distinct and definitive aromas as set forth in Table XXV below are imparted to the colognes. The perfume ingredients as indicated in Table XXV below are also added to the handkerchief perfumes at concentrations of 15%, 20%, 25%, 30% and 35% (in 75%, 80_{%}, 90% and 95% aqueous ethanol) and aroma profiles as set forth in Table XXV below are imparted to the handkerchief perfume:

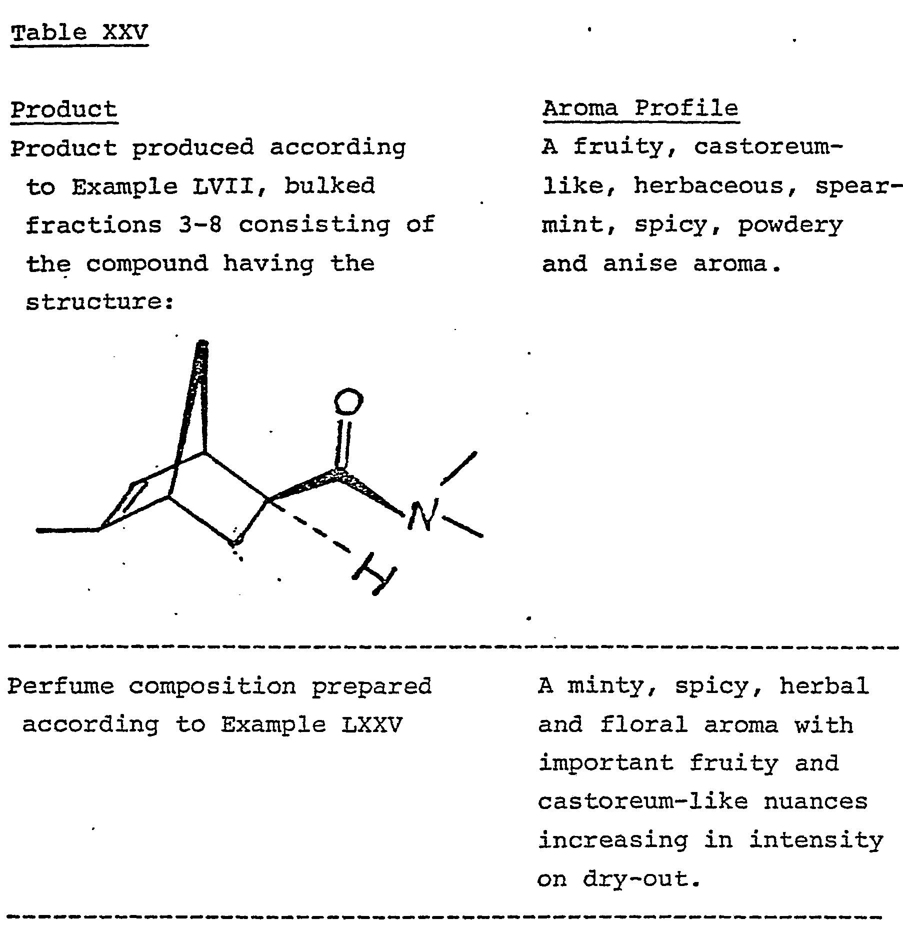

### Example LXXVII

### Perfumed liquid detergent

Concentrated liquid detergents (lysine salt of n-dodecyl benzene sulfonic acid as more specifically described in U.S. Patent No. 3,948,818, issued on April 6, 1976) with aromas as indicated in Table XXVI below are prepared containing 0.10%, 0.15%, 0.20%, 0.25%, 0.30%, 0.40%, 0.50% and 0.80% of the perfume ingredient as set forth in Table XXVI below. The detergents are prepared by adding and homogeneously mixing the appropriate quantity of perfume ingredient as set forth in Table XXVI below. The detergents all possess aromas as set forth in Table XXVI below with the intensity of each increasing with greater concentrations of the perfume ingredient as indicated in Table XXVI below.

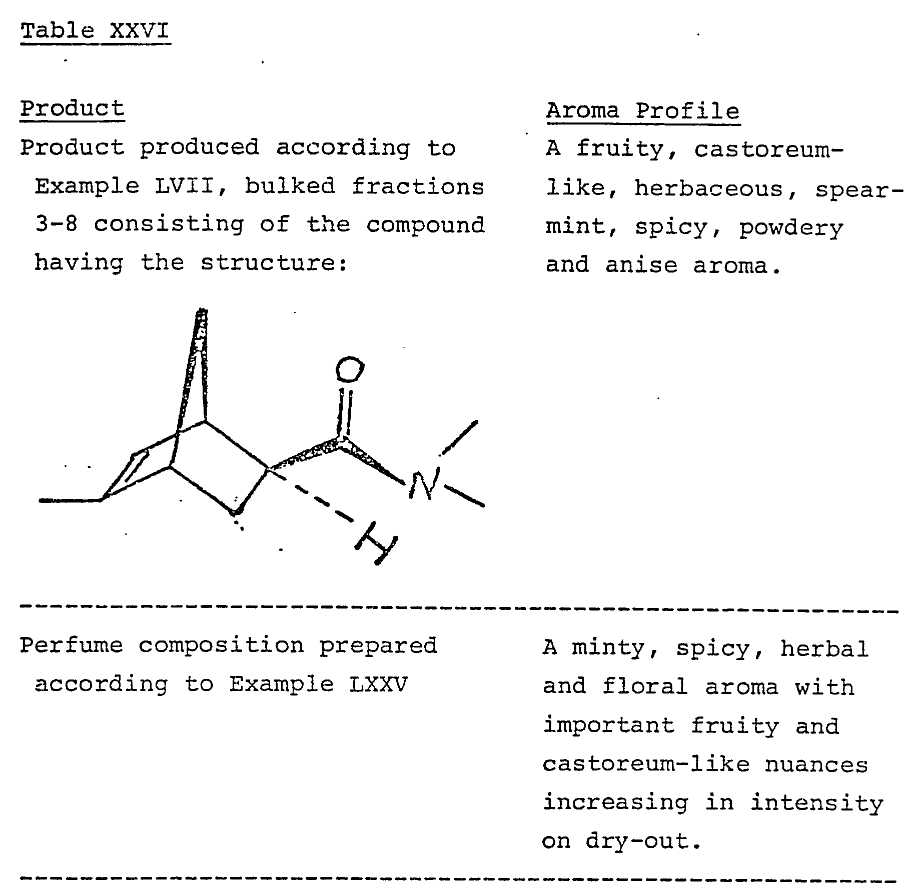

Preferred.features of the present invention are:-
1. A process for augmenting or enhancing the aroma or taste of a consumable material which is, in the alternative, a foodstuff, a chewing gum, a toothpaste, a medicinal product, a smoking tobacco, a chewing tobacco, a perfume, a perfumed article, a cologne, a smoking tobacco or a smoking tobacco article comprising a carbonyl norbornyl derivative admixed with the consumable material;
   characterized in that
   the carbonyl norbornyl derivative has the generic structure: 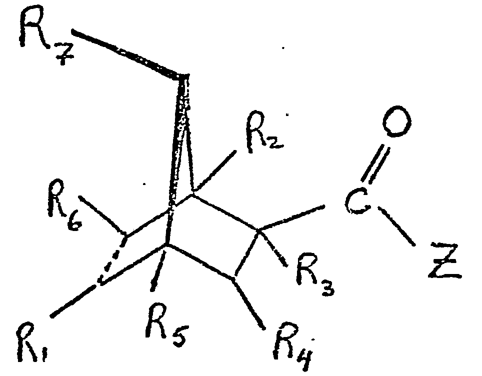 wherein the dashed line represents a carbon-carbon single bond or a carbon-carbon double bond; wherein Z is in the alternative one of the moieties: 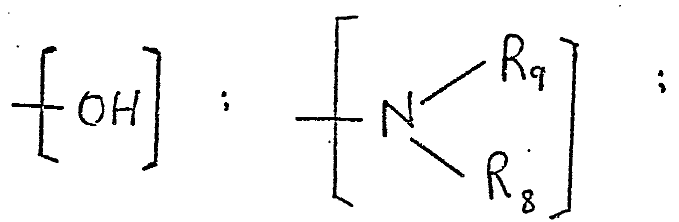 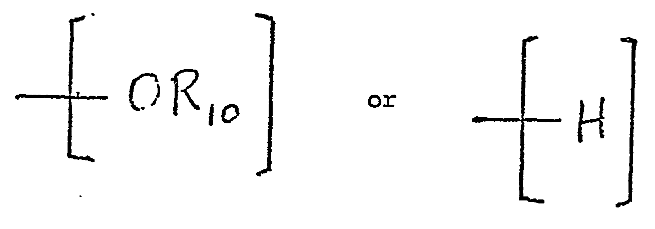 wherein R₁, R₂, R₃, R₄, R₅, R₆ and R₇ are each in the alternative hydrogen or methyl; wherein R₈ and R₉ are in the alternative the same or different and are hydrogen or C₁- _{C4} alkyl; with the proviso that one of R₁, R₂, R₅, R₆ or _{R}7 is methyl and the other of R₁, R₂, R₅, R and R₇ is hydrogen; and with the additional proviso that R₃ and R₄ are not both methyl.
2. The process of Claim 1 wherein the consumable material is a foodstuff.
3. The process of Claim 1 wherein the consumable material is a perfume composition or cologne.
4. The process of Claim 1 wherein the consumable material is a chewing gum.
5. The process of Claim 1 wherein the consumable material is a medicinal product.
6. The process of Claim 1 wherein the consumable material is a smoking tobacco.
7. The process of Claim 1 wherein the consumable material is a perfumed article and the perfumed article is a solid or liquid anionic, cationic, nonionic or zwitterionic detergent.
8. The process of Claim 1 wherein the consumable material is a perfumed article and the perfumed article is a fabric softener composition or a fabric softener article of manufacture.
9. The process of Claim 1 wherein the consumable material is a smoking tobacco or a smoking tobacco article.
10. A carbonyl norbornane compound characterized in that it has the generic structure: 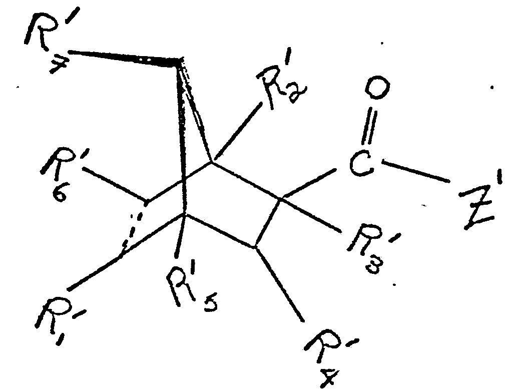 wherein the dashed line represents a carbon-carbon single bond or a carbon-carbon double bond; wherein Z' represents a moiety having the structure: 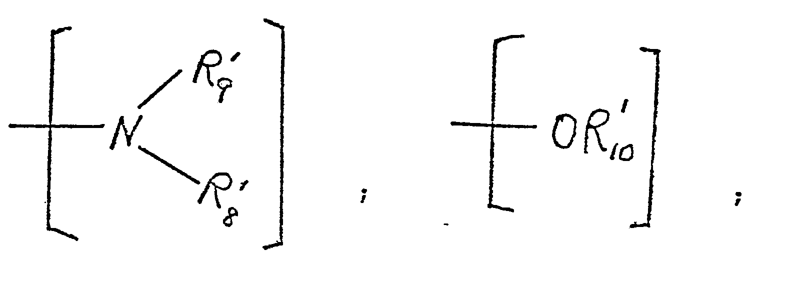 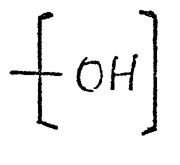 or 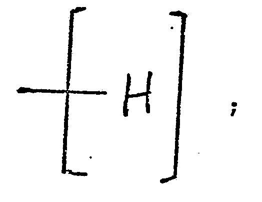
   wherein one of R₁', R₂', R₅', R₆' or R₇' is methyl and
   each of the other of R₁', R₂', R₅', R₆'and R₇' are hydrogen;
   where one of _{R3}' or R₄' is methyl and the other of R₃' or
   ^{R}₄' is hydrogen; with the provisos that;
      A. when Z'has the structure: 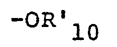 then when the dashed line is a carbon-carbon double bond, R₁', R₃', and R₄' are not all hydrogen when R₂' is methyl;
      B. when Z' is hydrogen, then:
         1. R₁' or R₂' are hydrogen or methyl;
         2. R₄', is hydrogen or methyl;
         3. and R₃', R₅', R₆' and R₇' are each hydrogen;
         and
      C. when Z' has the structure: 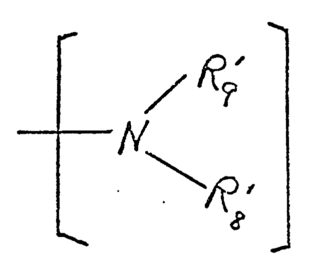 then one of R₁', R₂' and R₇' is hydrogen and the other of R₁', R₂' and R₇' is methyl; one of R₃', or R₄' is hydrogen and the other of R₃' or R₄' is methyl and R₅' and R₆' are both hydrogen;
   and wherein R₈' and R₉' are each hydrogen or C₁-₃ lower alkyl; and wherein R₁₀' is C₁-C₄ lower alkyl.
11. A carbonyl norbornane compound defined according to Claim 10 wherein the structure of the carbonyl norbornane compound is: 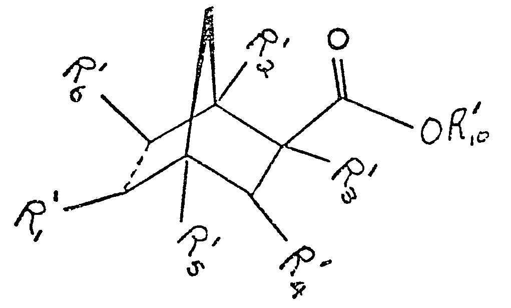 wherein one of R₁', R₂', R₅' or R₆' is methyl and each of the other of R₁', R₂', R_{S}' or R₆' is hydrogen; wherein one of R₃' or R₄' is hydrogen and the other of R₃' or R₄' is methyl; wherein R₁₀' is C₁-C₄ lower alkyl; wherein the dashed line represents a carbon-carbon single bond or a carbon-carbon double bond; with the proviso that when the dashed line is a carbon-carbon double bond, R₁', R₃', R₄', R_{S}' and R₆' are not all hydrogen when R₂' is methyl.
12. The process for preparing the carbonyl norbornane compound of Claim 11 comprising the step of intimately admixing a mixture of methyl cyclopentadienes having the structures: 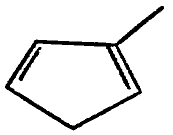 and 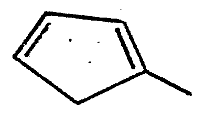 with at least one acrylic acid ester having the structure: 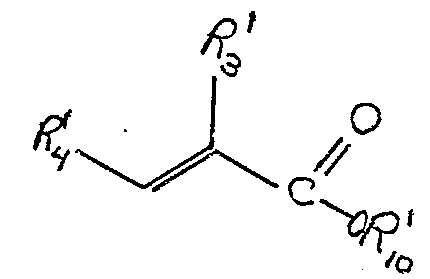 wherein in the acrylic acid ester R₃' and R₄' may be "cis" or "trans" in configuration, at a temperature of from 170°C up to 250°C at a pressure greater than atmospheric pressure.
13. The process of Claim 12 wherein in the carbonyl norbornane structure the dashed line is a carbon-carbon single bond and the process includes the additional step of reacting the resulting product wherein the dashed line in the carbonyl norbornane structure is a double bond, with hydrogen.
14. The product prepared according to the process of Claim 12.
15. The product prepared according to the process of Claim 13.
16. A methyl substituted carbonyl norbornane derivative defined according to Claim 10 further characterized in that it has the structure: 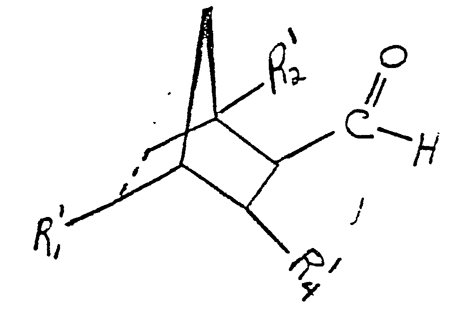 wherein the dashed line represents a carbon-carbon single bond or a carbon-carbon double bond; and wherein R₁', R₂' and R₃¹ are each selected from the group consisting of methyl and hydrogen with the proviso (i) when Rₗ' is hydrogen, R₃' is methyl and (ii) R₁' is methyl, R₃' is hydrogen.
17. A process for augmenting or enhancing the organoleptic properties of a consumable material which is in the alternative a perfume composition, a perfumed article, a co cologne, a foodstuff, a chewing gum, a toothpaste, a medicinal product, or a chewing tobacco comprising the step of adding an organoleptic property modifying quantity of at least one methyl substituted carbonyl norbornane derivative to said consumable material, characterized in that the carbonyl norbornane derivative is defined according to Claim 16.
18. A process for preparing at least one methyl substituted carbonyl norbornane derivative defined according to Claim 16 comprising the step of intimately admixing at least one methyl cyclopentadiene having the structure which is in the alternative: 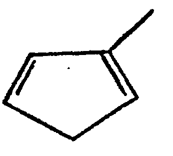 and 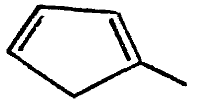 with an α,β-unsaturated aldehyde defined according to the structure: 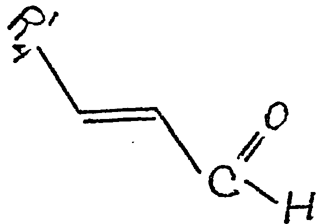 wherein R₄' is methyl or hydrogen, in the presence of a catalyst having the structure: 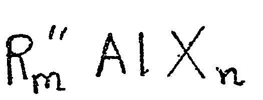 wherein R" is Cₗ-C₃ alkyl and X is chloro or bromo with m + n equal 3 and m being 1 or 2 and n being 1 or 2.
19. The process of Claim 18 comprising the additional step of reacting hydrogen with the resulting reaction product containing at least one compound having the structure in the alternative: 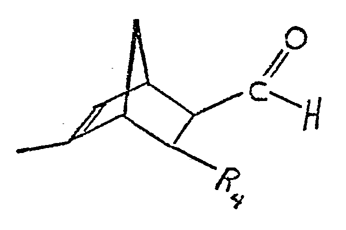 or 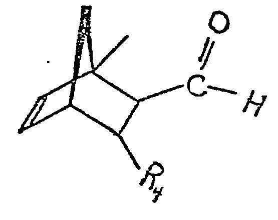 whereby at least one compound having a structure in the alternative: 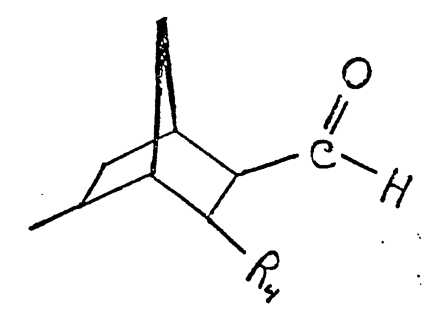 .or 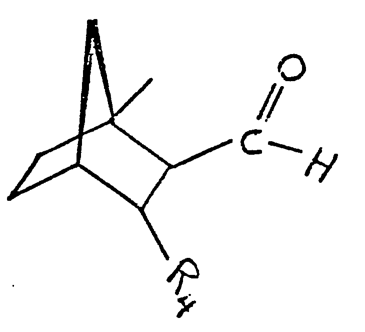 is formed wherein R₄ is hydrogen or methyl, the reaction taking place in the presence of a catalyst which is in the alternative supported palladium or Raney nickel.
20. A carbonyl norbornane derivative defined according to Claim 10 further characterized in that it has the structure: 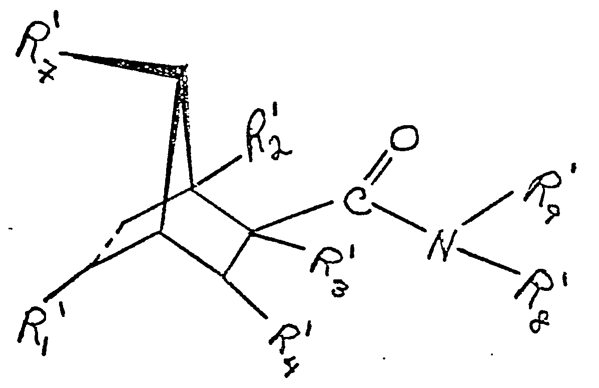 wherein the dashed line represents a carbon-carbon single bond or a carbon-carbon double bond and wherein R₁', R₂' and R₇' are methyl or hydrogen with one of R₁', R₂' or R₇' being methyl and the other of R₁', R₂' or R₇' being hydrogen; wherein R₃' and R₄' are hydrogen or methyl with one of _{R3}' or R₄' being hydrogen and the other of R₃' or R₄' being methyl; wherein R₈' and R₉' are the same or different and each represents hydrogen or C1-C₃ lower alkyl.
21. A process for augmenting or enhancing the aroma or taste of a consumable material which is in the alternative a perfume composition, a perfumed article, a cologne, or a foodstuff comprising the step of adding to a perfume base, a perfumed article base, a cologne base or a foodstuff base an organoleptic property modifying quantity of at least one compound defined according to Claim 20.
22. The compound of Claim 20 wherein the dashed line is a carbon-carbon double bond.
23. The process of Claim 21 wherein in the carbonyl norbornane derivative, the dashed line is a carbon-carbon double bond.
24. A process for preparing the compound defined according to Claim 20 comprising the step of reacting an acrylamide defined according to the structure: 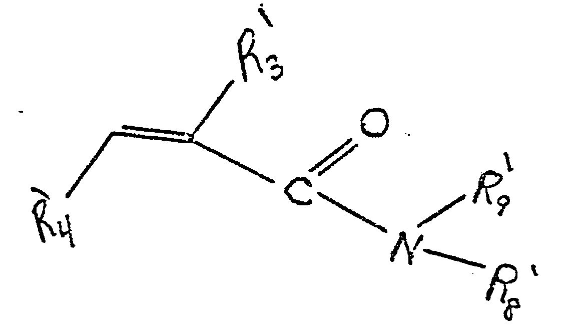 with one or more methyl cyclopentadienes defined according to the structure: 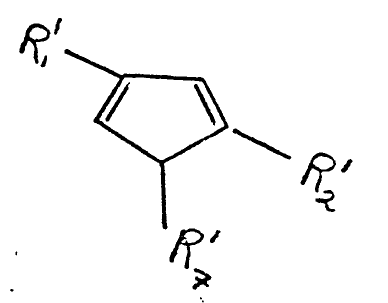 wherein R₁', R₂', R₃', R₄' and R₇' represent methyl or hydrogen; and R₈' or R₉' represent C₁-C₃ lower alkyl; said reaction taking place in the absence of a catalyst at a temperature in the range of 170-250°C and a pressure greater than atmospheric pressure.
25. The process of Claim 24 comprising the additional step of reacting the resulting compound with hydrogen in the presence of a hydrogenation catalyst, thus effecting the reaction sequence: 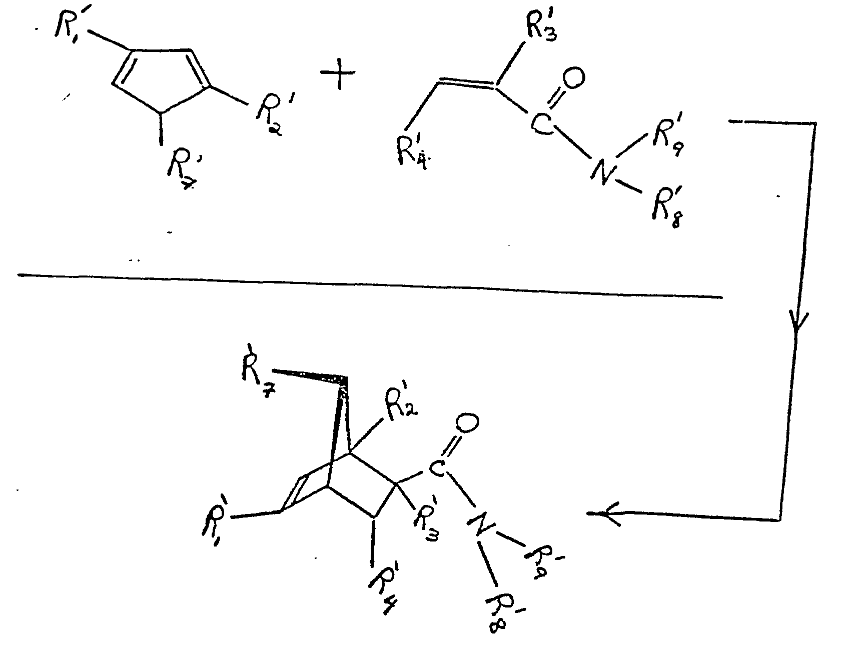 and 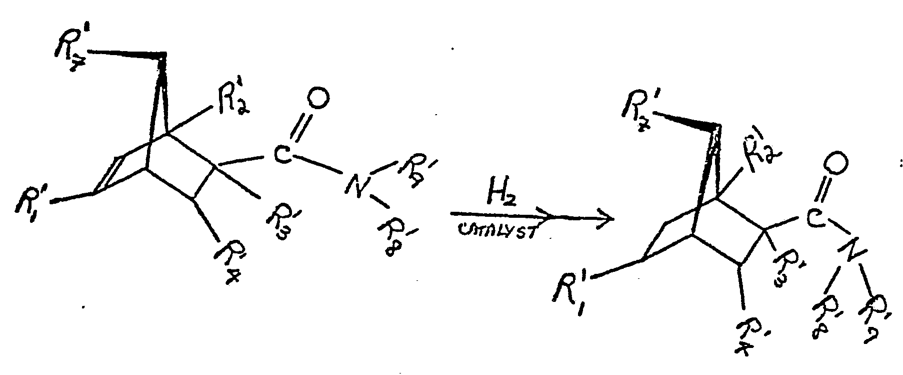 wherein R₁', R₂', R₃', R₄' and R₇¹ represent hydrogen or methyl and wherein R₈' and R₉' represent C₁-C₃ lower alkyl.
26. A process for augmenting or enhancing the grapefruit aroma or taste of a foodstuff which comprises adding thereto from about 0.02 ppm up to about 50 ppm by weight of said foodstuff of at least one compound defined according to Claim 20.
27. A composition for augmenting or enhancing the grapefruit flavor of a foodstuff comprising (i) from about 0.05% up to about 5% by weight of said flavor of at least one compound defined according to Claim 20, the remainder of said composition being (ii) at least one adjuvant which is in the alternative or in combination:
   grapefruit oil
   bergamot oil
   citral
   amyl alcohol, and/or
   5-phenyl 4-pentenal.
28. The carbonyl norbornane derivative defined according to Claim 20 having the structure: 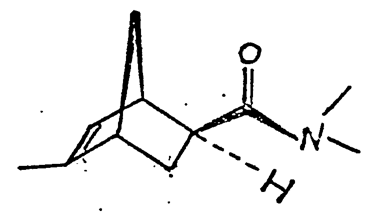
29. The process of Claim 24 according to the reaction: 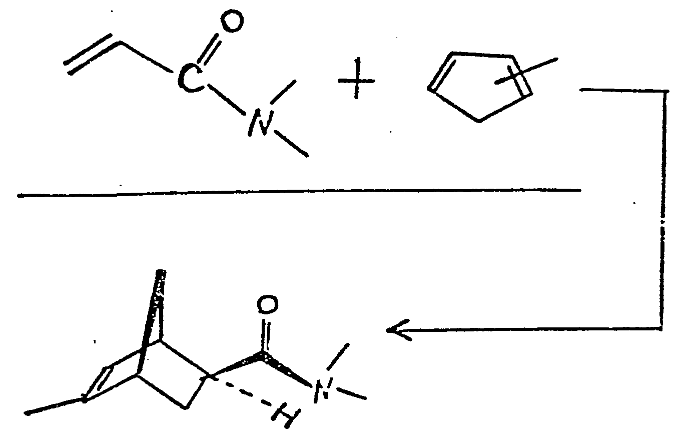
30. A process for augmenting or enhancing the aroma of a perfume composition, perfumed article or cologne comprising the step of adding to a perfume base, a cologne base or a perfumed article base, the compound of Claim 28.
31. The product produced according to the process of Claim 24.
32. The product produced according to the process of Claim 25.
33. The process for preparing a norbornyl carbonyl derivative according to the reaction sequence: 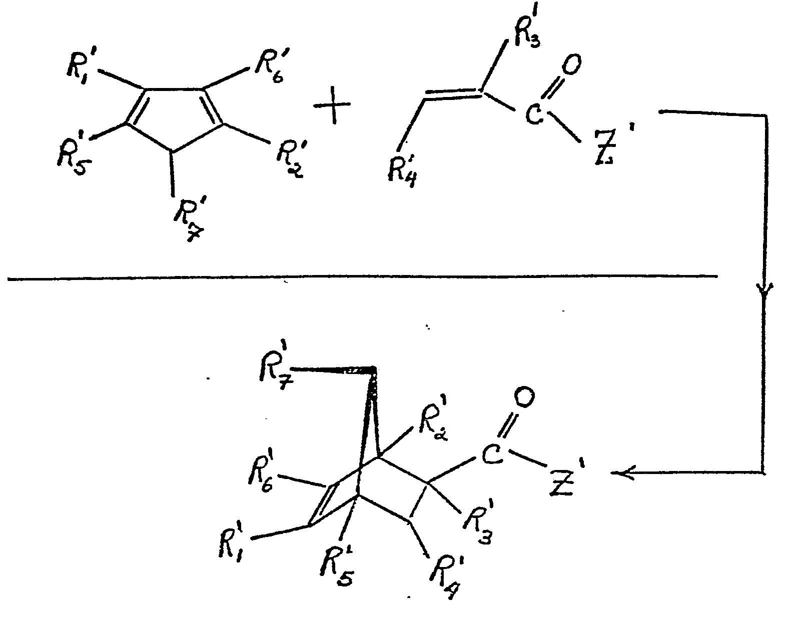 wherein R₁', R₂', R₃', R₄', R₅', R₆', and R₇' represent hydrogen or methyl; wherein one of R₁', R₂', R₅', R₆' and R₇' represent methyl and the other of R₁', R₂', R₅', R₆' and R₇' represents hydrogen; wherein one of R₃' or R₄' represents hydrogen and the other of R₃' or R₄' represents methyl; and wherein Z'represents one of the structure: 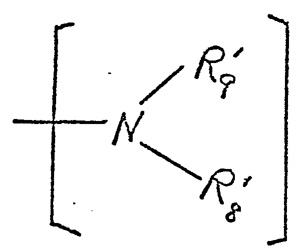 or 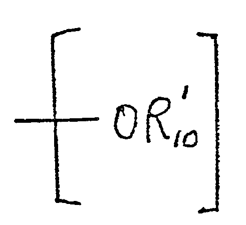 or hydrogen; wherein R₈' and R₉' are the same or different and each represents hydrogen or alkyl; wherein R₁₀' represents C₁-C₄ alkyl, comprising the steps of reacting at least one methyl-substituted cyclopentadiene having the structure: 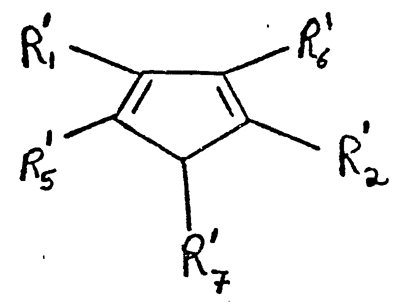 with at least one unsaturated carbonyl derivative having the structure: 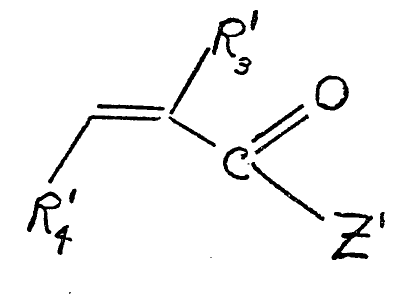 either
   (i) in the presence of a catalyst having the structure: 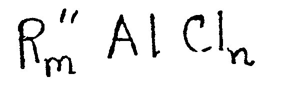 at from 0-50°C at atmospheric pressure or
   (ii) in the absence of a catalyst at super-atmospheric pressures at a temperature of from 170°C up to 250°C

   wherein R" represents C₁-C₃ alkyl and m + n = 3 with m being 1 or 2 and n being 1 or 2.
34. The process of Claim 33 wherein Z' is OR₁₀' and wherein the reaction is carried out in the presence of a catalyst at from 0-50°C and the products resulting from the process have the structures: 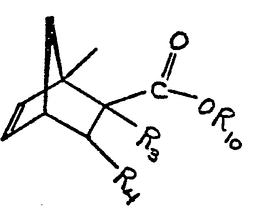 and 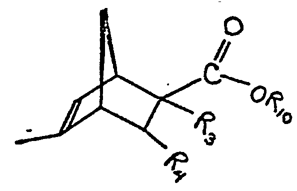 wherein one of R₃ or R₄ is methyl and the other of R₃ or R₄ is hydrogen and R₁₀ represents C₁-C₃ alkyl.

## Claims

1. A process for augmenting or enhancing the aroma or taste of a consumable material which is, in the alternative, a foodstuff, a chewing gum, a toothpaste, a medicinal product, a smoking tobacco, a chewing tobacco, a perfume, a perfumed article, a cologne, a smoking tobacco or a smoking tobacco article comprising a carbonyl norbornyl derivative admixed with the consumable material; characterized in that
the carbonyl norbornyl derivative has the generic structure: 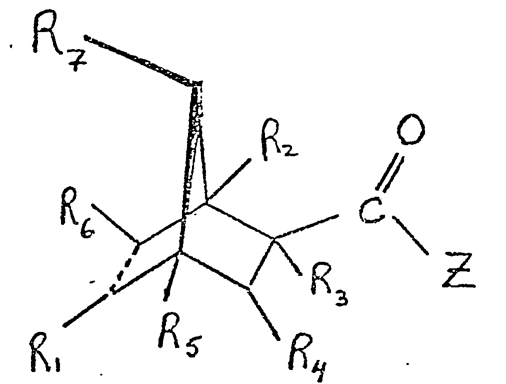 wherein the dashed line represents a carbon-carbon single bond or a carbon-carbon double bond; wherein Z is in the alternative one of the moieties: 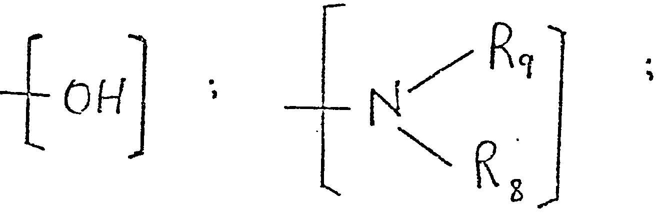 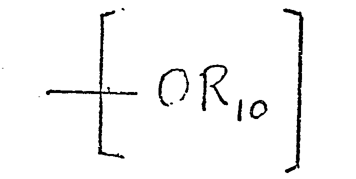 or 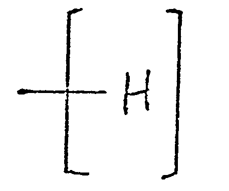 wherein R₁, R₂, R₃, R₄, R₅, R₆ and R₇ are each in the alternative hydrogen or methyl; wherein R₈ and R₉ are in the alternative the same or different and are hydrogen or C₁-C₄ alkyl; with the proviso that one of R₁, R₂, R₅, R₆ or R₇ is methyl and the other of R₁, R₂, R₅, R₆ and R₇ is hydrogen; and with the additional proviso that R₃ and R₄ are not both methyl.

2. A carbonyl norbornane compound characterized in that it has the generic structure: 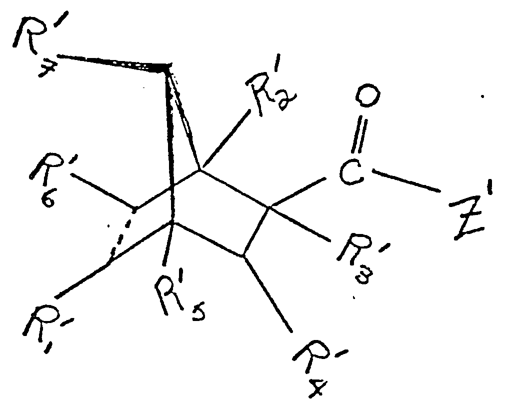 wherein the dashed line represents a carbon-carbon single bond or a carbon-carbon double bond; wherein Z' represents a moiety having the structure: 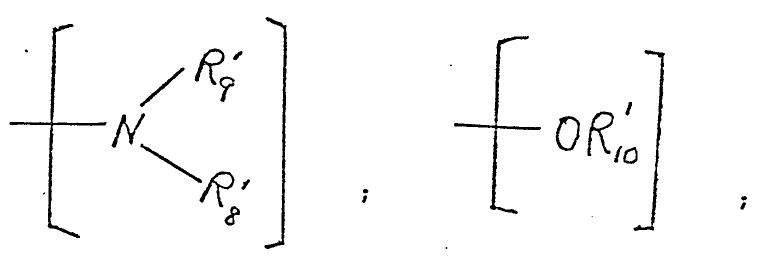 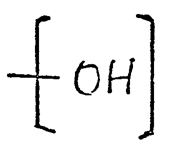 or 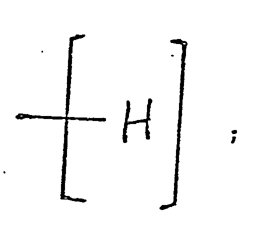 wherein one of R₁', R₂', R₅', R₆' or R₇' is methyl and each of the other of R₁', R₂', R₅', R₆' and R₇' are hydrogen; where one of R₃' or R₄' is methyl and the other of R₃' or R₄' is hydrogen; with the provisos that;
A. when Z'has the structure: 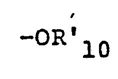 then when the dashed line is a carbon-carbon double bond, R₁', R₃', and R₄' are not all hydrogen when R₂' is methyl;
B. when Z' is hydrogen, then:
1. R₁' or R₂' are hydrogen or methyl;
2. R₄' is hydrogen or methyl;
3. and R₃', R₅', R₆' and R₇' are each hydrogen;
and
C. when Z' has the structure: 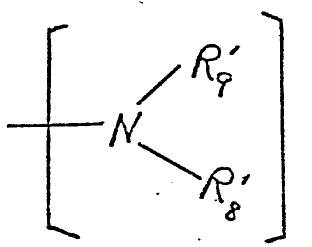 then one of R₁', R₂' and R₇' is hydrogen and the other of R₁', R₂' and R₇' is methyl; one of R₃', or R₄' is hydrogen and the other of R₃' or R₄' is methyl and R₅' and R₆' are both hydrogen;
and wherein R₈' and Rg' are each hydrogen or C₁-C₃ lower alkyl; and wherein R₁₀' is Cₗ-C₄ lower alkyl.

3 . A carbonyl norbornane compound defined according to Claim ₂, wherein the structure of the carbonyl norbornane compound is: 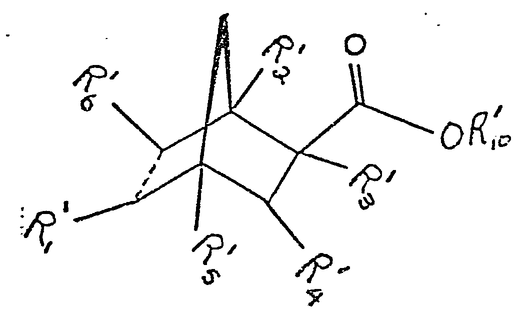 wherein one of R₁', R₂', R₅' or R₆' is methyl and each of the other of R₁', R₂', R₅' or R₆' is hydrogen; wherein one of R₃' or R₄' is hydrogen and the other of R₃' or R₄' is methyl; wherein R₁₀' is C₁-C₄ lower alkyl; wherein the dashed line represents a carbon-carbon single bond or a carbon-carbon double bond; with the proviso that when the dashed line is a carbon-carbon double bond, R₁', R₃', R₄', R₅' and R₆' are not all hydrogen when R₂' is methyl.

4. The process for preparing the carbonyl norbornane compound of Claim 3, comprising the step of intimately admixing a mixture of methyl cyclopentadienes having the structures: 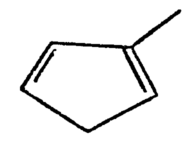 and 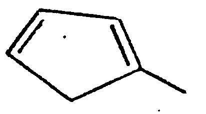 with at least one acrylic acid ester having the structure: 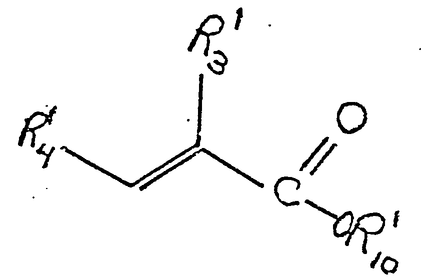 wherein in the acrylic acid ester R₃' and R₄' may be "cis" or "trans" in configuration, at a temperature of from 170°C up to 250°C at a pressure greater than atmospheric pressure.

5. The process of Claim 4, wherein in the carbonyl norbornane structure the dashed line is a carbon-carbon single bond and the process includes the additional step of reacting the resulting product wherein the dashed line in the carbonyl norbcrnane structure is a double bond, with hydrogen.

6. A methyl substituted carbonyl norbornane derivative defined according to Claim 2, further characterized in that it has the structure: 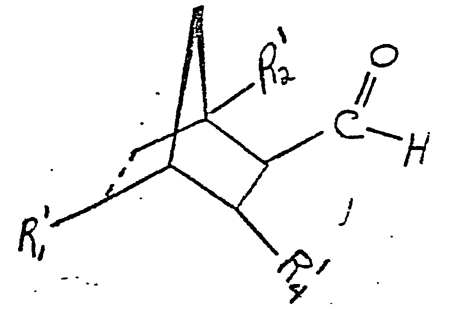 wherein the dashed line represents a carbon-carbon single bond or a carbon-carbon double bond; and wherein R1', R₂' and R₃' are each selected from the group consisting of methyl and hydrogen with the proviso (i) when R₁' is hydrogen, R₃¹ is methyl and (ii) R₁' is methyl, R₃' is hydrogen.

7. A process for augmenting or enhancing the organoleptic properties of a consumable material which is in the alternative a perfume composition, a perfumed article, a co cologne, a foodstuff, a chewing gum, a toothpaste, a medicinal product, or a chewing tobacco comprising the step of adding an organoleptic property modifying quantity of at least one methyl substituted carbonyl norbornane derivative to said consumable material, characterized in that the carbonyl norbornane derivative is defined according to Claim 6.

8. A carbonyl norbornane derivative defined according to Claim 2 further characterized in that it has the structure: 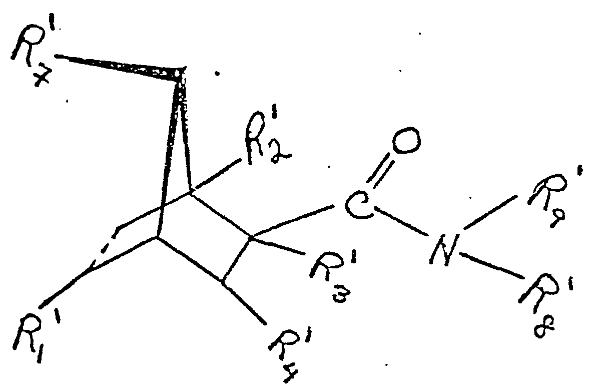 wherein the dashed line represents a carbon-carbon single bond or a carbon-carbon double bond and wherein R₁', R₂' and R₇' are methyl or hydrogen with one of R₁', R₂'or R₇' being methyl and the other of R₁', R₂' or R₇' being hydrogen; wherein R₃' and R₄' are hydrogen or methyl with one of R₃' or R₄' being hydrogen and the other of R₃' or _{R4'} being methyl; wherein R₈' and R₉' are the same or different and each represents hydrogen or C₁-C₃ lower alkyl.

9. The process for preparing a norbornyl carbonyl derivative according to the reaction sequence: 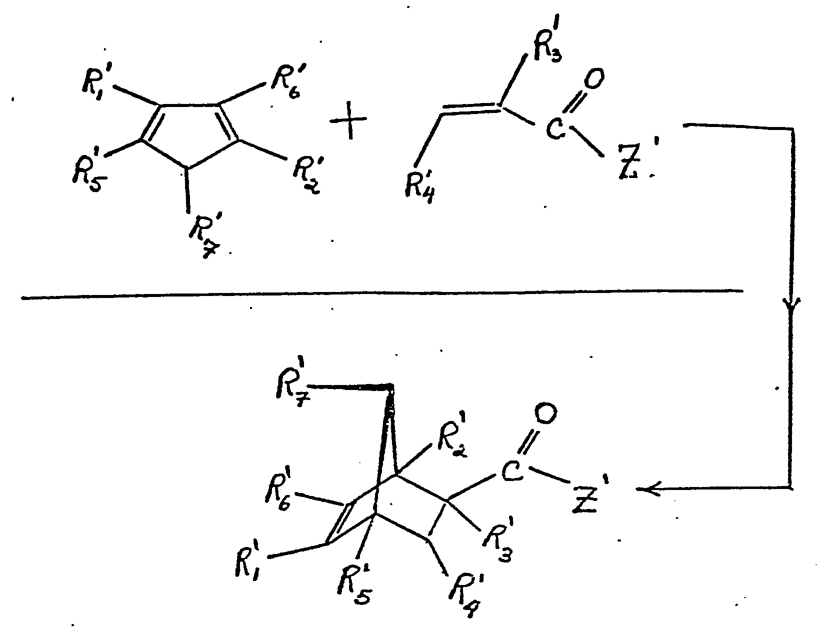 wherein R₁', R₂', R₃', R₄', R₅', R₆', and R₇' represent hydrogen or methyl; wherein one of R₁', R₂', R₅', R₆' and R₇' represent methyl and the other of R₁', R₂', R₅', R₆' and R₇' represents hydrogen; wherein one of R₃' or R₄' represents hydrogen and the other of R₃' or R₄' represents methyl; and wherein Z' represents one of the structures: 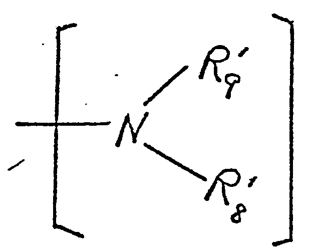 or 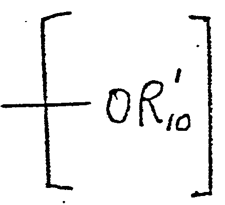 or hydrogen; wherein R₈' and R₉' are.the same or different and each represents hydrogen or alkyl; wherein R₁₀' represents C₁-C₄ alkyl, comprising the steps of reacting at least one methyl-substituted cyclopentadiene having the structure: 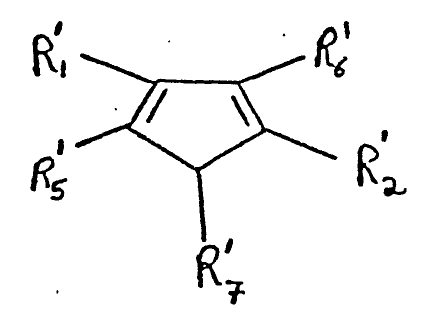 with at least one unsaturated carbonyl derivative having the structure: 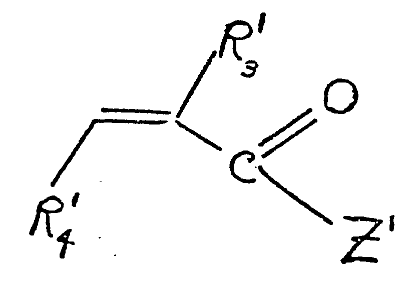 either
(i)' in the presence of a catalyst having the structure: 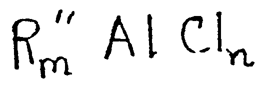 at from 0-50°C at atmospheric pressure or
(ii) in the absence of a catalyst at super-atmospheric pressures at a temperature of from 170°C up to 250°C
whereir R" represents C₁-C₃ alkyl and m + n = 3 with m being 1 or 2 and n being 1 or 2.

10 . The process of Claim 9 wherein Z' is OR₁₀' and wherein the reaction is carried out in the presence of a catalyst at from 0-50°C and the products resulting from the process have the structures: 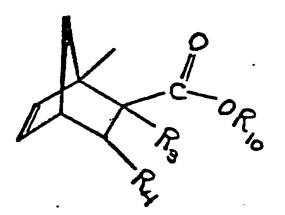 and 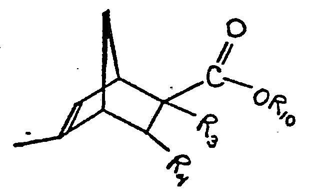 wherein one of R₃ or R₄ is methyl and the other of R₃ or R₄ is hydrogen and R₁₀ represents C₁-C₃ alkyl.
